(19)

Europäisches
Patentamt

European
Patent Office

Office européen
des brevets

(11)  **EP 2 848 690 B1**

(12)  **EUROPEAN PATENT SPECIFICATION**

(45) Date of publication and mention
of the grant of the patent:
**19.08.2020  Bulletin 2020/34**

(51) Int Cl.:
*C12N 15/63* *(2006.01)*          *C12N 9/22* *(2006.01)*
*C12N 15/70* *(2006.01)*         *C12N 15/74* *(2006.01)*
*C12N 15/85* *(2006.01)*         *C12N 15/10* *(2006.01)*

(21) Application number: **14190376.5**

(22) Date of filing: **12.12.2013**

(54) **Crispr-cas component systems, methods and compositions for sequence manipulation**

Systeme, Verfahren und Zusammensetzungen mit CRISPR-Cas-Komponenten zur
Sequenzmanipulation

Systèmes de composants CRISPR-cas, procédés et compositions pour la manipulation de séquence

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **12.12.2012  US 201261736527 P
02.01.2013  US 201361748427 P
29.01.2013  US 201361757972 P
25.02.2013  US 201361768959 P
15.03.2013  US 201361791409 P
17.06.2013  US 201361835931 P**

(43) Date of publication of application:
**18.03.2015  Bulletin 2015/12**

(62) Document number(s) of the earlier application(s) in
accordance with Art. 76 EPC:
**13812430.0 / 2 825 654**

(73) Proprietors:
• **The Broad Institute, Inc.**
**Cambridge, MA 02142 (US)**
• **MASSACHUSETTS INSTITUTE OF
TECHNOLOGY**
**Cambridge, MA 02142-1324 (US)**
• **President and Fellows of Harvard College**
**Cambridge, MA 02138-3876 (US)**
• **The Rockefeller University**
**New York, NY 10065 (US)**

(72) Inventors:
• **Zhang, Feng**
**Cambridge, MA 02139 (US)**
• **Bikard, David Olivier**
**New York, NY 10028 (US)**

• **Cox, David Benjamin Turitz**
**Cambridge, MA 02138 (US)**
• **Jiang, Wenyan**
**Whitestone, NY 11357 (US)**
• **Marraffini, Luciano**
**New York, NY 10065 (US)**
• **Habib, Naomi**
**Cambridge, MA 02139 (US)**
• **Cong, Le**
**Cambridge, MA 02142 (US)**

(74) Representative: **Williams, Gareth Owen
Marks & Clerk LLP
62-68 Hills Road
Cambridge CB2 1LA (GB)**

(56) References cited:
• **M. JINEK ET AL: "A Programmable
Dual-RNA-Guided DNA Endonuclease in
Adaptive Bacterial Immunity", SCIENCE, vol. 337,
no. 6096, 17 August 2012 (2012-08-17), pages
816-821, XP055067740, ISSN: 0036-8075, DOI:
10.1126/science.1225829 & M. JINEK ET AL: "A
Programmable Dual-RNA-Guided DNA
Endonuclease in Adaptive Bacterial Immunity
(Supplementary Material)", SCIENCE, vol. 337,
no. 6096, 28 June 2012 (2012-06-28), pages
816-821, XP055067747, ISSN: 0036-8075, DOI:
10.1126/science.1225829**

Note: Within nine months of the publication of the mention of the grant of the European patent in the European Patent
Bulletin, any person may give notice to the European Patent Office of opposition to that patent, in accordance with the
Implementing Regulations. Notice of opposition shall not be deemed to have been filed until the opposition fee has been
paid. (Art. 99(1) European Patent Convention).

**(Cont. next page)**

- G. GASIUNAS ET AL: "PNAS Plus: Cas9-crRNA ribonucleoprotein complex mediates specific DNA cleavage for adaptive immunity in bacteria", PROCEEDINGS OF THE NATIONAL ACADEMY OF SCIENCES, vol. 109, no. 39, 25 September 2012 (2012-09-25), pages E2579-E2586, XP055068588, ISSN: 0027-8424, DOI: 10.1073/pnas.1208507109
- L. CONG ET AL: "Multiplex Genome Engineering Using CRISPR/Cas Systems", SCIENCE, vol. 339, no. 6121, 15 February 2013 (2013-02-15), pages 819-823, XP055102030, ISSN: 0036-8075, DOI: 10.1126/science.1231143 & L. CONG ET AL: "Supplementary Material to : Multiplex Genome Engineering Using CRISPR/Cas Systems", SCIENCE, vol. 339, no. 6121, 3 January 2013 (2013-01-03), pages 819-823, XP055067744, ISSN: 0036-8075, DOI: 10.1126/science.1231143
- GAJ THOMAS ET AL: "ZFN, TALEN, and CRISPR/Cas-based methods for genome engineering", TRENDS IN BIOTECHNOLOGY, vol. 31, no. 7, 1 July 2013 (2013-07-01), pages 397-405, XP028571313, ISSN: 0167-7799, DOI: 10.1016/J.TIBTECH.2013.04.004
- WANG HAOYI ET AL: "One-Step Generation of Mice Carrying Mutations in Multiple Genes by CRISPR/Cas-Mediated Genome Engineering", CELL, vol. 153, no. 4, 9 May 2013 (2013-05-09), pages 910-918, XP028538358, ISSN: 0092-8674, DOI: 10.1016/J.CELL.2013.04.025
- LUKE A. GILBERT ET AL: "CRISPR-Mediated Modular RNA-Guided Regulation of Transcription in Eukaryotes", CELL, vol. 154, no. 2, 1 July 2013 (2013-07-01), pages 442-451, XP055115843, ISSN: 0092-8674, DOI: 10.1016/j.cell.2013.06.044
- KRZYSZTOF CHYLINSKI ET AL: "The tracrRNA and Cas9 families of type II CRISPR-Cas immunity systems", RNA BIOLOGY, vol. 10, no. 5, 1 May 2013 (2013-05-01), pages 726-737, XP055116068, ISSN: 1547-6286, DOI: 10.4161/rna.24321
- JIANG WENYAN ET AL: "RNA-guided editing of bacterial genomes using CRISPR-Cas systems", NATURE BIOTECHNOLOGY,, vol. 31, no. 3, 29 January 2013 (2013-01-29), pages 233-239, XP002699849,
- BLAKE WIEDENHEFT ET AL: "RNA-guided genetic silencing systems in bacteria and archaea", NATURE, vol. 482, no. 7385, 15 February 2012 (2012-02-15), pages 331-338, XP055116249, ISSN: 0028-0836, DOI: 10.1038/nature10886
- ELITZA DELTCHEVA ET AL: "CRISPR RNA maturation by trans-encoded small RNA and host factor RNase III", NATURE, vol. 471, no. 7340, 31 March 2011 (2011-03-31), pages 602-607, XP055068535, ISSN: 0028-0836, DOI: 10.1038/nature09886
- MICHAEL P TERNS ET AL: "CRISPR-based adaptive immune systems", CURRENT OPINION IN MICROBIOLOGY, vol. 14, no. 3, 1 June 2011 (2011-06-01), pages 321-327, XP055097823, ISSN: 1369-5274, DOI: 10.1016/j.mib.2011.03.005

**Description**

FIELD OF THE INVENTION

The present disclosure generally relates to systems, methods and compositions used for the control of gene expression involving sequence targeting, such as genome perturbation or gene-editing, that may use vector systems related to Clustered Regularly Interspaced Short Palindromic Repeats (CRISPR) and components thereof.

STATEMENT AS TO FEDERALLY SPONSORED RESEARCH

This invention was made with government support under the NIH Pioneer Award DP1MH100706, awarded by the National Institutes of Health. The government has certain rights in the invention.

BACKGROUND OF THE INVENTION

Recent advances in genome sequencing techniques and analysis methods have significantly accelerated the ability to catalog and map genetic factors associated with a diverse range of biological functions and diseases. Precise genome targeting technologies are needed to enable systematic reverse engineering of causal genetic variations by allowing selective perturbation of individual genetic elements, as well as to advance synthetic biology, biotechnological, and medical applications. Although genome-editing techniques such as designer zinc fingers, transcription activator-like effectors (TALEs), or homing meganucleases are available for producing targeted genome perturbations, there remains a need for new genome engineering technologies that are affordable, easy to set up, scalable, and amenable to targeting multiple positions within the eukaryotic genome.

SUMMARY OF THE INVENTION

There exists a pressing need for alternative and robust systems and techniques for sequence targeting with a wide array of applications. This invention addresses this need and provides related advantages. The CRISPR/Cas or the CRISPR-Cas system (both terms are used interchangeably throughout this application) does not require the generation of customized proteins to target specific sequences but rather a single Cas enzyme can be programmed by a short RNA molecule to recognize a specific DNA target, in other words the Cas enzyme can be recruited to a specific DNA target using said short RNA molecule. Adding the CRISPR-Cas system to the repertoire of genome sequencing techniques and analysis methods may significantly simplify the methodology and accelerate the ability to catalog and map genetic factors associated with a diverse range of biological functions and diseases. To utilize the CRISPR-Cas system effectively for genome editing without deleterious effects, it is critical to understand aspects of engineering and optimization of these genome engineering tools.

In one aspect, the invention provides a method of selecting one or more prokaryotic cell(s), the method being as set forth in claim 1. Further aspects of the invention are described in the dependent claims.

Also described herein is a vector system comprising one or more vectors. In some embodiments, the system comprises: (a) a first regulatory element operably linked to a tracr mate sequence and one or more insertion sites for inserting one or more guide sequences upstream of the tracr mate sequence, wherein when expressed, the guide sequence directs sequence-specific binding of a CRISPR complex to a target sequence in a eukaryotic cell, wherein the CRISPR complex comprises a CRISPR enzyme complexed with (1) the guide sequence that is hybridized to the target sequence, and (2) the tracr mate sequence that is hybridized to the tracr sequence; and (b) a second regulatory element operably linked to an enzyme-coding sequence encoding said CRISPR enzyme comprising a nuclear localization sequence; wherein components (a) and (b) are located on the same or different vectors of the system. In some embodiments, component (a) further comprises the tracr sequence downstream of the tracr mate sequence under the control of the first regulatory element. In some embodiments, component (a) further comprises two or more guide sequences operably linked to the first regulatory element, wherein when expressed, each of the two or more guide sequences direct sequence specific binding of a CRISPR complex to a different target sequence in a eukaryotic cell. In some embodiments, the system comprises the tracr sequence under the control of a third regulatory element, such as a polymerase III promoter. In some embodiments, the tracr sequence exhibits at least 50%, 60%, 70%, 80%, 90%, 95%, or 99% of sequence complementarity along the length of the tracr mate sequence when optimally aligned. Determining optimal alignment is within the purview of one of skill in the art. For example, there are publically and commercially available alignment algorithms and programs such as, but not limited to, ClustalW, Smith-Waterman in matlab, Bowtie, Geneious, Biopython and SeqMan. In some embodiments, the CRISPR complex comprises one or more nuclear localization sequences of sufficient strength to drive accumulation of said CRISPR complex in a detectable amount in the nucleus of a eukaryotic cell. Without wishing to be bound by theory, it is believed that a nuclear localization sequence is not necessary for CRISPR complex activity in eukaryotes, but that including such sequences enhances activity of the system, especially as to targeting nucleic acid molecules in the nucleus. In some embodiments, the CRISPR enzyme is a type II CRISPR system enzyme. In some embodiments, the CRISPR enzyme is a Cas9 enzyme. In some embodiments, the Cas9 enzyme is *S. pneumoniae, S. pyogenes,* or *S. thermophilus* Cas9, and may include mutated Cas9 derived from these organisms. The enzyme may be a Cas9 homolog or ortholog. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity. In some embodiments, the first regulatory element is a polymerase III promoter. In some embodiments, the second regulatory element is a polymerase II promoter. In some embodiments, the guide sequence is at least 15, 16, 17, 18, 19, 20, 25 nucleotides, or between 10-30, or between 15-25, or between 15-20 nucleotides in length. In general, and throughout this specification, the term "vector" refers to a nucleic acid molecule capable of transporting another nucleic acid to which it has been linked. Vectors include, but are not limited to, nucleic acid molecules that are single-stranded, double-stranded, or partially double-stranded; nucleic acid molecules that comprise one or more free ends, no free ends (e.g. circular); nucleic acid molecules that comprise DNA, RNA, or both; and other varieties of polynucleotides known in the art. One type of vector is a "plasmid," which refers to a circular double stranded DNA loop into which additional DNA segments can be inserted, such as by standard molecular cloning techniques. Another type of vector is a viral vector, wherein virally-derived DNA or RNA sequences are present in the vector for packaging into a virus (e.g. retroviruses, replication defective retroviruses, adenoviruses, replication defective adenoviruses, and adeno-associated viruses). Viral vectors also include polynucleotides carried by a virus for transfection into a host cell. Certain vectors are capable of autonomous replication in a host cell into which they are introduced (e.g. bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors are capable of directing the expression of genes to which they are operatively-linked. Such vectors are referred to herein as "expression vectors." Common expression vectors of utility in recombinant DNA techniques are often in the form of plasmids.

Recombinant expression vectors can comprise a nucleic acid as described herein in a form suitable for expression of the nucleic acid in a host cell, which means that the recombinant expression vectors include one or more regulatory elements, which may be selected on the basis of the host cells to be used for expression, that is operatively-linked to the nucleic acid sequence to be expressed. Within a recombinant expression vector, "operably linked" is intended to mean that the nucleotide sequence of interest is linked to the regulatory element(s) in a manner that allows for expression of the nucleotide sequence (e.g. in an in vitro transcription/translation system or in a host cell when the vector is introduced into the host cell).

The term "regulatory element" is intended to include promoters, enhancers, internal ribosomal entry sites (IRES), and other expression control elements (e.g. transcription termination signals, such as polyadenylation signals and poly-U sequences). Such regulatory elements are described, for example, in Goeddel, GENE EXPRESSION TECHNOLOGY: METHODS IN ENZYMOLOGY 185, Academic Press, San Diego, Calif. (1990). Regulatory elements include those that direct constitutive expression of a nucleotide sequence in many types of host cell and those that direct expression of the nucleotide sequence only in certain host cells (e.g., tissue-specific regulatory sequences). A tissue-specific promoter may direct expression primarily in a desired tissue of interest, such as muscle, neuron, bone, skin, blood, specific organs (e.g. liver, pancreas), or particular cell types (e.g. lymphocytes). Regulatory elements may also direct expression in a temporal-dependent manner, such as in a cell-cycle dependent or developmental stage-dependent manner, which may or may not also be tissue or cell-type specific. In some embodiments, a vector comprises one or more pol III promoter (e.g. 1, 2, 3, 4, 5, or more pol III promoters), one or more pol II promoters (e.g. 1, 2, 3, 4, 5, or more pol II promoters), one or more pol I promoters (e.g. 1, 2, 3, 4, 5, or more pol I promoters), or combinations thereof. Examples of pol III promoters include, but are not limited to, U6 and H1 promoters. Examples of pol II promoters include, but are not limited to, the retroviral Rous sarcoma virus (RSV) LTR promoter (optionally with the RSV enhancer), the cytomegalovirus (CMV) promoter (optionally with the CMV enhancer) [see, e.g., Boshart et al, Cell, 41:521-530 (1985)], the SV40 promoter, the dihydrofolate reductase promoter, the β-actin promoter, the phosphoglycerol kinase (PGK) promoter, and the EF1a promoter. Also encompassed by the term "regulatory element" are enhancer elements, such as WPRE; CMV enhancers; the R-U5' segment in LTR of HTLV-I (Mol. Cell. Biol., Vol. 8(1), p. 466-472, 1988); SV40 enhancer; and the intron sequence between exons 2 and 3 of rabbit β-globm (Proc. Natl. Acad. Sci. USA., Vol. 78(3), p. 1527-31, 1981). It will be appreciated by those skilled in the art that the design of the expression vector can depend on such factors as the choice of the host cell to be transformed, the level of expression desired, etc. A vector can be introduced into host cells to thereby produce transcripts, proteins, or peptides, including fusion proteins or peptides, encoded by nucleic acids as described herein (e.g., clustered regularly interspersed short palindromic repeats (CRISPR) transcripts, proteins, enzymes, mutant forms thereof, fusion proteins thereof, etc.).

Advantageous vectors include lentiviruses and adeno-associated viruses, and types of such vectors can also be selected for targeting particular types of cells.

In one aspect, described herein is a vector comprising a regulatory element operably linked to an enzyme-coding sequence encoding a CRISPR enzyme comprising one or more nuclear localization sequences. In some embodiments, said regulatory element drives transcription of the CRISPR enzyme in a eukaryotic cell such that said CRISPR enzyme accumulates in a detectable amount in the nucleus of the eukaryotic cell. In some embodiments, the regulatory element is a polymerase II promoter. In some embodiments, the CRISPR enzyme is a type II CRISPR system enzyme. In some embodiments, the CRISPR enzyme is a Cas9 enzyme. In some embodiments, the Cas9 enzyme is *S. pneumoniae, S. pyogenes* or *S. thermophilus* Cas9, and may include mutated Cas9 derived from these organisms. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity.

In one aspect, described herein is a CRISPR enzyme comprising one or more nuclear localization sequences of sufficient strength to drive accumulation of said CRISPR enzyme in a detectable amount in the nucleus of a eukaryotic cell. In some embodiments, the CRISPR enzyme is a type II CRISPR system enzyme. In some embodiments, the CRISPR enzyme is a Cas9 enzyme. In some embodiments, the Cas9 enzyme is *S. pneumoniae, S. pyogenes* or *S. thermophilus* Cas9, and may include mutated Cas9 derived from these organisms. The enzyme may be a Cas9 homolog or ortholog. In some embodiments, the CRISPR enzyme lacks the ability to cleave one or more strands of a target sequence to which it binds.

In one aspect, described herein is a eukaryotic host cell comprising (a) a first regulatory element operably linked to a tracr mate sequence and one or more insertion sites for inserting one or more guide sequences upstream of the tracr mate sequence, wherein when expressed, the guide sequence directs sequence-specific binding of a CRISPR complex to a target sequence in a eukaryotic cell, wherein the CRISPR complex comprises a CRISPR enzyme complexed with (1) the guide sequence that is hybridized to the target sequence, and (2) the tracr mate sequence that is hybridized to the tracr sequence; and/or (b) a second regulatory element operably linked to an enzyme-coding sequence encoding said CRISPR enzyme comprising a nuclear localization sequence. In some embodiments, the host cell comprises components (a) and (b). In some embodiments, component (a), component (b), or components (a) and (b) are stably integrated into a genome of the host eukaryotic cell. In some embodiments, component (a) further comprises the tracr sequence downstream of the tracr mate sequence under the control of the first regulatory element. In some embodiments, component (a) further comprises two or more guide sequences operably linked to the first regulatory element, wherein when expressed, each of the two or more guide sequences direct sequence specific binding of a CRISPR complex to a different target sequence in a eukaryotic cell. In some embodiments, the eukaryotic host cell further comprises a third regulatory element, such as a polymerase III promoter, operably linked to said tracr sequence. In some embodiments, the tracr sequence exhibits at least 50%, 60%, 70%, 80%, 90%, 95%, or 99% of sequence complementarity along the length of the tracr mate sequence when optimally aligned. In some embodiments, the CRISPR enzyme comprises one or more nuclear localization sequences of sufficient strength to drive accumulation of said CRISPR enzyme in a detectable amount in the nucleus of a eukaryotic cell. In some embodiments, the CRISPR enzyme is a type II CRISPR system enzyme. In some embodiments, the CRISPR enzyme is a Cas9 enzyme. In some embodiments, the Cas9 enzyme is *S. pneumoniae, S. pyogenes* or *S. thermophilus* Cas9, and may include mutated Cas9 derived from these organisms. The enzyme may be a Cas9 homolog or ortholog. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity. In some embodiments, the first regulatory element is a polymerase III promoter. In some embodiments, the second regulatory element is a polymerase II promoter. In some embodiments, the guide sequence is at least 15, 16, 17, 18, 19, 20, 25 nucleotides, or between 10-30, or between 15-25, or between 15-20 nucleotides in length. In an aspect, described herein is a non-human eukaryotic organism; preferably a multicellular eukaryotic organism, comprising a eukaryotic host cell according to any of the described embodiments. In other aspects, described herein is a eukaryotic organism; preferably a multicellular eukaryotic organism, comprising a eukaryotic host cell according to any of the described embodiments. The organism in some embodiments of these aspects may be an animal; for example a mammal. Also, the organism may be an arthropod such as an insect. The organism also may be a plant. Further, the organism may be a fungus.

In one aspect, described herein is a kit comprising one or more of the components described herein. In some embodiments, the kit comprises a vector system and instructions for using the kit. In some embodiments, the vector system comprises (a) a first regulatory element operably linked to a tracr mate sequence and one or more insertion sites for inserting one or more guide sequences upstream of the tracr mate sequence, wherein when expressed, the guide sequence directs sequence-specific binding of a CRISPR complex to a target sequence in a eukaryotic cell, wherein the CRISPR complex comprises a CRISPR enzyme complexed with (1) the guide sequence that is hybridized to the target sequence, and (2) the tracr mate sequence that is hybridized to the tracr sequence; and/or (b) a second regulatory element operably linked to an enzyme-coding sequence encoding said CRISPR enzyme comprising a nuclear localization sequence. In some embodiments, the kit comprises components (a) and (b) located on the same or different vectors of the system. In some embodiments, component (a) further comprises the tracr sequence downstream of the tracr mate sequence under the control of the first regulatory element. In some embodiments, component (a) further comprises two or more guide sequences operably linked to the first regulatory element, wherein when expressed, each of the two or more guide sequences direct sequence specific binding of a CRISPR complex to a different target sequence in a eukaryotic cell. In some embodiments, the system further comprises a third regulatory element, such as a polymerase III promoter, operably linked to said tracr sequence. In some embodiments, the tracr sequence exhibits at least 50%, 60%, 70%, 80%, 90%, 95%, or 99% of sequence complementarity along the length of the tracr mate sequence when optimally aligned. In some embodiments, the CRISPR enzyme comprises one or more nuclear localization sequences of sufficient strength to drive accumulation of said CRISPR enzyme in a detectable amount in the nucleus of a eukaryotic cell. In some embodiments, the CRISPR enzyme is a type II CRISPR system enzyme. In some embodiments, the CRISPR enzyme is a Cas9 enzyme. In some embodiments, the Cas9 enzyme is *S. pneumoniae, S. pyogenes* or *S. thermophilus* Cas9, and may include mutated Cas9 derived from these organisms. The enzyme may be a Cas9 homolog or ortholog. In some embodiments, the CRISPR enzyme is codon-optimized for expression in a eukaryotic cell. In some embodiments, the CRISPR enzyme directs cleavage of one or two strands at the location of the target sequence. In some embodiments, the CRISPR enzyme lacks DNA strand cleavage activity. In some embodiments, the first regulatory element is a polymerase III promoter. In some embodiments, the second regulatory element is a polymerase II promoter. In some embodiments, the guide sequence is at least 15, 16, 17, 18, 19, 20, 25 nucleotides, or between 10-30, or between 15-25, or between 15-20 nucleotides in length.

In one aspect, described herein is a method of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expressed from a gene comprising the target sequence. In some embodiments, the method further comprises delivering one or more vectors to said eukaryotic cell, wherein the one or more vectors drive expression of one or more of: the CRISPR enzyme, the guide sequence linked to the tracr mate sequence, and the tracr sequence. In some embodiments, said vectors are delivered to the eukaryotic cell in a subject. In some embodiments, said modifying takes place in said eukaryotic cell in a cell culture. In some embodiments, the method further comprises isolating said eukaryotic cell from a subject prior to said modifying. In some embodiments, the method further comprises returning said eukaryotic cell and/or cells derived therefrom to said subject.

In one aspect, described herein is a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence. In some embodiments, the method further comprises delivering one or more vectors to said eukaryotic cells, wherein the one or more vectors drive expression of one or more of: the CRISPR enzyme, the guide sequence linked to the tracr mate sequence, and the tracr sequence.

In one aspect, described herein is a method of generating a model eukaryotic cell comprising a mutated disease gene. In some embodiments, a disease gene is any gene associated an increase in the risk of having or developing a disease. In some embodiments, the method comprises (a) introducing one or more vectors into a eukaryotic cell, wherein the one or more vectors drive expression of one or more of: a CRISPR enzyme, a guide sequence linked to a tracr mate sequence, and a tracr sequence; and (b) allowing a CRISPR complex to bind to a target polynucleotide to effect cleavage of the target polynucleotide within said disease gene, wherein the CRISPR complex comprises the CRISPR enzyme complexed with (1) the guide sequence that is hybridized to the target sequence within the target polynucleotide, and (2) the tracr mate sequence that is hybridized to the tracr sequence, thereby generating a model eukaryotic cell comprising a mutated disease gene. In some embodiments, said cleavage comprises cleaving one or two strands at the location of the target sequence by said CRISPR enzyme. In some embodiments, said cleavage results in decreased transcription of a target gene. In some embodiments, the method further comprises repairing said cleaved target polynucleotide by homologous recombination with an exogenous template polynucleotide, wherein said repair results in a mutation comprising an insertion, deletion, or substitution of one or more nucleotides of said target polynucleotide. In some embodiments, said mutation results in one or more amino acid changes in a protein expression from a gene comprising the target sequence.

In one aspect, described herein is a method for developing a biologically active agent that modulates a cell signaling event associated with a disease gene. In some embodiments, a disease gene is any gene associated an increase in the risk of having or developing a disease. In some embodiments, the method comprises (a) contacting a test compound with a model cell of any one of the described embodiments; and (b) detecting a change in a readout that is indicative of a reduction or an augmentation of a cell signaling event associated with said mutation in said disease gene, thereby developing said biologically active agent that modulates said cell signaling event associated with said disease gene.

In one aspect, described herein is a recombinant polynucleotide comprising a guide sequence upstream of a tracr mate sequence, wherein the guide sequence when expressed directs sequence-specific binding of a CRISPR complex to a corresponding target sequence present in a eukaryotic cell. In some embodiments, the target sequence is a viral sequence present in a eukaryotic cell. In some embodiments, the target sequence is a proto-oncogene or an oncogene.

In one aspect described herein is a method of selecting one or more prokaryotic cell(s) by introducing one or more mutations in a gene in the one or more prokaryotic cell (s), the method comprising: introducing one or more vectors into the prokaryotic cell (s), wherein the one or more vectors drive expression of one or more of: a CRISPR enzyme, a guide sequence linked to a tracr mate sequence, a tracr sequence, and a editing template; wherein the editing template comprises the one or more mutations that abolish CRISPR enzyme cleavage; allowing homologous recombination of the editing template with the target polynucleotide in the cell(s) to be selected; allowing a CRISPR complex to bind to a target polynucleotide to effect cleavage of the target polynucleotide within said gene, wherein the CRISPR complex comprises the CRISPR enzyme complexed with (1) the guide sequence that is hybridized to the target sequence within the target polynucleotide, and (2) the tracr mate sequence that is hybridized to the tracr sequence, wherein binding of the CRISPR complex to the target polynucleotide induces cell death, thereby allowing one or more prokaryotic cell(s) in which one or more mutations have been introduced to be selected. In a preferred embodiment, the CRISPR enzyme is Cas9. In another aspect described herein the cell to be selected may be a eukaryotic cell. Aspects of the invention allow for selection of specific cells without requiring a selection marker or a two-step process that may include a counter-selection system.

BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

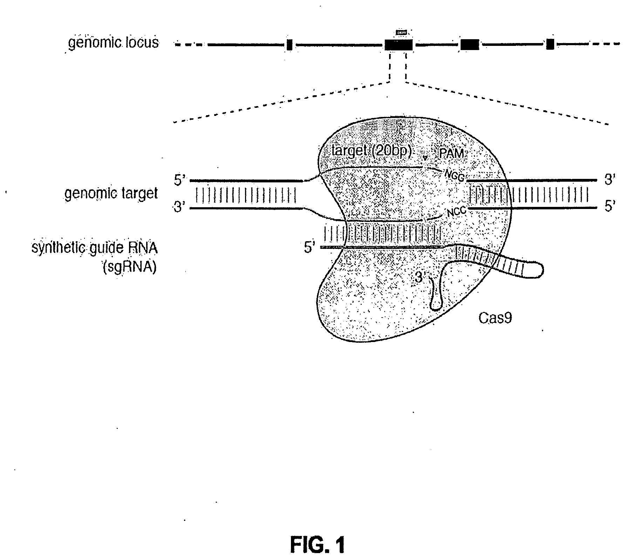

Figure 1 shows a schematic model of the CRISPR system. The Cas9 nuclease from Streptococcus pyogenes (yellow) is targeted to genomic DNA by a synthetic guide RNA (sgRNA) consisting of a 20-nt guide sequence (blue) and a scaffold (red). The guide sequence base-pairs with the DNA target (blue), directly upstream of a requisite 5'-NGG protospacer adjacent motif (PAM; magenta), and Cas9 mediates a double-stranded break (DSB) ~3 bp upstream of the PAM (red triangle).

Figure 2A-F shows an exemplary CRISPR system, a possible mechanism of action, an example adaptation for expression in eukaryotic cells, and results of tests assessing nuclear localization and CRISPR activity.

Figure 3 shows an exemplary expression cassette for expression of CRISPR system elements in eukaryotic cells, predicted structures of example guide sequences, and CRISPR system activity as measured in eukaryotic and prokaryotic cells.

Figure 4A-D shows results of an evaluation of SpCas9 specificity for an example target.

Figure 5A-G show an exemplary vector system and results for its use in directing homologous recombination in eukaryotic cells.

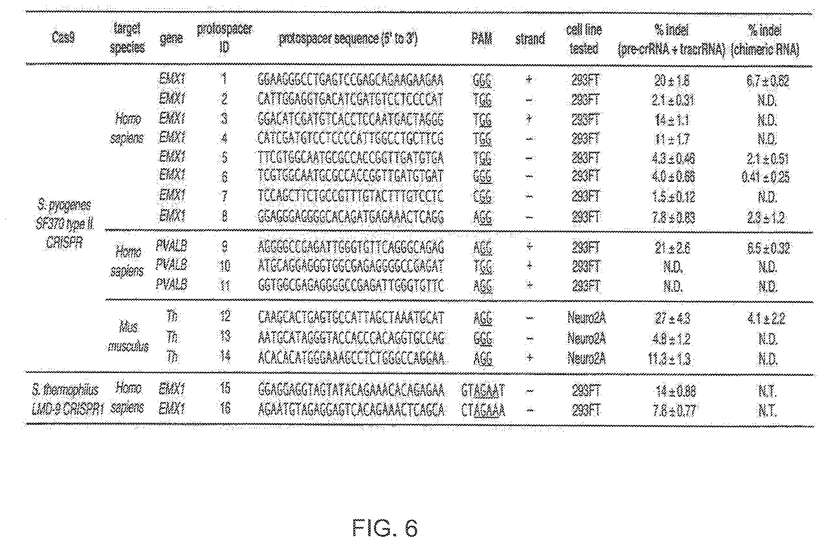

Figure 6 provides a table of protospacer sequences and summarizes modification efficiency results for protospacer targets designed based on exemplary *S. pyogenes* and *S. thermophilus* CRISPR systems with corresponding PAMs against loci in human and mouse genomes. Cells were transfected with Cas9 and either pre-crRNA/tracrRNA or chimeric RNA, and analyzed 72 hours after transfection. Percent indels are calculated based on Surveyor assay results from indicated cell lines (N=3 for all protospacer targets, errors are S.E.M., N.D. indicates not detectable using the Surveyor assay, and N.T. indicates not tested in this study).

Figure 7A-C shows a comparison of different tracrRNA transcripts for Cas9-mediated gene targeting.

Figure 8 shows a schematic of a surveyor nuclease assay for detection of double strand break-induced micro-insertions and -deletions.

Figure 9A-B shows exemplary bicistronic expression vectors for expression of CRISPR system elements in eukaryotic cells.

**Figure 10** shows a bacterial plasmid transformation interference assay, expression cassettes and plasmids used therein, and transformation efficiencies of cells used therein.

**Figure 11A-C** shows histograms of distances between adjacent *S. pyogenes* SF370 locus 1 PAM (NGG) (Figure 10A) and *S. thermophilus* LMD9 locus 2 PAM (NNAGAAW) (Figure 10B) in the human genome; and distances for each PAM by chromosome (Chr) (Figure 10C).

**Figure 12A-C** shows an exemplary CRISPR system, an example adaptation for expression in eukaryotic cells, and results of tests assessing CRISPR activity.

**Figure 13A-C** shows exemplary manipulations of a CRISPR system for targeting of genomic loci in mammalian cells.

**Figure 14A-B** shows the results of a Northern blot analysis of crRNA processing in mammalian cells.

**Figure 15** shows an exemplary selection of protospacers in the human *PVALB* and mouse *Th* loci.

**Figure 16** shows example protospacer and corresponding PAM sequence targets of the *S. thermophilus* CRISPR system in the human *EMX1* locus.

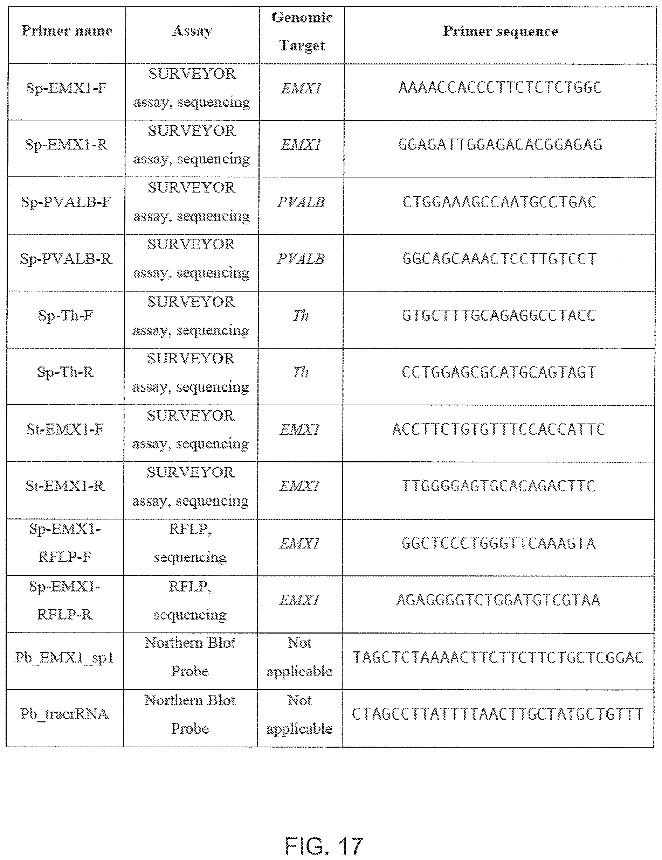

**Figure 17** provides a table of sequences for primers and probes used for Surveyor, RFLP, genomic sequencing, and Northern blot assays.

**Figure 18A-C** shows exemplary manipulation of a CRISPR system with chimeric RNAs and results of SURVEYOR assays for system activity in eukaryotic cells.

**Figure 19A-B** shows a graphical representation of the results of SURVEYOR assays for CRISPR system activity in eukaryotic cells.

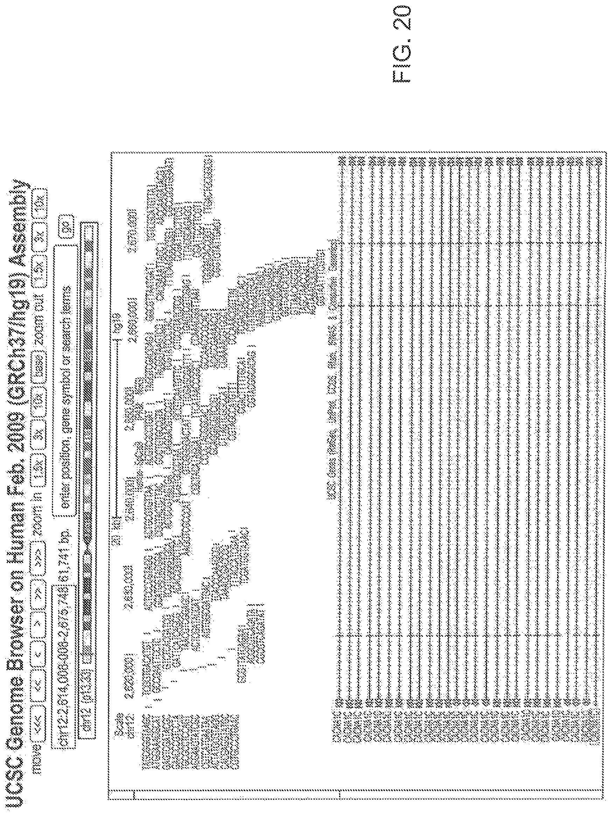

**Figure 20** shows an exemplary visualization of some *S. pyogenes* Cas9 target sites in the human genome using the UCSC genome browser.

**Figure 21** shows predicted secondary structures for exemplary chimeric RNAs comprising a guide sequence, tracr mate sequence, and tracr sequence.

**Figure 22** shows exemplary bicistronic expression vectors for expression of CRISPR system elements in eukaryotic cells.

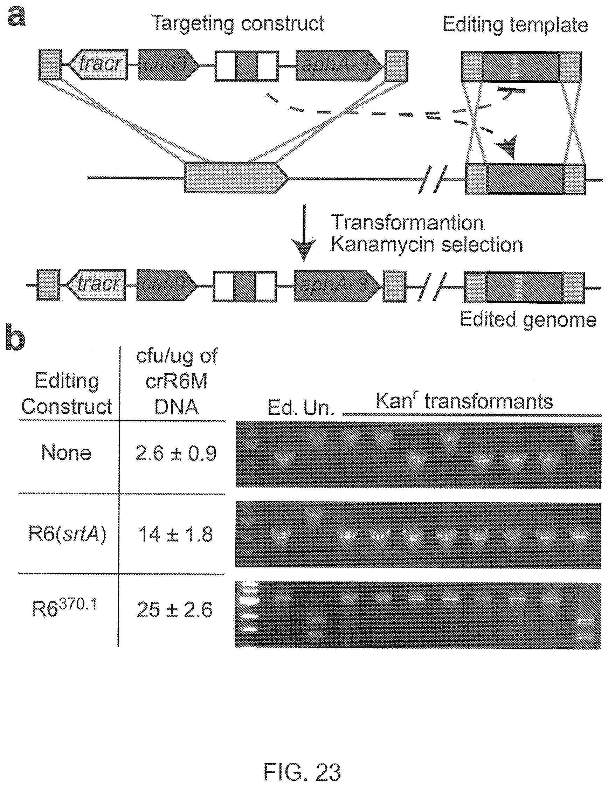

**Figure 23** shows that Cas9 nuclease activity against endogenous targets may be exploited for genome editing. (a) Concept of genome editing using the CRISPR system. The CRISPR targeting construct directed cleavage of a chromosomal locus and was co-transformed with an editing template that recombined with the target to prevent cleavage. Kanamycin-resistant transformants that survived CRISPR attack contained modifications introduced by the editing template. *tracr*, *trans*-activating CRISPR RNA; *aphA-3*, kanamycin resistance gene. (b) Transformation of crR6M DNA in R6$^{8232.5}$ cells with no editing template, the R6 wild-type srtA or the R6370.1 editing templates. Recombination of either R6 *srtA* or R6$^{370.1}$ prevented cleavage by Cas9. Transformation efficiency was calculated as colony forming units (cfu) per μg of crR6M DNA; the mean values with standard deviations from at least three independent experiments are shown. PCR analysis was performed on 8 clones in each transformation. "Un." indicates the unedited *srtA* locus of strain R6$^{8232.5}$; "Ed." shows the editing template. R6$^{8232.5}$ and R6$^{370.1}$ targets are distinguished by restriction with EaeI.

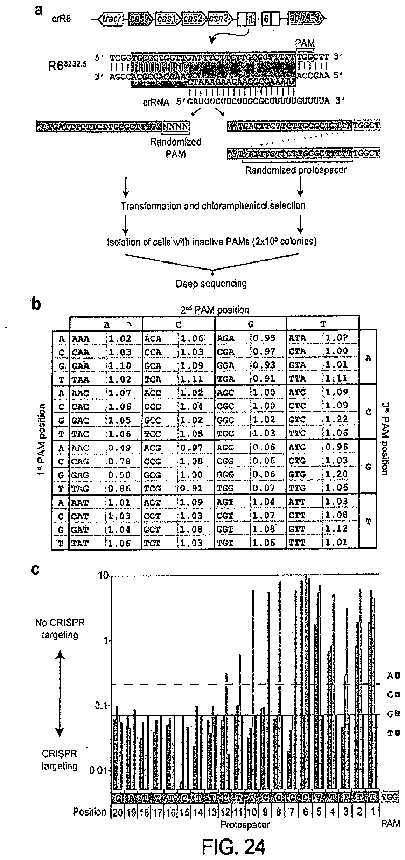

**Figure 24** shows analysis of PAM and seed sequences that eliminate Cas9 cleavage. (a) PCR products with randomized PAM sequences or randomized seed sequences were transformed in crR6 cells. These cells expressed Cas9 loaded with a crRNA that targeted a chromosomal region of R6$^{8232.5}$ cells (highlighted in pink) that is absent from the R6 genome. More than $2\times10^5$ chloramphenicol-resistant transformants, carrying inactive PAM or seed sequences, were combined for amplification and deep sequencing of the target region. (b) Relative proportion of number of reads after transformation of the random PAM constructs in crR6 cells (compared to number of reads in R6 transformants). The relative abundance for each 3-nucleotide PAM sequence is shown. Severely underrepresented sequences (NGG) are shown in red; partially underrepresented one in orange (NAG) (c) Relative proportion of number of reads after transformation of the random seed sequence constructs in crR6 cells (compared to number of reads in R6 transformants). The relative abundance of each nucleotide for each position of the first 20 nucleotides of the protospacer sequence is shown. High abundance indicates lack of cleavage by Cas9, i.e. a CRISPR inactivating mutation. The grey line shows the level of the WT sequence. The dotted line represents the level above which a mutation significantly disrupts cleavage (See section "Analysis of deep sequencing data" in Example 5)

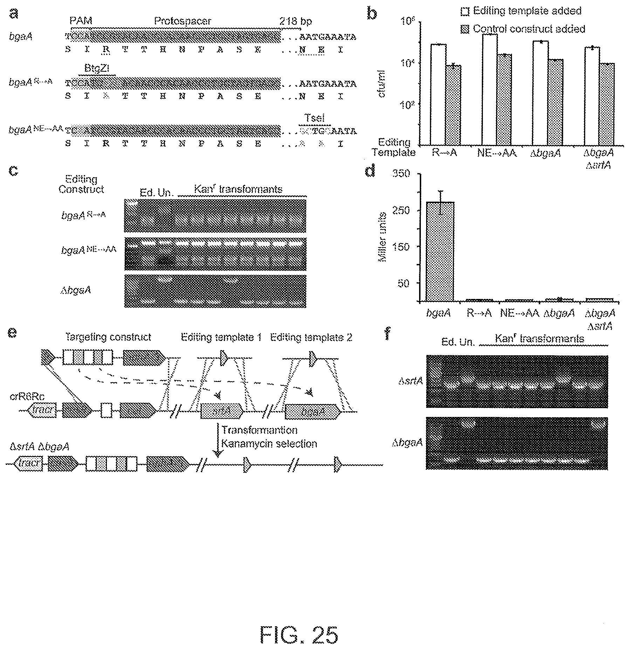

**Figure 25** shows introduction of single and multiple mutations using the CRISPR system in *S. pneumoniae.* (a) Nucleotide and amino acid sequences of the wild-type and edited (green nucleotides; underlined amino acid residues) *bgaA*. The protospacer, PAM and restriction sites are shown. (b) Transformation efficiency of cells transformed with targeting constructs in the presence of an editing template or control. (c) PCR analysis for 8 transformants of each editing experiment followed by digestion with BtgZI (R→A) and TseI (NE→AA). Deletion of *bgaA* was revealed as a smaller PCR product. (d) Miller assay to measure the β-galactosidase activity of WT and edited strains. (e) For a single-step, double deletion the targeting construct contained two spacers (in this case matching *srtA* and *bgaA*) and was co-transformed with two different editing templates (f) PCR analysis for 8 transformants to detect deletions in *srtA* and *bgaA* loci. 6/8 transformants contained deletions of both genes.

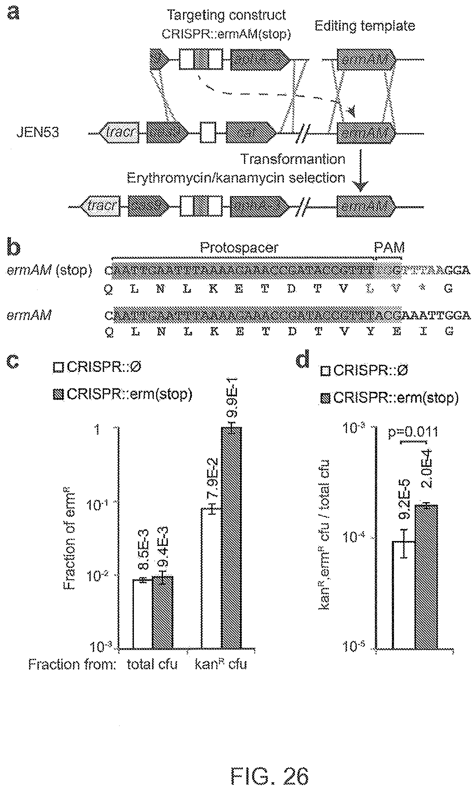

**Figure 26** provides mechanisms underlying editing using the CRISPR system. (a) A stop codon was introduced in the erythromycin resistance gene *ermAM* to generate strain JEN53. The wild-type sequence can be restored by targeting the stop codon with the CRISPR::ermAM(stop) construct, and using the ermAM wild-type sequence as an editing template. (b) Mutant and wild-type *ermAM* sequences. (c) Fraction of erythromicyn-resistant *(erm$^R$)* cfu calculated from total or kanamycin-resistant *(kan$^R$)* cfu. (d) Fraction of total cells that acquire both the CRISPR construct and the editing template. Co-transformation of the CRISPR targeting construct produced more transformants (t-test, p=0.011). In all cases the values show the mean±s.d. for three independent experiments.

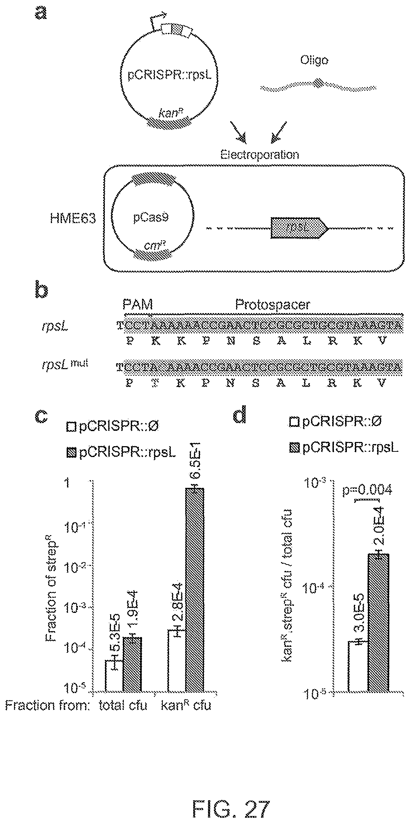

**Figure 27** illustrates genome editing with the CRISPR system in E. coli. (a) A kanamycin-resistant plasmid carrying the CRISPR array (pCRISPR) targeting the gene to edit may be transformed in the HME63 recombineering strain containing a chloramphenicol-resistant plasmid harboring cas9 and tracr (pCas9), together with an oligonucleotide specifying the mutation. (b) A K42T mutation conferring streptomycin resistance was introduced in the *rpsL* gene (c) Fraction of streptomicyn-resistant (strep$^R$) cfu calculated from total or kanamycin-resistant (kan$^R$) cfu. (d) Fraction of total cells that acquire both the pCRISPR plasmid and the editing oligonucleotide. Co-transformation of the pCRISPR targeting plasmid produced more transformants (t-test, p=0.004). In all cases the values showed the mean±s.d. for three independent experiments.

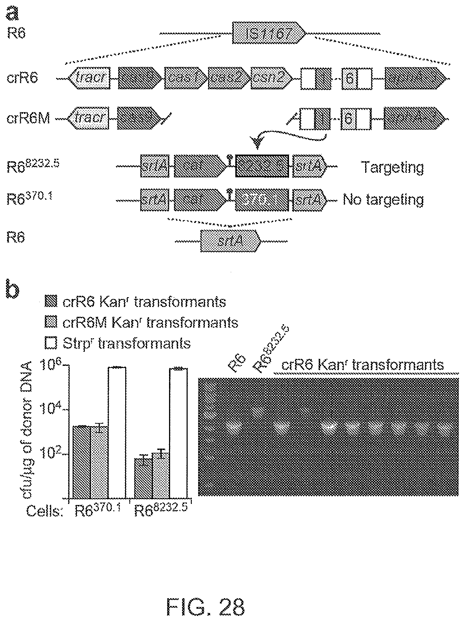

**Figure 28** illustrates the transformation of crR6 genomic DNA leads to editing of the targeted locus (a) The *IS1167* element of *S. pneumoniae* R6 was replaced by the CRISPR01 locus of *S. pyogenes* SF370 to generate crR6 strain. This locus encodes for the Cas9 nuclease, a CRISPR array with six spacers, the tracrRNA that is required for crRNA biogenesis and Cas1, Cas2 and Csn2, proteins not necessary for targeting. Strain crR6M contains a minimal functional CRISPR system without *cas1*, *cas2* and *csn2*. The *aphA-3* gene encodes kanamycin resistance. Protospacers from the streptococcal bacteriophages φ8232.5 and φ370.1 were fused to a chloramphenicol resistance gene (*cat*) and integrated in the *srtA* gene of strain R6 to generate strains R68232.5 and R6370.1. (b) Left panel: Transformation of crR6 and crR6M genomic DNA in R6$^{8232.5}$ and R$^{6370.1}$. As a control of cell competence a streptomycin resistant gene was also transformed. Right panel: PCR analysis of 8 R6$^{8232.5}$ transformants with crR6 genomic DNA. Primers that amplify the *srtA* locus were used for PCR. 7/8 genotyped colonies replaced the *R68232.5* srtA locus by the WT locus from the crR6 genomic DNA.

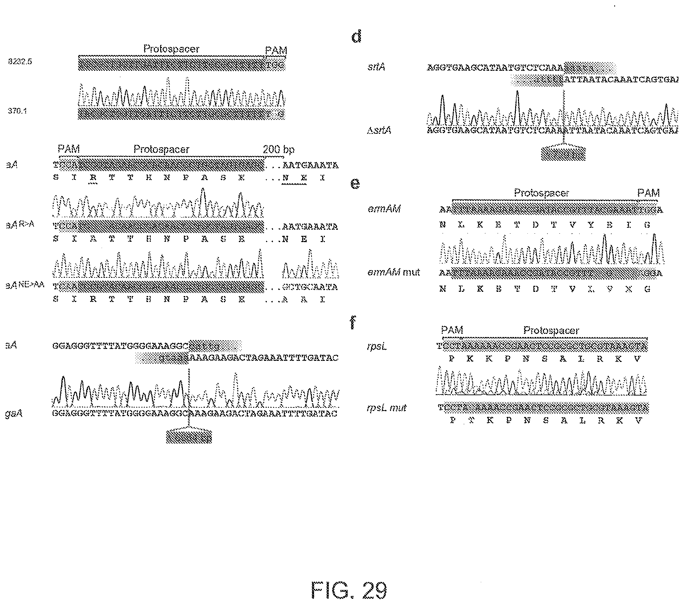

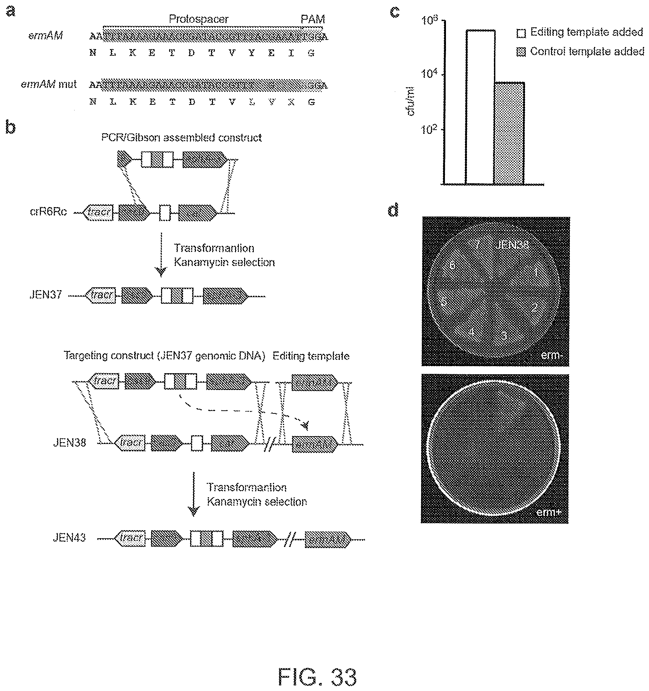

**Figure 29** provides chromatograms of DNA sequences of edited cells obtained in this study. In all cases the wild-type and mutant protospacer and PAM sequences (or their reverse complement) are indicated. When relevant, the amino acid sequence encoded by the protospacer is provided. For each editing experiment, all strains for which PCR and restriction analysis corroborated the introduction of the desired modification were sequenced. A representative chromatogram is shown. **(a)** Chromatogram for the introduction of a PAM mutation into the R6$^{8232.5}$ target (Figure 23d). **(b)** Chromatograms for the introduction of the R>A and NE>AA mutations into β-galactosidase (*bgaA*) (Figure 25c). **(c)** Chromatogram for the introduction of a 6664 bp deletion within *bgaA* ORF (Figures 25c and 25f). The dotted line indicates the limits of the deletion. **(d)** Chromatogram for the introduction of a 729 bp deletion within *srtA* ORF (Figure 25f). The dotted line indicates the limits of the deletion. **(e)** Chromatograms for the generation of a premature stop codon within *ermAM* (Figure 33). **(f)** *rpsL* editing in *E. coli* (Figure 27).

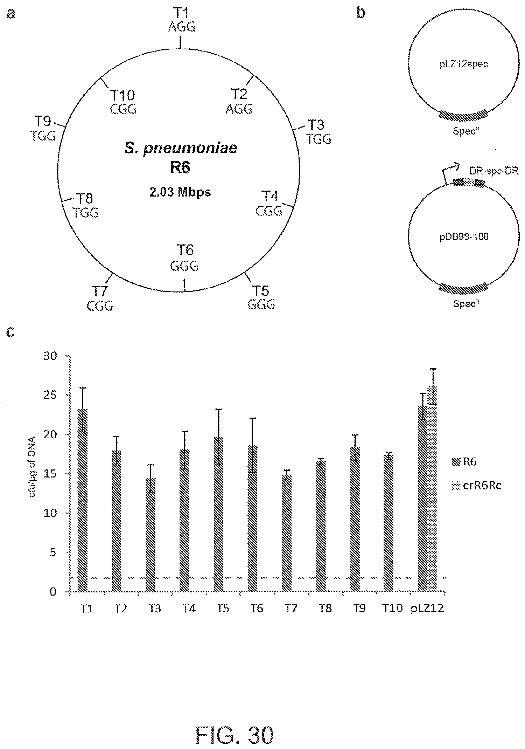

**Figure 30** illustrates CRISPR immunity against random *S. pneumoniae* targets containing different PAMs. **(a)** Position of the 10 random targets on the *S. pneumoniae* R6 genome. The chosen targets have different PAMs and are on both strands. **(b)** Spacers corresponding to the targets were cloned in a minimal CRISPR array on plasmid pLZ12 and transformed into strain crR6Rc, which supplies the processing and targeting machinery in trans. **(c)** Transformation efficiency of the different plasmids in strain R6 and crR6Rc. No colonies were recovered for the transformation of pDB99-108 (T1-T10) in crR6Rc. The dashed line represents limit of detection of the assay.

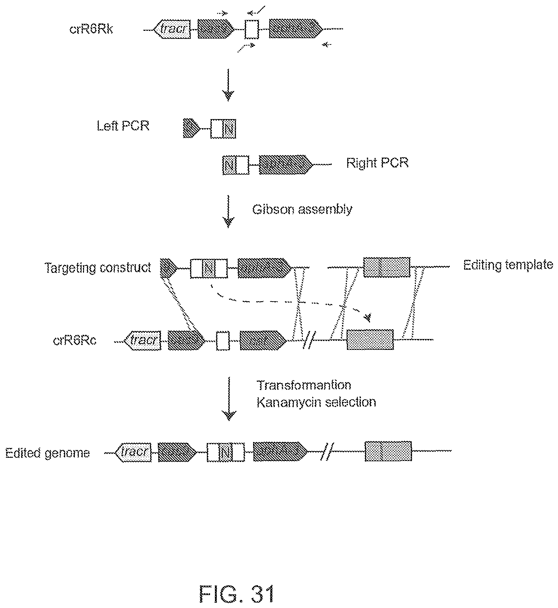

**Figure 31** provides a general scheme for targeted genome editing. To facilitate targeted genome editing, crR6M was further engineered to contain tracrRNA, Cas9 and only one repeat of the CRISPR array followed by kanamycin resistance marker (*aphA-3*), generating strain crR6Rk. DNA from this strain is used as a template for PCR with primers designed to introduce a new spacer (green box designated with N). The left and right PCRs are assembled using the Gibson method to create the targeting construct. Both the targeting and editing constructs are then transformed into strain crR6Rc, which is a strain equivalent to crR6Rk but has the kanamycin resistance marker replaced by a chloramphenicol resistance marker (*cat*). About 90 % of the kanamycin-resistant transformants contain the desired mutation.

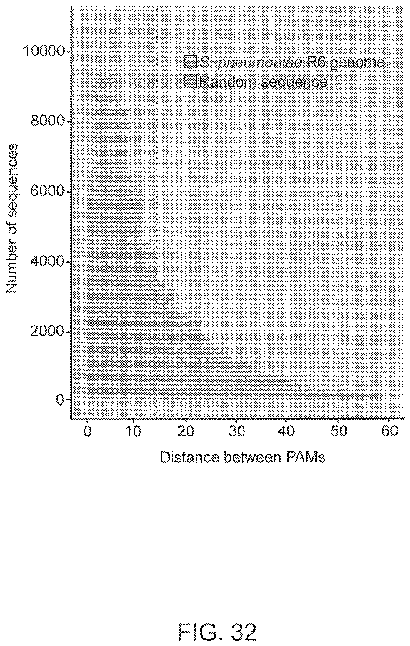

**Figure 32** illustrates the distribution of distances between PAMs. NGG and CCN that are considered to be valid PAMs. Data is shown for the *S. pneumoniae* R6 genome as well as for a random sequence of the same length and with the same GC-content (39.7 %). The dotted line represents the average distance (12) between PAMs in the R6 genome.

**Figure 33** illustrates CRISPR-mediated editing of the *ermAM* locus using genomic DNA as targeting construct. To use genomic DNA as targeting construct it is necessary to avoid CRISPR autoimmunity, and therefore a spacer against a sequence not present in the chromosome must be used (in this case the *ermAM* erythromycin resistance gene). **(a)** Nucleotide and amino acid sequences of the wild-type and mutated (red letters) *ermAM* gene. The protospacer and PAM sequences are shown. **(b)** A schematic for CRISPR-mediated editing of the *ermAM* locus using genomic DNA. A construct carrying an *ermAM*-targeting spacer (blue box) is made by PCR and Gibson assembly, and transformed into strain crR6Rc, generating strain JEN37. The genomic DNA of JEN37 was then used as a targeting construct, and was co-transformed with the editing template into JEN38, a strain in which the *srtA* gene was replaced by a wild-type copy of *ermAM* Kanamycin-resistant transformants contain the edited genotype (JEN43). **(c)** Number of kanamycin-resistant cells obtained after co-transformation of targeting and editing or control templates. In the presence of the control template $5.4 \times 10^3$ cfu/ml were obtained, and $4.3 \times 10^5$ cfu/ml when the editing template was used. This difference indicates an editing efficiency of about 99% $[(4.3 \times 10^5 - 5.4 \times 10^3)/4.3 \times 10^5]$. **(d)** To check for the presence of edited cells seven kanamycin-resistant clones and JEN38 were streaked on agar plates with (erm+) or without (erm-) erythromycin. Only the positive control displayed resistance to erythromycin. The *ermAM* mut genotype of one of these transformants was also verified by DNA sequencing (Figure 29e).

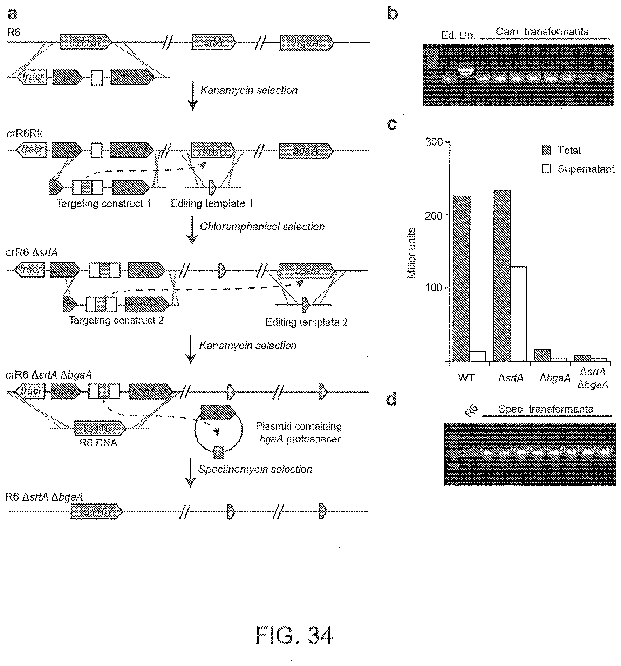

**Figure 34** illustrates sequential introduction of mutations by CRISPR-mediated genome editing, **(a)** A schematic for sequential introduction of mutations by CRISPR-mediated genome editing. First, R6 is engineered to generate crR6Rk. crR6Rk is co-transformed with a *srtA*-targeting construct fused to cat for chloramphenicol selection of edited cells, along with an editing construct for a *ΔsrtA* in-frame deletion. Strain crR6 *ΔsrtA* is generated by selection on chlramphenicol. Subsequently, the *ΔsrtA* strain is co-transformed with a *bgaA*-targeting construct fused to *aphA-3* for kanamycin selection of edited cells, and an editing construct containing a *ΔbgaA* in-frame deletion. Finally, the engineered CRISPR locus can be erased from the chromosome by first co-transforming R6 DNA containing the wild-type *IS1167* locus and a plasmid carrying a *bgaA* protospacer (pDB97), and selection on spectinomycin. **(b)** PCR analysis for 8 chloramphenicol (Cam)-resistant transformants to detect the deletion in the *srtA* locus. **(c)** β-galactosidase activity as measured by Miller assay. In *S. pneumoniae,* this enzyme is anchored to the cell wall by sortase A. Deletion of the *srtA* gene results in the release of β-galactosidase into the supernatant. *ΔbgaA* mutants show no activity. **(d)** PCR analysis for 8 spectinomycin (Spec)-resistant transformants to detect the replacement of the CRISPR locus by wild-type *IS1167.*

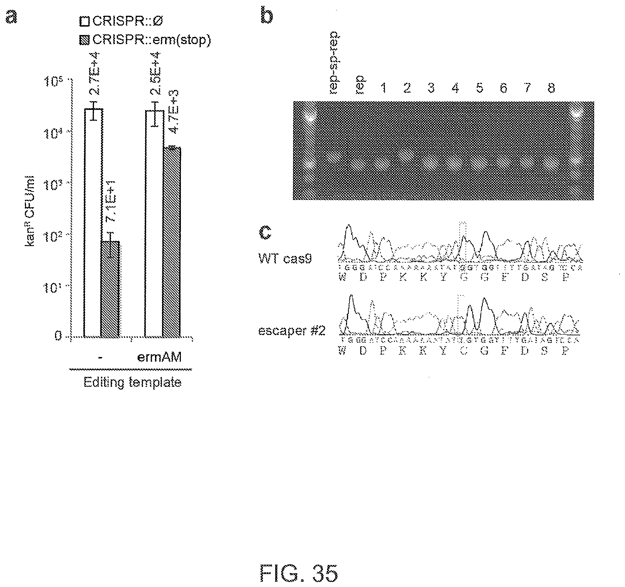

**Figure 35** illustrates the background mutation frequency of CRISPR in *S. pneumoniae.* **(a)** Transformation of the CRISPR::Ø or CRISPR::erm(stop) targeting constructs in JEN53, with or without the ermAM editing template. The difference in kan$^R$ CFU between CRISPR::Ø and CRISPR::erm(stop) indicates that Cas9 cleavage kills non-edited cells. Mutants that escape CRISPR interference in the absence of editing template are observed at a frequency of $3 \times 10^{-3}$. **(b)** PCR analysis of the CRISPR locus of escapers shows that 7/8 have a spacer deletion. **(c)** Escaper #2 carries a point mutation in *cas9.*

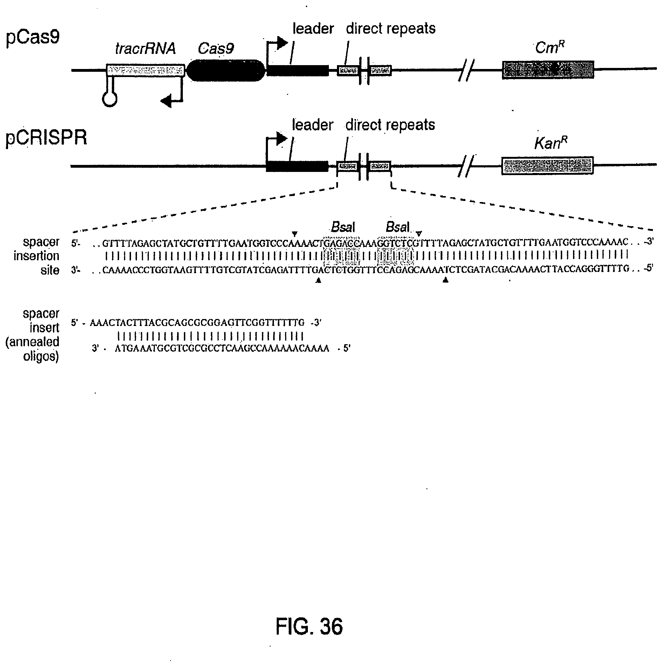

**Figure 36** illustrates that the essential elements of the *S. pyogenes* CRISPR locus 1 are reconstituted in *E. coli* using pCas9. The plasmid contained tracrRNA, Cas9, as well as a leader sequence driving the crRNA array. The pCRISPR plasmids contained the leader and the array only. Spacers may be inserted into the crRNA array between *BsaI* sites using annealed oligonucleotides. Oligonucleotide design is shown at bottom. pCas9 carried chloramphenicol resistance (CmR) and is based on the low-copy pACYC184 plasmid backbone. pCRISPR is based on the high-copy number pZE21 plasmid. Two plasmids were required because a pCRISPR plasmid containing a spacer targeting the *E. coli* chromosome may not be constructed using this organism as a cloning host if Cas9 is also present (it will kill the host).

**Figure 37** illustrates CRISPR-directed editing in *E.coli* MG1655. An oligonucleotide (W542) carrying a point mutation that both confers streptomycin resistance and abolishes CRISPR immunity, together with a plasmid targeting *rpsL* (pCRISPR: :rpsL) or a control plasmid (pCRISPR::Ø) were co-transformed into wild-type *E.coli* strain MG1655 containing pCas9. Transformants were selected on media containing either streptomycin or kanamycin. Dashed line indicates limit of detection of the transformation assay.

**Figure 38** illustrates the background mutation frequency of CRISPR in *E. coli* HME63. **(a)** Transformation of the pCRISPR:: Ø or pCRISPR::rpsL plasmids into HME63 competent cells. Mutants that escape CRISPR interference were observed at a frequency of $2.6 \times 10^{-4}$. **(b)** Amplification of the CRISPR array of escapers showed that 8/8 have deleted the spacer.

**Figure 39A-D** shows a circular depiction of the phylogenetic analysis revealing five families of Cas9s, including three groups of large Cas9s (~1400 amino acids) and two of small Cas9s (~1100 amino acids).

**Figure 40A-F** shows the linear depiction of the phylogenetic analysis revealing five families of Cas9s, including three groups of large Cas9s (~1400 amino acids) and two of small Cas9s (~1100 amino acids).

**Figure 41A-M** shows sequences where the mutation points are located within the SpCas9 gene.

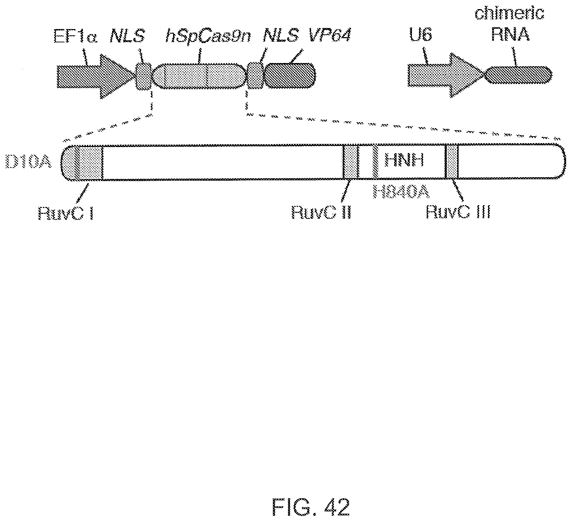

**Figure 42** shows a schematic construct in which the transcriptional activation domain (VP64) is fused to Cas9 with two mutations in the catalytic domains (D10 and H840).

**Figure 43A-D** shows genome editing via homologous recombination. (a) Schematic of SpCas9 nickase, with D10A mutation in the RuvC I catalytic domain. (b) Schematic representing homologous recombination (HR) at the human *FMX1* locus using either sense or antisense single stranded oligonucleotides as repair templates. Red arrow above indicates sgRNA cleavage site; PCR primers for genotyping (Tables J and K) are indicated as arrows in right panel.

(c) Sequence of region modified by HR. d, SURVEYOR assay for wildtype (wt) and nickase (D10A) SpCas9-mediated indels at the *EMX1* target 1 locus (*n=3*). Arrows indicate positions of expected fragment sizes.

**Figure 44A-B** shows single vector designs for SpCas9.

**Figure 45** shows quantification of cleavage of NLS-Csn1 constructs NLS-Csn1, Csn1, Csn1-NLS, NLS-Csn1-NLS, NLS-Csn1-GFP-NLS and UnTFN.

**Figure 46** shows index frequency of NLS-Cas9, Cas9, Cas9-NLS and NLS-Cas9-NLS.

**Figure 47** shows a gel demonstrating that SpCas9 with nickase mutations (individually) do not induce double strand breaks.

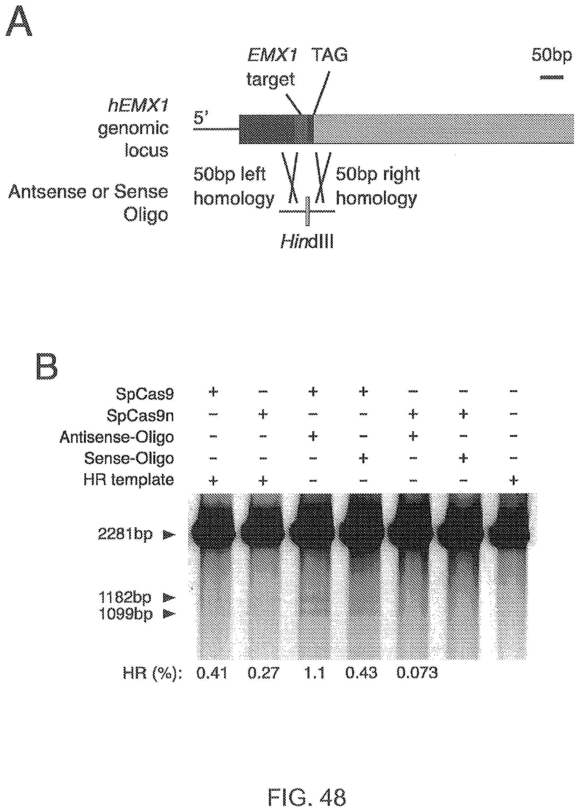

**Figure 48** shows a design of the oligo DNA used as Homologous Recombination (HR) template in this experiment and a comparison of HR efficiency induced by different combinations of Cas9 protein and HR template.

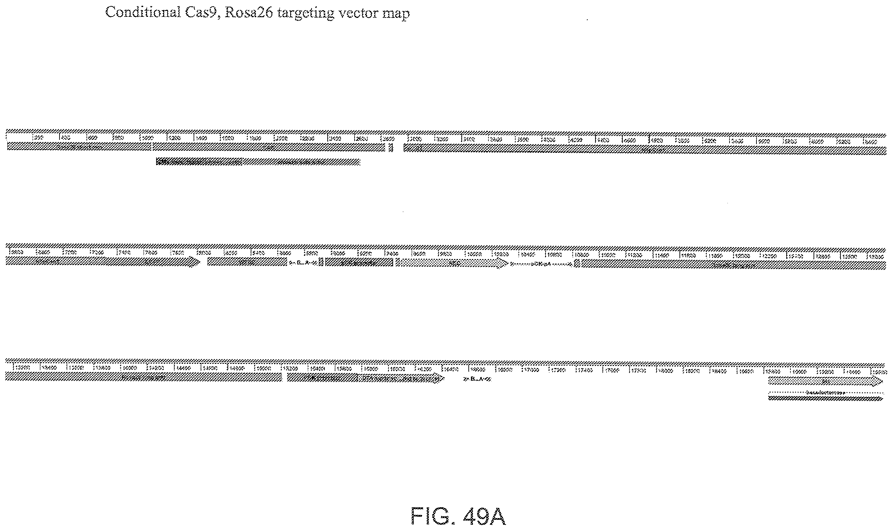

**Figure 49A** shows the Conditional Cas9, Rosa26 targeting vector map.

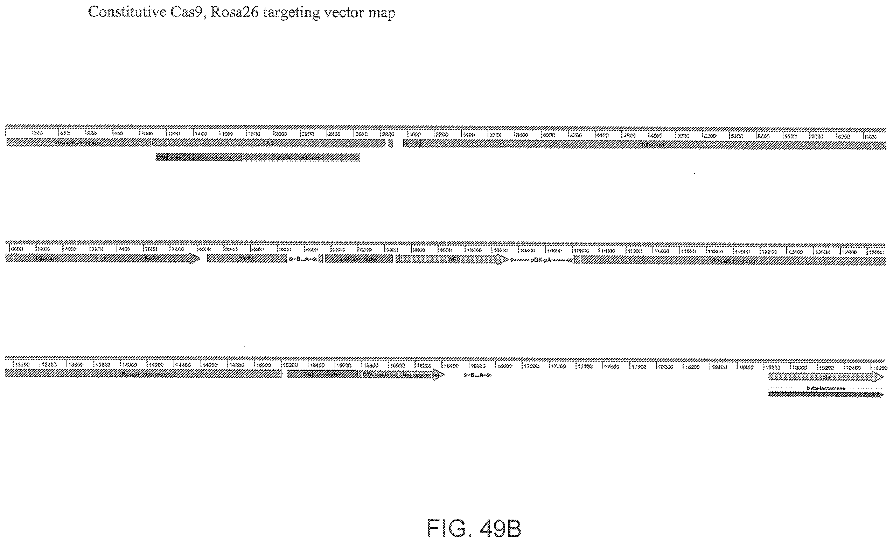

**Figure 49B** shows the Constitutive Cas9, Rosa26 targeting vector map.

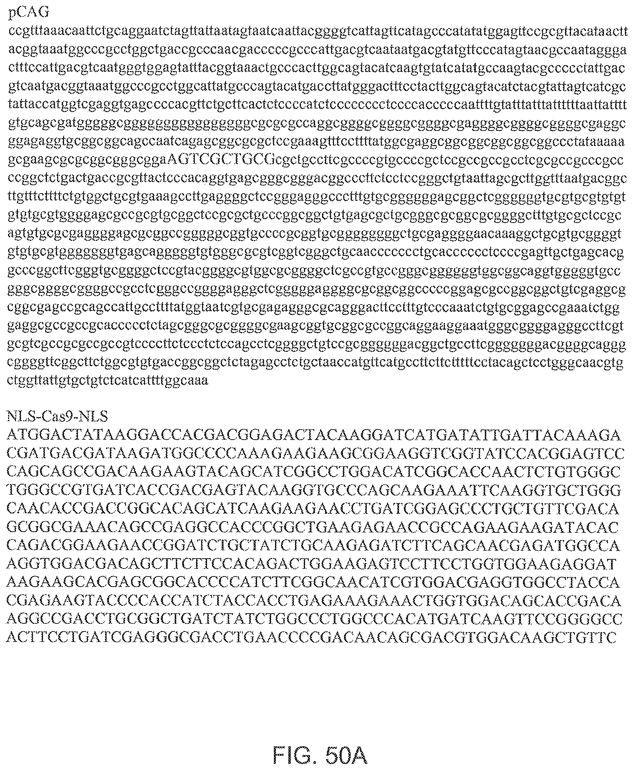

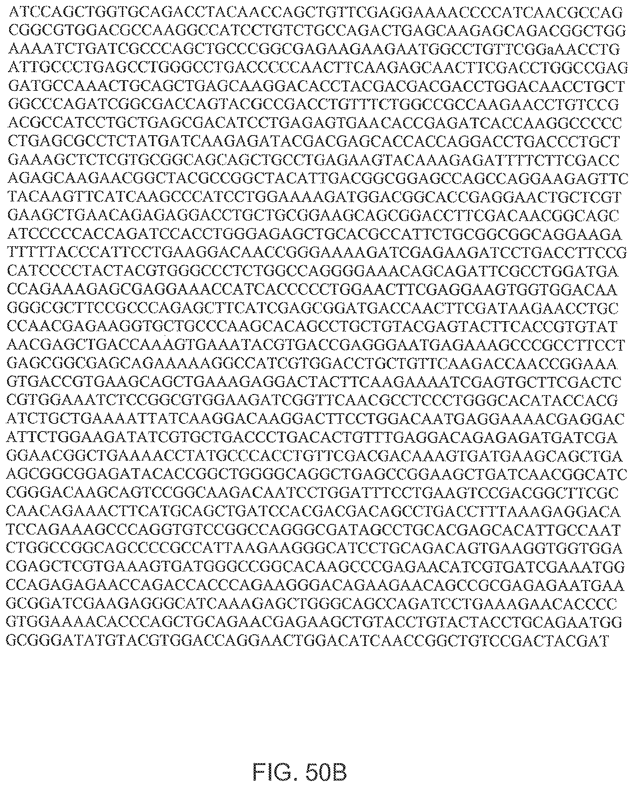

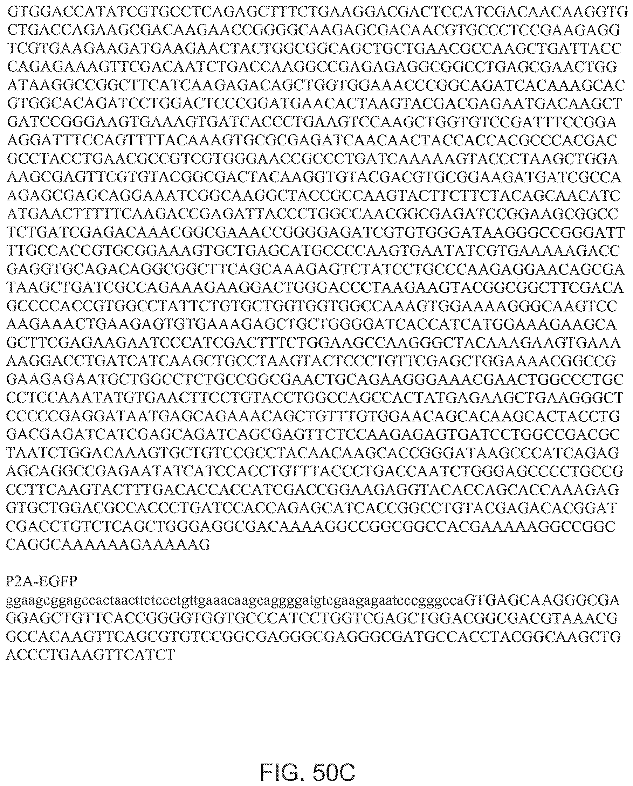

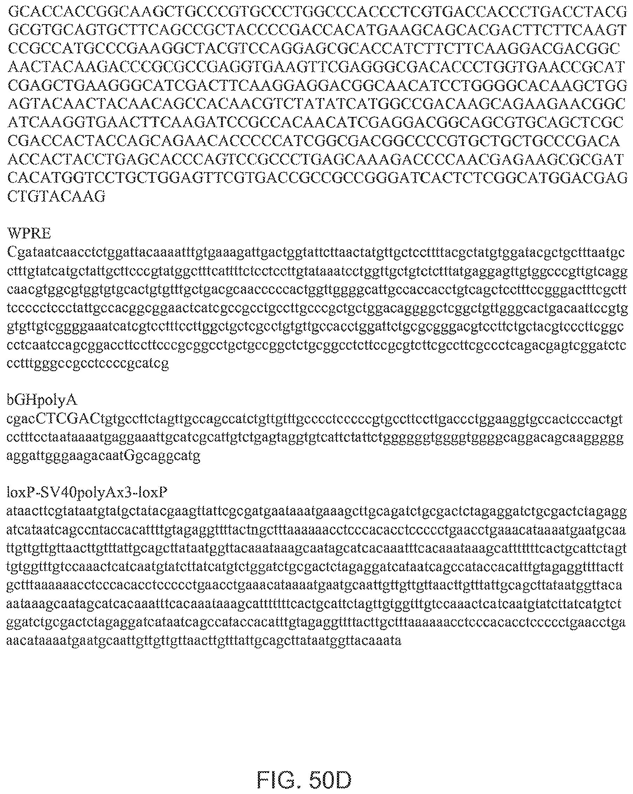

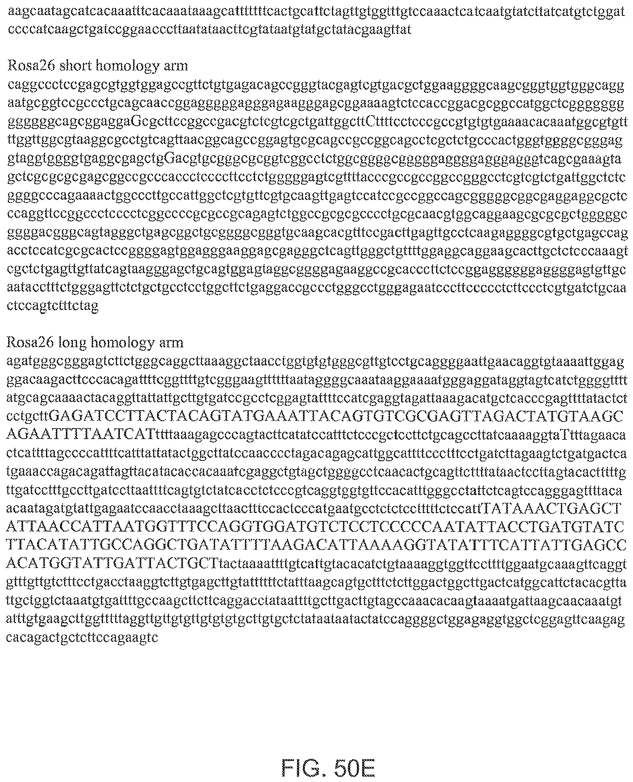

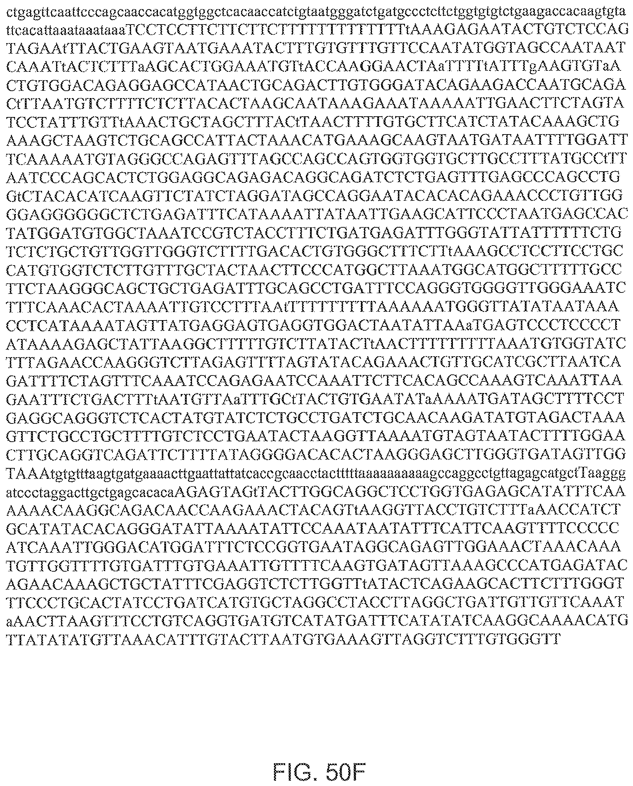

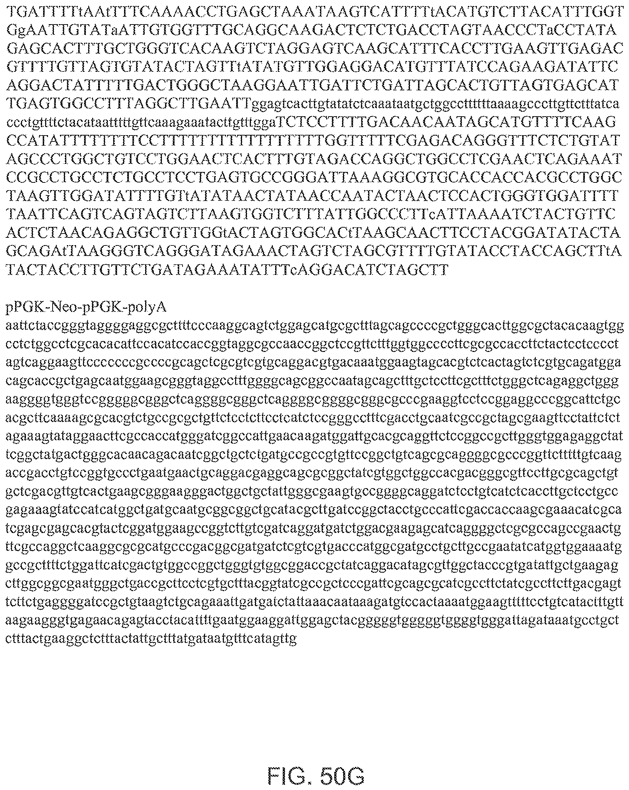

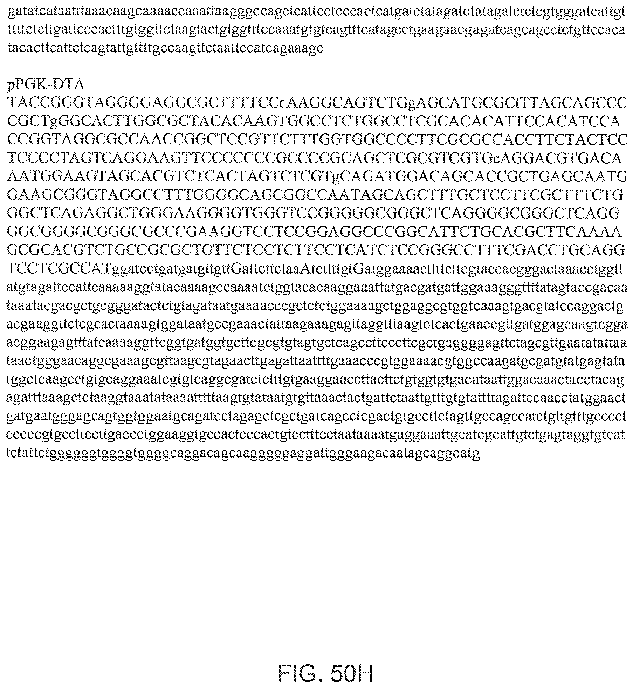

**Figure 50A-H** show the sequences of each element present in the vector maps of Figures 49A-B.

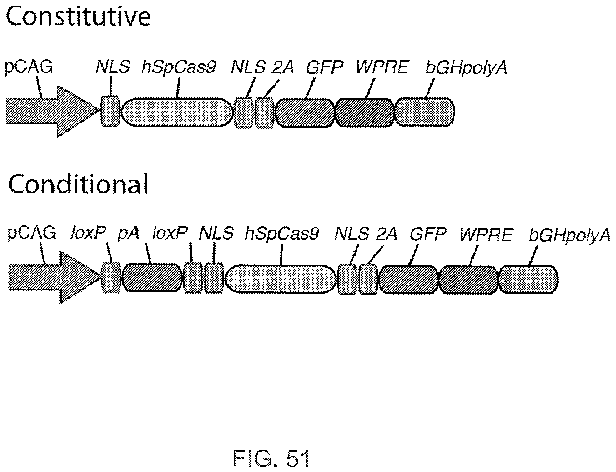

**Figure 51** shows a schematic of the important elements in the Constitutive and Conditional Cas9 constructs.

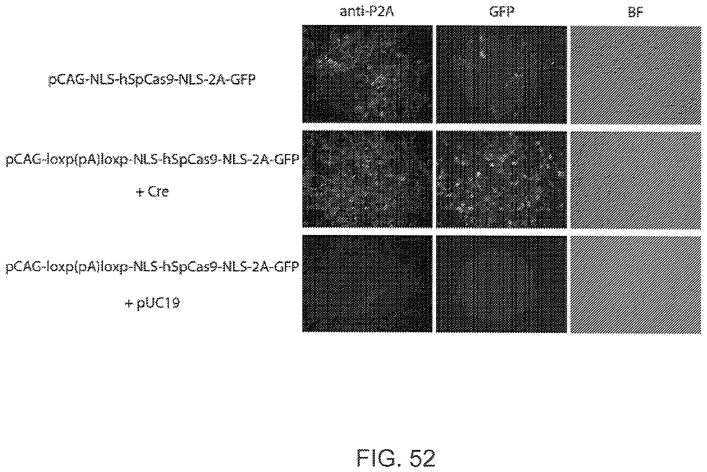

**Figure 52** shows the functional validation of the expression of Constitutive and Conditional Cas9 constructs.

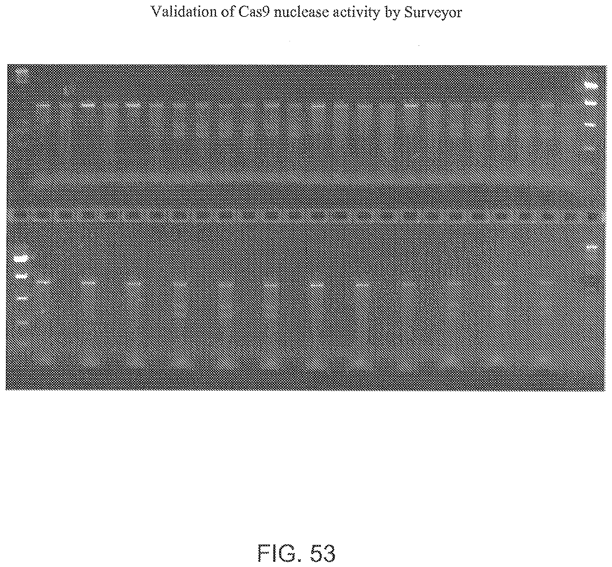

**Figure 53** shows the validation of Cas9 nuclease activity by Surveyor.

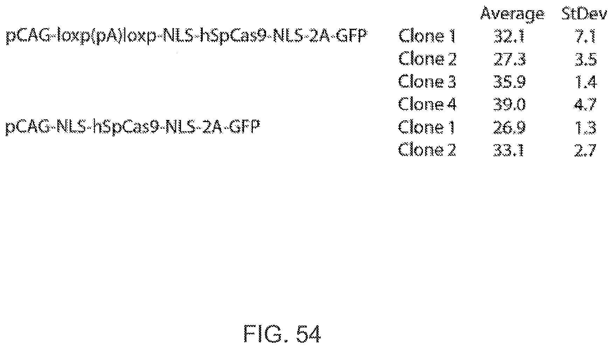

**Figure 54** shows the quantification of Cas9 nuclease activity.

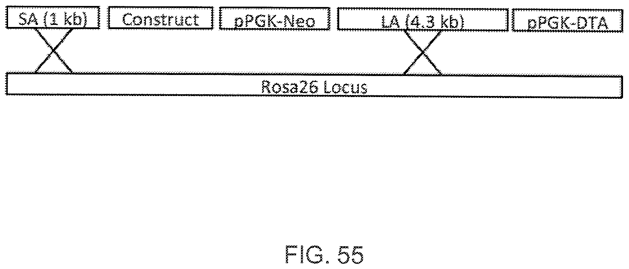

**Figure 55** shows construct design and homologous recombination (HR) strategy.

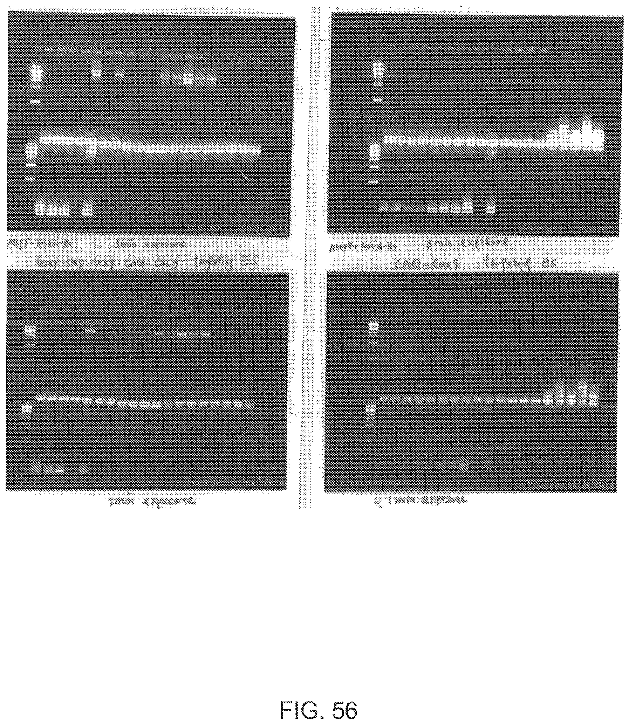

**Figure 56** shows the genomic PCR genotyping results for the constitutive (Right) and conditional (Left) constructs at two different gel exposure times (top row for 3 min and bottom row for 1 min).

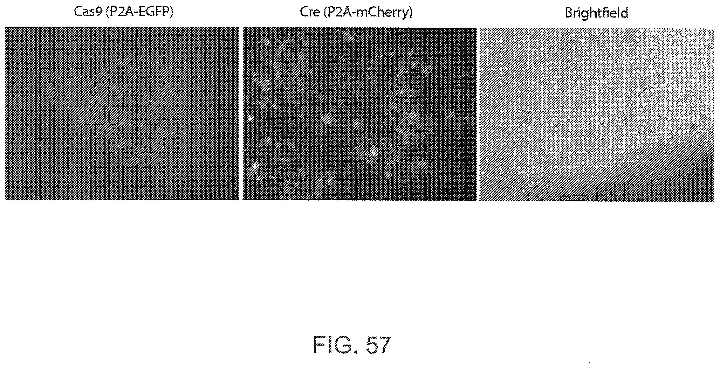

**Figure 57** shows Cas9 activation in mESCs.

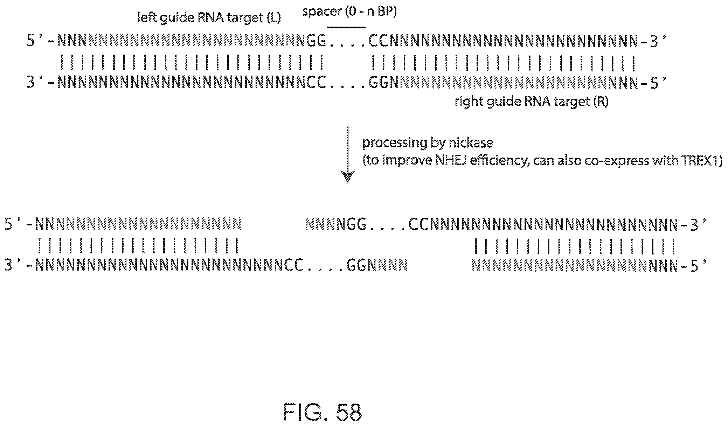

**Figure 58** shows a schematic of the strategy used to mediate gene knockout via NHEJ using a nickase version of Cas9 along with two guide RNAs.

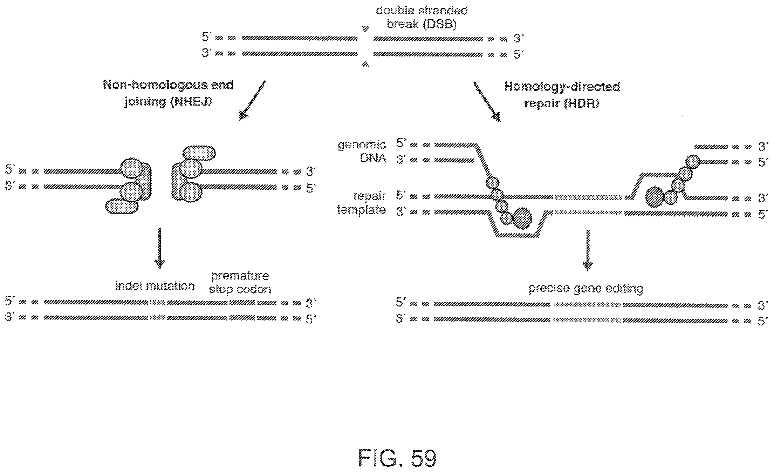

**Figure 59** shows how DNA double-strand break (DSB) repair promotes gene editing. In the error-prone non-homologous end joining (NHEJ) pathway, the ends of a DSB are processed by endogenous DNA repair machineries and rejoined together, which can result in random insertion/deletion (indel) mutations at the site of junction. Indel mutations occurring within the coding region of a gene can result in frame-shift and a premature stop codon, leading to gene knockout. Alternatively, a repair template in the form of a plasmid or single-stranded oligodeoxynucleotides (ssODN) can be supplied to leverage the homology-directed repair (HDR) pathway, which allows high fidelity and precise editing.

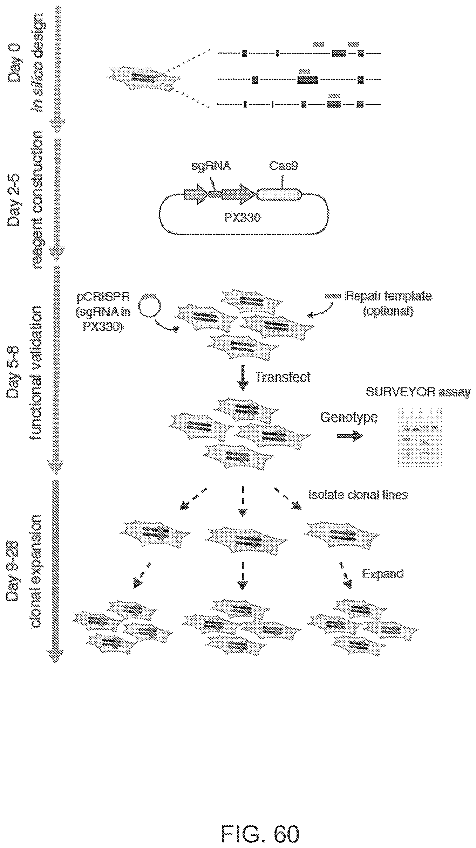

**Figure 60** shows the timeline and overview of experiments. Steps for reagent design, construction, validation, and cell line expansion. Custom sgRNAs (light blue bars) for each target, as well as genotyping primers, are designed in silico via our online design tool (available at the website genome-engineering.org/tools). sgRNA expression vectors are then cloned into a plasmid containing Cas9 (PX330) and verified via DNA sequencing. Completed plasmids (pCRISPRs), and optional repair templates for facilitating homology directed repair, are then transfected into cells and assayed for ability to mediate targeted cleavage. Finally, transfected cells can be clonally expanded to derive isogenic cell lines with defined mutations.

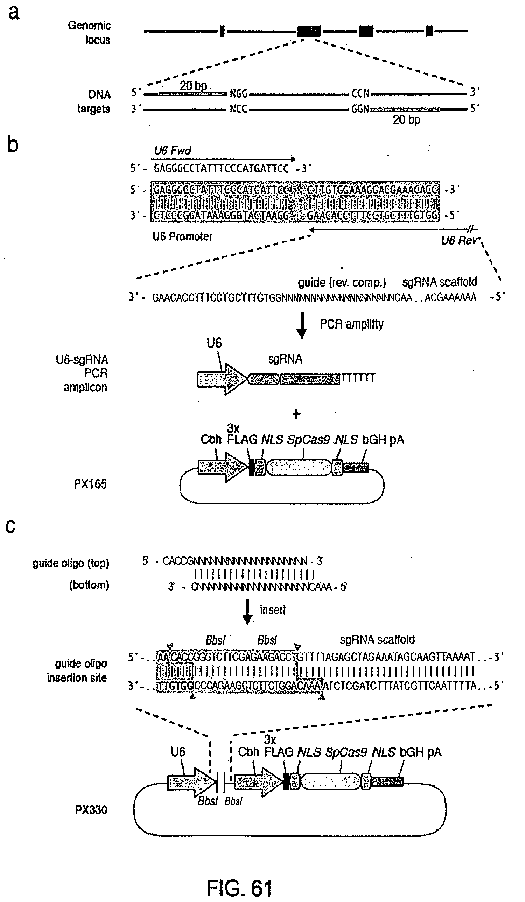

**Figure 61A-C** shows Target selection and reagent preparation. (a) For S. pyogenes Cas9, 20-bp targets (highlighted in blue) must be followed by 5'-NGG, which can occur in either strand on genomic DNA. We recommend using the online tool described in this protocol in aiding target selection (www.genome-engineering.org/tools). (b) Schematic for co-transfection of Cas9 expression plasmid (PX165) and PCR-amplified U6-driven sgRNA expression cassette. Using a U6 promoter-containing PCR template and a fixed forward primer (U6 Fwd), sgRNA-encoding DNA can appended onto the U6 reverse primer (U6 Rev) and synthesized as an extended DNA oligo (Ultramer oligos from IDT). Note the guide sequence (blue N's) in U6 Rev is the reverse complement of the 5'-NGG flanking target sequence. (c) Schematic for scarless cloning of the guide sequence oligos into a plasmid containing Cas9 and sgRNA scaffold (PX330). The guide oligos (blue N's) contain overhangs for ligation into the pair of BbsI sites on PS330, with the top and bottom strand orientations matching those of the genomic target (i.e. top oligo is the 20-bp sequence preceding 5'-NGG in genomic DNA). Digestion of PX330 with BbsI allows the replacement of the Type IIs restriction sites (blue outline) with direct insertion of annealed oligos. It is worth noting that an extra G was placed before the first base of the guide sequence. Applicants have found that an extra G in front of the guide sequence does not adversely affect targeting efficiency. In cases when the 20-nt guide sequence of choice does not begin with guanine, the extra guanine will ensure the sgRNA is efficiently transcribed by the U6 promoter, which prefers a guanine in the first base of the transcript.

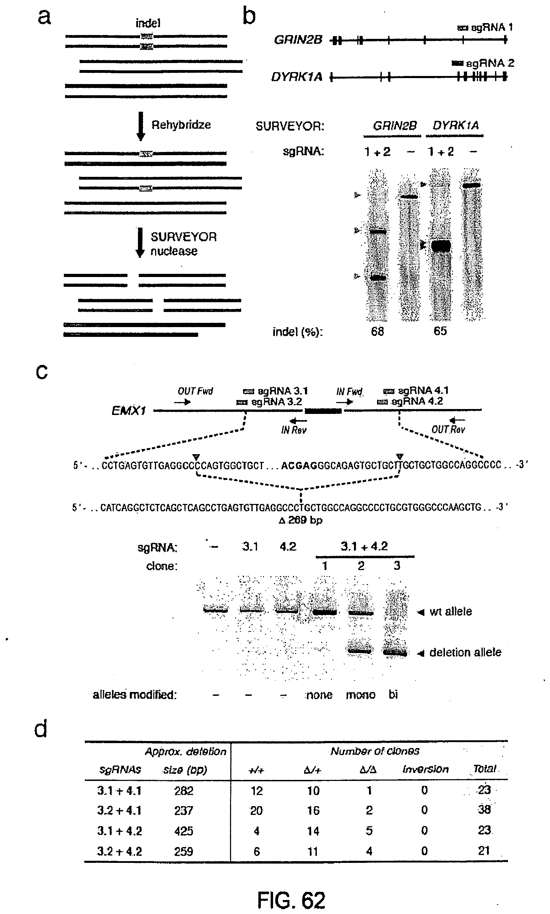

**Figure 62A-D** shows the anticipated results for multiplex NHEJ. (a) Schematic of the SURVEYOR assay used to determine indel percentage. First, genomic DNA from the heterogeneous population of Cas9-targeted cells is amplified by PCR. Amplicons are then reannealed slowly to generate heteroduplexes. The reannealed heteroduplexes are cleaved by SURVEYOR nuclease, whereas homoduplexes are left intact. Cas9-mediated cleavage efficiency (% indel) is calculated based on the fraction of cleaved DNA, as determined by integrated intensity of gel bands.

(b) Two sgRNAs (orange and blue bars) are designed to target the human GRIN2B and DYRK1A loci. SURVEYOR gel shows modification at both loci in transfected cells. Colored arrows indicated expected fragment sizes for each locus. (c) A pair of sgRNAs (light blue and green bars) are designed to excise an exon (dark blue) in the human EMX1 locus. Target sequences and PAMs (red) are shown in respective colors, and sites of cleavage indicated by red triangle. Predicted junction is shown below. Individual clones isolated from cell populations transfected with sgRNA 3, 4, or both are assayed by PCR (OUT Fwd, OUT Rev), reflecting a deletion of ~270-bp. Representative clones with no modification (12/23), mono-allelic (10/23), and bi-allelic (1/23) modifications are shown. IN Fwd and IN Rev primers are used to screen for inversion events (Fig. 6d). (d) Quantification of clonal lines with EMX1 exon deletions. Two pairs of sgRNAs (3.1, 3.2 left-flanking sgRNAs; 4.1, 4.2, right flanking sgRNAs) are used to mediate deletions of variable sizes around one EMX1 exon. Transfected cells are clonally isolated and expanded for genotyping analysis for deletions and inversion events. Of the 105 clones are screened, 51 (49%) and 11 (10%) carrying heterozygous and homozygous deletions, respectively. Approximate deletion sizes are given since junctions may be variable.

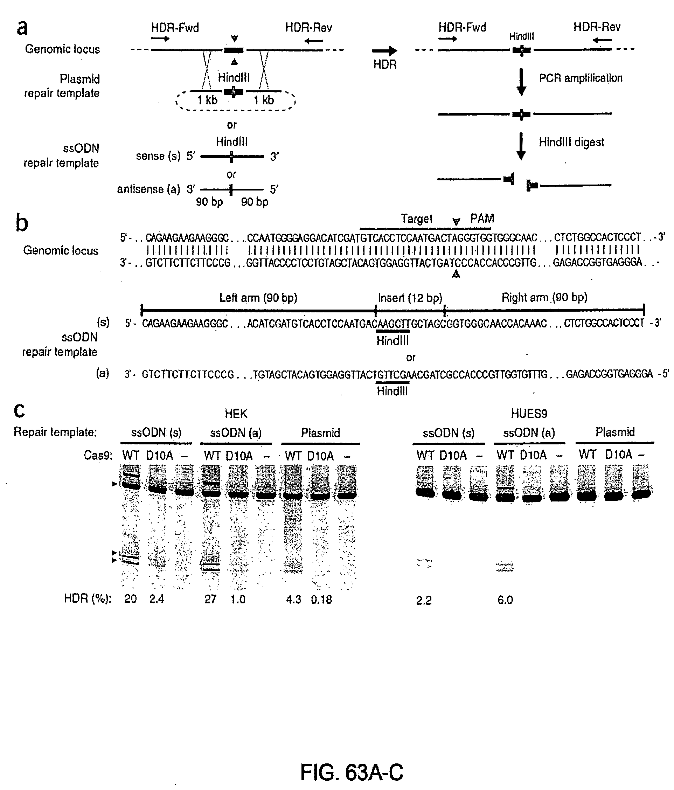

**Figure 63A-C** shows the application of ssODNs and targeting vector to mediate HR with both wildtype and nickase mutant of Cas9 in HEK293FT and HUES9 cells with efficiencies ranging from 1.0-27%.

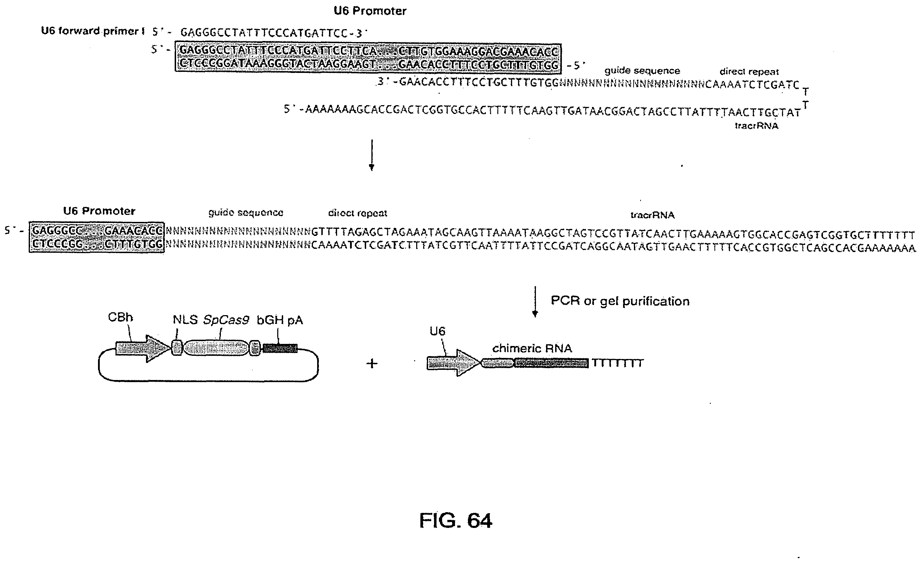

**Figure 64** shows a schematic of a PCR-based method for rapid and efficient CRISPR targeting in mammalian cells. A plasmid containing the human RNA polymerase III promoter U6 is PCR-amplified using a U6-specific forward primer and a reverse primer carrying the reverse complement of part of the U6 promoter, the sgRNA(+85) scaffold with guide sequence, and 7 T nucleotides for transcriptional termination. The resulting PCR product is purified and co-delivered with a plasmid carrying Cas9 driven by the CBh promoter.

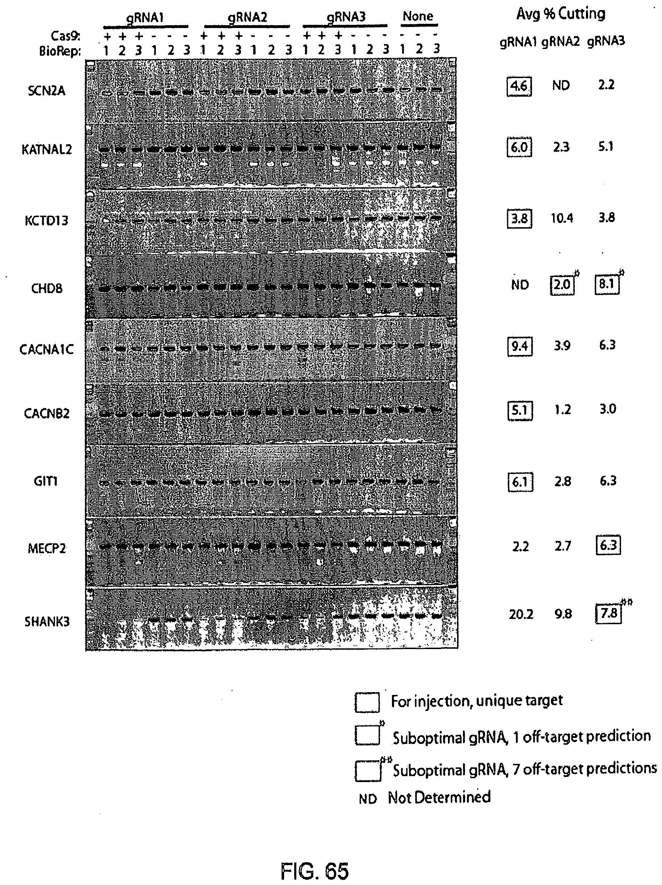

**Figure 65** shows SURVEYOR Mutation Detection Kit from Transgenomics results for each gRNA and respective controls. A positive SURVEYOR result is one large band corresponding to the genomic PCR and two smaller bands that are the product of the SURVEYOR nuclease making a double-strand break at the site of a mutation. Each gRNA was validated in the mouse cell line, Neuro-N2a, by liposomal transient co-transfection with hSpCas9. 72 hours post-transfection genomic DNA was purified using QuickExtract DNA from Epicentre. PCR was performed to amplify the locus of interest.

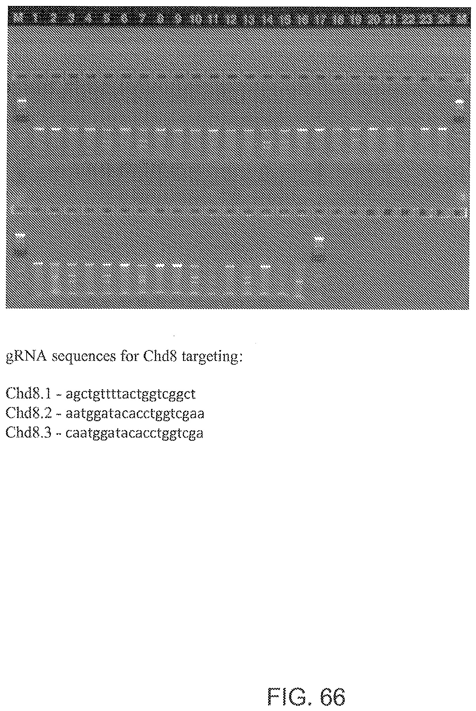

**Figure 66** shows Surveyor results for 38 live pups (lanes 1-38) 1 dead pup (lane 39) and 1 wild-type pup for comparison (lane 40). Pups 1-19 were injected with gRNA Chd8.2 and pups 20-38 were injected with gRNA Chd8.3. Of the 38 live pups, 13 were positive for a mutation. The one dead pup also had a mutation. There was no mutation detected in the wild-type sample. Genomic PCR sequencing was consistent with the SURVEYOR assay findings.

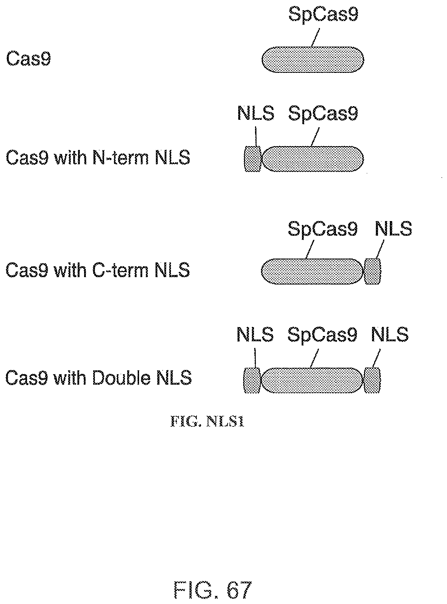

**Figure 67** shows a design of different Cas9 NLS constructs. All Cas9 were the human-codon-optimized version of the Sp Cas9. NLS sequences are linked to the cas9 gene at either N-terminus or C-terminus. All Cas9 variants with different NLS designs were cloned into a backbone vector containing so it is driven by EF1a promoter. On the same vector there is a chimeric RNA targeting human EMX1 locus driven by U6 promoter, together forming a two-component system.

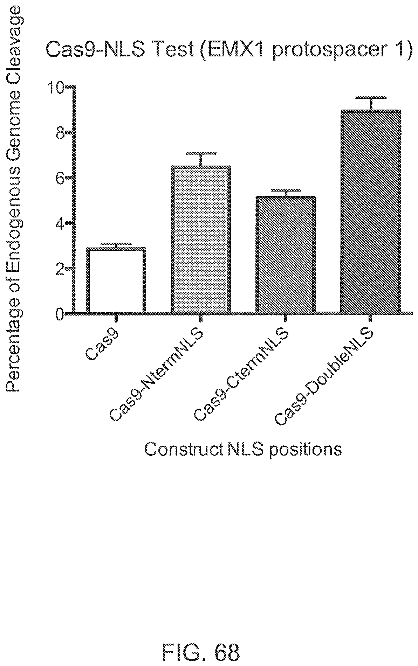

**Figure 68** shows the efficiency of genomic cleavage induced by Cas9 variants bearing different NLS designs. The percentage indicate the portion of human EMX1 genomic DNA that were cleaved by each construct. All experiments are from 3 biological replicates. n = 3, error indicates S.E.M.

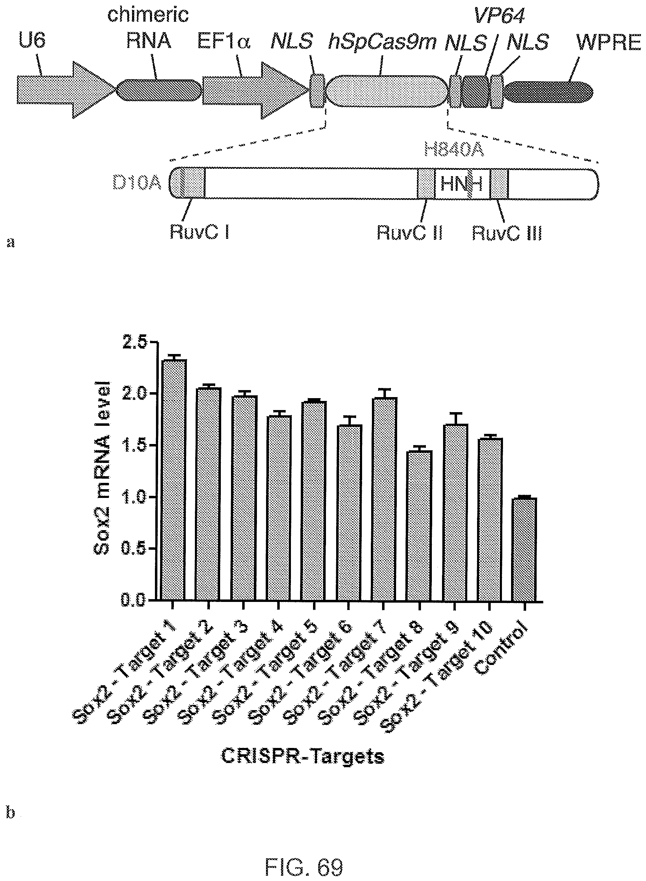

**Figure 69A** shows a design of the CRISPR-TF (Transcription Factor) with transcriptional activation activity. The chimeric RNA is expressed by U6 promoter, while a human-codon-optimized, double-mutant version of the Cas9 protein (hSpCas9m), operably linked to triple NLS and a VP64 functional domain is expressed by a EF1a promoter. The double mutations, D10A and H840A, renders the cas9 protein unable to introduce any cleavage but maintained its capacity to bind to target DNA when guided by the chimeric RNA.

**Figure 69B** shows transcriptional activation of the human SOX2 gene with CRISPR-TF system (Chimeric RNA and the Cas9-NLS-VP64 fusion protein). 293FT cells were transfected with plasmids bearing two components: (1) U6-driven different chimeric RNAs targeting 20-bp sequences within or around the human SOX2 genomic locus, and (2) EF1a-driven hSpCas9m (double mutant)-NLS-VP64 fusion protein. 96 hours post transfection, 293FT cells were harvested and the level of activation is measured by the induction of mRNA expression using a qRT-PCR assay. All expression levels are normalized against the control group (grey bar), which represents results from cells transfected with the CRISPR-TF backbone plasmid without chimeric RNA. The qRT-PCR probes used for detecting the SOX2 mRNA is Taqman Human Gene Expression Assay (Life Technologies). All experiments represents data from 3 biological replicates, n=3, error bars show s.e.m.

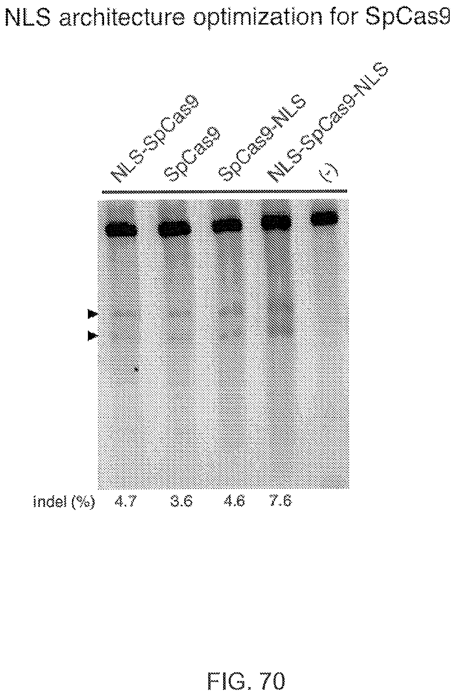

**Figure 70** depicts NLS architecture optimization for SpCas9.

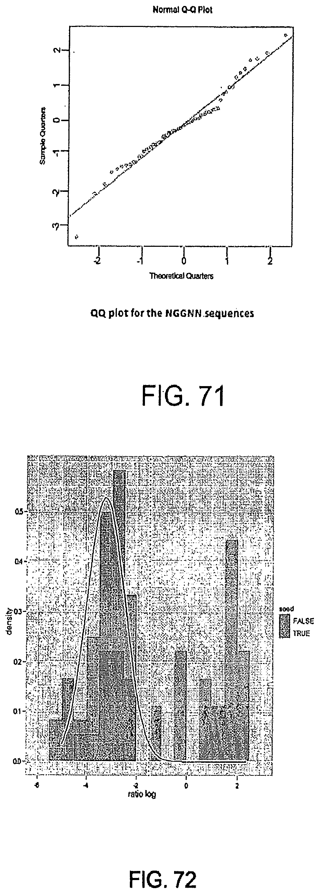

**Figure 71** shows a QQ plot for NGGNN sequences.

**Figure 72** shows a histogram of the data density with fitted normal distribution (black line) and .99 quantile (dotted line).

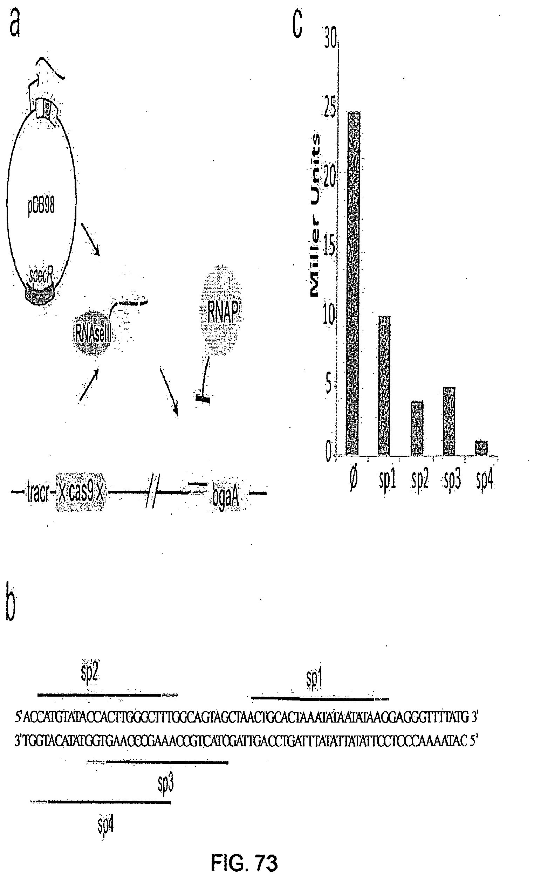

**Figure 73A-C** shows RNA-guided repression of *bgaA* expression by dgRNA::cas9**. **a.** The Cas9 protein binds to the tracrRNA, and to the precursor CRISPR RNA which is processed by RNAseIII to form the crRNA. The crRNA directs binding of Cas9 to the *bgaA* promoter and represses transcription. **b.** The targets used to direct Cas9** to the bgaA promoter are represented. Putative -35, -10 as well as the *bgaA* start codon are in bold. **c.** Betagalactosidase activity as measure by Miller assay in the absence of targeting and for the four different targets.

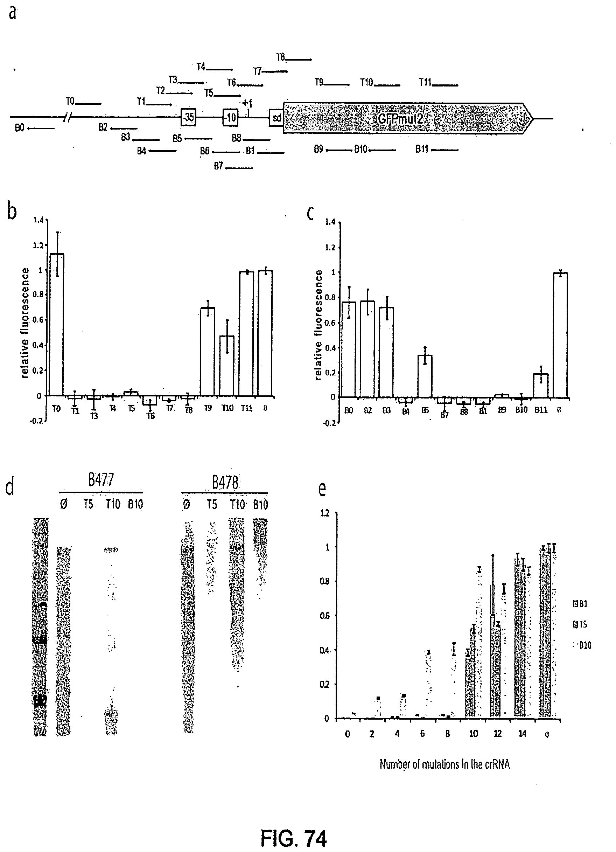

**Figure 74A-E** shows characterization of Cas9** mediated repression. **a.** The *gfpmut2* gene and its promoter, including the -35 and -10 signals are represented together with the position of the different target sites used the study. **b.** Relative fluorescence upon targeting of the coding strand. **c.** Relative fluorescence upon targeting of the non-coding strand. **d.** Northern blot with probes B477 and B478 on RNA extracted from T5, T10, B10 or a control strain without a target. **e.** Effect of an increased number of mutations in the 5' end of the crRNA of B1, T5 and B10.

The figures herein are for illustrative purposes only and are not necessarily drawn to scale.

DETAILED DESCRIPTION OF THE INVENTION

The terms "polynucleotide", "nucleotide", "nucleotide sequence", "nucleic acid" and "oligonucleotide" are used interchangeably. They refer to a polymeric form of nucleotides of any length, either deoxyribonucleotides or ribonucleotides, or analogs thereof. Polynucleotides may have any three dimensional structure, and may perform any function, known or unknown. The following are non-limiting examples of polynucleotides: coding or non-coding regions of a gene or gene fragment, loci (locus) defined from linkage analysis, exons, introns, messenger RNA (mRNA), transfer RNA, ribosomal RNA, short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, nucleic acid probes, and primers. A polynucleotide may comprise one or more modified nucleotides, such as methylated nucleotides and nucleotide analogs. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component.

In aspects of the disclosure the terms "chimeric RNA", "chimeric guide RNA", "guide RNA", "single guide RNA" and "synthetic guide RNA" are used interchangeably and refer to the polynucleotide sequence comprising the guide sequence, the tracr sequence and the tracr mate sequence. The term "guide sequence" refers to the about 20bp sequence within the guide RNA that specifies the target site and may be used interchangeably with the terms "guide" or "spacer". The term "tracr mate sequence" may also be used interchangeably with the term "direct repeat(s)".

As used herein the term "wild type" is a term of the art understood by skilled persons and means the typical form of an organism, strain, gene or characteristic as it occurs in nature as distinguished from mutant or variant forms.

As used herein the term "variant" should be taken to mean the exhibition of qualities that have a pattern that deviates from what occurs in nature.

The terms "non-naturally occurring" or "engineered" are used interchangeably and indicate the involvement of the hand of man. The terms, when referring to nucleic acid molecules or polypeptides mean that the nucleic acid molecule or the polypeptide is at least substantially free from at least one other component with which they are naturally associated in nature and as found in nature.

"Complementarity" refers to the ability of a nucleic acid to form hydrogen bond(s) with another nucleic acid sequence by either traditional Watson-Crick base pairing or other non-traditional types. A percent complementarity indicates the percentage of residues in a nucleic acid molecule which can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 60%, 65%, 70%, 75%, 80%, 85%, 90%, 95%, 97%, 98%, 99%, or 100% over a region of 8, 9, 10, 11, 12, 13, 14, 15, 16, 17, 18, 19, 20, 21, 22, 23, 24, 25, 30, 35, 40, 45, 50, or more nucleotides, or refers to two nucleic acids that hybridize under stringent conditions.

As used herein, "stringent conditions" for hybridization refer to conditions under which a nucleic acid having complementarity to a target sequence predominantly hybridizes with the target sequence, and substantially does not hybridize to non-target sequences. Stringent conditions are generally sequence-dependent, and vary depending on a number of factors. In general, the longer the sequence, the higher the temperature at which the sequence specifically hybridizes to its target sequence. Non-limiting examples of stringent conditions are described in detail in Tijssen (1993), Laboratory Techniques In Biochemistry And Molecular Biology-Hybridization With Nucleic Acid Probes Part I, Second Chapter "Overview of principles of hybridization and the strategy of nucleic acid probe assay", Elsevier, N.Y.

"Hybridization" refers to a reaction in which one or more polynucleotides react to form a complex that is stabilized via hydrogen bonding between the bases of the nucleotide residues. The hydrogen bonding may occur by Watson Crick base pairing, Hoogstein binding, or in any other sequence specific manner. The complex may comprise two strands forming a duplex structure, three or more strands forming a multi stranded complex, a single self hybridizing strand, or any combination of these. A hybridization reaction may constitute a step in a more extensive process, such as the initiation of PCR, or the cleavage of a polynucleotide by an enzyme. A sequence capable of hybridizing with a given sequence is referred to as the "complement" of the given sequence.

As used herein, "expression" refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The terms "polypeptide", "peptide" and "protein" are used interchangeably herein to refer to polymers of amino acids of any length. The polymer may be linear or branched, it may comprise modified amino acids, and it may be interrupted by non amino acids. The terms also encompass an amino acid polymer that has been modified; for example, disulfide bond formation, glycosylation, lipidation, acetylation, phosphorylation, or any other manipulation, such as conjugation with a labeling component. As used herein the term "amino acid" includes natural and/or unnatural or synthetic amino acids, including glycine and both the D or L optical isomers, and amino acid analogs and peptidomimetics.

The terms "subject," "individual," and "patient" are used interchangeably herein to refer to a vertebrate, preferably a mammal, more preferably a human. Mammals include, but are not limited to, murines, simians, humans, farm animals, sport animals, and pets. Tissues, cells and their progeny of a biological entity obtained in vivo or cultured in vitro are also encompassed.

The terms "therapeutic agent", "therapeutic capable agent" or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose may vary depending on one or more of: the particular agent chosen, the dosing regimen to be followed, whether it is administered in combination with other compounds, timing of administration, the tissue to be imaged, and the physical delivery system in which it is carried.

The practice of the present disclosure employs, unless otherwise indicated, conventional techniques of immunology, biochemistry, chemistry, molecular biology, microbiology, cell biology, genomics and recombinant DNA, which are within the skill of the art. See Sambrook, Fritsch and Maniatis, MOLECULAR CLONING: A LABORATORY MANUAL, 2nd edition (1989); CURRENT PROTOCOLS IN MOLECULAR BIOLOGY (F. M. Ausubel, et al. eds., (1987)); the series METHODS IN ENZYMOLOGY (Academic Press, Inc.): PCR 2: A PRACTICAL APPROACH (M.J. MacPherson, B.D. Hames and G.R. Taylor eds. (1995)), Harlow and Lane, eds. (1988) ANTIBODIES, A LABORATORY MANUAL, and ANIMAL CELL CULTURE (R.I. Freshney, ed. (1987)).

Several aspects of the disclosure relate to vector systems comprising one or more vectors, or vectors as such. Vectors can be designed for expression of CRISPR transcripts (e.g. nucleic acid transcripts, proteins, or enzymes) in prokaryotic or eukaryotic cells. For example, CRISPR transcripts can be expressed in bacterial cells such as *Escherichia coli*, insect cells (using baculovirus expression vectors), yeast cells, or mammalian cells. Suitable host cells are discussed further in Goeddel, GENE EXPRESSION TECHNOLOGY: METHODS IN ENZYMOLOGY 185, Academic Press, San Diego, Calif. (1990). Alternatively, the recombinant expression vector can be transcribed and translated in vitro, for example using T7 promoter regulatory sequences and T7 polymerase.

Vectors may be introduced and propagated in a prokaryote. In some embodiments, a prokaryote is used to amplify copies of a vector to be introduced into a eukaryotic cell or as an intermediate vector in the production of a vector to be introduced into a eukaryotic cell (e.g. amplifying a plasmid as part of a viral vector packaging system). In some embodiments, a prokaryote is used to amplify copies of a vector and express one or more nucleic acids, such as to provide a source of one or more proteins for delivery to a host cell or host organism. Expression of proteins in prokaryotes is most often carried out in *Escherichia coli* with vectors containing constitutive or inducible promoters directing the expression of either fusion or non-fusion proteins. Fusion vectors add a number of amino acids to a protein encoded therein, such as to the amino terminus of the recombinant protein. Such fusion vectors may serve one or more purposes, such as: (i) to increase expression of recombinant protein; (ii) to increase the solubility of the recombinant protein; and (iii) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Example fusion expression vectors include pGEX (Pharmacia Biotech Inc; Smith and Johnson, 1988. Gene 67: 31-40), pMAL (New England Biolabs, Beverly, Mass.) and pRIT5 (Pharmacia, Piscataway, N.J.) that fuse glutathione S-transferase (GST), maltose E binding protein, or protein A, respectively, to the target recombinant protein.

Examples of suitable inducible non-fusion *E. coli* expression vectors include pTrc (Amrann et al., (1988) Gene 69:301-315) and pET 11d (Studier et al., GENE EXPRESSION TECHNOLOGY: METHODS IN ENZYMOLOGY 185, Academic Press, San Diego, Calif. (1990) 60-89).

In some embodiments, a vector is a yeast expression vector. Examples of vectors for expression in yeast *Saccharomyces cerivisae* include pYepSecl (Baldari, et al., 1987. EMBO J. 6: 229-234), pMFa (Kuijan and Herskowitz, 1982. Cell 30: 933-943), pJRY88 (Schultz et al., 1987. Gene 54: 113-123), pYES2 (Invitrogen Corporation, San Diego, Calif.), and picZ (InVitrogen Corp, San Diego, Calif.).

In some embodiments, a vector drives protein expression in insect cells using baculovirus expression vectors. Baculovirus vectors available for expression of proteins in cultured insect cells (e.g., SF9 cells) include the pAc series (Smith, et al., 1983. Mol. Cell. Biol. 3: 2156-2165) and the pVL series (Lucklow and Summers, 1989. Virology 170: 31-39).

In some embodiments, a vector is capable of driving expression of one or more sequences in mammalian cells using a mammalian expression vector. Examples of mammalian expression vectors include pCDM8 (Seed, 1987. Nature 329: 840) and pMT2PC (Kaufman, et al., 1987. EMBO J. 6: 187-195). When used in mammalian cells, the expression vector's control functions are typically provided by one or more regulatory elements. For example, commonly used promoters are derived from polyoma, adenovirus 2, cytomegalovirus, simian virus 40, and others disclosed herein and known in the art. For other suitable expression systems for both prokaryotic and eukaryotic cells see, e.g., Chapters 16 and 17 of Sambrook, etal., MOLECULAR CLONING: A LABORATORY MANUAL. 2nd ed., Cold Spring Harbor Laboratory, Cold Spring Harbor Laboratory Press, Cold Spring Harbor, N.Y., 1989.

In some embodiments, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert, et al., 1987. Genes Dev. 1: 268-277), lymphoid-specific promoters (Calame and Eaton, 1988. Adv. Immunol. 43: 235-275), in particular promoters of T cell receptors (Winoto and Baltimore, 1989. EMBO J. 8: 729-733) and immunoglobulins (Baneiji, et al., 1983. Cell 33: 729-740; Queen and Baltimore, 1983. Cell 33: 741-748), neuron-specific promoters (e.g., the neurofilament promoter; Byrne and Ruddle, 1989. Proc. Natl. Acad. Sci. USA 86: 5473-5477), pancreas-specific promoters (Edlund, et al., 1985. Science 230: 912-916), and mammary gland-specific promoters (e.g., milk whey promoter; U.S. Pat. No. 4,873,316 and European Application Publication No. 264,166). Developmentally-regulated promoters are also encompassed, e.g., the murine hox promoters (Kessel and Gruss, 1990. Science 249: 374-379) and the $\alpha$-fetoprotein promoter (Campes and Tilghman, 1989. Genes Dev. 3: 537-546).

In some embodiments, a regulatory element is operably linked to one or more elements of a CRISPR system so as to drive expression of the one or more elements of the CRISPR system. In general, CRISPRs (Clustered Regularly Interspaced Short Palindromic Repeats), also known as SPIDRs (SPacer Interspersed Direct Repeats), constitute a family of DNA loci that are usually specific to a particular bacterial species. The CRISPR locus comprises a distinct class of interspersed short sequence repeats (SSRs) that were recognized in *E. coli* (Ishino et al., J. Bacteriol., 169:5429-5433 [1987]; and Nakata et al., J. Bacteriol., 171:3553-3556 [1989]), and associated genes. Similar interspersed SSRs have been identified in *Haloferax mediterranei, Streptococcus pyogenes, Anabaena,* and *Mycobacterium tuberculosis* (See, Groenen et al., Mol. Microbiol., 10:1057-1065 [1993]; Hoe et al., Emerg. Infect. Dis., 5:254-263 [1999]; Masepohl et al., Biochim. Biophys. Acta 1307:26-30 [1996]; and Mojica et al., Mol. Microbiol., 17:85-93 [1995]). The CRISPR loci typically differ from other SSRs by the structure of the repeats, which have been termed short regularly spaced repeats (SRSRs) (Janssen et al., OMICS J. Integ. Biol., 6:23-33 [2002]; and Mojica et al., Mol. Microbiol., 36:244-246 [2000]). In general, the repeats are short elements that occur in clusters that are regularly spaced by unique intervening sequences with a substantially constant length (Mojica et al., [2000], supra). Although the repeat sequences are highly conserved between strains, the number of interspersed repeats and the sequences of the spacer regions typically differ from strain to strain (van Embden et al., J. Bacteriol., 182:2393-2401 [2000]). CRISPR loci have been identified in more than 40 prokaryotes (See e.g., Jansen et al., Mol. Microbiol., 43:1565-1575 [2002]; and Mojica et al., [2005]) including, but not limited to *Aeropyrum, Pyrobaculum, Sulfolobus, Archaeoglobus, Halocarcula, Methanobacterium, Methanococcus, Methanosarcina, Methanopyrus, Pyrococcus, Picrophilus, Thermoplasma, Corynebacterium, Mycobacterium, Streptomyces,*

*Aquifex, Porphyromonas, Chlorobium, Thermus, Bacillus, Listeria, Staphylococcus, Clostridium, Thermoanaerobacter, Mycoplasma, Fusobacterium, Azarcus, Chromobacterium, Neisseria, Nitrosomonas, Desulfovibrio, Geobacter, Myxococcus, Campylobacter, Wolinella, Acinetobacter, Erwinia, Escherichia, Legionella, Methylococcus, Pasteurella, Photobacterium, Salmonella, Xanthomonas, Yersinia, Treponema,* and *Thermotoga.*

In general, "CRISPR system" refers collectively to transcripts and other elements involved in the expression of or directing the activity of CRISPR-associated ("Cas") genes, including sequences encoding a Cas gene, a tracr (trans-activating CRISPR) sequence (e.g. tracrRNA or an active partial tracrRNA), a tracr-mate sequence (encompassing a "direct repeat" and a tracrRNA-processed partial direct repeat in the context of an endogenous CRISPR system), a guide sequence (also referred to as a "spacer" in the context of an endogenous CRISPR system), or other sequences and transcripts from a CRISPR locus. In some embodiments, one or more elements of a CRISPR system is derived from a type I, type II, or type III CRISPR system. In some embodiments, one or more elements of a CRISPR system is derived from a particular organism comprising an endogenous CRISPR system, such as *Streptococcus pyogenes.* In general, a CRISPR system is characterized by elements that promote the formation of a CRISPR complex at the site of a target sequence (also referred to as a protospacer in the context of an endogenous CRISPR system). In the context of formation of a CRISPR complex, "target sequence" refers to a sequence to which a guide sequence is designed to have complementarity, where hybridization between a target sequence and a guide sequence promotes the formation of a CRISPR complex. Full complementarity is not necessarily required, provided there is sufficient complementarity to cause hybridization and promote formation of a CRISPR complex. A target sequence may comprise any polynucleotide, such as DNA or RNA polynucleotides. In some embodiments, a target sequence is located in the nucleus or cytoplasm of a cell. In some embodiments, the target sequence may be within an organelle of a eukaryotic cell, for example, mitochondrion or chloroplast. A sequence or template that may be used for recombination into the targeted locus comprising the target sequences is referred to as an "editing template" or "editing polynucleotide" or "editing sequence". In aspects of the disclosure, an exogenous template polynucleotide may be referred to as an editing template. In an aspect of the disclosure the recombination is homologous recombination.

Typically, in the context of an endogenous CRISPR system, formation of a CRISPR complex (comprising a guide sequence hybridized to a target sequence and complexed with one or more Cas proteins) results in cleavage of one or both strands in or near (e.g. within 1, 2, 3, 4, 5, 6, 7, 8, 9, 10, 20, 50, or more base pairs from) the target sequence. Without wishing to be bound by theory, the tracr sequence, which may comprise or consist of all or a portion of a wild-type tracr sequence (e.g. about or more than about 20, 26, 32, 45, 48, 54, 63, 67, 85, or more nucleotides of a wild-type tracr sequence), may also form part of a CRISPR complex, such as by hybridization along at least a portion of the tracr sequence to all or a portion of a tracr mate sequence that is operably linked to the guide sequence. In some embodiments, the tracr sequence has sufficient complementarity to a tracr mate sequence to hybridize and participate in formation of a CRISPR complex. As with the target sequence, it is believed that complete complementarity is not needed, provided there is sufficient to be functional. In some embodiments, the tracr sequence has at least 50%, 60%, 70%, 80%, 90%, 95% or 99% of sequence complementarity along the length of the tracr mate sequence when optimally aligned. In some embodiments, one or more vectors driving expression of one or more elements of a CRISPR system are introduced into a host cell such that expression of the elements of the CRISPR system direct formation of a CRISPR complex at one or more target sites. For example, a Cas enzyme, a guide sequence linked to a tracr-mate sequence, and a tracr sequence could each be operably linked to separate regulatory elements on separate vectors. Alternatively, two or more of the elements expressed from the same or different regulatory elements, may be combined in a single vector, with one or more additional vectors providing any components of the CRISPR system not included in the first vector. CRISPR system elements that are combined in a single vector may be arranged in any suitable orientation, such as one element located 5' with respect to ("upstream" of) or 3' with respect to ("downstream" of) a second element. The coding sequence of one element may be located on the same or opposite strand of the coding sequence of a second element, and oriented in the same or opposite direction. In some embodiments, a single promoter drives expression of a transcript encoding a CRISPR enzyme and one or more of the guide sequence, tracr mate sequence (optionally operably linked to the guide sequence), and a tracr sequence embedded within one or more intron sequences (e.g. each in a different intron, two or more in at least one intron, or all in a single intron). In some embodiments, the CRISPR enzyme, guide sequence, tracr mate sequence, and tracr sequence are operably linked to and expressed from the same promoter.

In some embodiments, a vector comprises one or more insertion sites, such as a restriction endonuclease recognition sequence (also referred to as a "cloning site"). In some embodiments, one or more insertion sites (e.g. about or more than about 1, 2, 3, 4, 5, 6, 7, 8, 9, 10, or more insertion sites) are located upstream and/or downstream of one or more sequence elements of one or more vectors. In some embodiments, a vector comprises an insertion site upstream of a tracr mate sequence, and optionally downstream of a regulatory element operably linked to the tracr mate sequence, such that following insertion of a guide sequence into the insertion site and upon expression the guide sequence directs sequence-specific binding of a CRISPR complex to a target sequence in a eukaryotic cell. In some embodiments, a vector comprises two or more insertion sites, each insertion site being located between two tracr mate sequences so as to allow insertion of a guide sequence at each site. In such an arrangement, the two or more guide sequences may comprise two or more copies of a single guide sequence, two or more different guide sequences, or combinations of these. When multiple different guide sequences are used, a single expression construct may be used to target CRISPR activity to multiple different, corresponding target sequences within a cell. For example, a single vector may comprise about or more than about 1, 2, 3, 4, 5, 6, 7, 8, 9, 10, 15, 20, or more guide sequences. In some embodiments, about or more than about 1, 2, 3, 4, 5, 6, 7, 8, 9, 10, or more such guide-sequence-containing vectors may be provided, and optionally delivered to a cell.

In some embodiments, a vector comprises a regulatory element operably linked to an enzyme-coding sequence encoding a CRISPR enzyme, such as a Cas protein. Non-limiting examples of Cas proteins include Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Cscl, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, homologs thereof, or modified versions thereof. These enzymes are known; for example, the amino acid sequence of *S. pyogenes* Cas9 protein may be found in the SwissProt database under accession number Q99ZW2. In some embodiments, the unmodified CRISPR enzyme has DNA cleavage activity, such as Cas9. In some embodiments the CRISPR enzyme is Cas9, and may be Cas9 from *S. pyogenes* or *S. pneumoniae.* In some embodiments, the CRISPR enzyme directs cleavage of one or both strands at the location of a target sequence, such as within the target sequence and/or within the complement of the target sequence. In some embodiments, the CRISPR enzyme directs cleavage of one or both strands within about 1, 2, 3, 4, 5, 6, 7, 8, 9, 10, 15, 20, 25, 50, 100, 200, 500, or more base pairs from the first or last nucleotide of a target sequence. In some embodiments, a vector encodes a CRISPR enzyme that is mutated to with respect to a corresponding wild-type enzyme such that the mutated CRISPR enzyme lacks the ability to cleave one or both strands of a target polynucleotide containing a target sequence. For example, an aspartate-to-alanine substitution (D10A) in the RuvC I catalytic domain of Cas9 from *S. pyogenes* converts Cas9 from a nuclease that cleaves both strands to a nickase (cleaves a single strand). Other examples of mutations that render Cas9 a nickase include, without limitation, H840A, N854A, and N863A. In some embodiments, a Cas9 nickase may be used in combination with guide sequenc(es), e.g., two guide sequences, which target respectively sense and antisense strands of the DNA target. This combination allows both strands to be nicked and used to induce NHEJ. Applicants have demonstrated (data not shown) the efficacy of two nickase targets (i.e., sgRNAs targeted at the same location but to different strands of DNA) in inducing mutagenic NHEJ. A single nickase (Cas9-D10A with a single sgRNA) is unable to induce NHEJ and create indels but Applicants have shown that double nickase (Cas9-D10A and two sgRNAs targeted to different strands at the same location) can do so in human embryonic stem cells (hESCs). The efficiency is about 50% of nuclease (i.e., regular Cas9 without D10 mutation) in hESCs.

As a further example, two or more catalytic domains of Cas9 (RuvC I, RuvC II, and RuvC III) may be mutated to produce a mutated Cas9 substantially lacking all DNA cleavage activity. In some embodiments, a D10A mutation is combined with one or more of H840A, N854A, or N863A mutations to produce a Cas9 enzyme substantially lacking all DNA cleavage activity. In some embodiments, a CRISPR enzyme is considered to substantially lack all DNA cleavage activity when the DNA cleavage activity of the mutated enzyme is less than about 25%, 10%, 5%, 1%, 0.1%, 0.01%, or lower with respect to its non-mutated form. Other mutations may be useful; where the Cas9 or other CRISPR enzyme is from a species other than *S. pyogenes,* mutations in corresponding amino acids may be made to achieve similar effects.

In some embodiments, an enzyme coding sequence encoding a CRISPR enzyme is codon optimized for expression in particular cells, such as eukaryotic cells. The eukaryotic cells may be those of or derived from a particular organism, such as a mammal, including but not limited to human, mouse, rat, rabbit, dog, or non-human primate. In general, codon optimization refers to a process of modifying a nucleic acid sequence for enhanced expression in the host cells of interest by replacing at least one codon (e.g. about or more than about 1, 2, 3, 4, 5, 10, 15, 20, 25, 50, or more codons) of the native sequence with codons that are more frequently or most frequently used in the genes of that host cell while maintaining the native amino acid sequence. Various species exhibit particular bias for certain codons of a particular amino acid. Codon bias (differences in codon usage between organisms) often correlates with the efficiency of translation of messenger RNA (mRNA), which is in turn believed to be dependent on, among other things, the properties of the codons being translated and the availability of particular transfer RNA (tRNA) molecules. The predominance of selected tRNAs in a cell is generally a reflection of the codons used most frequently in peptide synthesis. Accordingly, genes can be tailored for optimal gene expression in a given organism based on codon optimization. Codon usage tables are readily available, for example, at the "Codon Usage Database", and these tables can be adapted in a number of ways. See Nakamura, Y., et al. "Codon usage tabulated from the international DNA sequence databases: status for the year 2000" Nucl. Acids Res. 28:292 (2000). Computer algorithms for codon optimizing a particular sequence for expression in a particular host cell are also available, such as Gene Forge (Aptagen; Jacobus, PA), are also available. In some embodiments, one or more codons (e.g. 1, 2, 3, 4, 5, 10, 15, 20, 25, 50, or more, or all codons) in a sequence encoding a CRISPR enzyme correspond to the most frequently used codon for a particular amino acid.

In some embodiments, a vector encodes a CRISPR enzyme comprising one or more nuclear localization sequences (NLSs), such as about or more than about 1, 2, 3, 4, 5, 6, 7, 8, 9, 10, or more NLSs. In some embodiments, the CRISPR enzyme comprises about or more than about 1, 2, 3, 4, 5, 6, 7, 8, 9, 10, or more NLSs at or near the amino-terminus, about or more than about 1, 2, 3, 4, 5, 6, 7, 8, 9, 10, or more NLSs at or near the carboxy-terminus, or a combination of these (e.g. one or more NLS at the amino-terminus and one or more NLS at the carboxy terminus). When more than one NLS is present, each may be selected independently of the others, such that a single NLS may be present in more than one copy and/or in combination with one or more other NLSs present in one or more copies. In a preferred embodiment of the disclosure, the CRISPR enzyme comprises at most 6 NLSs. In some embodiments, an NLS is considered near the N- or C-terminus when the nearest amino acid of the NLS is within about 1, 2, 3, 4, 5, 10, 15, 20, 25, 30, 40, 50, or more amino acids along the polypeptide chain from the N- or C-terminus. Typically, an NLS consists of one or more short sequences of positively charged lysines or arginines exposed on the protein surface, but other types of NLS are known. Non-limiting examples of NLSs include an NLS sequence derived from: the NLS of the SV40 virus large T-antigen, having the amino acid sequence PKKKRKV; the NLS from nucleoplasmin (e.g. the nucleoplasmin bipartite NLS with the sequence KRPAATKKAGQAKKKK); the c-myc NLS having the amino acid sequence PAAKRVKLD or RQRRNELKRSP; the hRNPA1 M9 NLS having the sequence NQSSNFGPMKGGNFGGRSSGPYGGGGGQYFAK-PRNQGGY; the sequence RMRIZFKNKGKDTAELRRRRVEVSVELRKAKKDEQILKRRNV of the IBB domain from importin-alpha; the sequences VSRKRPRP and PPKKARED of the myoma T protein; the sequence POPKKKPL of human p53; the sequence SALIKKKKKMAP of mouse c-abl IV; the sequences DRLRR and PKQKKRK of the influenza virus NS1; the sequence RKLKKKIKKL of the Hepatitis virus delta antigen; the sequence REKKKFLKRR of the mouse Mx1 protein; the sequence KRKGDEVDGVDEVAKKKSKK of the human poly(ADP-ribose) polymerase; and the sequence RKCLQAGMNLEARKTKK of the steroid hormone receptors (human) glucocorticoid.

In general, the one or more NLSs are of sufficient strength to drive accumulation of the CRISPR enzyme in a detectable amount in the nucleus of a eukaryotic cell. In general, strength of nuclear localization activity may derive from the number of NLSs in the CRISPR enzyme, the particular NLS(s) used, or a combination of these factors. Detection of accumulation in the nucleus may be performed by any suitable technique. For example, a detectable marker may be fused to the CRISPR enzyme, such that location within a cell may be visualized, such as in combination with a means for detecting the location of the nucleus (e.g. a stain specific for the nucleus such as DAPI). Examples of detectable markers include fluorescent proteins (such as Green fluorescent proteins, or GFP; RFP; CFP), and epitope tags (HA tag, flag tag, SNAP tag). Cell nuclei may also be isolated from cells, the contents of which may then be analyzed by any suitable process for detecting protein, such as immunohistochemistry, Western blot, or enzyme activity assay. Accumulation in the nucleus may also be determined indirectly, such as by an assay for the effect of CRISPR complex formation (e.g. assay for DNA cleavage or mutation at the target sequence, or assay for altered gene expression activity affected by CRISPR complex formation and/or CRISPR enzyme activity), as compared to a control no exposed to the CRISPR enzyme or complex, or exposed to a CRISPR enzyme lacking the one or more NLSs.

In general, a guide sequence is any polynucleotide sequence having sufficient complementarity with a target polynucleotide sequence to hybridize with the target sequence and direct sequence-specific binding of a CRISPR complex to the target sequence. In some embodiments, the degree of complementarity between a guide sequence and its corresponding target sequence, when optimally aligned using a suitable alignment algorithm, is about or more than about 50%, 60%, 75%, 80%, 85%, 90%, 95%, 97.5%, 99%, or more. Optimal alignment may be determined with the use of any suitable algorithm for aligning sequences, non-limiting example of which include the Smith-Waterman algorithm, the Needleman-Wunsch algorithm, algorithms based on the Burrows-Wheeler Transform (e.g. the Burrows Wheeler Aligner), ClustalW, Clustal X, BLAT, Novoalign (Novocraft Technologies, ELAND (Illumina, San Diego, CA), SOAP (available at soap.genomics.org.cn), and Maq (available at maq.sourceforge.net). In some embodiments, a guide sequence is about or more than about 5, 10, 11, 12, 13, 14, 15, 16, 17, 18, 19, 20, 21, 22, 23, 24, 25, 26, 27, 28, 29, 30, 35, 40, 45, 50, 75, or more nucleotides in length. In some embodiments, a guide sequence is less than about 75, 50, 45, 40, 35, 30, 25, 20, 15, 12, or fewer nucleotides in length. The ability of a guide sequence to direct sequence-specific binding of a CRISPR complex to a target sequence may be assessed by any suitable assay. For example, the components of a CRISPR system sufficient to form a CRISPR complex, including the guide sequence to be tested, may be provided to a host cell having the corresponding target sequence, such as by transfection with vectors encoding the components of the CRISPR sequence, followed by an assessment of preferential cleavage within the target sequence, such as by Surveyor assay as described herein. Similarly, cleavage of a target polynucleotide sequence may be evaluated in a test tube by providing the target sequence, components of a CRISPR complex, including the guide sequence to be tested and a control guide sequence different from the test guide sequence, and comparing binding or rate of cleavage at the target sequence between the test and control guide sequence reactions. Other assays are possible, and will occur to those skilled in the art.

A guide sequence may be selected to target any target sequence. In some embodiments, the target sequence is a sequence within a genome of a cell. Exemplary target sequences include those that are unique in the target genome. For example, for the *S. pyogenes* Cas9, a unique target sequence in a genome may include a Cas9 target site of the form MMMMMMMMNNNNNNNNNNNNXGG where NNNNNNNNNNNNXGG (N is A, G, T, or C; and X can be anything) has a single occurrence in the genome. A unique target sequence in a genome may include an *S. pyogenes* Cas9 target site of the form GG where NNNNNNNNNNNNXGG (N is A, G, T, or C; and X can be anything) has a single occurrence in the genome. For the *S. thermophilus* CRISPR1 Cas9, a unique target sequence in a genome may include a Cas9 target site of the form MMMMMMMMNNNNNNNNNNNNXXAGAAW where NNNNNNNNNNNNXXAGAAW (N is A, G, T, or C; X can be anything; and W is A or T) has a single occurrence in the genome. A unique target sequence in a genome may include an *S. thermophilus* CRISPR1 Cas9 target site of the form MMMMMMMMMNNNNNNNNNNNNNXX-AGAAW where NNNNNNNNNNNNXXAGAAW (N is A, G, T, or C; X can be anything; and W is A or T) has a single occurrence in the genome. For the *S. pyogenes* Cas9, a unique target sequence in a genome may include a Cas9 target site of the form MMMMMMMMNNNNNNNNNNNNXGGXG where NNNNNNNNNNNNXGGXG (N is A, G, T, or C; and X can be anything) has a single occurrence in the genome. A unique target sequence in a genome may include an *S. pyogenes* Cas9 target site of the form MMMMMMMMMNNNNNNNNNNNNNXGGXG where NNNNNNNNNNNNXGGXG (N is A, G, T, or C; and X can be anything) has a single occurrence in the genome. In each of these sequences "M" may be A, G, T, or C, and need not be considered in identifying a sequence as unique.

In some embodiments, a guide sequence is selected to reduce the degree of secondary structure within the guide sequence. Secondary structure may be determined by any suitable polynucleotide folding algorithm. Some programs are based on calculating the minimal Gibbs free energy. An example of one such algorithm is mFold, as described by Zuker and Stiegler (Nucleic Acids Res. 9 (1981), 133-148). Another example folding algorithm is the online webserver RNAfold, developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

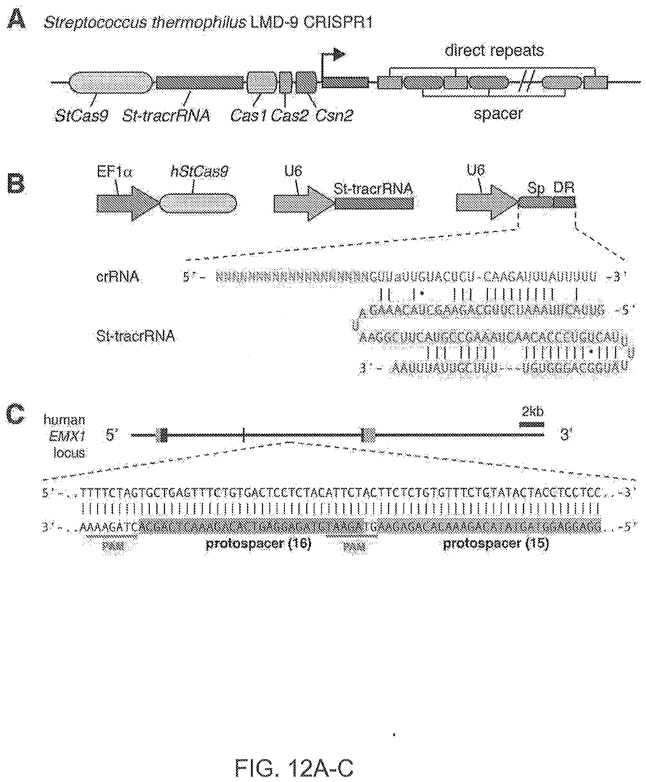

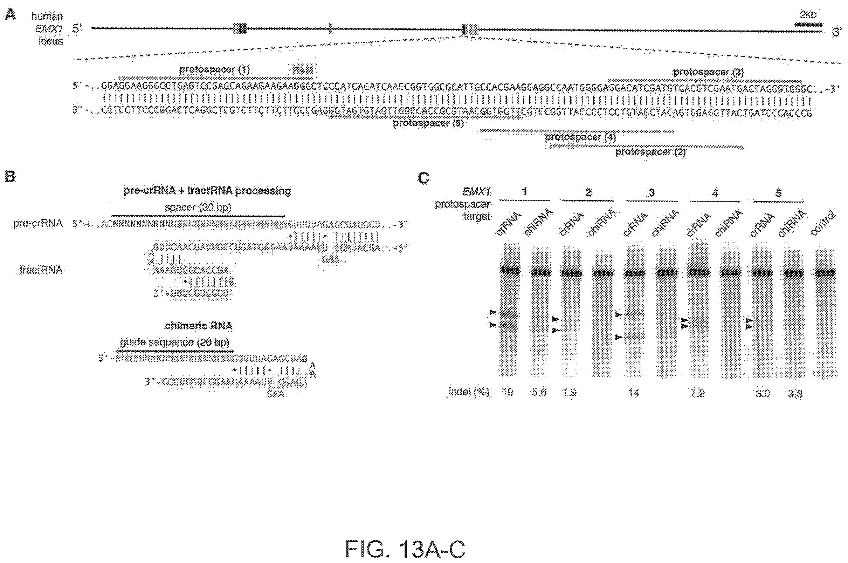

In general, a tracr mate sequence includes any sequence that has sufficient complementarity with a tracr sequence to promote one or more of: (1) excision of a guide sequence flanked by tracr mate sequences in a cell containing the corresponding tracr sequence; and (2) formation of a CRISPR complex at a target sequence, wherein the CRISPR complex comprises the tracr mate sequence hybridized to the tracr sequence. In general, degree of complementarity is with reference to the optimal alignment of the tracr mate sequence and tracr sequence, along the length of the shorter of the two sequences. Optimal alignment may be determined by any suitable alignment algorithm, and may further account for secondary structures, such as self-complementarity within either the tracr sequence or tracr mate sequence. In some embodiments, the degree of complementarity between the tracr sequence and tracr mate sequence along the length of the shorter of the two when optimally aligned is about or more than about 25%, 30%, 40%, 50%, 60%, 70%, 80%, 90%, 95%, 97.5%, 99%, or higher. Example illustrations of optimal alignment between a tracr sequence and a tracr mate sequence are provided in Figures 12B and 13B. In some embodiments, the tracr sequence is about or more than about 5, 6, 7, 8, 9, 10, 11, 12, 13, 14, 15, 16, 17, 18, 19, 20, 25, 30, 40, 50, or more nucleotides in length. In some embodiments, the tracr sequence and tracr mate sequence are contained within a single transcript, such that hybridization between the two produces a transcript having a secondary structure, such as a hairpin. Preferred loop forming sequences for use in hairpin structures are four nucleotides in length, and most preferably have the sequence GAAA. However, longer or shorter loop sequences may be used, as may alternative sequences. The sequences preferably include a nucleotide triplet (for example, AAA), and an additional nucleotide (for example C or G). Examples of loop forming sequences include CAAA and AAAG. In an embodiment of the disclosure, the transcript or transcribed polynucleotide sequence has at least two or more hairpins. In preferred embodiments, the transcript has two, three, four or five hairpins. In a further embodiment of the disclosure, the transcript has at most five hairpins. In some embodiments, the single transcript further includes a transcription termination sequence; preferably this is a polyT sequence, for example six T nucleotides. An example illustration of such a hairpin structure is provided in the lower portion of Figure 13B, where the portion of the sequence 5' of the final "N" and upstream of the loop corresponds to the tracr mate sequence, and the portion of the sequence 3' of the loop corresponds to the tracr sequence. Further non-limiting examples of single polynucleotides comprising a guide sequence, a tracr mate sequence, and a tracr sequence are as follows (listed 5' to 3'), where "N" represents a base of a guide sequence, the first block of lower case letters represent the tracr mate sequence, and the second block of lower case letters represent the tracr sequence, and the final poly-T sequence represents the transcription terminator: (1) NNNNNNNNNNNNNNNNNNNNgttttgtactctcaagatttaGAAAtaaatcttgca-gaagctacaaagataaggctt catgccgaaatcaacaccctgtcattttatggcagggtgtttcgttatttaaTTTTTT; (2) NNNNNNNNNNNNNNNNNNNNgttttgtactctcaGAAAtgcagaagctacaaagataaggcttcatgccgaaatca acaccctgtcattttat-ggcagggtgtttcgttatttaaTTTTTT; (3) NNNNNNNNNNNNNNNNNNNNgttttgtactctcaGAAAtgcagaagctacaaagataag-gcttcatgccgaaatca acaccctgtcattttatggcagggtgtTTTTTT; (4) NNNNNNNNNNNNNNNNNNNNgtttagagctaGAAAtagcaagttaaaataaggctagtccgttatcaacttgaaaa agtggcaccgagtcggt-gcTTTTTT; (5) NNNNNNNNNNNNNNNNNNNNgtttagagctaGAAATAGcaagttaaaataaggctagtccgttatcaacttgaa aaagt-gTTTTTTT; and (6) NNNNNNNNNNNNNNNNNNNNgtttagagctagAAATAGcaagttaaaataaggctagtccgttatcaTTTTT TTT. In some embodiments, sequences (1) to (3) are used in combination with Cas9 from *S. thermophilus* CRISPR1. In some embodiments, sequences (4) to (6) are used in combination with Cas9 from *S. pyogenes.* In some embodiments, the tracr sequence is a separate transcript from a transcript comprising the tracr mate sequence (such as illustrated in the top portion of Figure 13B).

In some embodiments, a recombination template is also provided. A recombination template may be a component of another vector as described herein, contained in a separate vector, or provided as a separate polynucleotide. In some embodiments, a recombination template is designed to serve as a template in homologous recombination, such as within or near a target sequence nicked or cleaved by a CRISPR enzyme as a part of a CRISPR complex. A template polynucleotide may be of any suitable length, such as about or more than about 10, 15, 20, 25, 50, 75, 100, 150, 200, 500, 1000, or more nucleotides in length. In some embodiments, the template polynucleotide is complementary to a portion of a polynucleotide comprising the target sequence. When optimally aligned, a template polynucleotide might overlap with one or more nucleotides of a target sequences (e.g. about or more than about 1, 5, 10, 15, 20, 25, 30, 35, 40, 45, 50, 60, 70, 80, 90, 100 or more nucleotides). In some embodiments, when a template sequence and a polynucleotide comprising a target sequence are optimally aligned, the nearest nucleotide of the template polynucleotide is within about 1, 5, 10, 15, 20, 25, 50, 75, 100, 200, 300, 400, 500, 1000, 5000, 10000, or more nucleotides from the target sequence.

In some embodiments, the CRISPR enzyme is part of a fusion protein comprising one or more heterologous protein domains (e.g. about or more than about 1, 2, 3, 4, 5, 6, 7, 8, 9, 10, or more domains in addition to the CRISPR enzyme). A CRISPR enzyme fusion protein may comprise any additional protein sequence, and optionally a linker sequence between any two domains. Examples of protein domains that may be fused to a CRISPR enzyme include, without limitation, epitope tags, reporter gene sequences, and protein domains having one or more of the following activities: methylase activity, demethylase activity, transcription activation activity, transcription repression activity, transcription release factor activity, histone modification activity, RNA cleavage activity and nucleic acid binding activity. Non-limiting examples of epitope tags include histidine (His) tags, V5 tags, FLAG tags, influenza hemagglutinin (HA) tags, Myc tags, VSV-G tags, and thioredoxin (Trx) tags. Examples of reporter genes include, but are not limited to, glutathione-S-transferase (GST), horseradish peroxidase (HRP), chloramphenicol acetyltransferase (CAT) beta-galactosidase, beta-glucuronidase, luciferase, green fluorescent protein (GFP), HcRed, DsRed, cyan fluorescent protein (CFP), yellow fluorescent protein (YFP), and autofluorescent proteins including blue fluorescent protein (BFP). A CRISPR enzyme may be fused to a gene sequence encoding a protein or a fragment of a protein that bind DNA molecules or bind other cellular molecules, including but not limited to maltose binding protein (MBP), Stag, Lex A DNA binding domain (DBD) fusions, GAL4 DNA binding domain fusions, and herpes simplex virus (HSV) BP16 protein fusions. Additional domains that may form part of a fusion protein comprising a CRISPR enzyme are described in US20110059502. In some embodiments, a tagged CRISPR enzyme is used to identify the location of a target sequence.

In some aspects, described herein are methods comprising delivering one or more polynucleotides, such as or one or more vectors as described herein, one or more transcripts thereof, and/or one or proteins transcribed therefrom, to a host cell. In some aspects, described herein are cells produced by such methods, and organisms (such as animals, plants, or fungi) comprising or produced from such cells. In some embodiments, a CRISPR enzyme in combination with (and optionally complexed with) a guide sequence is delivered to a cell. Conventional viral and non-viral based gene transfer methods can be used to introduce nucleic acids in mammalian cells or target tissues. Such methods can be used to administer nucleic acids encoding components of a CRISPR system to cells in culture, or in a host organism. Non-viral vector delivery systems include DNA plasmids, RNA (e.g. a transcript of a vector described herein), naked nucleic acid, and nucleic acid complexed with a delivery vehicle, such as a liposome. Viral vector delivery systems include DNA and RNA viruses, which have either episomal or integrated genomes after delivery to the cell. For a review of gene therapy procedures, see Anderson, Science 256:808-813 (1992); Nabel & Felgner, TIBTECH 11:211-217 (1993); Mitani & Caskey, TIBTECH 11:162-166 (1993); Dillon, TIBTECH 11:167-175 (1993); Miller, Nature 357:455-460 (1992); Van Brunt, Biotechnology 6(10):1149-1154 (1988); Vigne, Restorative Neurology and Neuroscience 8:35-36 (1995); Kremer & Perricaudet, British Medical Bulletin 51(1):31-44 (1995); Haddada et al., in Current Topics in Microbiology and Immunology, Doerfler and Böhm (eds) (1995); and Yu et al., Gene Therapy 1:13-26 (1994).

Methods of non-viral delivery of nucleic acids include lipofection, nucleofection, microinjection, biolistics, virosomes, liposomes, immunoliposomes, polycation or lipid:nucleic acid conjugates, naked DNA, artificial virions, and agent-enhanced uptake of DNA. Lipofection is described in e.g., U.S. Pat. Nos. 5,049,386, 4,946,787; and 4,897,355) and lipofection reagents are sold commercially (e.g., Transfectam™ and Lipofectin™). Cationic and neutral lipids that are suitable for efficient receptor-recognition lipofection of polynucleotides include those of Felgner, WO 91/17424; WO 91/16024. Delivery can be to cells (e.g. in vitro or ex vivo administration) or target tissues (e.g. in vivo administration).

The preparation of lipid:nucleic acid complexes, including targeted liposomes such as immunolipid complexes, is well known to one of skill in the art (see, e.g., Crystal, Science 270:404-410 (1995); Blaese et al., Cancer Gene Ther. 2:291-297 (1995); Behr et al., Bioconjugate Chem. 5:382-389 (1994); Remy et al., Bioconjugate Chem. 5:647-654 (1994); Gao et al., Gene Therapy 2:710-722 (1995); Ahmad et al., Cancer Res. 52:4817-4820 (1992); U.S. Pat. Nos. 4,186,183, 4,217,344, 4,235,871, 4,261,975, 4,485,054, 4,501,728, 4,774,085, 4,837,028, and 4,946,787).

The use of RNA or DNA viral based systems for the delivery of nucleic acids takes advantage of highly evolved processes for targeting a virus to specific cells in the body and trafficking the viral payload to the nucleus. Viral vectors can be administered directly to patients (in vivo) or they can be used to treat cells in vitro, and the modified cells may optionally be administered to patients (ex vivo). Conventional viral based systems could include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long term expression of the inserted transgene. Additionally, high transduction efficiencies have been observed in many different cell types and target tissues.

The tropism of a retrovirus can be altered by incorporating foreign envelope proteins, expanding the potential target population of target cells. Lentiviral vectors are retroviral vectors that are able to transduce or infect non-dividing cells and typically produce high viral titers. Selection of a retroviral gene transfer system would therefore depend on the target tissue. Retroviral vectors are comprised of cis-acting long terminal repeats with packaging capacity for up to 6-10 kb of foreign sequence. The minimum cis-acting LTRs are sufficient for replication and packaging of the vectors, which are then used to integrate the therapeutic gene into the target cell to provide permanent transgene expression. Widely used retroviral vectors include those based upon murine leukemia virus (MuLV), gibbon ape leukemia virus (GaLV), Simian Immuno deficiency virus (SIV), human immuno deficiency virus (HIV), and combinations thereof (see, e.g., Buchscher et al., J. Virol. 66:2731-2739 (1992); Johann et al., J. Virol. 66:1635-1640 (1992); Sommnerfelt et al., Virol. 176:58-59 (1990); Wilson et al., J. Virol. 63:2374-2378 (1989); Miller et al., J. Virol. 65:2220-2224 (1991); PCT/US94/05700).In applications where transient expression is preferred, adenoviral based systems may be used. Adenoviral based vectors are capable of very high transduction efficiency in many cell types and do not require cell division. With such vectors, high titer and levels of expression have been obtained. This vector can be produced in large quantities in a relatively simple system. Adeno-associated virus ("AAV") vectors may also be used to transduce cells with target nucleic acids, e.g., in the in vitro production of nucleic acids and peptides, and for in vivo and ex vivo gene therapy procedures (see, e.g., West et al., Virology 160:38-47 (1987); U.S. Pat. No. 4,797,368; WO 93/24641; Kotin, Human Gene Therapy 5:793-801 (1994); Muzyczka, J. Clin. Invest. 94:1351 (1994). Construction of recombinant AAV vectors are described in a number of publications, including U.S. Pat. No. 5,173,414; Tratschin et al., Mol. Cell. Biol. 5:3251-3260 (1985); Tratschin, et al., Mol. Cell. Biol. 4:2072-2081 (1984); Hermonat & Muzyczka, PNAS 81:6466-6470 (1984); and Samulski et al., J. Virol. 63:03822-3828 (1989).

Packaging cells are typically used to form virus particles that are capable of infecting a host cell. Such cells include 293 cells, which package adenovirus, and ψ2 cells or PA317 cells, which package retrovirus. Viral vectors used in gene therapy are usually generated by producing a cell line that packages a nucleic acid vector into a viral particle. The vectors typically contain the minimal viral sequences required for packaging and subsequent integration into a host, other viral sequences being replaced by an expression cassette for the polynucleotide(s) to be expressed. The missing viral functions are typically supplied in trans by the packaging cell line. For example, AAV vectors used in gene therapy typically only possess ITR sequences from the AAV genome which are required for packaging and integration into the host genome. Viral DNA is packaged in a cell line, which contains a helper plasmid encoding the other AAV genes, namely rep and cap, but lacking ITR sequences. The cell line may also be infected with adenovirus as a helper. The helper virus promotes replication of the AAV vector and expression of AAV genes from the helper plasmid. The helper plasmid is not packaged in significant amounts due to a lack of ITR sequences. Contamination with adenovirus can be reduced by, e.g., heat treatment to which adenovirus is more sensitive than AAV. Additional methods for the delivery of nucleic acids to cells are known to those skilled in the art. See, for example, US20030087817.

In some embodiments, a host cell is transiently or non-transiently transfected with one or more vectors described herein. In some embodiments, a cell is transfected as it naturally occurs in a subject. In some embodiments, a cell that is transfected is taken from a subject. In some embodiments, the cell is derived from cells taken from a subject, such as a cell line. A wide variety of cell lines for tissue culture are known in the art. Examples of cell lines include, but are not limited to, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepalclc7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)). In some embodiments, a cell transfected with one or more vectors described herein is used to establish a new cell line comprising one or more vector-derived sequences.

In some embodiments, a cell transiently transfected with the components of a CRISPR system as described herein (such as by transient transfection of one or more vectors, or transfection with RNA), and modified through the activity of a CRISPR complex, is used to establish a new cell line comprising cells containing the modification but lacking any other exogenous sequence. In some embodiments, cells transiently or non-transiently transfected with one or more vectors described herein, or cell lines derived from such cells are used in assessing one or more test compounds.

In some embodiments, one or more vectors described herein are used to produce a non-human transgenic animal or transgenic plant. In some embodiments, the transgenic animal is a mammal, such as a mouse, rat, or rabbit. In certain embodiments, the organism or subject is a plant. In certain embodiments, the organism or subject or plant is algae. Methods for producing transgenic plants and animals are known in the art, and generally begin with a method of cell transfection, such as described herein.

In one aspect, described herein are methods of modifying a target polynucleotide in a eukaryotic cell. In some embodiments, the method comprises allowing a CRISPR complex to bind to the target polynucleotide to effect cleavage of said target polynucleotide thereby modifying the target polynucleotide, wherein the CRISPR complex comprises a CRISPR enzyme complexed with a guide sequence hybridized to a target sequence within said target polynucleotide, wherein said guide sequence is linked to a tracr mate sequence which in turn hybridizes to a tracr sequence.

In one aspect, described herein is a method of modifying expression of a polynucleotide in a eukaryotic cell. In some embodiments, the method comprises allowing a CRISPR complex to bind to the polynucleotide such that said binding results in increased or decreased expression of said polynucleotide; wherein the CRISPR complex comprises a CRISPR enzyme complexed with a guide sequence hybridized to a target sequence within said polynucleotide, wherein said guide sequence is linked to a tracr mate sequence which in turn hybridizes to a tracr sequence.

With recent advances in crop genomics, the ability to use CRISPR-Cas systems to perform efficient and cost effective gene editing and manipulation will allow the rapid selection and comparison of single and multiplexed genetic manipulations to transform such genomes for improved production and enhanced traits. In this regard reference is made to US patents and publications: US Patent No. 6,603,061 - Agrobacterium-Mediated Plant Transformation Method; US Patent No. 7,868,149 - Plant Genome Sequences and Uses Thereof and US 2009/0100536 - Transgenic Plants with Enhanced Agronomic Traits. In the practice of the disclosure, the contents and disclosure of Morrell et al "Crop genomics:advances and applications" Nat Rev Genet. 2011 Dec 29;13(2):85-96 are also referred to. In an advantageous embodiment of the disclosure, the CRISPR/Cas9 system is used to engineer microalgae (Example 15). Accordingly, reference herein to animal cells may also apply, mutatis mutandis, to plant cells unless otherwise apparent.

In one aspect, described herein are methods of modifying a target polynucleotide in a eukaryotic cell, which may be in vivo, ex vivo or in vitro. In some embodiments, the method comprises sampling a cell or population of cells from a human or non-human animal or plant (including micro-algae), and modifying the cell or cells. Culturing may occur at any stage ex vivo. The cell or cells may even be re-introduced into the non-human animal or plant (including micro-algae).

In plants, pathogens are often host-specific. For example, *Fusarium oxysporum* f. sp. *lycopersici* causes tomato wilt but attacks only tomato, and *F. oxysporum f dianthii Puccinia graminis* f. sp. *tritici* attacks only wheat. Plants have existing and induced defenses to resist most pathogens. Mutations and recombination events across plant generations lead to genetic variability that gives rise to susceptibility, especially as pathogens reproduce with more frequency than plants. In plants there can be non-host resistance, e.g., the host and pathogen are incompatible. There can also be Horizontal Resistance, e.g., partial resistance against all races of a pathogen, typically controlled by many genes and Vertical Resistance, e.g., complete resistance to some races of a pathogen but not to other races, typically controlled by a few genes. In a Gene-for-Gene level, plants and pathogens evolve together, and the genetic changes in one balance changes in other. Accordingly, using Natural Variability, breeders combine most useful genes for Yield, Quality, Uniformity, Hardiness, Resistance. The sources of resistance genes include native or foreign Varieties, Heirloom Varieties, Wild Plant Relatives, and Induced Mutations, e.g., treating plant material with mutagenic agents. Using the present disclosure, plant breeders are provided with a new tool to induce mutations. Accordingly, one skilled in the art can analyze the genome of sources of resistance genes, and in Varieties having desired characteristics or traits employ the present disclosure to induce the rise of resistance genes, with more precision than previous mutagenic agents and hence accelerate and improve plant breeding programs.

In one aspect, described herein are kits containing any one or more of the elements disclosed in the above methods and compositions. In some embodiments, the kit comprises a vector system and instructions for using the kit. In some embodiments, the vector system comprises (a) a first regulatory element operably linked to a tracr mate sequence and one or more insertion sites for inserting a guide sequence upstream of the tracr mate sequence, wherein when expressed, the guide sequence directs sequence-specific binding of a CRISPR complex to a target sequence in a eukaryotic cell, wherein the CRISPR complex comprises a CRISPR enzyme complexed with (1) the guide sequence that is hybridized to the target sequence, and (2) the tracr mate sequence that is hybridized to the tracr sequence; and/or (b) a second regulatory element operably linked to an enzyme-coding sequence encoding said CRISPR enzyme comprising a nuclear localization sequence. Elements may be provide individually or in combinations, and may be provided in any suitable container, such as a vial, a bottle, or a tube. In some embodiments, the kit includes instructions in one or more languages, for example in more than one language.

In some embodiments, a kit comprises one or more reagents for use in a process utilizing one or more of the elements described herein. Reagents may be provided in any suitable container. For example, a kit may provide one or more reaction or storage buffers. Reagents may be provided in a form that is usable in a particular assay, or in a form that requires addition of one or more other components before use (e.g. in concentrate or lyophilized form). A buffer can be any buffer, including but not limited to a sodium carbonate buffer, a sodium bicarbonate buffer, a borate buffer, a Tris buffer, a MOPS buffer, a HEPES buffer, and combinations thereof. In some embodiments, the buffer is alkaline. In some embodiments, the buffer has a pH from about 7 to about 10. In some embodiments, the kit comprises one or more oligonucleotides corresponding to a guide sequence for insertion into a vector so as to operably link the guide sequence and a regulatory element. In some embodiments, the kit comprises a homologous recombination template polynucleotide.

In one aspect, described herein are methods for using one or more elements of a CRISPR system. The CRISPR complex described herein provides an effective means for modifying a target polynucleotide. The CRISPR complex described herein has a wide variety of utility including modifying (e.g., deleting, inserting, translocating, inactivating, activating) a target polynucleotide in a multiplicity of cell types. As such the CRISPR complex described herein has a broad spectrum of applications in, e.g., gene therapy, drug screening, disease diagnosis, and prognosis. An exemplary CRISPR complex comprises a CRISPR enzyme complexed with a guide sequence hybridized to a target sequence within the target polynucleotide. The guide sequence is linked to a tracr mate sequence, which in turn hybridizes to a tracr sequence.

The target polynucleotide of a CRISPR complex can be any polynucleotide endogenous or exogenous to the eukaryotic cell. For example, the target polynucleotide can be a polynucleotide residing in the nucleus of the eukaryotic cell. The target polynucleotide can be a sequence coding a gene product (e.g., a protein) or a non-coding sequence (e.g., a regulatory polynucleotide or a junk DNA). Without wishing to be bound by theory, it is believed that the target sequence should be associated with a PAM (protospacer adjacent motif); that is, a short sequence recognized by the CRISPR complex. The precise sequence and length requirements for the PAM differ depending on the CRISPR enzyme used, but PAMs are typically 2-5 base pair sequences adjacent the protospacer (that is, the target sequence) Examples of PAM sequences are given in the examples section below, and the skilled person will be able to identify further PAM sequences for use with a given CRISPR enzyme.

The target polynucleotide of a CRISPR complex may include a number of disease-associated genes and polynucleotides as well as signaling biochemical pathway-associated genes and polynucleotides.

Examples of target polynucleotides include a sequence associated with a signaling biochemical pathway, e.g., a signaling biochemical pathway-associated gene or polynucleotide. Examples of target polynucleotides include a disease associated gene or polynucleotide. A "disease-associated" gene or polynucleotide refers to any gene or polynucleotide which is yielding transcription or translation products at an abnormal level or in an abnormal form in cells derived from a disease-affected tissues compared with tissues or cells of a non disease control. It may be a gene that becomes expressed at an abnormally high level; it may be a gene that becomes expressed at an abnormally low level, where the altered expression correlates with the occurrence and/or progression of the disease. A disease-associated gene also refers to a gene possessing mutation(s) or genetic variation that is directly responsible or is in linkage disequilibrium with a gene(s) that is responsible for the etiology of a disease. The transcribed or translated products may be known or unknown, and may be at a normal or abnormal level.

Examples of disease-associated genes and polynucleotides are available from McKusick-Nathans Institute of Genetic Medicine, Johns Hopkins University (Baltimore, Md.) and National Center for Biotechnology Information, National Library of Medicine (Bethesda, Md.), available on the World Wide Web.

Examples of disease-associated genes and polynucleotides are listed in Tables A and B. Disease specific information is available from McKusick-Nathans Institute of Genetic Medicine, Johns Hopkins University (Baltimore, Md.) and National Center for Biotechnology Information, National Library of Medicine (Bethesda, Md.), available on the World Wide Web. Examples of signaling biochemical pathway-associated genes and polynucleotides are listed in Table C.

Mutations in these genes and pathways can result in production of improper proteins or proteins in improper amounts which affect function. Further examples of genes, diseases and proteins are given in US Provisional application 61/736,527 filed on December 12, 2012 and 61/748,427 filed on February 2, 2013. Such genes, proteins and pathways may be the target polynucleotide of a CRISPR complex.

**Table A**

| DISEASE/DISORDER S | GENE(S) |
| --- | --- |
| Neoplasia | PTEN; ATM; ATR; EGFR; ERBB2; ERBB3; ERBB4; |
|  | Notch1; Notch2; Notch3; Notch4; AKT; AKT2; AKT3; HIF; |

(continued)

| DISEASE/DISORDER S | GENE(S) |
|---|---|
| | HIF1a; HIF3a; Met; HRG; Bcl2; PPAR alpha; PPAR gamma; WT1 (Wilms Tumor); FGF Receptor Family members (5 members: 1, 2, 3, 4, 5); CDKN2a; APC; RB (retinoblastoma); MEN1; VHL; BRCA1; BRCA2; AR (Androgen Receptor); TSG101; IGF; IGF Receptor; Igf1 (4 variants); Igf2 (3 variants); Igf 1 Receptor; Igf2 Receptor; Bax; Bcl2; caspases family (9 members: 1, 2, 3, 4, 6, 7, 8, 9, 12); Kras; Apc |
| Age-related Macular Degeneration | Abcr; Cc12; Cc2; cp (ceruloplasmin); Timp3; cathepsinD; Vldlr; Ccr2 |
| Schizophrenia | Neuregulin1 (Nrg1); Erb4 (receptor for Neuregulin); Complexin1 (Cplx1); Tph1 Tryptophan hydroxylase; Tph2 Tryptophan hydroxylase 2; Neurexin 1; GSK3; GSK3a; GSK3b |
| Disorders | 5-HTT (Slc6a4); COMT; DRD (Drdla); SLC6A3; DAOA; DTNBP1; Dao (Dao1) |
| Trinucleotide Repeat Disorders | HTT (Huntington's Dx); SBMA/SMAX1/AR (Kennedy's Dx); FXN/X25 (Friedrich's Ataxia); ATX3 (Machado-Joseph's Dx); ATXN1 and ATXN2 (spinocerebellar ataxias); DMPK (myotonic dystrophy); Atrophin-1 and Atnl (DRPLA Dx); CBP (Creb-BP - global instability); VLDLR (Alzheimer's); Atxn7; AtxnlO |
| Fragile X Syndrome | FMR2; FXR1; FXR2; mGLUR5 |
| Secretase Related Disorders | APH-1 (alpha and beta); Presenilin (Psen1); nicastrin (Ncstn); PEN-2 |
| Others | Nos1; Parp1; Nat1; Nat2 |
| Prion - related disorders | Prp |
| ALS | SOD1; ALS2; STEX; FUS; TARDBP; VEGF (VEGF-a; VEGF-b; VEGF-c) |
| Drug addiction | Prkce (alcohol); Drd2; Drd4; ABAT (alcohol); GRIA2; Grm5; Grin1; Htr1b; Grin2a; Drd3; Pdyn; Gria1 (alcohol) |
| Autism | Mecp2; BZRAP1; MDGA2; Sema5A; Neurexin 1; Fragile X (FMR2 (AFF2); FXR1; FXR2; Mglur5) |
| Alzheimer's Disease | E1; CHIP; UCH; UBB; Tau; LRP; PICALM; Clusterin; PS1; SORL1; CR1; Vldlr; Uba1; Uba3; CHIP28 (Aqp1, Aquaporin 1); Uchl1; Uchl3; APP |
| Inflammation | IL-10; IL-1 (IL-1a; IL-1b); IL-13; IL-17 (IL-17a (CTLA8); IL-17b; IL-17c; IL-17d; IL-17f); Il-23; Cx3cr1; ptpn22; TNFa; |

(continued)

| DISEASE/DISORDER S | GENE(S) |
|---|---|
| | NOD2/CARD15 for IBD; IL-6; IL-12 (IL-12a; IL-12b); |
| | CTLA4; Cx3cl1 |
| Parkinson's Disease | x-Synuclein; DJ-1; LRRK2; Parkin; PINK1 |

**Table B:**

| Blood and coagulation diseases and disorders | Anemia (CDAN1, CDA1, RPS19, DBA, PKLR, PK1, NT5C3, UMPH1, PSN1, RHAG, RH50A, NRAMP2, SPTB, ALAS2, ANH1, ASB, ABCB7, ABC7, ASAT); Bare lymphocyte syndrome (TAPBP, TPSN, TAP2, ABCB3, PSF2, RING11, MHC2TA, C2TA, RFX5, RFXAP, |
|---|---|
| | RFX5), Bleeding disorders (TBXA2R, P2RX1, P2X1); Factor H and factor H-like 1 (HF1, CFH, HUS); Factor V and factor VIII (MCFD2); Factor VII deficiency (F7); Factor X deficiency (F10); Factor XI deficiency (F11); Factor XII deficiency (F12, HAF); Factor XIIIA deficiency (F13A1, F13A); Factor XIIIB deficiency (F13B); Fanconi anemia (FANCA, FACA, FA1, FA, FAA, FAAP95, FAAP90, FLJ34064, FANCB, FANCC, FACC, BRCA2, FANCD1, FANCD2, FANCD, FACD, FAD, FANCE, FACE, FANCF, XRCC9, FANCG, BRIP1, BACH1, FANCJ, PHF9, FANCL, FANCM, KIAA1596); Hemophagocytic lymphohistiocytosis disorders (PRF1, HPLH2, UNC13D, MUNC13-4, HPLH3, HLH3, FHL3); Hemophilia A (F8, F8C, HEMA); Hemophilia B (F9, HEMB), Hemorrhagic disorders (PI, ATT, F5); Leukocyde deficiencies and disorders (ITGB2, CD18, LCAMB, LAD, EIF2B1, EIF2BA, EIF2B2, EIF2B3, EIF2B5, LVWM, CACH, CLE, EIF2B4); Sickle cell anemia (HBB); Thalassemia (HBA2, HBB, HBD, LCRB, HBA1). |
| | |
| Cell dysregulation and oncology diseases and disorders | B-cell non-Hodgkin lymphoma (BCL7A, BCL7); Leukemia (TAL1, TCL5, SCL, TAL2, FLT3, NBS1, NBS, ZNFNIAI, IK1, LYF1, HOXD4, HOX4B, BCR, CML, PHL, ALL, ARNT, KRAS2, RASK2, GMPS, AF10, ARHGEF12, LARG, KIAA0382, CALM, CLTH, CEBPA, CEBP, CHIC2, BTL, FLT3, KIT, PBT, LPP, NPM1, NUP214, D9S46E, CAN, CAIN, RUNX1, CBFA2, AML1, WHSC1L1, NSD3, FLT3, AF1Q, NPM1, NUMA1, ZNF145, PLZF, PML, MYL, STAT5B, AF10, CALM, CLTH, ARL11, ARLTS1, P2RX7, P2X7, BCR, CML, PHL, ALL, GRAF, NF1, VRNF, WSS, NFNS, PTPN11, PTP2C, SHP2, NS1, BCL2, CCND1, PRAD1, BCL1, TCRA, GATA1, GF1, ERYF1, NFE1, ABL1, NQO1, DIA4, NMOR1, NUP214, D9S46E, CAN, CAIN). |
| | |
| Inflammation and immune related diseases and disorders | AIDS (KIR3DL1, NKAT3, NKB1, AMB11, KIR3DS1, IFNG, CXCL12, SDF1); Autoimmune lymphoproliferative syndrome (TNFRSF6, APT1, FAS, CD95, ALPS1A); Combined immunodeficiency, (IL2RG, SCIDX1, SCIDX, IMD4); HIV-1 (CCL5, SCYA5, D17S136E, TCP228), HIV susceptibility or infection (IL10, CSIF, CMKBR2, CCR2, CMKBR5, CCCKR5 (CCR5)); Immunodeficiencies (CD3E, CD3G, AICDA, AID, HIGM2, TNFRSF5, CD40, UNG, DGU, HIGM4, TNFSF5, CD40LG, HIGM1, IGM, FOXP3, IPEX, AIID, XPID, PIDX, TNFRSF14B, TACI); Inflammation (IL-10, IL-1 (IL-1a, IL-1b), IL-13, IL-17 (IL-17a (CTLA8), IL-17b, IL-17c, IL-17d, IL-17f), Il-23, Cx3cr1, ptpn22, TNFa, NOD2/CARD15 for IBD, IL-6, IL-12 (IL-12a, IL-12b), CTLA4, Cx3cl1); Severe combined immunodeficiencies (SCIDs)(JAK3, JAKL, DCLREIC, ARTEMIS, SCIDA, RAG1, RAG2, ADA, PTPRC, CD45, LCA, IL7R, CD3D, T3D, IL2RG, SCIDX1, SCIDX, IMD4). |

(continued)

| Metabolic, liver, kidney and protein diseases and disorders | Amyloid neuropathy (TTR, PALB); Amyloidosis (APOA1, APP, AAA, CVAP, AD1, GSN, FGA, LYZ, TTR, PALB); Cirrhosis (KRT18, KRT8, CIRHIA, NAIC, TEX292, KIAA1988); Cystic fibrosis (CFTR, ABCC7, CF, MRP7); Glycogen storage diseases (SLC2A2, GLUT2, G6PC, G6PT, G6PT1, GAA, LAMP2, LAMPB, AGL, GDE, GBE1, GYS2, PYGL, PFKM); Hepatic adenoma, 142330 (TCF1, HNF1A, MODY3), Hepatic failure, early onset, and neurologic disorder (SCOD1, SCO1), Hepatic lipase deficiency (LIPC), Hepatoblastoma, cancer and carcinomas (CTNNB1, PDGFRL, PDGRL, PRLTS, AXIN1, AXIN, CTNNB1, TP53, P53, LFS1, IGF2R, MPRI, MET, CASP8, MCH5; Medullary cystic kidney disease (UMOD, HNFJ, FJHN, MCKD2, ADMCKD2); Phenylketonuria (PAH, PKU1, QDPR, DHPR, PTS); Polycystic kidney and hepatic disease (FCYT, PKHD1, ARPKD, PKD1, PKD2, PKD4, PKDTS, PRKCSH, G19P1, PCLD, SEC63). |
|---|---|
| Muscular / Skeletal diseases and disorders | Becker muscular dystrophy (DMD, BMD, MYF6), Duchenne Muscular Dystrophy (DMD, BMD); Emery-Dreifuss muscular dystrophy (LMNA, LMN1, EMD2, FPLD, CMD1A, HGPS, LGMD1B, LMNA, LMN1, EMD2, FPLD, CMD1A); Facioscapulohumeral muscular dystrophy (FSHMD1A, FSHD1A); Muscular dystrophy (FKRP, MDC1C, LGMD2I, LAMA2, LAMM, LARGE, KIAA0609, MDC1D, FCMD, TTID, MYOT, CAPN3, CANP3, DYSF, LGMD2B, SGCG, LGMD2C, DMDA1, SCG3, SGCA, ADL, DAG2, LGMD2D, DMDA2, SGCB, LGMD2E, SGCD, SGD, LGMD2F, CMD1L, TCAP, LGMD2G, CMD1N, TRIM32, HT2A, LGMD2H, FKRP, MDC1C, LGMD2I, TTN, CMD1G, TMD, LGMD2J, POMT1, CAV3, LGMD1C, SEPN1, SELN, RSMD1, PLEC1, PLTN, EBS1); Osteopetrosis (LRP5, BMND1, LRP7, LR3, OPPG, VBCH2, CLCN7, CLC7, OPTA2, OSTM1, GL, TCIRG1, TIRC7, OC116, OPTB1); Muscular atrophy (VAPB, VAPC, ALS8, SMN1, SMA1, SMA2, SMA3, SMA4, BSCL2, SPG17, GARS, SMAD1, CMT2D, HEXB, IGHMBP2, SMUBP2, CATF1, SMARD1). |
| Neurological and neuronal diseases and disorders | ALS (SOD1, ALS2, STEX, FUS, TARDBP, VEGF (VEGF-a, VEGF-b, VEGF-c); Alzheimer disease (APP, AAA, CVAP, AD1, APOE, AD2, PSEN2, AD4, STM2, APBB2, FE65L1, NOS3, PLAU, URK, ACE, DCP1, ACE1, MPO, PACIP1, PAXIP1L, PTIP, A2M, BLMH, BMH, PSEN1, AD3); Autism (Mecp2, BZRAP1, MDGA2, Sema5A, Neurexin 1, GLO1, MECP2, RTT, PPMX, MRX16, MRX79, NLGN3, NLGN4, KIAA1260, AUTSX2); Fragile X Syndrome (FMR2, FXR1, FXR2, mGLUR5); Huntington's disease and disease like disorders (HD, IT15, PRNP, PRIP, JPH3, JP3, HDL2, TBP, SCA17); Parkinson disease (NR4A2, NURR1, NOT, TINUR, SNCAIP, TBP, SCA17, SNCA, NACP, PARK1, PARK4, DJ1, PARK7, LRRK2, PARK8, PINK1, PARK6, UCHL1, PARK5, SNCA, NACP, PARK1, PARK4, PRKN, PARK2, PDJ, DBH, NDUFV2); Rett syndrome (MECP2, RTT, PPMX, MRX16, MRX79, CDKL5, STK9, MECP2, RTT, PPMX, MRX16, |
| | MRX79, x-Synuclein, DJ-1); Schizophrenia (Neuregulin1 (Nrg1), Erb4 (receptor for Neuregulin), Complexin1 (Cplx1), Tph1 Tryptophan hydroxylase, Tph2, Tryptophan hydroxylase 2, Neurexin 1, GSK3, GSK3a, GSK3b, 5-HTT (Slc6a4), COMT, DRD (Drd1a), SLC6A3, DAOA, DTNBP1, Dao (Dao1)); Secretase Related Disorders (APH-1 (alpha and beta), Presenilin (Psen1), nicastrin, (Ncstn), PEN-2, Nos1, Parp1, Natl, Nat2); Trinucleotide Repeat Disorders (HTT (Huntington's Dx), SBMA/SMAX1/AR (Kennedy's Dx), FXN/X25 (Friedrich's Ataxia), ATX3 (Machado-Joseph's Dx), ATXN1 and ATXN2 (spinocerebellar ataxias), DMPK (myotonic dystrophy), Atrophin-1 and Atn1 (DRPLA Dx), CBP (Creb-BP - global instability), VLDLR (Alzheimer's), Atxn7, Atxn10). |

(continued)

| Occular diseases and disorders | Age-related macular degeneration (Abcr, Ccl2, Cc2, cp (ceruloplasmin), Timp3, cathepsinD, Vldlr, Ccr2); Cataract (CRYAA, CRYA1, CRYBB2, CRYB2, PITX3, BFSP2, CP49, CP47, CRYAA, CRYA1, PAX6, AN2, MGDA, CRYBA1, CRYB1, CRYGC, CRYG3, CCL, LIM2, MP19, CRYGD, CRYG4, BFSP2, CP49, CP47, HSF4, CTM, HSF4, CTM, MIP, AQP0, CRYAB, CRYA2, CTPP2, CRYBB1, CRYGD, CRYG4, CRYBB2, CRYB2, CRYGC, CRYG3, CCL, CRYAA, CRYA1, GJA8, CX50, CAE1, GJA3, CX46, CZP3, CAE3, CCM1, CAM, KRIT1); Corneal clouding and dystrophy (APOA1, TGFBI, CSD2, CDGG1, CSD, BIGH3, CDG2, TACSTD2, TROP2, M1S1, VSX1, RINX, PPCD, PPD, KTCN, COL8A2, FECD, PPCD2, PIP5K3, CFD); Cornea plana congenital (KERA, CNA2); Glaucoma (MYOC, TIGR, GLC1A, JOAG, GPOA, OPTN, GLC1E, FIP2, HYPL, NRP, CYP1B1, GLC3A, OPA1, NTG, NPG, CYP1B1, GLC3A); Leber congenital amaurosis (CRB1, RP12, CRX, CORD2, CRD, RPGRIP1, LCA6, CORD9, RPE65, RP20, AIPL1, LCA4, GUCY2D, GUC2D, LCA1, CORD6, RDH12, LCA3); Macular dystrophy (ELOVL4, ADMD, STGD2, STGD3, RDS, RP7, PRPH2, PRPH, AVMD, AOFMD, VMD2). |
| --- | --- |

**Table C:**

| CELLULAR FUNCTION | GENES |
| --- | --- |
| PI3K/AKT Signaling | PRKCE; ITGAM; ITGA5; IRAK1; PRKAA2; EIF2AK2; |
| | PTEN; EIF4E; PRKCZ; GRK6; MAPK1; TSC1; PLK1; |
| | AKT2; IKBKB; PIK3CA; CDK8; CDKN1B; NFKB2; BCL2; |
| | PIK3CB; PPP2R1A; MAPK8; BCL2L1; MAPK3; TSC2; |
| | ITGA1; KRAS; EIF4EBP1; RELA; PRKCD; NOS3; |
| | PRKAA1; MAPK9; CDK2; PPP2CA; PIM1; ITGB7; |
| | YWHAZ; ILK; TP53; RAF1; IKBKG; RELB; DYRK1A; |
| | CDKN1A; ITGB1; MAP2K2; JAK1; AKT1; JAK2; PIK3R1; |
| | CHUK; PDPK1; PPP2R5C; CTNNB1; MAP2K1; NFKB1; |
| | PAK3; ITGB3; CCND1; GSK3A; FRAP1; SFN; ITGA2; |
| | TTK; CSNK1A1; BRAF; GSK3B; AKT3; FOXO1; SGK; |
| | HSP90AA1; RPS6KB1 |
| ERK/MAPK Signaling | PRKCE; ITGAM; ITGA5; HSPB1; IRAK1; PRKAA2; |
| | EIF2AK2; RAC1; RAP1A; TLN1; EIF4E; ELK1; GRK6; |
| | MAPK1; RAC2; PLK1; AKT2; PIK3CA; CDK8; CREB1; |
| | PRKCI; PTK2; FOS; RPS6KA4; PIK3CB; PPP2R1A; |
| | PIK3C3; MAPK8; MAPK3; ITGA1; ETS1; KRAS; MYCN; |
| | EIF4EBP1; PPARG; PRKCD; PRKAA1; MAPK9; SRC; |
| | CDK2; PPP2CA; PIM1; PIK3C2A; ITGB7; YWHAZ; |
| | PPP1CC; KSR1; PXN; RAF1; FYN; DYRK1A; ITGB1; |
| | MAP2K2; PAK4; PIK3R1; STAT3; PPP2R5C; MAP2K1; |
| | PAK3; ITGB3; ESR1; ITGA2; MYC; TTK; CSNK1A1; |
| | CRKL; BRAF; ATF4; PRKCA; SRF; STAT1; SGK |
| Glucocorticoid Receptor | RAC1; TAF4B; EP300; SMAD2; TRAF6; PCAF; ELK1; |
| Signaling | MAPK1; SMAD3; AKT2; IKBKB; NCOR2; UBE2I; |
| | PIK3CA; CREB1; FOS; HSPA5; NFKB2; BCL2; |

(continued)

| CELLULAR FUNCTION | GENES |
|---|---|
| | MAP3K14; STAT5B; PIK3CB; PIK3C3; MAPK8; BCL2L1; |
| | MAPK3; TSC22D3; MAPK10; NRIP1; KRAS; MAPK13; |
| | RELA; STAT5A; MAPK9; NOS2A; PBX1; NR3C1; |
| | PIK3C2A; CDKN1C; TRAF2; SERPINE1; NCOA3; |
| | MAPK14; TNF; RAF1; IKBKG; MAP3K7; CREBBP; |
| | CDKN1A; MAP2K2; JAK1; IL8; NCOA2; AKT1; JAK2; |
| | PIK3R1; CHUK; STAT3; MAP2K1; NFKB1; TGFBR1; |
| | ESR1; SMAD4; CEBPB; JUN; AR; AKT3; CCL2; MMP1; |
| | STAT1; IL6; HSP90AA1 |
| Axonal Guidance Signaling | PRKCE; ITGAM; ROCK1; ITGA5; CXCR4; ADAM12; |
| | IGF1; RAC1; RAP1A; EIF4E; PRKCZ; NRP1; NTRK2; |
| | ARHGEF7; SMO; ROCK2; MAPK1; PGF; RAC2; |
| | PTPN11; GNAS; AKT2; PIK3CA; ERBB2; PRKCI; PTK2; |
| | CFL1; GNAQ; PIK3CB; CXCL12; PIK3C3; WNT11; |
| | PRKD1; GNB2L1; ABL1; MAPK3; ITGA1; KRAS; RHOA; |
| | PRKCD; PIK3C2A; ITGB7; GLI2; PXN; VASP; RAF1; |
| | FYN; ITGB1; MAP2K2; PAK4; ADAM17; AKT1; PIK3R1; |
| | GLI1; WNT5A; ADAM10; MAP2K1; PAK3; ITGB3; |
| | CDC42; VEGFA; ITGA2; EPHA8; CRKL; RND1; GSK3B; |
| | AKT3; PRKCA |
| Ephrin Receptor Signaling | PRKCE; ITGAM; ROCK1; ITGA5; CXCR4; IRAKI; |
| | PRKAA2; EIF2AK2; RAC1; RAP1A; GRK6; ROCK2; |
| | MAPK1; PGF; RAC2; PTPN11; GNAS; PLK1; AKT2; |
| | DOK1; CDK8; CREB1; PTK2; CFL1; GNAQ; MAP3K14; |
| | CXCL12; MAPK8; GNB2L1; ABL1; MAPK3; ITGA1; |
| | KRAS; RHOA; PRKCD; PRKAA1; MAPK9; SRC; CDK2; |
| | PIM1; ITGB7; PXN; RAF1; FYN; DYRK1A; ITGB1; |
| | MAP2K2; PAK4; AKT1; JAK2; STAT3; ADAM10; |
| | MAP2K1; PAK3; ITGB3; CDC42; VEGFA; ITGA2; |
| | EPHA8; TTK; CSNK1A1; CRKL; BRAF; PTPN13; ATF4; |
| | AKT3; SGK |
| Actin Cytoskeleton | ACTN4; PRKCE; ITGAM; ROCK1; ITGA5; IRAK1; |
| Signaling | PRKAA2; EIF2AK2; RAC1; INS; ARHGEF7; GRK6; |
| | ROCK2; MAPK1; RAC2; PLK1; AKT2; PIK3CA; CDK8; |
| | PTK2; CFL1; PIK3CB; MYH9; DIAPH1; PIK3C3; MAPK8; |
| | F2R; MAPK3; SLC9A1; ITGA1; KRAS; RHOA; PRKCD; |
| | PRKAA1; MAPK9; CDK2; PIM1; PIK3C2A; ITGB7; |
| | PPP1CC; PXN; VIL2; RAF1; GSN; DYRK1A; ITGB1; |

| CELLULAR FUNCTION | GENES |
|---|---|
| | MAP2K2; PAK4; PIP5K1A; PIK3R1; MAP2K1; PAK3; |
| | ITGB3; CDC42; APC; ITGA2; TTK; CSNK1A1; CRKL; |
| | BRAF; VAV3; SGK |
| Huntington's Disease | PRKCE; IGF1; EP300; RCOR1; PRKCZ; HDAC4; TGM2; |
| Signaling | MAPK1; CAPNS1; AKT2; EGFR; NCOR2; SP1; CAPN2; |
| | PIK3CA; HDAC5; CREB1; PRKCI; HSPA5; REST; |
| | GNAQ; PIK3CB; PIK3C3; MAPK8; IGF1R; PRKD1; |
| | GNB2L1; BCL2L1; CAPN1; MAPK3; CASP8; HDAC2; |
| | HDAC7A; PRKCD; HDAC11; MAPK9; HDAC9; PIK3C2A; |
| | HDAC3; TP53; CASP9; CREBBP; AKT1; PIK3R1; |
| | PDPK1; CASP1; APAF1; FRAP1; CASP2; JUN; BAX; |
| | ATF4; AKT3; PRKCA; CLTC; SGK; HDAC6; CASP3 |
| Apoptosis Signaling | PRKCE; ROCK1; BID; IRAK1; PRKAA2; EIF2AK2; BAK1; |
| | BIRC4; GRK6; MAPK1; CAPNS 1; PLK1; AKT2; IKBKB; |
| | CAPN2; CDK8; FAS; NFKB2; BCL2; MAP3K14; MAPK8; |
| | BCL2L1; CAPN1; MAPK3; CASP8; KRAS; RELA; |
| | PRKCD; PRKAA1; MAPK9; CDK2; PIM1; TP53; TNF; |
| | RAF1; IKBKG; RELB; CASP9; DYRK1A; MAP2K2; |
| | CHUK; APAF1; MAP2K1; NFKB1; PAK3; LMNA; CASP2; |
| | BIRC2; TTK; CSNK1A1; BRAF; BAX; PRKCA; SGK; |
| | CASP3; BIRC3; PARP1 |
| B Cell Receptor Signaling | RAC1; PTEN; LYN; ELK1; MAPK1; RAC2; PTPN11; |
| | AKT2; IKBKB; PIK3CA; CREB1; SYK; NFKB2; CAMK2A; |
| | MAP3K14; PIK3CB; PIK3C3; MAPK8; BCL2L1; ABL1; |
| | MAPK3; ETS1; KRAS; MAPK13; RELA; PTPN6; MAPK9; |
| | EGR1; PIK3C2A; BTK; MAPK14; RAF1; IKBKG; RELB; |
| | MAP3K7; MAP2K2; AKT1; PIK3R1; CHUK; MAP2K1; |
| | NFKB1; CDC42; GSK3A; FRAP1; BCL6; BCL10; JUN; |
| | GSK3B; ATF4; AKT3; VAV3; RPS6KB1 |
| Leukocyte Extravasation | ACTN4; CD44; PRKCE; ITGAM; ROCK1; CXCR4; CYBA; |
| Signaling | RAC1; RAP1A; PRKCZ; ROCK2; RAC2; PTPN11; |
| | MMP14; PIK3CA; PRKCI; PTK2; PIK3CB; CXCL12; |
| | PIK3C3; MAPK8; PRKD1; ABL1; MAPK10; CYBB; |
| | MAPK13; RHOA; PRKCD; MAPK9; SRC; PIK3C2A; BTK; |
| | MAPK14; NOX1; PXN; VIL2; VASP; ITGB1; MAP2K2; |
| | CTNND1; PIK3R1; CTNNB1; CLDN1; CDC42; F11R; ITK; |
| | CRKL; VAV3; CTTN; PRKCA; MMP1; MMP9 |
| Integrin Signaling | ACTN4; ITGAM; ROCK1; ITGA5; RAC1; PTEN; RAP1A; |

(continued)

| CELLULAR FUNCTION | GENES |
|---|---|
| | TLN1; ARHGEF7; MAPK1; RAC2; CAPNS1; AKT2; |
| | CAPN2; PIK3CA; PTK2; PIK3CB; PIK3C3; MAPK8; |
| | CAV1; CAPN1; ABL1; MAPK3; ITGA1; KRAS; RHOA; |
| | SRC; PIK3C2A; ITGB7; PPP1CC; ILK; PXN; VASP; |
| | RAF1; FYN; ITGB1; MAP2K2; PAK4; AKT1; PIK3R1; |
| | TNK2; MAP2K1; PAK3; ITGB3; CDC42; RND3; ITGA2; |
| | CRKL; BRAF; GSK3B; AKT3 |
| Acute Phase Response Signaling | IRAKI; SOD2; MYD88; TRAF6; ELK1; MAPK1; PTPN11; |
| | AKT2; IKBKB; PIK3CA; FOS; NFKB2; MAP3K14; |
| | PIK3CB; MAPK8; RIPK1; MAPK3; IL6ST; KRAS; |
| | MAPK13; IL6R; RELA; SOCS1; MAPK9; FTL; NR3C1; |
| | TRAF2; SERPINE1; MAPK14; TNF; RAF1; PDK1; |
| | IKBKG; RELB; MAP3K7; MAP2K2; AKT1; JAK2; PIK3R1; |
| | CHUK; STAT3; MAP2K1; NFKB1; FRAP1; CEBPB; JUN; |
| | AKT3; IL1R1; IL6 |
| PTEN Signaling | ITGAM; ITGA5; RAC1; PTEN; PRKCZ; BCL2L11; |
| | MAPK1; RAC2; AKT2; EGFR; IKBKB; CBL; PIK3CA; |
| | CDKN1B; PTK2; NFKB2; BCL2; PIK3CB; BCL2L1; |
| | MAPK3; ITGA1; KRAS; ITGB7; ILK; PDGFRB; INSR; |
| | RAF1; IKBKG; CASP9; CDKN1A; ITGB1; MAP2K2; |
| | AKT1; PIK3R1; CHUK; PDGFRA; PDPK1; MAP2K1; |
| | NFKB1; ITGB3; CDC42; CCND1; GSK3A; ITGA2; |
| | GSK3B; AKT3; FOXO1; CASP3; RPS6KB1 |
| p53 Signaling | PTEN; EP300; BBC3; PCAF; FASN; BRCA1; GADD45A; |
| | BIRC5; AKT2; PIK3CA; CHEK1; TP53INP1; BCL2; |
| | PIK3CB; PIK3C3; MAPK8; THBS1; ATR; BCL2L1; E2F1; |
| | PMAIP1; CHEK2; TNFRSF10B; TP73; RB1; HDAC9; |
| | CDK2; PIK3C2A; MAPK14; TP53; LRDD; CDKN1A; |
| | HIPK2; AKT1; PIK3R1; RRM2B; APAF1; CTNNB1; |
| | SIRT1; CCND1; PRKDC; ATM; SFN; CDKN2A; JUN; |
| | SNAI2; GSK3B; BAX; AKT3 |
| Aryl Hydrocarbon Receptor Signaling | HSPB1; EP300; FASN; TGM2; RXRA; MAPK1; NQO1; |
| | NCOR2; SP1; ARNT; CDKN1B; FOS; CHEK1; |
| | SMARCA4; NFKB2; MAPK8; ALDH1A1; ATR; E2F1; |
| | MAPK3; NRIP1; CHEK2; RELA; TP73; GSTP1; RB1; |
| | SRC; CDK2; AHR; NFE2L2; NCOA3; TP53; TNF; |
| | CDKN1A; NCOA2; APAF1; NFKB1; CCND1; ATM; ESR1; |
| | CDKN2A; MYC; JUN; ESR2; BAX; IL6; CYP1B1; |

(continued)

| CELLULAR FUNCTION | GENES |
|---|---|
| | HSP90AA1 |
| Xenobiotic Metabolism | PRKCE; EP300; PRKCZ; RXRA; MAPK1; NQO1; |
| Signaling | NCOR2; PIK3CA; ARNT; PRKCI; NFKB2; CAMK2A; |
| | PIK3CB; PPP2R1A; PIK3C3; MAPK8; PRKD1; |
| | ALDH1A1; MAPK3; NRIP1; KRAS; MAPK13; PRKCD; |
| | GSTP1; MAPK9; NOS2A; ABCB1; AHR; PPP2CA; FTL; |
| | NFE2L2; PIK3C2A; PPARGC1A; MAPK14; TNF; RAF1; |
| | CREBBP; MAP2K2; PIK3R1; PPP2R5C; MAP2K1; |
| | NFKB1; KEAP1; PRKCA; EIF2AK3; IL6; CYP1B1; |
| | HSP90AA1 |
| SAPK/JNK Signaling | PRKCE; IRAKI; PRKAA2; EIF2AK2; RAC1; ELK1; |
| | GRK6; MAPK1; GADD45A; RAC2; PLK1; AKT2; PIK3CA; |
| | FADD; CDK8; PIK3CB; PIK3C3; MAPK8; RIPK1; |
| | GNB2L1; IRS1; MAPK3; MAPK10; DAXX; KRAS; |
| | PRKCD; PRKAA1; MAPK9; CDK2; PIM1; PIK3C2A; |
| | TRAF2; TP53; LCK; MAP3K7; DYRK1A; MAP2K2; |
| | PIK3R1; MAP2K1; PAK3; CDC42; JUN; TTK; CSNK1A1; |
| | CRKL; BRAF; SGK |
| PPAr/RXR Signaling | PRKAA2; EP300; INS; SMAD2; TRAF6; PPARA; FASN; |
| | RXRA; MAPK1; SMAD3; GNAS; IKBKB; NCOR2; |
| | ABCA1; GNAQ; NFKB2; MAP3K14; STAT5B; MAPK8; |
| | IRS1; MAPK3; KRAS; RELA; PRKAA1; PPARGC1A; |
| | NCOA3; MAPK14; INSR; RAF1; IKBKG; RELB; MAP3K7; |
| | CREBBP; MAP2K2; JAK2; CHUK; MAP2K1; NFKB1; |
| | TGFBR1; SMAD4; JUN; IL1R1; PRKCA; IL6; HSP90AA1; |
| | ADIPOQ |
| NF-KB Signaling | IRAK1; EIF2AK2; EP300; INS; MYD88; PRKCZ; TRAF6; |
| | TBK1; AKT2; EGFR; IKBKB; PIK3CA; BTRC; NFKB2; |
| | MAP3K14; PIK3CB; PIK3C3; MAPK8; RIPK1; HDAC2; |
| | KRAS; RELA; PIK3C2A; TRAF2; TLR4; PDGFRB; TNF; |
| | INSR; LCK; IKBKG; RELB; MAP3K7; CREBBP; AKT1; |
| | PIK3R1; CHUK; PDGFRA; NFKB1; TLR2; BCL10; |
| | GSK3B; AKT3; TNFAIP3; IL1R1 |
| Neuregulin Signaling | ERBB4; PRKCE; ITGAM; ITGA5; PTEN; PRKCZ; ELK1; |
| | MAPK1; PTPN11; AKT2; EGFR; ERBB2; PRKCI; |
| | CDKN1B; STAT5B; PRKD1; MAPK3; ITGA1; KRAS; |
| | PRKCD; STAT5A; SRC; ITGB7; RAF1; ITGB1; MAP2K2; |
| | ADAM17; AKT1; PIK3R1; PDPK1; MAP2K1; ITGB3; |

(continued)

| CELLULAR FUNCTION | GENES |
|---|---|
| | EREG; FRAP1; PSEN1; ITGA2; MYC; NRG1; CRKL; |
| | AKT3; PRKCA; HSP90AA1; RPS6KB1 |
| Wnt & Beta catenin | CD44; EP300; LRP6; DVL3; CSNK1E; GJA1; SMO; |
| Signaling | AKT2; PIN1; CDH1; BTRC; GNAQ; MARK2; PPP2R1A; |
| | WNT11; SRC; DKK1; PPP2CA; SOX6; SFRP2; ILK; |
| | LEF1; SOX9; TP53; MAP3K7; CREBBP; TCF7L2; AKT1; |
| | PPP2R5C; WNT5A; LRP5; CTNNB1; TGFBR1; CCND1; |
| | GSK3A; DVL1; APC; CDKN2A; MYC; CSNK1A1; GSK3B; |
| | AKT3; SOX2 |
| Insulin Receptor Signaling | PTEN; INS; EIF4E; PTPN1; PRKCZ; MAPK1; TSC1; |
| | PTPN11; AKT2; CBL; PIK3CA; PRKCI; PIK3CB; PIK3C3; |
| | MAPK8; IRS1; MAPK3; TSC2; KRAS; EIF4EBP1; |
| | SLC2A4; PIK3C2A; PPP1CC; INSR; RAF1; FYN; |
| | MAP2K2; JAK1; AKT1; JAK2; PIK3R1; PDPK1; MAP2K1; |
| | GSK3A; FRAP1; CRKL; GSK3B; AKT3; FOXO1; SGK; |
| | RPS6KB1 |
| IL-6 Signaling | HSPB1; TRAF6; MAPKAPK2; ELK1; MAPK1; PTPN11; |
| | IKBKB; FOS; NFKB2; MAP3K14; MAPK8; MAPK3; |
| | MAPK10; IL6ST; KRAS; MAPK13; IL6R; RELA; SOCS1; |
| | MAPK9; ABCB1; TRAF2; MAPK14; TNF; RAF1; IKBKG; |
| | RELB; MAP3K7; MAP2K2; IL8; JAK2; CHUK; STAT3; |
| | MAP2K1; NFKB1; CEBPB; JUN; IL1R1; SRF; IL6 |
| Hepatic Cholestasis | PRKCE; IRAK1; INS; MYD88; PRKCZ; TRAF6; PPARA; |
| | RXRA; IKBKB; PRKCI; NFKB2; MAP3K14; MAPK8; |
| | PRKD1; MAPK10; RELA; PRKCD; MAPK9; ABCB1; |
| | TRAF2; TLR4; TNF; INSR; IKBKG; RELB; MAP3K7; IL8; |
| | CHUK; NR1H2; TJP2; NFKB1; ESR1; SREBF1; FGFR4; |
| | JUN; IL1R1; PRKCA; IL6 |
| IGF-1 Signaling | IGF1; PRKCZ; ELK1; MAPK1; PTPN11; NEDD4; AKT2; |
| | PIK3CA; PRKCI; PTK2; FOS; PIK3CB; PIK3C3; MAPK8; |
| | IGF1R; IRS1; MAPK3; IGFBP7; KRAS; PIK3C2A; |
| | YWHAZ; PXN; RAF1; CASP9; MAP2K2; AKT1; PIK3R1; |
| | PDPK1; MAP2K1; IGFBP2; SFN; JUN; CYR61; AKT3; |
| | FOXO1; SRF; CTGF; RPS6KB1 |
| NRF2-mediated Oxidative | PRKCE; EP300; SOD2; PRKCZ; MAPK1; SQSTM1; |
| Stress Response | NQO1; PIK3CA; PRKCI; FOS; PIK3CB; PIK3C3; MAPK8; |
| | PRKD1; MAPK3; KRAS; PRKCD; GSTP1; MAPK9; FTL; |
| | NFE2L2; PIK3C2A; MAPK14; RAF1; MAP3K7; CREBBP; |

(continued)

| CELLULAR FUNCTION | GENES |
|---|---|
| | MAP2K2; AKT1; PIK3R1; MAP2K1; PPIB; JUN; KEAP1; |
| | GSK3B; ATF4; PRKCA; EIF2AK3; HSP90AA1 |
| Hepatic Fibrosis/Hepatic | EDN1; IGF1; KDR; FLT1; SMAD2; FGFR1; MET; PGF; |
| Stellate Cell Activation | SMAD3; EGFR; FAS; CSF1; NFKB2; BCL2; MYH9; |
| | IGF1R; IL6R; RELA; TLR4; PDGFRB; TNF; RELB; IL8; |
| | PDGFRA; NFKB1; TGFBR1; SMAD4; VEGFA; BAX; |
| | IL1R1; CCL2; HGF; MMP1; STAT1; IL6; CTGF; MMP9 |
| PPAR Signaling | EP300; INS; TRAF6; PPARA; RXRA; MAPK1; IKBKB; |
| | NCOR2; FOS; NFKB2; MAP3K14; STAT5B; MAPK3; |
| | NRIP1; KRAS; PPARG; RELA; STAT5A; TRAF2; |
| | PPARGC1A; PDGFRB; TNF; INSR; RAF1; IKBKG; |
| | RELB; MAP3K7; CREBBP; MAP2K2; CHUK; PDGFRA; |
| | MAP2K1; NFKB1; JUN; IL1R1; HSP90AA1 |
| Fc Epsilon RI Signaling | PRKCE; RAC1; PRKCZ; LYN; MAPK1; RAC2; PTPN11; |
| | AKT2; PIK3CA; SYK; PRKCI; PIK3CB; PIK3C3; MAPK8; |
| | PRKD1; MAPK3; MAPK10; KRAS; MAPK13; PRKCD; |
| | MAPK9; PIK3C2A; BTK; MAPK14; TNF; RAF1; FYN; |
| | MAP2K2; AKT1; PIK3R1; PDPK1; MAP2K1; AKT3; |
| | VAV3; PRKCA |
| G-Protein Coupled | PRKCE; RAP1A; RGS16; MAPK1; GNAS; AKT2; IKBKB; |
| Receptor Signaling | PIK3CA; CREB1; GNAQ; NFKB2; CAMK2A; PIK3CB; |
| | PIK3C3; MAPK3; KRAS; RELA; SRC; PIK3C2A; RAF1; |
| | IKBKG; RELB; FYN; MAP2K2; AKT1; PIK3R1; CHUK; |
| | PDPK1; STAT3; MAP2K1; NFKB1; BRAF; ATF4; AKT3; |
| | PRKCA |
| Inositol Phosphate | PRKCE; IRAK1; PRKAA2; EIF2AK2; PTEN; GRK6; |
| Metabolism | MAPK1; PLK1; AKT2; PIK3CA; CDK8; PIK3CB; PIK3C3; |
| | MAPK8; MAPK3; PRKCD; PRKAA1; MAPK9; CDK2; |
| | PIM1; PIK3C2A; DYRK1A; MAP2K2; PIP5K1A; PIK3R1; |
| | MAP2K1; PAK3; ATM; TTK; CSNK1A1; BRAF; SGK |
| PDGF Signaling | EIF2AK2; ELK1; ABL2; MAPK1; PIK3CA; FOS; PIK3CB; |
| | PIK3C3; MAPK8; CAV1; ABL1; MAPK3; KRAS; SRC; |
| | PIK3C2A; PDGFRB; RAF1; MAP2K2; JAK1; JAK2; |
| | PIK3R1; PDGFRA; STAT3; SPHK1; MAP2K1; MYC; |
| | JUN; CRKL; PRKCA; SRF; STAT1; SPHK2 |
| VEGF Signaling | ACTN4; ROCK1; KDR; FLT1; ROCK2; MAPK1; PGF; |
| | AKT2; PIK3CA; ARNT; PTK2; BCL2; PIK3CB; PIK3C3; |
| | BCL2L1; MAPK3; KRAS; HIF1A; NOS3; PIK3C2A; PXN; |

(continued)

| CELLULAR FUNCTION | GENES |
|---|---|
| | RAF1; MAP2K2; ELAVL1; AKT1; PIK3R1; MAP2K1; SFN; |
| | VEGFA; AKT3; FOXO1; PRKCA |
| Natural Killer Cell Signaling | PRKCE; RAC1; PRKCZ; MAPK1; RAC2; PTPN11; |
| | KIR2DL3; AKT2; PIK3CA; SYK; PRKCI; PIK3CB; |
| | PIK3C3; PRKD1; MAPK3; KRAS; PRKCD; PTPN6; |
| | PIK3C2A; LCK; RAF1; FYN; MAP2K2; PAK4; AKT1; |
| | PIK3R1; MAP2K1; PAK3; AKT3; VAV3; PRKCA |
| Cell Cycle: G1/S | HDAC4; SMAD3; SUV39H1; HDAC5; CDKN1B; BTRC; |
| Checkpoint Regulation | ATR; ABL1; E2F1; HDAC2; HDAC7A; RB1; HDAC11; |
| | HDAC9; CDK2; E2F2; HDAC3; TP53; CDKN1A; CCND1; |
| | E2F4; ATM; RBL2; SMAD4; CDKN2A; MYC; NRG1; |
| | GSK3B; RBL1; HDAC6 |
| T Cell Receptor Signaling | RAC1; ELK1; MAPK1; IKBKB; CBL; PIK3CA; FOS; |
| | NFKB2; PIK3CB; PIK3C3; MAPK8; MAPK3; KRAS; |
| | RELA; PIK3C2A; BTK; LCK; RAF1; IKBKG; RELB; FYN; |
| | MAP2K2; PIK3R1; CHUK; MAP2K1; NFKB1; ITK; BCL10; |
| | JUN; VAV3 |
| Death Receptor Signaling | CRADD; HSPB1; BID; BIRC4; TBK1; IKBKB; FADD; |
| | FAS; NFKB2; BCL2; MAP3K14; MAPK8; RIPK1; CASP8; |
| | DAXX; TNFRSF10B; RELA; TRAF2; TNF; IKBKG; RELB; |
| | CASP9; CHUK; APAF1; NFKB1; CASP2; BIRC2; CASP3; |
| | BIRC3 |
| FGF Signaling | RAC1; FGFR1; MET; MAPKAPK2; MAPK1; PTPN11; |
| | AKT2; PIK3CA; CREB1; PIK3CB; PIK3C3; MAPK8; |
| | MAPK3; MAPK13; PTPN6; PIK3C2A; MAPK14; RAF1; |
| | AKT1; PIK3R1; STAT3; MAP2K1; FGFR4; CRKL; ATF4; |
| | AKT3; PRKCA; HGF |
| GM-CSF Signaling | LYN; ELK1; MAPK1; PTPN11; AKT2; PIK3CA; CAMK2A; |
| | STAT5B; PIK3CB; PIK3C3; GNB2L1; BCL2L1; MAPK3; |
| | ETS1; KRAS; RUNX1; PIM1; PIK3C2A; RAF1; MAP2K2; |
| | AKT1; JAK2; PIK3R1; STAT3; MAP2K1; CCND1; AKT3; |
| | STAT1 |
| Amyotrophic Lateral | BID; IGF1; RAC1; BIRC4; PGF; CAPNS1; CAPN2; |
| Sclerosis Signaling | PIK3CA; BCL2; PIK3CB; PIK3C3; BCL2L1; CAPN1; |
| | PIK3C2A; TP53; CASP9; PIK3R1; RAB5A; CASP1; |
| | APAF1; VEGFA; BIRC2; BAX; AKT3; CASP3; BIRC3 |
| JAK/Stat Signaling | PTPN1; MAPK1; PTPN11; AKT2; PIK3CA; STAT5B; |
| | PIK3CB; PIK3C3; MAPK3; KRAS; SOCS1; STAT5A; |

(continued)

| CELLULAR FUNCTION | GENES |
|---|---|
| | PTPN6; PIK3C2A; RAF1; CDKN1A; MAP2K2; JAK1; |
| | AKT1; JAK2; PIK3R1; STAT3; MAP2K1; FRAP1; AKT3; |
| | STAT1 |
| Nicotinate and Nicotinamide | PRKCE; IRAK1; PRKAA2; EIF2AK2; GRK6; MAPK1; |
| Metabolism | PLK1; AKT2; CDK8; MAPK8; MAPK3; PRKCD; PRKAA1; |
| | PBEF1; MAPK9; CDK2; PIM1; DYRK1A; MAP2K2; |
| | MAP2K1; PAK3; NT5E; TTK; CSNK1A1; BRAF; SGK |
| Chemokine Signaling | CXCR4; ROCK2; MAPK1; PTK2; FOS; CFL1; GNAQ; |
| | CAMK2A; CXCL12; MAPK8; MAPK3; KRAS; MAPK13; |
| | RHOA; CCR3; SRC; PPP1CC; MAPK14; NOX1; RAF1; |
| | MAP2K2; MAP2K1; JUN; CCL2; PRKCA |
| IL-2 Signaling | ELK1; MAPK1; PTPN11; AKT2; PIK3CA; SYK; FOS; |
| | STAT5B; PIK3CB; PIK3C3; MAPK8; MAPK3; KRAS; |
| | SOCS1; STAT5A; PIK3C2A; LCK; RAF1; MAP2K2; |
| | JAK1; AKT1; PIK3R1; MAP2K1; JUN; AKT3 |
| Synaptic Long Term | PRKCE; IGF1; PRKCZ; PRDX6; LYN; MAPK1; GNAS; |
| Depression | PRKCI; GNAQ; PPP2R1 A; IGF1R; PRKD1; MAPK3; |
| | KRAS; GRN; PRKCD; NOS3; NOS2A; PPP2CA; |
| | YWHAZ; RAF1; MAP2K2; PPP2R5C; MAP2K1; PRKCA |
| Estrogen Receptor | TAF4B; EP300; CARM1; PCAF; MAPK1; NCOR2; |
| Signaling | SMARCA4; MAPK3; NRIP1; KRAS; SRC; NR3C1; |
| | HDAC3; PPARGC1A; RBM9; NCOA3; RAF1; CREBBP; |
| | MAP2K2; NCOA2; MAP2K1; PRKDC; ESR1; ESR2 |
| Protein Ubiquitination | TRAF6; SMURF1; BIRC4; BRCA1; UCHL1; NEDD4; |
| Pathway | CBL; UBE2I; BTRC; HSPA5; USP7; USP10; FBXW7; |
| | USP9X; STUB1; USP22; B2M; BIRC2; PARK2; USP8; |
| | USP1; VHL; HSP90AA1; BIRC3 |
| IL-10 Signaling | TRAF6; CCR1; ELK1; IKBKB; SP1; FOS; NFKB2; |
| | MAP3K14; MAPK8; MAPK13; RELA; MAPK14; TNF; |
| | IKBKG; RELB; MAP3K7; JAK1; CHUK; STAT3; NFKB1; |
| | JUN; IL1R1; IL6 |
| VDR/RXR Activation | PRKCE; EP300; PRKCZ; RXRA; GADD45A; HES1; |
| | NCOR2; SP1; PRKCI; CDKN1B; PRKD1; PRKCD; |
| | RUNX2; KLF4; YY1; NCOA3; CDKN1A; NCOA2; SPP1; |
| | LRP5; CEBPB; FOXO1; PRKCA |
| TGF-beta Signaling | EP300; SMAD2; SMURF1; MAPK1; SMAD3; SMAD1; |
| | FOS; MAPK8; MAPK3; KRAS; MAPK9; RUNX2; |
| | SERPINE1; RAF1; MAP3K7; CREBBP; MAP2K2; |

(continued)

| CELLULAR FUNCTION | GENES |
|---|---|
| | MAP2K1; TGFBR1; SMAD4; JUN; SMAD5 |
| Toll-like Receptor Signaling | IRAK1; EIF2AK2; MYD88; TRAF6; PPARA; ELK1; |
| | IKBKB; FOS; NFKB2; MAP3K14; MAPK8; MAPK13; |
| | RELA; TLR4; MAPK14; IKBKG; RELB; MAP3K7; CHUK; |
| | NFKB1; TLR2; JUN |
| p38 MAPK Signaling | HSPB1; IRAK1; TRAF6; MAPKAPK2; ELK1; FADD; FAS; |
| | CREB1; DDIT3; RPS6KA4; DAXX; MAPK13; TRAF2; |
| | MAPK14; TNF; MAP3K7; TGFBR1; MYC; ATF4; IL1R1; |
| | SRF; STAT1 |
| Neurotrophin/TRK Signaling | NTRK2; MAPK1; PTPN11; PIK3CA; CREB1; FOS; |
| | PIK3CB; PIK3C3; MAPK8; MAPK3; KRAS; PIK3C2A; |
| | RAF1; MAP2K2; AKT1; PIK3R1; PDPK1; MAP2K1; |
| | CDC42; JUN; ATF4 |
| FXR/RXR Activation | INS; PPARA; FASN; RXRA; AKT2; SDC1; MAPK8; |
| | APOB; MAPK10; PPARG; MTTP; MAPK9; PPARGC1A; |
| | TNF; CREBBP; AKT1; SREBF1; FGFR4; AKT3; FOXO1 |
| Synaptic Long Term | PRKCE; RAP1A; EP300; PRKCZ; MAPK1; CREB1; |
| Potentiation | PRKCI; GNAQ; CAMK2A; PRKD1; MAPK3; KRAS; |
| | PRKCD; PPP1CC; RAF1; CREBBP; MAP2K2; MAP2K1; |
| | ATF4; PRKCA |
| Calcium Signaling | RAP1A; EP300; HDAC4; MAPK1; HDAC5; CREB1; |
| | CAMK2A; MYH9; MAPK3; HDAC2; HDAC7A; HDAC11; |
| | HDAC9; HDAC3; CREBBP; CALR; CAMKK2; ATF4; |
| | HDAC6 |
| EGF Signaling | ELK1; MAPK1; EGFR; PIK3CA; FOS; PIK3CB; PIK3C3; |
| | MAPK8; MAPK3; PIK3C2A; RAF1; JAK1; PIK3R1; |
| | STAT3; MAP2K1; JUN; PRKCA; SRF; STAT1 |
| Hypoxia Signaling in the | EDN1; PTEN; EP300; NQO1; UBE2I; CREB1; ARNT; |
| Cardiovascular System | HIF1A; SLC2A4; NOS3; TP53; LDHA; AKT1; ATM; |
| | VEGFA; JUN; ATF4; VHL; HSP90AA1 |
| LPS/IL-1 Mediated Inhibition | IRAKI; MYD88; TRAF6; PPARA; RXRA; ABCA1; |
| of RXR Function | MAPK8; ALDH1A1; GSTP1; MAPK9; ABCB1; TRAF2; |
| | TLR4; TNF; MAP3K7; NR1H2; SREBF1; JUN; IL1R1 |
| LXR/RXR Activation | FASN; RXRA; NCOR2; ABCA1; NFKB2; IRF3; RELA; |
| | NOS2A; TLR4; TNF; RELB; LDLR; NR1H2; NFKB1; |
| | SREBF1; IL1R1; CCL2; IL6; MMP9 |
| Amyloid Processing | PRKCE; CSNK1E; MAPK1; CAPNS1; AKT2; CAPN2; |
| | CAPN1; MAPK3; MAPK13; MAPT; MAPK14; AKT1; |

(continued)

| CELLULAR FUNCTION | GENES |
|---|---|
|  | PSEN1; CSNK1A1; GSK3B; AKT3; APP |
| IL-4 Signaling | AKT2; PIK3CA; PIK3CB; PIK3C3; IRS1; KRAS; SOCS1; |
|  | PTPN6; NR3C1; PIK3C2A; JAK1; AKT1; JAK2; PIK3R1; |
|  | FRAP1; AKT3; RPS6KB1 |
| Cell Cycle: G2/M DNA | EP300; PCAF; BRCA1; GADD45A; PLK1; BTRC; |
| Damage Checkpoint | CHEK1; ATR; CHEK2; YWHAZ; TP53; CDKN1A; |
| Regulation | PRKDC; ATM; SFN; CDKN2A |
| Nitric Oxide Signaling in the | KDR; FLT1; PGF; AKT2; PIK3CA; PIK3CB; PIK3C3; |
| Cardiovascular System | CAV1; PRKCD; NOS3; PIK3C2A; AKT1; PIK3R1; |
|  | VEGFA; AKT3; HSP90AA1 |
| Purine Metabolism | NME2; SMARCA4; MYH9; RRM2; ADAR; EIF2AK4; |
|  | PKM2; ENTPD1; RAD51; RRM2B; TJP2; RAD51C; |
|  | NT5E; POLD1; NME1 |
| cAMP-mediated Signaling | RAP1A; MAPK1; GNAS; CREB1; CAMK2A; MAPK3; |
|  | SRC; RAF1; MAP2K2; STAT3; MAP2K1; BRAF; ATF4 |
| Mitochondrial Dysfunction | SOD2; MAPK8; CASP8; MAPK10; MAPK9; CASP9; |
|  | PARK7; PSEN1; PARK2; APP; CASP3 |
| Notch Signaling | HES1; JAG1; NUMB; NOTCH4; ADAM17; NOTCH2; |
|  | PSEN1; NOTCH3; NOTCH1; DLL4 |
| Endoplasmic Reticulum | HSPA5; MAPK8; XBP1; TRAF2; ATF6; CASP9; ATF4; |
| Stress Pathway | EIF2AK3; CASP3 |
| Pyrimidine Metabolism | NME2; AICDA; RRM2; EIF2AK4; ENTPD 1; RRM2B; |
|  | NT5E; POLD1; NME1 |
| Parkinson's Signaling | UCHL1; MAPK8; MAPK13; MAPK14; CASP9; PARK7; |
|  | PARK2; CASP3 |
| Cardiac & Beta Adrenergic | GNAS; GNAQ; PPP2R1A; GNB2L1; PPP2CA; PPP1CC; |
| Signaling | PPP2R5C |
| Glycolysis/Gluconeogene sis | HK2; GCK; GPI; ALDH1A1; PKM2; LDHA; HK1 |
| Interferon Signaling | IRF1; SOCS1; JAK1; JAK2; IFITM1; STAT1; IFIT3 |
| Sonic Hedgehog Signaling | ARRB2; SMO; GLI2; DYRK1A; GLI1; GSK3B; DYRK1B |
| Glycerophospholipid | PLD1; GRN; GPAM; YWHAZ; SPHK1; SPHK2 |
| Metabolism |  |
| Phospholipid Degradation | PRDX6; PLD1; GRN; YWHAZ; SPHK1; SPHK2 |
| Tryptophan Metabolism | SIAH2; PRMT5; NEDD4; ALDH1A1; CYP1B1; SIAH1 |
| Lysine Degradation | SUV39H1; EHMT2; NSD1; SETD7; PPP2R5C |
| Nucleotide Excision Repair | ERCC5; ERCC4; XPA; XPC; ERCC1 |
| Pathway |  |
| Starch and Sucrose | UCHL1; HK2; GCK; GPI; HK1 |

(continued)

| CELLULAR FUNCTION | GENES |
|---|---|
| Metabolism | |
| Aminosugars Metabolism | NQO1; HK2; GCK; HK1 |
| Arachidonic Acid Metabolism | PRDX6; GRN; YWHAZ; CYP1B1 |
| Circadian Rhythm Signaling | CSNK1E; CREB1; ATF4; NR1D1 |
| Coagulation System | BDKRB1; F2R; SERPINE1; F3 |
| Dopamine Receptor Signaling | PPP2R1A; PPP2CA; PPP1CC; PPP2R5C |
| Glutathione Metabolism | IDH2; GSTP1; ANPEP; IDH1 |
| Glycerolipid Metabolism | ALDH1A1; GPAM; SPHK1; SPHK2 |
| Linoleic Acid Metabolism | PRDX6; GRN; YWHAZ; CYP1B1 |
| Methionine Metabolism | DNMT1; DNMT3B; AHCY; DNMT3A |
| Pyruvate Metabolism | GLO1; ALDH1A1; PKM2; LDHA |
| Arginine and Proline Metabolism | ALDH1A1; NOS3; NOS2A |
| Eicosanoid Signaling | PRDX6; GRN; YWHAZ |
| Fructose and Mannose Metabolism | HK2; GCK; HK1 |
| Galactose Metabolism | HK2; GCK; HK 1 |
| Stilbene, Coumarine and Lignin Biosynthesis | PRDX6; PRDX1; TYR |
| Antigen Presentation Pathway | CALR; B2M |
| Biosynthesis of Steroids | NQO1; DHCR7 |
| Butanoate Metabolism | ALDH1A1; NLGN1 |
| Citrate Cycle | IDH2; IDH1 |
| Fatty Acid Metabolism | ALDH1A1; CYP1B1 |
| Glycerophospholipid Metabolism | PRDX6; CHKA |
| Histidine Metabolism | PRMT5; ALDH1A1 |
| Inositol Metabolism | ERO1L; APEX1 |
| Metabolism of Xenobiotics by Cytochrome p450 | GSTP1; CYP1B1 |
| Methane Metabolism | PRDX6; PRDX1 |
| Phenylalanine Metabolism | PRDX6; PRDX1 |
| Propanoate Metabolism | ALDH1A1; LDHA |
| Selenoamino Acid Metabolism | PRMT5; AHCY |
| Sphingolipid Metabolism | SPHK1; SPHK2 |

(continued)

| CELLULAR FUNCTION | GENES |
|---|---|
| Aminophosphonate Metabolism | PRMT5 |
| Androgen and Estrogen Metabolism | PRMT5 |
| Ascorbate and Aldarate Metabolism | ALDH1A1 |
| Bile Acid Biosynthesis | ALDH1A1 |
| Cysteine Metabolism | LDHA |
| Fatty Acid Biosynthesis | FASN |
| Glutamate Receptor Signaling | GNB2L1 |
| NRF2-mediated Oxidative Stress Response | PRDX1 |
| Pentose Phosphate Pathway | GPI |
| Pentose and Glucuronate Interconversions | UCHL1 |
| Retinol Metabolism | ALDH1A1 |
| Riboflavin Metabolism | TYR |
| Tyrosine Metabolism | PRMT5, TYR |
| Ubiquinone Biosynthesis | PRMT5 |
| Valine, Leucine and Isoleucine Degradation | ALDH1A1 |
| Glycine, Serine and Threonine Metabolism | CHKA |
| Lysine Degradation | ALDH1A1 |
| Pain/Taste | TRPM5; TRPA1 |
| Pain | TRPM7; TRPC5; TRPC6; TRPC1; Cnr1; cnr2; Grk2; |
| | Trpa1; Pomc; Cgrp; Crf; Pka; Era; Nr2b; TRPM5; Prkaca; |
| | Prkacb; Prkar1a; Prkar2a |
| Mitochondrial Function | AIF; CytC; SMAC (Diablo); Aifm-1; Aifm-2 |
| Developmental Neurology | BMP-4; Chordin (Chrd); Noggin (Nog); WNT (Wnt2; |
| | Wnt2b; Wnt3a; Wnt4; Wnt5a; Wnt6; Wnt7b; Wnt8b; |
| | Wnt9a; Wnt9b; Wnt10a; Wnt10b; Wnt16); beta-catenin; |
| | Dkk-1; Frizzled related proteins; Otx-2; Gbx2; FGF-8; |
| | Reelin; Dab1; unc-86 (Pou4f1 or Brn3a); Numb; Rein |

Embodiments of the disclosure also relate to methods and compositions related to knocking out genes, amplifying genes and repairing particular mutations associated with DNA repeat instability and neurological disorders (Robert D. Wells, Tetsuo Ashizawa, Genetic Instabilities and Neurological Diseases, Second Edition, Academic Press, Oct 13, 2011 - Medical). Specific aspects of tandem repeat sequences have been found to be responsible for more than twenty human diseases (New insights into repeat instability: role of RNA•DNA hybrids. McIvor EI, Polak U, Napierala M. RNA Biol. 2010 Sep-Oct;7(5):551-8). The CRISPR-Cas system may be harnessed to correct these defects of genomic instability.

A further aspect of the disclosure relates to utilizing the CRISPR-Cas system for correcting defects in the EMP2A and EMP2B genes that have been identified to be associated with Lafora disease. Lafora disease is an autosomal recessive condition which is characterized by progressive myoclonus epilepsy which may start as epileptic seizures in adolescence. A few cases of the disease may be caused by mutations in genes yet to be identified. The disease causes seizures, muscle spasms, difficulty walking, dementia, and eventually death. There is currently no therapy that has proven effective against disease progression. Other genetic abnormalities associated with epilepsy may also be targeted by the CRISPR-Cas system and the underlying genetics is further described in Genetics of Epilepsy and Genetic Epilepsies, edited by Giuliano Avanzini, Jeffrey L. Noebels, Mariani Foundation Paediatric Neurology:20; 2009).

In yet another aspect of the disclosure, the CRISPR-Cas system may be used to correct ocular defects that arise from several genetic mutations further described in Genetic Diseases of the Eye, Second Edition, edited by Elias I. Traboulsi, Oxford University Press, 2012.

Several further aspects of the disclosure relate to correcting defects associated with a wide range of genetic diseases which are further described on the website of the National Institutes of Health under the topic subsection Genetic Disorders (website at health.nih.gov/topic/GeneticDisorders). The genetic brain diseases may include but are not limited to Adrenoleukodystrophy, Agenesis of the Corpus Callosum, Aicardi Syndrome, Alpers' Disease, Alzheimer's Disease, Barth Syndrome, Batten Disease, CADASIL, Cerebellar Degeneration, Fabry's Disease, Gerstmann-Straussler-Scheinker Disease, Huntington's Disease and other Triplet Repeat Disorders, Leigh's Disease, Lesch-Nyhan Syndrome, Menkes Disease, Mitochondrial Myopathies and NINDS Colpocephaly. These diseases are further described on the website of the National Institutes of Health under the subsection Genetic Brain Disorders.

In some embodiments, the condition may be neoplasia. In some embodiments, where the condition is neoplasia, the genes to be targeted are any of those listed in Table A (in this case PTEN and so forth). In some embodiments, the condition may be Age-related Macular Degeneration. In some embodiments, the condition may be a Schizophrenic Disorder. In some embodiments, the condition may be a Trinucleotide Repeat Disorder. In some embodiments, the condition may be Fragile X Syndrome. In some embodiments, the condition may be a Secretase Related Disorder. In some embodiments, the condition may be a Prion - related disorder. In some embodiments, the condition may be ALS. In some embodiments, the condition may be a drug addiction. In some embodiments, the condition may be Autism. In some embodiments, the condition may be Alzheimer's Disease. In some embodiments, the condition may be inflammation. In some embodiments, the condition may be Parkinson's Disease.

Examples of proteins associated with Parkinson's disease include but are not limited to $\alpha$-synuclein, DJ-1, LRRK2, PINK1, Parkin, UCHL1, Synphilin-1, and NURR1.

Examples of addiction-related proteins may include ABAT for example.

Examples of inflammation-related proteins may include the monocyte chemoattractant protein-1 (MCP1) encoded by the Ccr2 gene, the C-C chemokine receptor type 5 (CCR5) encoded by the Ccr5 gene, the IgG receptor IIB (FCGR2b, also termed CD32) encoded by the Fcgr2b gene, or the Fc epsilon R1g (FCER1g) protein encoded by the Fcerlg gene, for example.

Examples of cardiovascular diseases associated proteins may include IL1B (interleukin 1, beta), XDH (xanthine dehydrogenase), TP53 (tumor protein p53), PTGIS (prostaglandin I2 (prostacyclin) synthase), MB (myoglobin), IL4 (interleukin 4), ANGPT1 (angiopoietin 1), ABCG8 (ATP-binding cassette, sub-family G (WHITE), member 8), or CTSK (cathepsin K), for example.

Examples of Alzheimer's disease associated proteins may include the very low density lipoprotein receptor protein (VLDLR) encoded by the VLDLR gene, the ubiquitin-like modifier activating enzyme 1 (UBA1) encoded by the UBA1 gene, or the NEDD8-activating enzyme E1 catalytic subunit protein (UBE1C) encoded by the UBA3 gene, for example.

Examples of proteins associated Autism Spectrum Disorder may include the benzodiazapine receptor (peripheral) associated protein 1 (BZRAP1) encoded by the BZRAP1 gene, the AF4/FMR2 family member 2 protein (AFF2) encoded by the AFF2 gene (also termed MFR2), the fragile X mental retardation autosomal homolog 1 protein (FXR1) encoded by the FXR1 gene, or the fragile X mental retardation autosomal homolog 2 protein (FXR2) encoded by the FXR2 gene, for example.

Examples of proteins associated Macular Degeneration may include the ATP-binding cassette, sub-family A (ABC1) member 4 protein (ABCA4) encoded by the ABCR gene, the apolipoprotein E protein (APOE) encoded by the APOE gene, or the chemokine (C-C motif) Ligand 2 protein (CCL2) encoded by the CCL2 gene, for example.

Examples of proteins associated Schizophrenia may include NRG1, ErbB4, CPLX1, TPH1, TPH2, NRXN1, GSK3A, BDNF, DISCI, GSK3B, and combinations thereof.

Examples of proteins involved in tumor suppression may include ATM (ataxia telangiectasia mutated), ATR (ataxia telangiectasia and Rad3 related), EGFR (epidermal growth factor receptor), ERBB2 (v-erb-b2 erythroblastic leukemia viral oncogene homolog 2), ERBB3 (v-erb-b2 erythroblastic leukemia viral oncogene homolog 3), ERBB4 (v-erb-b2 erythroblastic leukemia viral oncogene homolog 4), Notch 1, Notch2, Notch 3, or Notch 4, for example.

Examples of proteins associated with a secretase disorder may include PSENEN (presenilin enhancer 2 homolog (C. elegans)), CTSB (cathepsin B), PSEN1 (presenilin 1), APP (amyloid beta (A4) precursor protein), APH1B (anterior pharynx defective 1 homolog B (C. elegans)), PSEN2 (presenilin 2 (Alzheimer disease 4)), or BACE1 (beta-site APP-cleaving enzyme 1), for example.

Examples of proteins associated with Amyotrophic Lateral Sclerosis may include SOD 1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins associated with prion diseases may include SOD1 (superoxide dismutase 1), ALS2 (amyotrophic lateral sclerosis 2), FUS (fused in sarcoma), TARDBP (TAR DNA binding protein), VAGFA (vascular endothelial growth factor A), VAGFB (vascular endothelial growth factor B), and VAGFC (vascular endothelial growth factor C), and any combination thereof.

Examples of proteins related to neurodegenerative conditions in prion disorders may include A2M (Alpha-2-Macroglobulin), AATF (Apoptosis antagonizing transcription factor), ACPP (Acid phosphatase prostate), ACTA2 (Actin alpha 2 smooth muscle aorta), ADAM22 (ADAM metallopeptidase domain), ADORA3 (Adenosine A3 receptor), or ADRA1D (Alpha-ID adrenergic receptor for Alpha-1D adrenoreceptor), for example.

Examples of proteins associated with Immunodeficiency may include A2M [alpha-2-macroglobulin]; AANAT [arylalkylamine N-acetyltransferase]; ABCA1 [ATP-binding cassette, sub-family A (ABC1), member 1]; ABCA2 [ATP-binding cassette, sub-family A (ABC1), member 2]; or ABCA3 [ATP-binding cassette, sub-family A (ABC1), member 3]; for example.

Examples of proteins associated with Trinucleotide Repeat Disorders include AR (androgen receptor), FMR1 (fragile X mental retardation 1), HTT (huntingtin), or DMPK (dystrophia myotonica-protein kinase), FXN (frataxin), ATXN2 (ataxin 2), for example.

Examples of proteins associated with Neurotransmission Disorders include SST (somatostatin), NOS1 (nitric oxide synthase 1 (neuronal)), ADRA2A (adrenergic, alpha-2A-, receptor), ADRA2C (adrenergic, alpha-2C-, receptor), TACR1 (tachykinin receptor 1), or HTR2c (5-hydroxytryptamine (serotonin) receptor 2C), for example.

Examples of neurodevelopmental-associated sequences include A2BP1 [ataxin 2-binding protein 1], AADAT [aminoadipate aminotransferase], AANAT [arylalkylamine N-acetyltransferase], ABAT [4-aminobutyrate aminotransferase], ABCA1 [ATP-binding cassette, sub-family A (ABC1), member 1], or ABCA13 [ATP-binding cassette, sub-family A (ABC1), member 13], for example.

Further examples of preferred conditions treatable with the present system include may be selected from: Aicardi-Goutières Syndrome; Alexander Disease; Allan-Herndon-Dudley Syndrome; POLG-Related Disorders; Alpha-Mannosidosis (Type II and III); Alström Syndrome; Angelman; Syndrome; Ataxia-Telangiectasia; Neuronal Ceroid-Lipofuscinoses; Beta-Thalassemia; Bilateral Optic Atrophy and (Infantile) Optic Atrophy Type 1; Retinoblastoma (bilateral); Canavan Disease; Cerebrooculofacioskeletal Syndrome 1 [COFS1]; Cerebrotendinous Xanthomatosis; Cornelia de Lange Syndrome; MAPT-Related Disorders; Genetic Prion Diseases; Dravet Syndrome; Early-Onset Familial Alzheimer Disease; Friedreich Ataxia [FRDA]; Fryns Syndrome; Fucosidosis; Fukuyama Congenital Muscular Dystrophy; Galactosialidosis; Gaucher Disease; Organic Acidemias; Hemophagocytic Lymphohistiocytosis; Hutchinson-Gilford Progeria Syndrome; Mucolipidosis II; Infantile Free Sialic Acid Storage Disease; PLA2G6-Associated Neurodegeneration; Jervell and Lange-Nielsen Syndrome; Junctional Epidermolysis Bullosa; Huntington Disease; Krabbe Disease (Infantile); Mitochondrial DNA-Associated Leigh Syndrome and NARP; Lesch-Nyhan Syndrome; LIS1-Associated Lissencephaly; Lowe Syndrome; Maple Syrup Urine Disease; MECP2 Duplication Syndrome; ATP7A-Related Copper Transport Disorders; LAMA2-Related Muscular Dystrophy; Arylsulfatase A Deficiency; Mucopolysaccharidosis Types I, II or III; Peroxisome Biogenesis Disorders, Zellweger Syndrome Spectrum; Neurodegeneration with Brain Iron Accumulation Disorders; Acid Sphingomyelinase Deficiency; Niemann-Pick Disease Type C; Glycine Encephalopathy; ARX-Related Disorders; Urea Cycle Disorders; COL1A1/2-Related Osteogenesis Imperfecta; Mitochondrial DNA Deletion Syndromes; PLP1-Related Disorders; Perry Syndrome; Phelan-McDermid Syndrome; Glycogen Storage Disease Type II (Pompe Disease) (Infantile); MAPT-Related Disorders; MECP2-Related Disorders; Rhizomelic Chondrodysplasia Punctata Type 1; Roberts Syndrome; Sandhoff Disease; Schindler Disease - Type 1; Adenosine Deaminase Deficiency; Smith-Lemli-Opitz Syndrome; Spinal Muscular Atrophy; Infantile-Onset Spinocerebellar Ataxia; Hexosaminidase A Deficiency; Thanatophoric Dysplasia Type 1; Collagen Type VI-Related Disorders; Usher Syndrome Type I; Congenital Muscular Dystrophy; Wolf-Hirschhorn Syndrome; Lysosomal Acid Lipase Deficiency; and Xeroderma Pigmentosum.

As will be apparent, it is envisaged that the present system can be used to target any polynucleotide sequence of interest. Some examples of conditions or diseases that might be usefully treated using the present system are included in the Tables above and examples of genes currently associated with those conditions are also provided there. However, the genes exemplified are not exhaustive.

**EXAMPLES**

The following examples are given for the purpose of illustrating various embodiments of the disclosure and are not meant to limit the present invention in any fashion. The present examples, along with the methods described herein are presently representative of preferred embodiments, are exemplary, and are not intended as limitations on the scope of the invention.

*Example 1: CRISPR Complex Activity in the Nucleus of a Eukaryotic Cell*

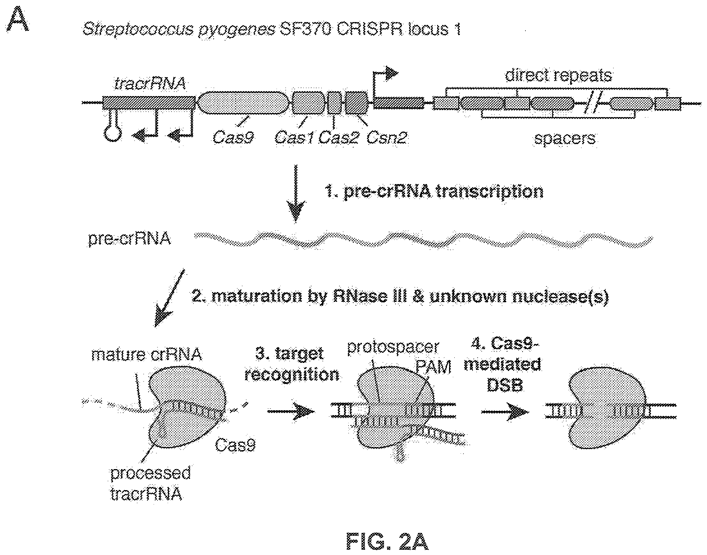

An example type II CRISPR system is the type II CRISPR locus from *Streptococcus pyogenes* SF370, which contains a cluster of four genes Cas9, Cas1, Cas2, and Csn1, as well as two non-coding RNA elements, tracrRNA and a characteristic array of repetitive sequences (direct repeats) interspaced by short stretches of non-repetitive sequences (spacers, about 30bp each). In this system, targeted DNA double-strand break (DSB) is generated in four sequential steps (Figure 2A). First, two non-coding RNAs, the pre-crRNA array and tracrRNA, are transcribed from the CRISPR locus. Second, tracrRNA hybridizes to the direct repeats of pre-crRNA, which is then processed into mature crRNAs containing individual spacer sequences. Third, the mature crRNA:tracrRNA complex directs Cas9 to the DNA target consisting of the protospacer and the corresponding PAM via heteroduplex formation between the spacer region of the crRNA and the protospacer DNA. Finally, Cas9 mediates cleavage of target DNA upstream of PAM to create a DSB within the protospacer (Figure 2A). This example describes an example process for adapting this RNA-programmable nuclease system to direct CRISPR complex activity in the nuclei of eukaryotic cells.

*Cell culture and transfection*

Human embryonic kidney (HEK) cell line HEK 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100$\mu$g/mL streptomycin at 37°C with 5% $CO_2$ incubation. Mouse neuro2A (N2A) cell line (ATCC) was maintained with DMEM supplemented with 5% fetal bovine serum (HyClone), 2mM GlutaMAX (Life Technologies), 100U/mL penicillin, and 100$\mu$g/mL streptomycin at 37°C with 5% $CO_2$.

HEK 293FT or N2A cells were seeded into 24-well plates (Corning) one day prior to transfection at a density of 200,000 cells per well. Cells were transfected using Lipofectamine 2000 (Life Technologies) following the manufacturer's recommended protocol. For each well of a 24-well plate a total of 800ng of plasmids were used.

*Surveyor assay and sequencing analysis for genome modification*

HEK 293FT or N2A cells were transfected with plasmid DNA as described above. After transfection, the cells were incubated at 37°C for 72 hours before genomic DNA extraction. Genomic DNA was extracted using the QuickExtract DNA extraction kit (Epicentre) following the manufacturer's protocol. Briefly, cells were resuspended in QuickExtract solution and incubated at 65°C for 15 minutes and 98°C for 10 minutes. Extracted genomic DNA was immediately processed or stored at -20°C.

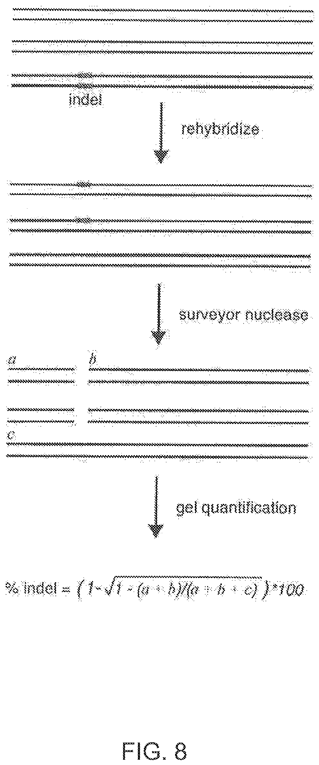

The genomic region surrounding a CRISPR target site for each gene was PCR amplified, and products were purified using QiaQuick Spin Column (Qiagen) following manufacturer's protocol. A total of 400ng of the purified PCR products were mixed with 2$\mu$l 10X Taq polymerase PCR buffer (Enzymatics) and ultrapure water to a final volume of 20$\mu$l, and subjected to a re-annealing process to enable heteroduplex formation: 95°C for 10min, 95°C to 85°C ramping at - 2°C/s, 85°C to 25°C at - 0.25°C/s, and 25°C hold for 1 minute. After re-annealing, products were treated with Surveyor nuclease and Surveyor enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities, as a measure of the fraction of cleaved DNA. Figure 8 provides a schematic illustration of this Surveyor assay.

*Restriction fragment length polymorphism assay for detection of homologous recombination*

HEK 293FT and N2A cells were transfected with plasmid DNA, and incubated at 37°C for 72 hours before genomic DNA extraction as described above. The target genomic region was PCR amplified using primers outside the homology arms of the homologous recombination (HR) template. PCR products were separated on a 1% agarose gel and extracted with MinElute GelExtraction Kit (Qiagen). Purified products were digested with HindIII (Fermentas) and analyzed on a 6% Novex TBE poly-acrylamide gel (Life Technologies).

RNA secondary structure prediction and analysis

RNA secondary structure prediction was performed using the online webserver RNAfold developed at Institute for Theoretical Chemistry at the University of Vienna, using the centroid structure prediction algorithm (see e.g. A.R. Gruber et al., 2008, Cell 106(1): 23-24; and PA Carr and GM Church, 2009, Nature Biotechnology 27(12): 1151-62).

Bacterial plasmid transformation interference assay

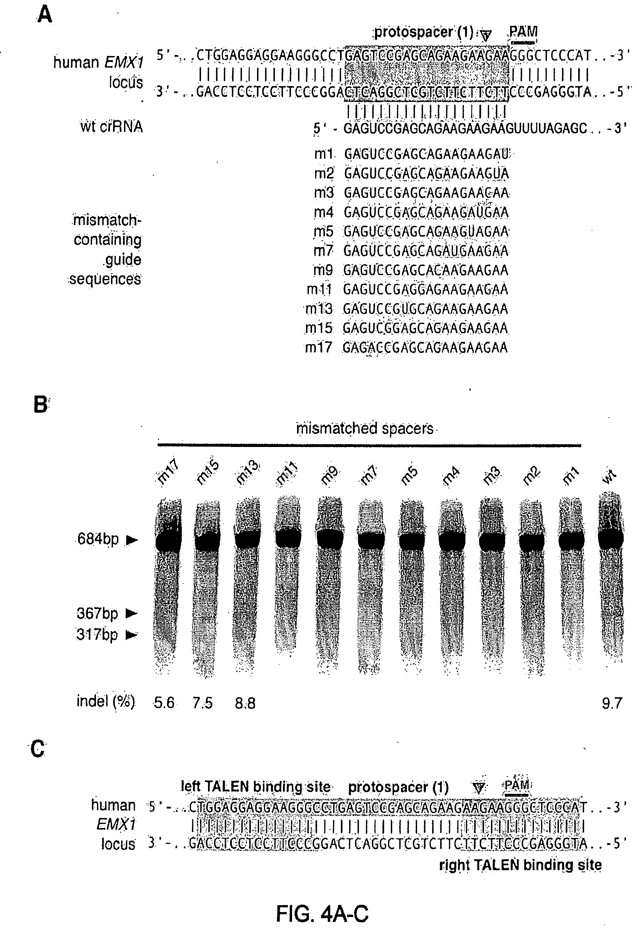

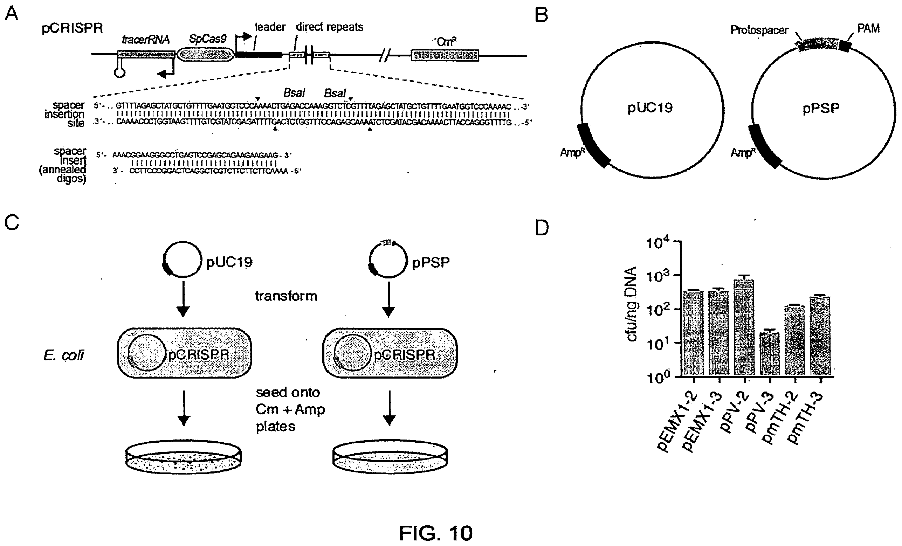

Elements of the S. pyogenes CRISPR locus 1 sufficient for CRISPR activity were reconstituted in E. coli using pCRISPR plasmid (schematically illustrated in Figure 10A). pCRISPR contained tracrRNA, SpCas9, and a leader sequence driving the crRNA array. Spacers (also referred to as "guide sequences") were inserted into the crRNA array between BsaI sites using annealed oligonucleotides, as illustrated. Challenge plasmids used in the interference assay were constructed by inserting the protospacer (also referred to as a "target sequence") sequence along with an adjacent CRISPR motif sequence (PAM) into pUC19 (see Figure 10B). The challenge plasmid contained ampicillin resistance. Figure 10C provides a schematic representation of the interference assay. Chemically competent E. coli strains already carrying pCRISPR and the appropriate spacer were transformed with the challenge plasmid containing the corresponding protospacer-PAM sequence. pUC19 was used to assess the transformation efficiency of each pCRISPR-carrying competent strain. CRISPR activity resulted in cleavage of the pPSP plasmid carrying the protospacer, precluding ampicillin resistance otherwise conferred by pUC19 lacking the protospacer. Figure 10D illustrates competence of each pCRISPR-carrying E. coli strain used in assays illustrated in Figure 4C.

RNA purification

HEK 293FT cells were maintained and transfected as stated above. Cells were harvested by trypsinization followed by washing in phosphate buffered saline (PBS). Total cell RNA was extracted with TRI reagent (Sigma) following manufacturer's protocol. Extracted total RNA was quantified using Naonodrop (Thermo Scientific) and normalized to same concentration.

*Northern blot analysis of crRNA and tracrRNA expression in mammalian cells*

RNAs were mixed with equal volumes of 2X loading buffer (Ambion), heated to 95°C for 5 min, chilled on ice for 1 min, and then loaded onto 8% denaturing polyacrylamide gels (SequaGel, National Diagnostics) after pre-running the gel for at least 30 minutes. The samples were electrophoresed for 1.5 hours at 40W limit. Afterwards, the RNA was transferred to Hybond N+ membrane (GE Healthcare) at 300 mA in a semi-dry transfer apparatus (Bio-rad) at room temperature for 1.5 hours. The RNA was crosslinked to the membrane using autocrosslink button on Stratagene UV Crosslinker the Stratalinker (Stratagene). The membrane was pre-hybridized in ULTRAhyb-Oligo Hybridization Buffer (Ambion) for 30 min with rotation at 42°C, and probes were then added and hybridized overnight. Probes were ordered from IDT and labeled with [gamma-$^{32}$P] ATP (Perkin Elmer) with T4 polynucleotide kinase (New England Biolabs). The membrane was washed once with pre-warmed (42°C) 2xSSC, 0.5% SDS for 1 min followed by two 30 minute washes at 42°C. The membrane was exposed to a phosphor screen for one hour or overnight at room temperature and then scanned with a phosphorimager (Typhoon).

*Bacterial CRISPR system construction and evaluation*

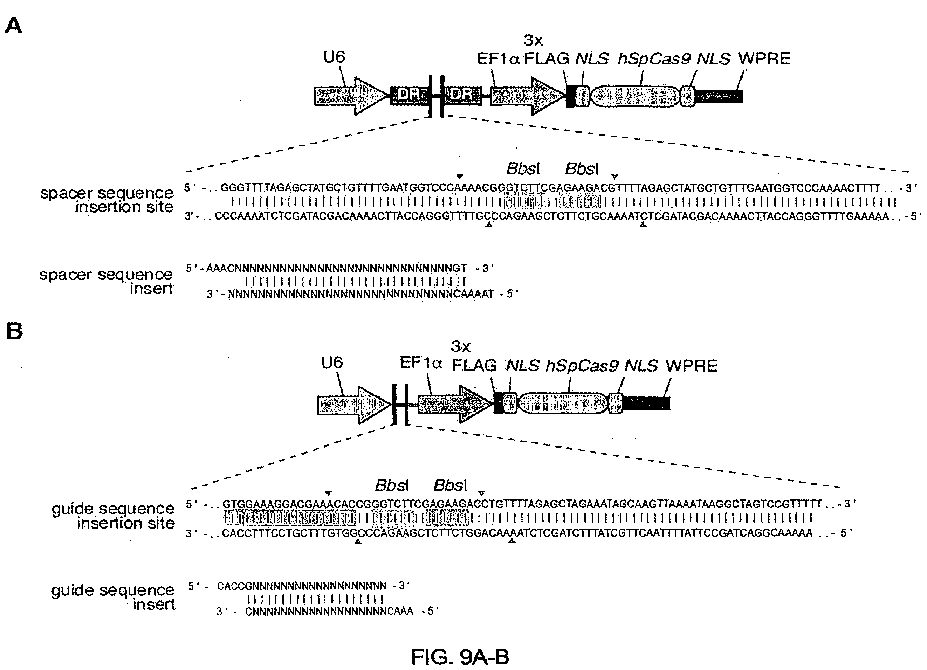

CRISPR locus elements, including tracrRNA, *Cas9,* and leader were PCR amplified from *Streptococcus pyogenes* SF370 genomic DNA with flanking homology arms for Gibson Assembly. Two *Bsa*I type IIS sites were introduced in between two direct repeats to facilitate easy insertion of spacers (Figure 9). PCR products were cloned into EcoRV-digested pACYC184 downstream of the tet promoter using Gibson Assembly Master Mix (NEB). Other endogenous CRISPR system elements were omitted, with the exception of the last 50bp of Csn2. Oligos (Integrated DNA Technology) encoding spacers with complimentary overhangs were cloned into the *Bsa*I-digested vector pDC000 (NEB) and then ligated with T7 ligase (Enzymatics) to generate pCRISPR plasmids. Challenge plasmids containing spacers with PAM sequences (also referred to herein as "CRISPR motif sequences") were created by ligating hybridized oligos carrying compatible overhangs (Integrated DNA Technology) into *Bam*HI-digested pUC19. Cloning for all constructs was performed in E. *coli* strain JM109 (Zymo Research).

pCRISPR-carrying cells were made competent using the Z-Competent *E. coli* Transformation Kit and Buffer Set (Zymo Research, T3001) according to manufacturer's instructions. In the transformation assay, 50uL aliquots of competent cells carrying pCRISPR were thawed on ice and transformed with 1ng of spacer plasmid or pUC19 on ice for 30 minutes, followed by 45 second heat shock at 42°C and 2 minutes on ice. Subsequently, 250ul SOC (Invitrogen) was added followed by shaking incubation at 37°C for 1hr, and 100 uL of the post-SOC outgrowth was plated onto double selection plates (12.5 ug/ml chloramphenicol, 100 ug/ml ampicillin). To obtain cfu/ng of DNA, total colony numbers were multiplied by 3.

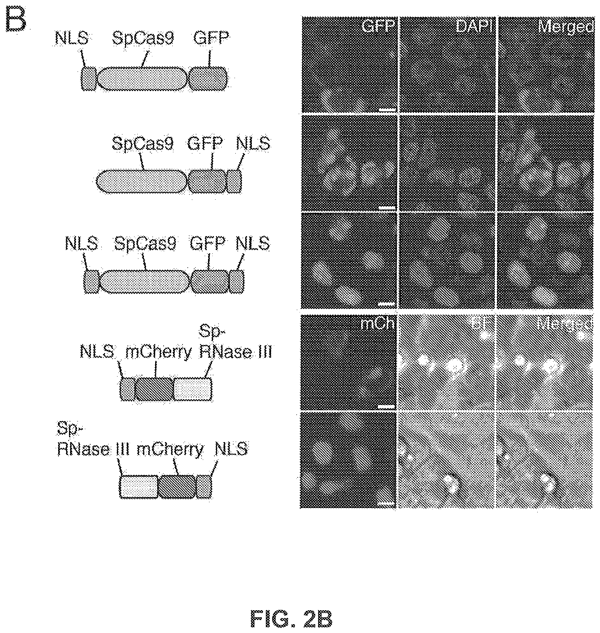

To improve expression of CRISPR components in mammalian cells, two genes from the SF370 locus 1 of *Streptococcus pyogenes* (*S. pyogenes*) were codon-optimized, *Cas9* (*SpCas9*) and *RNase III* (*SpRNase III*). To facilitate nuclear localization, a nuclear localization signal (NLS) was included at the amino (N)- or carboxyl (C)-termini of both SpCas9 and SpRNase III (Figure 2B). To facilitate visualization of protein expression, a fluorescent protein marker was also included at the N- or C-termini of both proteins (Figure 2B). A version of SpCas9 with an NLS attached to both N- and C-termini (2xNLS-SpCas9) was also generated. Constructs containing NLS-fused SpCas9 and SpRNase III were transfected into 293FT human embryonic kidney (HEK) cells, and the relative positioning of the NLS to SpCas9 and SpRNase III was found to affect their nuclear localization efficiency. Whereas the C-terminal NLS was sufficient to target SpRNase III to the nucleus, attachment of a single copy of these particular NLS's to either the N- or C-terminus of SpCas9 was unable to achieve adequate nuclear localization in this system. In this example, the C-terminal NLS was that of nucleoplasmin (KRPAATKKAGQAKKKK), and the C-terminal NLS was that of the SV40 large T-antigen (PKKKRKV). Of the versions of SpCas9 tested, only 2xNLS-SpCas9 exhibited nuclear localization (Figure 2B).

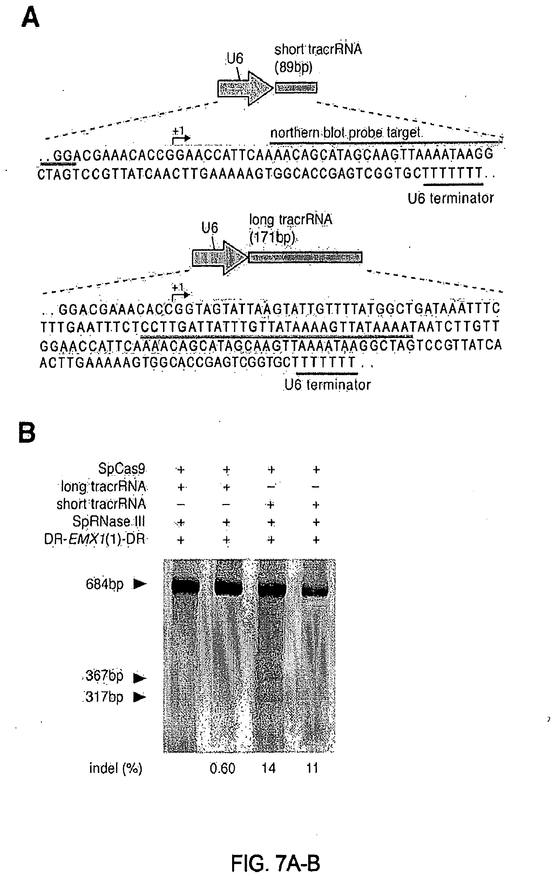

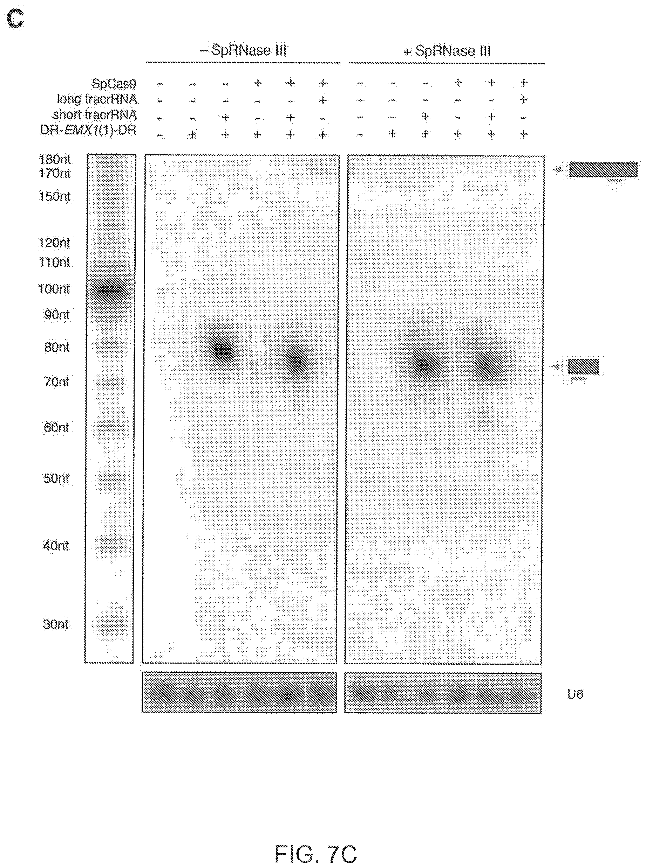

The tracrRNA from the CRISPR locus of *S. pyogenes* SF370 has two transcriptional start sites, giving rise to two transcripts of 89-nucleotides (nt) and 171nt that are subsequently processed into identical 75nt mature tracrRNAs. The shorter 89nt tracrRNA was selected for expression in mammalian cells (expression constructs illustrated in Figure 7A, with functionality as determined by results of the Surveyor assay shown in Figure 7B). Transcription start sites are marked as +1, and transcription terminator and the sequence probed by northern blot are also indicated. Expression of processed tracrRNA was also confirmed by Northern blot. Figure 7C shows results of a Northern blot analysis of total RNA extracted from 293FT cells transfected with U6 expression constructs carrying long or short tracrRNA, as well as SpCas9 and DR-EMX1(1)-DR. Left and right panels are from 293FT cells transfected without or with SpRNase III, respectively. U6 indicate loading control blotted with a probe targeting human U6 snRNA. Transfection of the short tracrRNA expression construct led to abundant levels of the processed form of tracrRNA (~75bp). Very low amounts of long tracrRNA are detected on the Northern blot.

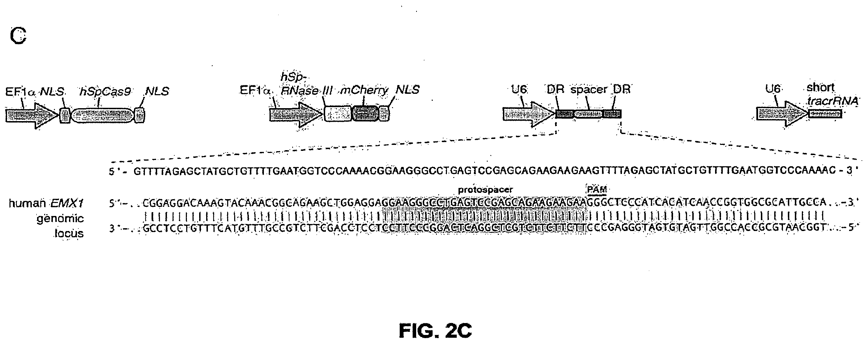

To promote precise transcriptional initiation, the RNA polymerase III-based U6 promoter was selected to drive the expression of tracrRNA (Figure 2C). Similarly, a U6 promoter-based construct was developed to express a pre-crRNA array consisting of a single spacer flanked by two direct repeats (DRs, also encompassed by the term "tracr-mate sequences"; Figure 2C). The initial spacer was designed to target a 33-base-pair (bp) target site (30-bp protospacer plus a 3-bp CRISPR motif (PAM) sequence satisfying the NGG recognition motif of Cas9) in the human *EMX1* locus (Figure 2C), a key gene in the development of the cerebral cortex.

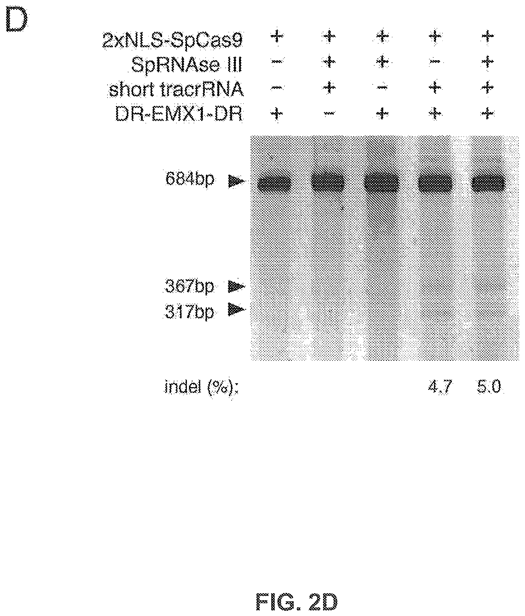

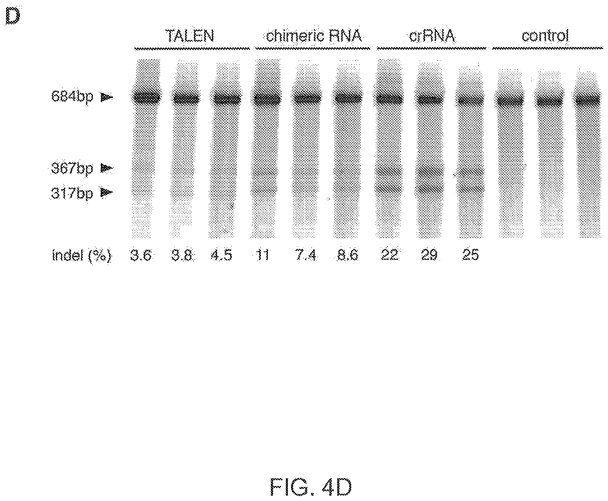

To test whether heterologous expression of the CRISPR system (SpCas9, SpRNase III, tracrRNA, and pre-crRNA) in mammalian cells can achieve targeted cleavage of mammalian chromosomes, HEK 293FT cells were transfected with combinations of CRISPR components. Since DSBs in mammalian nuclei are partially repaired by the non-homologous end joining (NHEJ) pathway, which leads to the formation of indels, the Surveyor assay was used to detect potential cleavage activity at the target *EMX1* locus (Figure 8) (see e.g. Guschin et al., 2010, Methods Mol Biol 649: 247). Co-transfection of all four CRISPR components was able to induce up to 5.0% cleavage in the protospacer (see Figure 2D). Co-transfection of all CRISPR components minus SpRNase III also induced up to 4.7% indel in the protospacer, suggesting that there may be endogenous mammalian RNases that are capable of assisting with crRNA maturation, such as for example the related Dicer and Drosha enzymes. Removing any of the remaining three components abolished the genome cleavage activity of the CRISPR system (Figure 2D). Sanger sequencing of amplicons containing the target locus verified the cleavage activity: in 43 sequenced clones, 5 mutated alleles (11.6%) were found. Similar experiments using a variety of guide sequences produced indel percentages as high as 29% (see Figures 4-7, 12, and 13). These results define a three-component system for efficient CRISPR-mediated genome modification in mammalian cells. To optimize the cleavage efficiency, Applicants also tested whether different isoforms of tracrRNA affected the cleavage efficiency and found that, in this example system, only the short (89-bp) transcript form was able to mediate cleavage of the human *EMX1* genomic locus (Figure 7B).

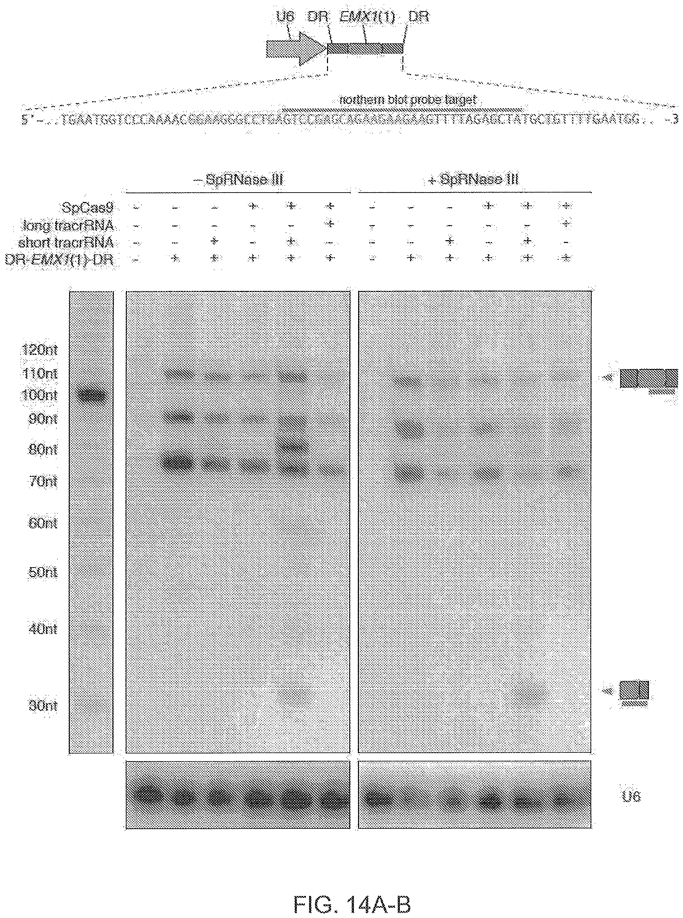

Figure 14 provides an additional Northern blot analysis of crRNA processing in mammalian cells. Figure 14A illustrates a schematic showing the expression vector for a single spacer flanked by two direct repeats (DR-EMX1(1)-DR). The 30bp spacer targeting the human EMX1 locus protospacer 1 (see Figure 6) and the direct repeat sequences are shown in the sequence beneath Figure 14A. The line indicates the region whose reverse-complement sequence was used to generate Northern blot probes for EMX1(1) crRNA detection. Figure 14B shows a Northern blot analysis of total RNA extracted from 293FT cells transfected with U6 expression constructs carrying DR-EMX1(1)-DR. Left and right panels are from 293FT cells transfected without or with SpRNase III respectively. DR-EMX1(1)-DR was processed into mature crRNAs only in the presence of SpCas9 and short tracrRNA and was not dependent on the presence of SpRNase III. The mature crRNA detected from transfected 293FT total RNA is ~33bp and is shorter than the 39-42bp mature crRNA from *S. pyogenes.* These results demonstrate that a CRISPR system can be transplanted into eukaryotic cells and reprogrammed to facilitate cleavage of endogenous mammalian target polynucleotides.

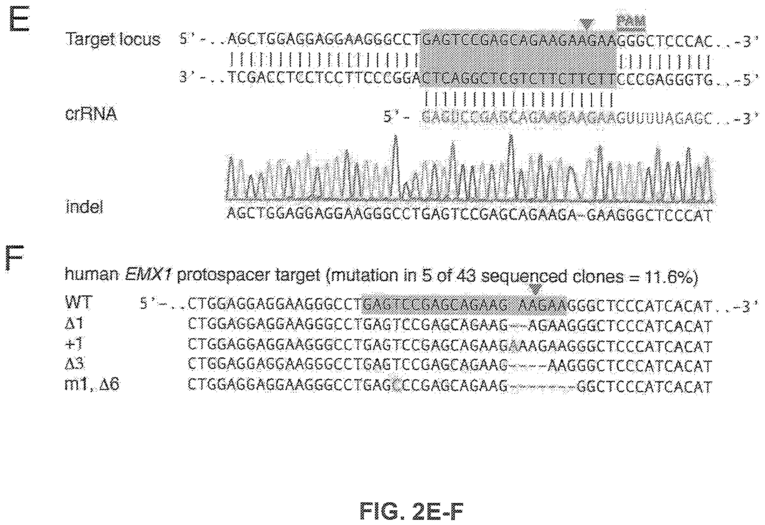

Figure 2 illustrates the bacterial CRISPR system described in this example. Figure 2A illustrates a schematic showing the CRISPR locus 1 from *Streptococcus pyogenes* SF370 and a proposed mechanism of CRISPR-mediated DNA cleavage by this system. Mature crRNA processed from the direct repeat-spacer array directs Cas9 to genomic targets consisting of complimentary protospacers and a protospacer-adjacent motif (PAM). Upon target-spacer base pairing, Cas9 mediates a double-strand break in the target DNA. Figure 2B illustrates engineering of *S. pyogenes* Cas9 (SpCas9) and RNase III (SpRNase III) with nuclear localization signals (NLSs) to enable import into the mammalian nucleus. Figure 2C illustrates mammalian expression of SpCas9 and SpRNase III driven by the constitutive EF1a promoter and tracrRNA and pre-crRNA array (DR-Spacer-DR) driven by the RNA Pol3 promoter U6 to promote precise transcription initiation and termination. A protospacer from the human *EMX1* locus with a satisfactory PAM sequence is used as the spacer in the pre-crRNA array. Figure 2D illustrates surveyor nuclease assay for SpCas9-mediated minor insertions and deletions. SpCas9 was expressed with and without SpRNase III, tracrRNA, and a pre-crRNA array carrying the *EMX1*-target spacer. Figure 2E illustrates a schematic representation of base pairing between target locus and *EMX1*-targeting crRNA, as well as an example chromatogram showing a micro deletion adjacent to the SpCas9 cleavage site. Figure 2F illustrates mutated alleles identified from sequencing analysis of 43 clonal amplicons showing a variety of micro insertions and deletions. Dashes indicate deleted bases, and non-aligned or mismatched bases indicate insertions or mutations. Scale bar = 10μm.

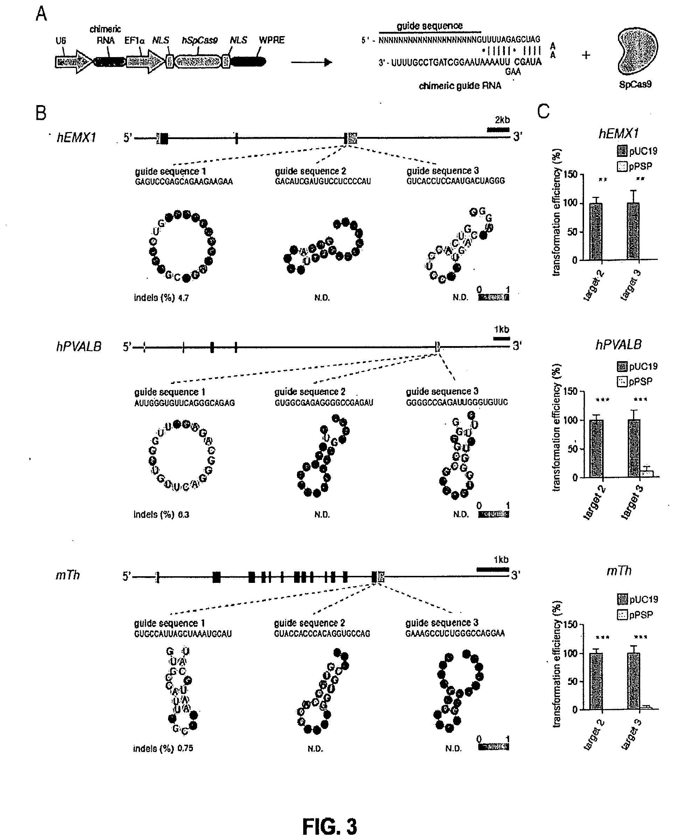

To further simplify the three-component system, a chimeric crRNA-tracrRNA hybrid design was adapted, where a mature crRNA (comprising a guide sequence) is fused to a partial tracrRNA via a stem-loop to mimic the natural crRNA:tracrRNA duplex (Figure 3A). To increase co-delivery efficiency, a bicistronic expression vector was created to drive co-expression of a chimeric RNA and SpCas9 in transfected cells (Figures 3A and 8). In parallel, the bicistronic vectors were used to express a pre-crRNA (DR-guide sequence-DR) with SpCas9, to induce processing into crRNA with a separately expressed tracrRNA (compare Figure 13B top and bottom). Figure 9 provides schematic illustrations of bicistronic expression vectors for pre-crRNA array (Figure 9A) or chimeric crRNA (represented by the short line downstream of the guide sequence insertion site and upstream of the EF1α promoter in Figure 9B) with hSpCas9, showing location of various elements and the point of guide sequence insertion. The expanded sequence around the location of the guide sequence insertion site in Figure 9B also shows a partial DR sequence (GTTTAGAGCTA) and a partial tracrRNA sequence (TAGCAAGTTAAAATAAGGCTAGTCCGTTTTT). Guide sequences can be inserted between BbsI sites using annealed oligonucleotides. Sequence design for the oligonucleotides are shown below the schematic illustrations in Figure 9, with appropriate ligation adapters indicated. WPRE represents the Woodchuck hepatitis virus post-transcriptional regulatory element. The efficiency of chimeric RNA-mediated cleavage was tested by targeting the same *EMX1* locus described above. Using both Surveyor assay and Sanger sequencing of amplicons, Applicants confirmed that the chimeric RNA design facilitates cleavage of human *EMX1* locus with approximately a 4.7% modification rate (Figure 4).

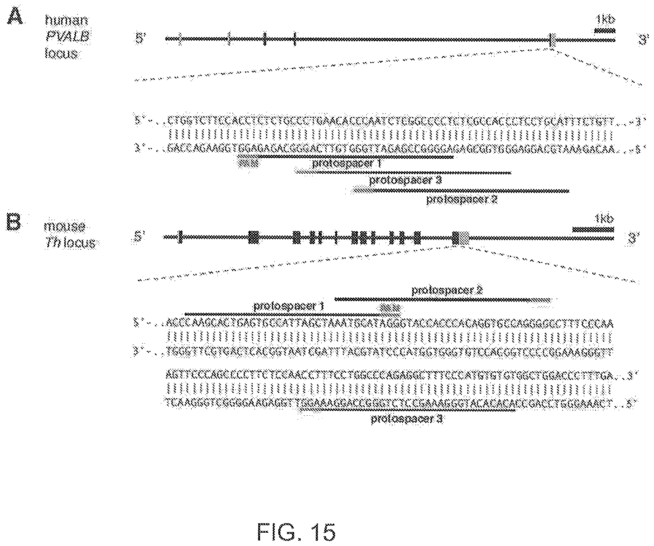

Generalizability of CRISPR-mediated cleavage in eukaryotic cells was tested by targeting additional genomic loci in both human and mouse cells by designing chimeric RNA targeting multiple sites in the human *EMX1* and *PVALB*, as well as the mouse *Th* loci. Figure 15 illustrates the selection of some additional targeted protospacers in human *PVALB* (Figure 15A) and mouse *Th* (Figure 15B) loci. Schematics of the gene loci and the location of three protospacers within the last exon of each are provided. The underlined sequences include 30bp of protospacer sequence and 3bp at the 3' end corresponding to the PAM sequences. Protospacers on the sense and anti-sense strands are indicated above and below the DNA sequences, respectively. A modification rate of 6.3% and 0.75% was achieved for the human *PVALB* and mouse *Th* loci respectively, demonstrating the broad applicability of the CRISPR system in modifying different loci across multiple organisms (Figures 3B and 6). While cleavage was only detected with one out of three spacers for each locus using the chimeric constructs, all target sequences were cleaved with efficiency of indel production reaching 27% when using the co-expressed pre-crRNA arrangement (Figure 6).

Figure 13 provides a further illustration that SpCas9 can be reprogrammed to target multiple genomic loci in mammalian cells. Figure 13A provides a schematic of the human *EMX1* locus showing the location of five protospacers, indicated by the underlined sequences. Figure 13B provides a schematic of the pre-crRNA/trcrRNA complex showing hybridization between the direct repeat region of the pre-crRNA and tracrRNA (top), and a schematic of a chimeric RNA design comprising a 20bp guide sequence, and tracr mate and tracr sequences consisting of partial direct repeat and tracrRNA sequences hybridized in a hairpin structure (bottom). Results of a Surveyor assay comparing the efficacy of Cas9-mediated cleavage at five protospacers in the human *EMX1* locus is illustrated in Figure 13C. Each protospacer is targeted using either processed pre-crRNA/tracrRNA complex (crRNA) or chimeric RNA (chiRNA).

Since the secondary structure of RNA can be crucial for intermolecular interactions, a structure prediction algorithm based on minimum free energy and Boltzmann-weighted structure ensemble was used to compare the putative secondary structure of all guide sequences used in our genome targeting experiment (Figure 3B) (see e.g. Gruber et al., 2008, Nucleic Acids Research, 36: W70). Analysis revealed that in most cases, the effective guide sequences in the chimeric crRNA context were substantially free of secondary structure motifs, whereas the ineffective guide sequences were more likely to form internal secondary structures that could prevent base pairing with the target protospacer DNA. It is thus possible that variability in the spacer secondary structure might impact the efficiency of CRISPR-mediated interference when using a chimeric crRNA.

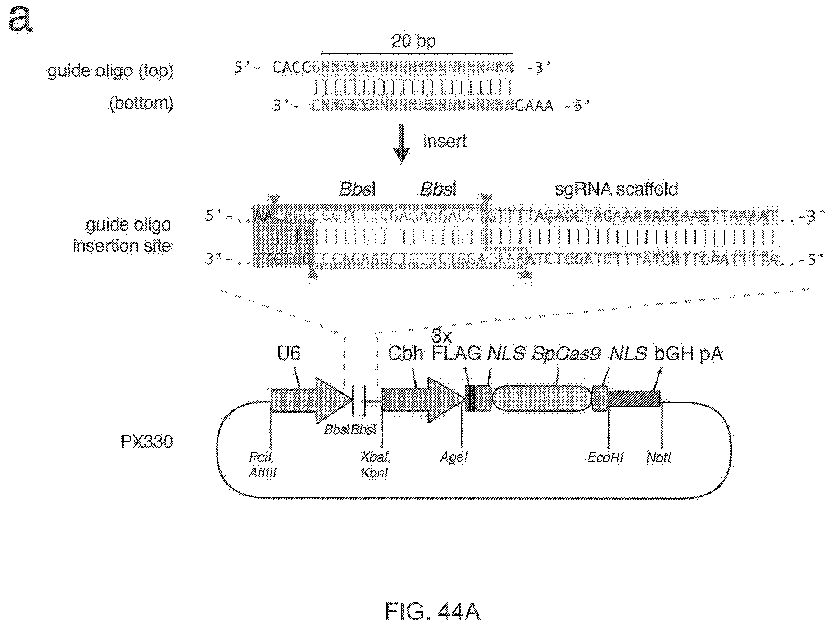

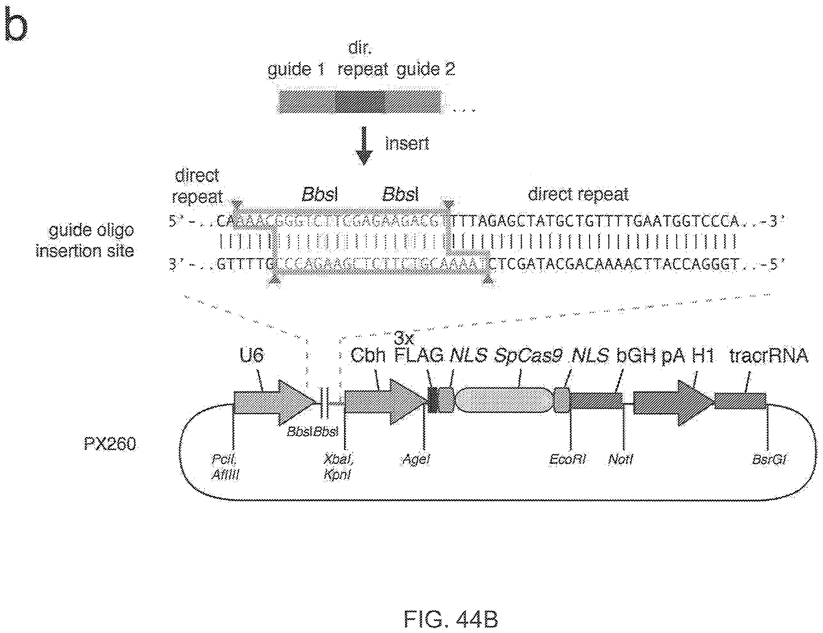

Figure 3 illustrates example expression vectors. Figure 3A provides a schematic of a bi-cistronic vector for driving the expression of a synthetic crRNA-tracrRNA chimera (chimeric RNA) as well as SpCas9. The chimeric guide RNA contains a 20-bp guide sequence corresponding to the protospacer in the genomic target site. Figure 3B provides a schematic showing guide sequences targeting the human *EMX1*, *PVALB*, and mouse *Th* loci, as well as their predicted secondary structures. The modification efficiency at each target site is indicated below the RNA secondary structure drawing (*EMX1*, n = 216 amplicon sequencing reads; *PVALB*, n = 224 reads; *Th*, n = 265 reads). The folding algorithm produced an output with each base colored according to its probability of assuming the predicted secondary structure, as indicated by a rainbow scale that is reproduced in Figure 3B in gray scale. Further vector designs for SpCas9 are shown in Figure 44, which illustrates single expression vectors incorporating a U6 promoter linked to an insertion site for a guide oligo, and a Cbh promoter linked to SpCas9 coding sequence. The vector shown in Figure 44b includes a tracrRNA coding sequence linked to an H1 promoter.

To test whether spacers containing secondary structures are able to function in prokaryotic cells where CRISPRs naturally operate, transformation interference of protospacer-bearing plasmids were tested in an *E. coli* strain heterologously expressing the *S. pyogenes* SF370 CRISPR locus 1 (Figure 10). The CRISPR locus was cloned into a low-copy *E. coli* expression vector and the crRNA array was replaced with a single spacer flanked by a pair of DRs (pCRISPR). *E. coli* strains harboring different pCRISPR plasmids were transformed with challenge plasmids containing the corresponding protospacer and PAM sequences (Figure 10C). In the bacterial assay, all spacers facilitated efficient CRISPR interference (Figure 4C). These results suggest that there may be additional factors affecting the efficiency of CRISPR activity in mammalian cells.

To investigate the specificity of CRISPR-mediated cleavage, the effect of single-nucleotide mutations in the guide sequence on protospacer cleavage in the mammalian genome was analyzed using a series of *EMX1*-targeting chimeric crRNAs with single point mutations (Figure 4A). Figure 4B illustrates results of a Surveyor nuclease assay comparing the cleavage efficiency of Cas9 when paired with different mutant chimeric RNAs. Single-base mismatch up to 12-bp 5' of the PAM substantially abrogated genomic cleavage by SpCas9, whereas spacers with mutations at farther upstream positions retained activity against the original protospacer target (Figure 4B). In addition to the PAM, SpCas9 has single-base specificity within the last 12-bp of the spacer. Furthermore, CRISPR is able to mediate genomic cleavage as efficiently as a pair of TALE nucleases (TALEN) targeting the same *EMX1* protospacer. Figure 4C provides a schematic showing the design of TALENs targeting *EMX1*, and Figure 4D shows a Surveyor gel comparing the efficiency of TALEN and Cas9 (n=3).

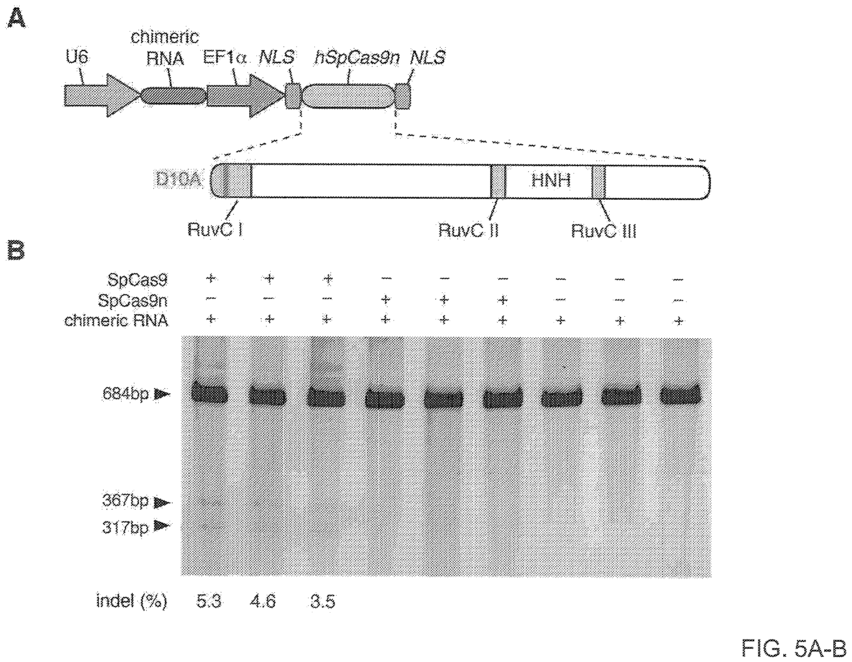

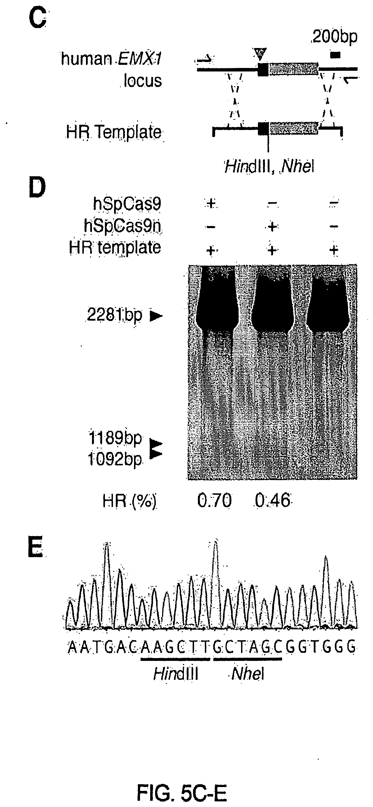

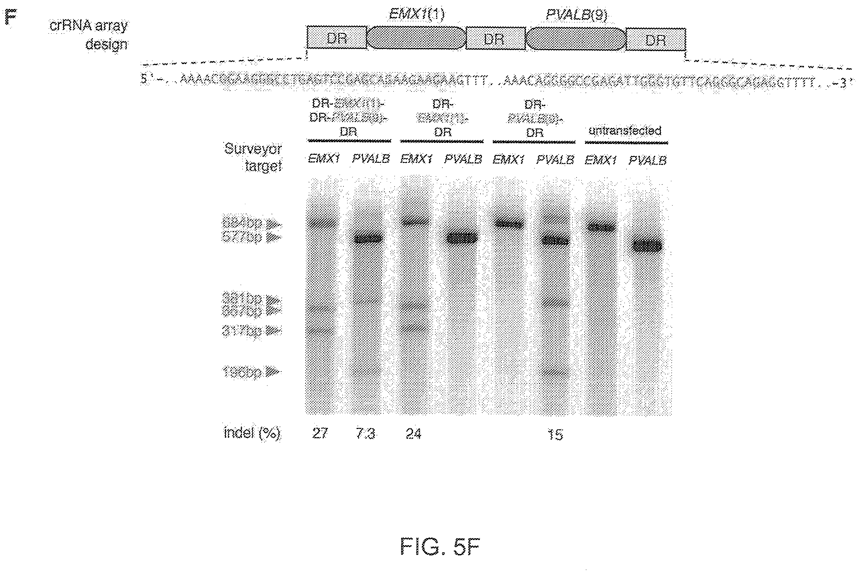

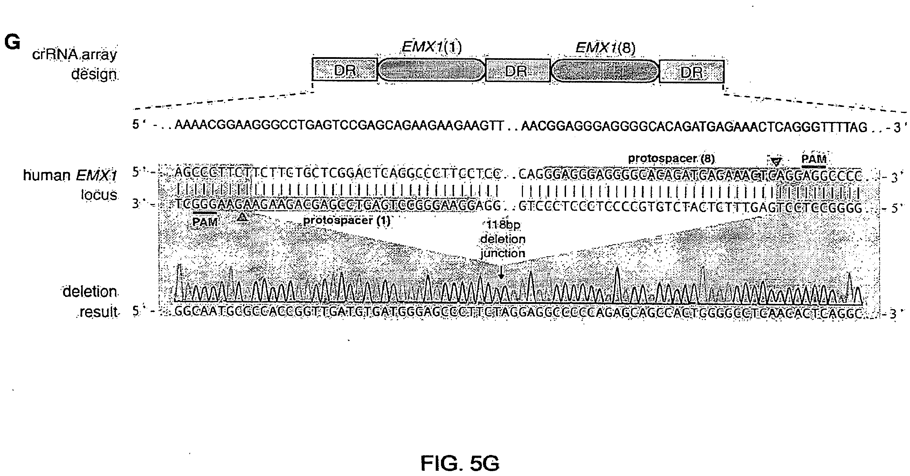

Having established a set of components for achieving CRISPR-mediated gene editing in mammalian cells through the error-prone NHEJ mechanism, the ability of CRISPR to stimulate homologous recombination (HR), a high fidelity gene repair pathway for making precise edits in the genome, was tested. The wild type SpCas9 is able to mediate site-specific DSBs, which can be repaired through both NHEJ and HR. In addition, an aspartate-to-alanine substitution (D10A) in the RuvC I catalytic domain of SpCas9 was engineered to convert the nuclease into a nickase (SpCas9n; illustrated in Figure 5A) (see e.g. Sapranauskas et al., 2011, Nucleic Acids Research, 39: 9275; Gasiunas et al., 2012, Proc. Natl. Acad. Sci. USA, 109:E2579), such that nicked genomic DNA undergoes the high-fidelity homology-directed repair (HDR). Surveyor assay confirmed that SpCas9n does not generate indels at the *EMX1* protospacer target. As illustrated in Figure 5B, co-expression of *EMX1*-targeting chimeric crRNA with SpCas9 produced indels in the target site, whereas co-expression with SpCas9n did not (n=3). Moreover, sequencing of 327 amplicons did not detect any indels induced by SpCas9n. The same locus was selected to test CRISPR-mediated HR by co-transfecting HEK 293FT cells with the chimeric RNA targeting *EMX1*, hSpCas9 or hSpCas9n, as well as a HR template to introduce a pair of restriction sites (*Hin*dIII and *Nhe*I) near the protospacer. Figure 5C provides a schematic illustration of the HR strategy, with relative locations of recombination points and primer annealing sequences (arrows). SpCas9 and SpCas9n indeed catalyzed integration of the HR template into the *EMX1* locus. PCR amplification of the target region followed by restriction digest with *Hin*dIII revealed cleavage products corresponding to expected fragment sizes (arrows in restriction fragment length polymorphism gel analysis shown in Figure 5D), with SpCas9 and SpCas9n mediating similar levels of HR efficiencies. Applicants further verified HR using Sanger sequencing of genomic amplicons (Figure 5E). These results demonstrate the utility of CRISPR for facilitating targeted gene insertion in the mammalian genome. Given the 14-bp (12-bp from the spacer and 2-bp from the PAM) target specificity of the wild type SpCas9, the availability of a nickase can significantly reduce the likelihood of off-target modifications, since single strand breaks are not substrates for the error-prone NHEJ pathway.

Expression constructs mimicking the natural architecture of CRISPR loci with arrayed spacers (Figure 2A) were constructed to test the possibility of multiplexed sequence targeting. Using a single CRISPR array encoding a pair of *EMY1-* and *PVALB*-targeting spacers, efficient cleavage at both loci was detected (Figure 4F, showing both a schematic design of the crRNA array and a Surveyor blot showing efficient mediation of cleavage). Targeted deletion of larger genomic regions through concurrent DSBs using spacers against two targets within *EMX1* spaced by 119bp was also tested, and a 1.6% deletion efficacy (3 out of 182 amplicons; Figure 4G) was detected. This demonstrates that the CRISPR system can mediate multiplexed editing within a single genome.

*Example 2: CRISPR system modifications and alternatives*

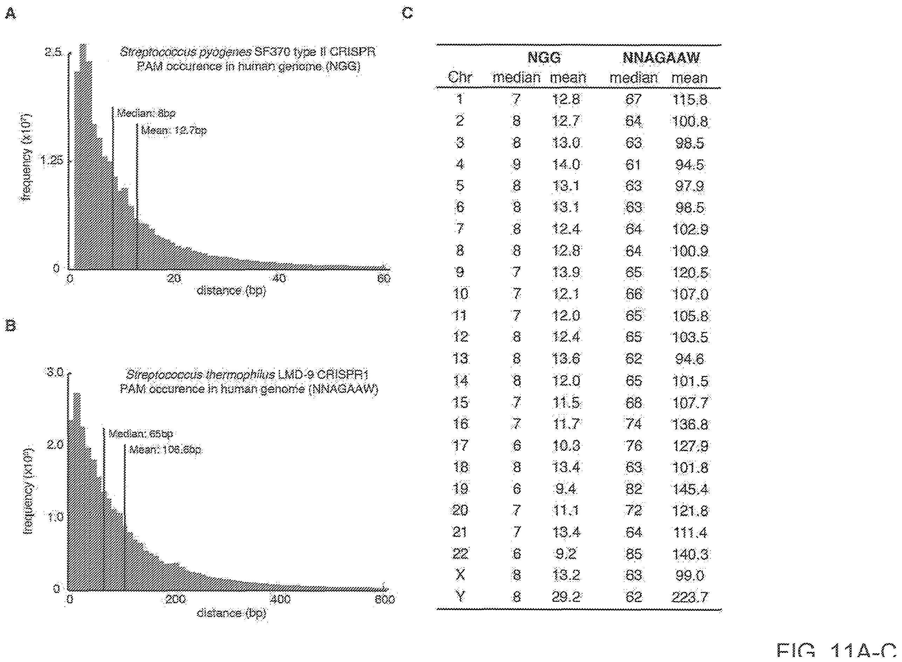

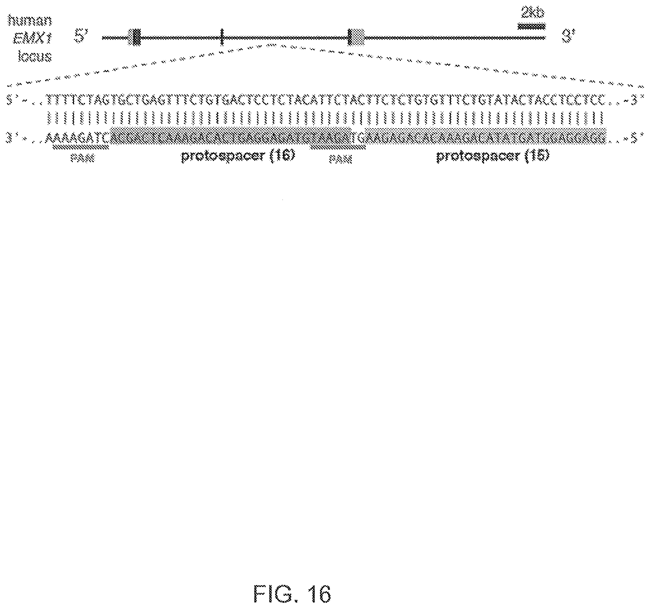

The ability to use RNA to program sequence-specific DNA cleavage defines a new class of genome engineering tools for a variety of research and industrial applications. Several aspects of the CRISPR system can be further improved to increase the efficiency and versatility of CRISPR targeting. Optimal Cas9 activity may depend on the availability of free $Mg^{2+}$ at levels higher than that present in the mammalian nucleus (see e.g. Jinek et al., 2012, Science, 337:816), and the preference for an NGG motif immediately downstream of the protospacer restricts the ability to target on average every 12-bp in the human genome (Figure 11, evaluating both plus and minus strands of human chromosomal sequences). Some of these constraints can be overcome by exploring the diversity of CRISPR loci across the microbial metagenome (see e.g. Makarova et al., 2011, Nat Rev Microbiol, 9:467). Other CRISPR loci may be transplanted into the mammalian cellular milieu by a process similar to that described in Example 1. For example, Figure 12 illustrates adaptation of the Type II CRISPR system from CRISPR 1 of *Streptococcus thermophilus* LMD-9 for heterologous expression in mammalian cells to achieve CRISPR-mediated genome editing. Figure 12A provides a Schematic illustration of CRISPR 1 from S. *thermophilus* LMD-9. Figure 12B illustrates the design of an expression system for the *S. thermophilus* CRISPR system. Human codon-optimized *hStCas9* is expressed using a constitutive EF1$\alpha$ promoter. Mature versions of tracrRNA and crRNA are expressed using the U6 promoter to promote precise transcription initiation. Sequences from the mature crRNA and tracrRNA are illustrated. A single base indicated by the lower case "a" in the crRNA sequence is used to remove the polyU sequence, which serves as a RNA polIII transcriptional terminator. Figure 12C provides a schematic showing guide sequences targeting the human *EMX1* locus as well as their predicted secondary structures. The modification efficiency at each target site is indicated below the RNA secondary structures. The algorithm generating the structures colors each base according to its probability of assuming the predicted secondary structure, which is indicated by a rainbow scale reproduced in Figure 12C in gray scale. Figure 12D shows the results of hStCas9-mediated cleavage in the target locus using the Surveyor assay. RNA guide spacers 1 and 2 induced 14% and 6.4%, respectively. Statistical analysis of cleavage activity across biological replica at these two protospacer sites is also provided in Figure 6. Figure 16 provides a schematic of additional protospacer and corresponding PAM sequence targets of the *S. thermophilus* CRISPR system in the human *EMX1* locus. Two protospacer sequences are highlighted and their corresponding PAM sequences satisfying NNAGAAW motif are indicated by underlining 3' with respect to the corresponding highlighted sequence. Both protospacers target the anti-sense strand.

*Example 3: Sample target sequence selection algorithm*

A software program is designed to identify candidate CRISPR target sequences on both strands of an input DNA sequence based on desired guide sequence length and a CRISPR motif sequence (PAM) for a specified CRISPR enzyme. For example, target sites for Cas9 from S. *pyogenes,* with PAM sequences NGG, may be identified by searching for 5'-$N_x$-NGG-3' both on the input sequence and on the reverse-complement of the input. Likewise, target sites for Cas9 of *S. thermophilus* CRISPR1, with PAM sequence NNAGAAW, may be identified by searching for 5'-$N_x$-NNAGAAW-3' both on the input sequence and on the reverse-complement of the input. Likewise, target sites for Cas9 of *S. thermophilus* CRISPR3, with PAM sequence NGGNG, may be identified by searching for 5'-$N_x$-NGGNG-3' both on the input sequence and on the reverse-complement of the input. The value "x" in $N_x$ may be fixed by the program or specified by the user, such as 20.

Since multiple occurrences in the genome of the DNA target site may lead to nonspecific genome editing, after identifying all potential sites, the program filters out sequences based on the number of times they appear in the relevant reference genome. For those CRISPR enzymes for which sequence specificity is determined by a 'seed' sequence, such as the 11-12bp 5' from the PAM sequence, including the PAM sequence itself, the filtering step may be based on the seed sequence. Thus, to avoid editing at additional genomic loci, results are filtered based on the number of occurrences of the seed:PAM sequence in the relevant genome. The user may be allowed to choose the length of the seed sequence. The user may also be allowed to specify the number of occurrences of the seed:PAM sequence in a genome for purposes of passing the filter. The default is to screen for unique sequences. Filtration level is altered by changing both the length of the seed sequence and the number of occurrences of the sequence in the genome. The program may in addition or alternatively provide the sequence of a guide sequence complementary to the reported target sequence(s) by providing the reverse complement of the identified target sequence(s).

*Example 4: Evaluation of multiple chimeric crRNA-tracrRNA hybrids*

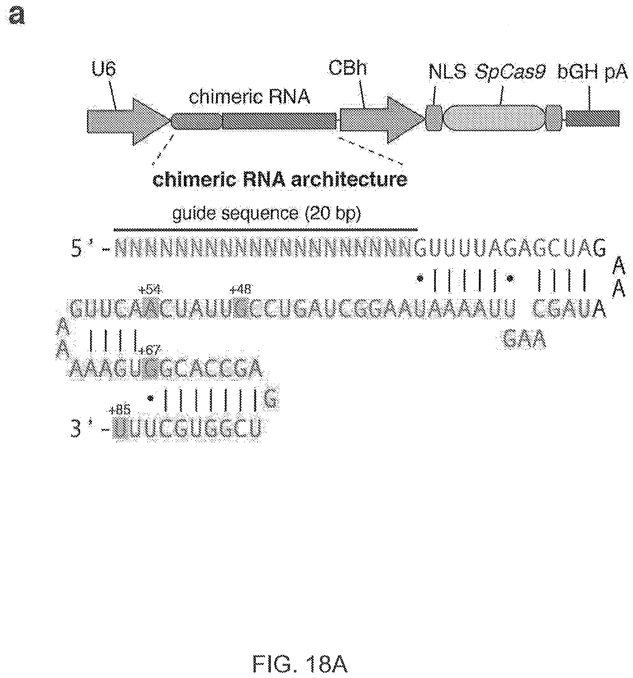

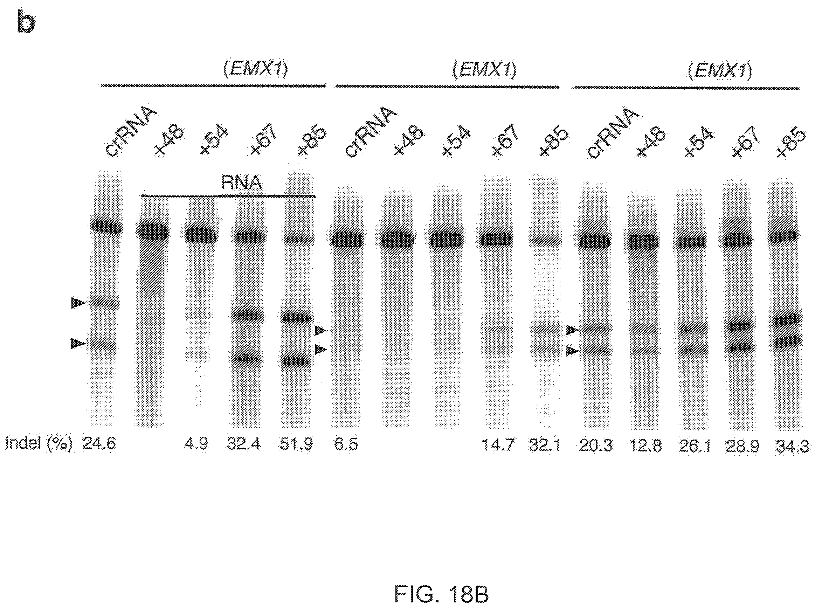

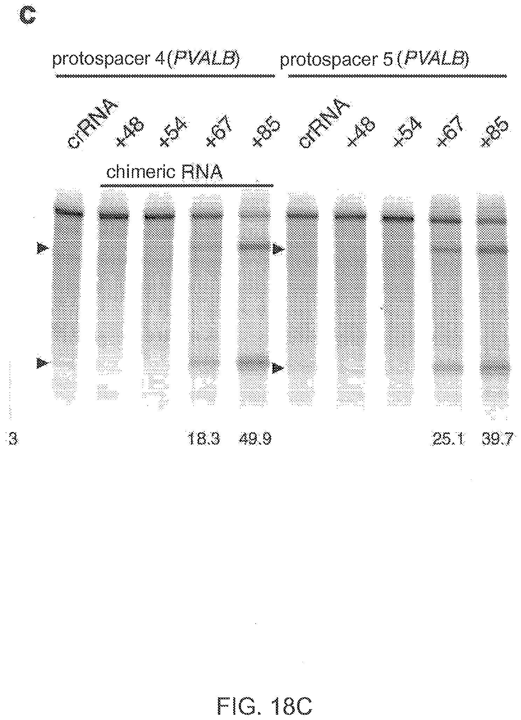

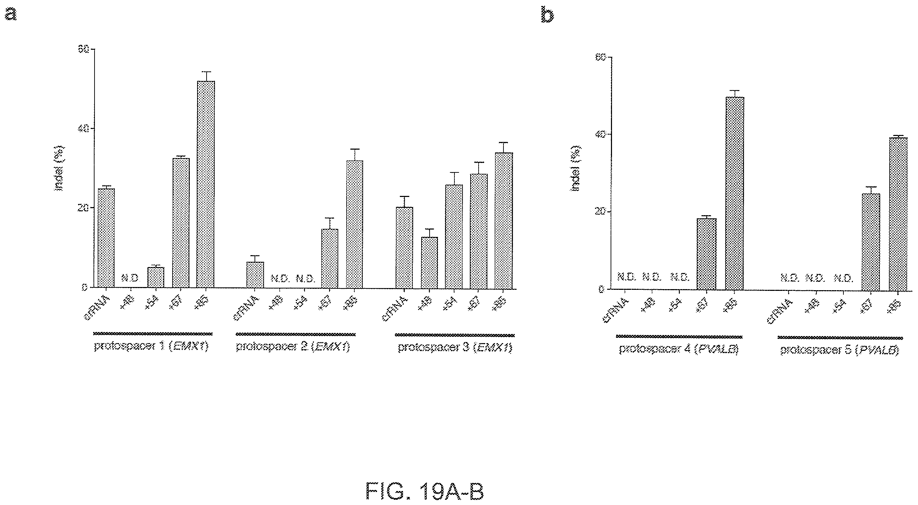

This example describes results obtained for chimeric RNAs (chiRNAs; comprising a guide sequence, a tracr mate sequence, and a tracr sequence in a single transcript) having tracr sequences that incorporate different lengths of wild-type tracrRNA sequence. Figure 18a illustrates a schematic of a bicistronic expression vector for chimeric RNA and Cas9. Cas9 is driven by the CBh promoter and the chimeric RNA is driven by a U6 promoter. The chimeric guide RNA consists of a 20bp guide sequence (Ns) joined to the tracr sequence (running from the first "U" of the lower strand to the end of the transcript), which is truncated at various positions as indicated. The guide and tracr sequences are separated by the tracr-mate sequence GUUUUAGAGCUA followed by the loop sequence GAAA. Results of SURVEYOR assays for Cas9-mediated indels at the human *EMX1* and *PVALB* loci are illustrated in Figure 18b and 18c, respectively. Arrows indicate the expected SURVEYOR fragments. ChiRNAs are indicated by their "+n" designation, and crRNA refers to a hybrid RNA where guide and tracr sequences are expressed as separate transcripts. Quantification of these results, performed in triplicate, are illustrated by histogram in Figures 19a and 19b, corresponding to Figures 18b and 18c, respectively ("N.D." indicates no indels detected). Protospacer IDs and their corresponding genomic target, protospacer sequence, PAM sequence, and strand location are provided in Table D. Guide sequences were designed to be complementary to the entire protospacer sequence in the case of separate transcripts in the hybrid system, or only to the underlined portion in the case of chimeric RNAs.

**Table D:**

| protospacer ID | genomic target | protospacer sequence (5' to 3') | PAM | strand |
|---|---|---|---|---|
| 1 | *EMX1* | GGACATCGAT<u>GTCACCTCCAATGACTAG</u><u>GG</u> | TGG | + |
| 2 | *EMX1* | CATTGGAGGT<u>GACATCGATGTCCTCCCC</u><u>AT</u> | TGG | - |
| 3 | *EMX1* | GGAAGGGCCT<u>GAGTCCGAGCAGAAGAA</u><u>GAA</u> | GGG | + |
| 4 | *PVALB* | GGTGGCGAGA<u>GGGGCCGAGATTGGGTGT</u><u>TC</u> | AGG | + |
| 5 | *PVALB* | ATGCAGGAGG<u>GTGGCGAGAGGGGCCGA</u><u>GAT</u> | TGG | + |

Cell culture and transfection

Human embryonic kidney (HEK) cell line 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100μg/mL streptomycin at 37°C with 5% $CO_2$ incubation. 293FT cells were seeded onto 24-well plates (Corning) 24 hours prior to transfection at a density of 150,000 cells per well. Cells were transfected using Lipofectamine 2000 (Life Technologies) following the manufacturer's recommended protocol. For each well of a 24-well plate, a total of 500ng plasmid was used.

SURVEYOR assay for genome modification

293FT cells were transfected with plasmid DNA as described above. Cells were incubated at 37°C for 72 hours post-transfection prior to genomic DNA extraction. Genomic DNA was extracted using the QuickExtract DNA Extraction Solution (Epicentre) following the manufacturer's protocol. Briefly, pelleted cells were resuspended in QuickExtract solution and incubated at 65°C for 15 minutes and 98°C for 10 minutes. The genomic region flanking the CRISPR target site for each gene was PCR amplified (primers listed in Table E), and products were purified using QiaQuick Spin Column (Qiagen) following the manufacturer's protocol. 400ng total of the purified PCR products were mixed with 2μl 10X Taq DNA Polymerase PCR buffer (Enzymatics) and ultrapure water to a final volume of 20μl, and subjected to a re-annealing process to enable heteroduplex formation: 95°C for 10min, 95°C to 85°C ramping at - 2°C/s, 85°C to 25°C at - 0.25°C/s, and 25°C hold for 1 minute. After re-annealing, products were treated with SURVEYOR nuclease and SURVEYOR enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities.

**Table E:**

| primer name | genomic target | primer sequence (5' to 3') |
|---|---|---|
| Sp-EMX1-F | *EMX1* | AAAACCACCCTTCTCTCTGGC |
| Sp-EMX1-R | *EMX1* | GGAGATTGGAGACACGGAGAG |
| Sp-PVALB-F | *PVALB* | CTGGAAAGCCAATGCCTGAC |
| Sp-PVALB-R | *PVALB* | GGCAGCAAACTCCTTGTCCT |

Computational identification of unique CRISPR target sites

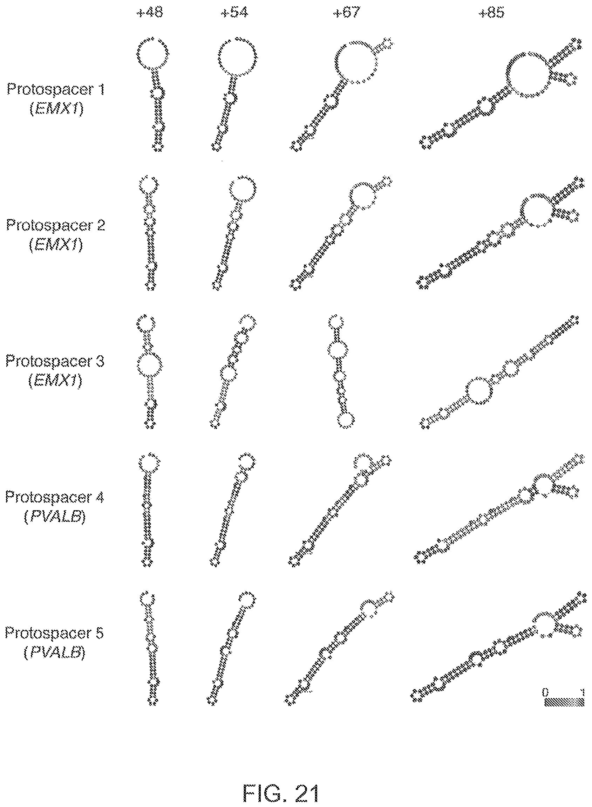

To identify unique target sites for the S. pyogenes SF370 Cas9 (SpCas9) enzyme in the human, mouse, rat, zebrafish, fruit fly, and C. elegans genome, we developed a software package to scan both strands of a DNA sequence and identify all possible SpCas9 target sites. For this example, each SpCas9 target site was operationally defined as a 20bp sequence followed by an NGG protospacer adjacent motif (PAM) sequence, and we identified all sequences satisfying this $5'-N_{20}-NGG-3'$ definition on all chromosomes. To prevent non-specific genome editing, after identifying all potential sites, all target sites were filtered based on the number of times they appear in the relevant reference genome. To take advantage of sequence specificity of Cas9 activity conferred by a 'seed' sequence, which can be, for example, approximately 11-12bp sequence 5' from the PAM sequence, 5'-NNNNNNNNNN-NGG-3' sequences were selected to be unique in the relevant genome. All genomic sequences were downloaded from the UCSC Genome Browser (Human genome hg19, Mouse genome mm9, Rat genome rn5, Zebrafish genome danRer7, D. melanogaster genome dm4 and C. elegans genome ce10). The full search results are available to browse using UCSC Genome Browser information. An example visualization of some target sites in the human genome is provided in Figure 21.

Initially, three sites within the EMX1 locus in human HEK 293FT cells were targeted. Genome modification efficiency of each chiRNA was assessed using the SURVEYOR nuclease assay, which detects mutations resulting from DNA double-strand breaks (DSBs) and their subsequent repair by the non-homologous end joining (NHEJ) DNA damage repair pathway. Constructs designated chiRNA(+n) indicate that up to the +n nucleotide of wild-type tracrRNA is included in the chimeric RNA construct, with values of 48, 54, 67, and 85 used for n. Chimeric RNAs containing longer fragments of wild-type tracrRNA (chiRNA(+67) and chiRNA(+85)) mediated DNA cleavage at all three *EMX1* target sites, with chiRNA(+85) in particular demonstrating significantly higher levels of DNA cleavage than the corresponding crRNA/tracrRNA hybrids that expressed guide and tracr sequences in separate transcripts (Figures 18b and 19a). Two sites in the PVALB locus that yielded no detectable cleavage using the hybrid system (guide sequence and tracr sequence expressed as separate transcripts) were also targeted using chiRNAs. chiRNA(+67) and chiRNA(+85) were able to mediate significant cleavage at the two PVALB protospacers (Figures 18c and 19b).

For all five targets in the *EMX*1 and PVALB loci, a consistent increase in genome modification efficiency with increasing tracr sequence length was observed. Without wishing to be bound by any theory, the secondary structure formed by the 3' end of the tracrRNA may play a role in enhancing the rate of CRISPR complex formation. An illustration of predicted secondary structures for each of the chimeric RNAs used in this example is provided in Figure 21. The secondary structure was predicted using RNAfold (http://rna.tbi.univie.ac.at/cgi-bin/RNAfold.cgi) using minimum free energy and partition function algorithm. Pseudocolor for each based (reproduced in grayscale) indicates the probability of pairing. Because chiRNAs with longer tracr sequences were able to cleave targets that were not cleaved by native CRISPR crRNA/tracrRNA hybrids, it is possible that chimeric RNA may be loaded onto Cas9 more efficiently than its native hybrid counterpart. To facilitate the application of Cas9 for site-specific genome editing in eukaryotic cells and organisms, all predicted unique target sites for the S. pyogenes Cas9 were computationally identified in the human, mouse, rat, zebra fish, C. elegans, and D. melanogaster genomes. Chimeric RNAs can be designed for Cas9 enzymes from other microbes to expand the target space of CRISPR RNA-programmable nucleases.

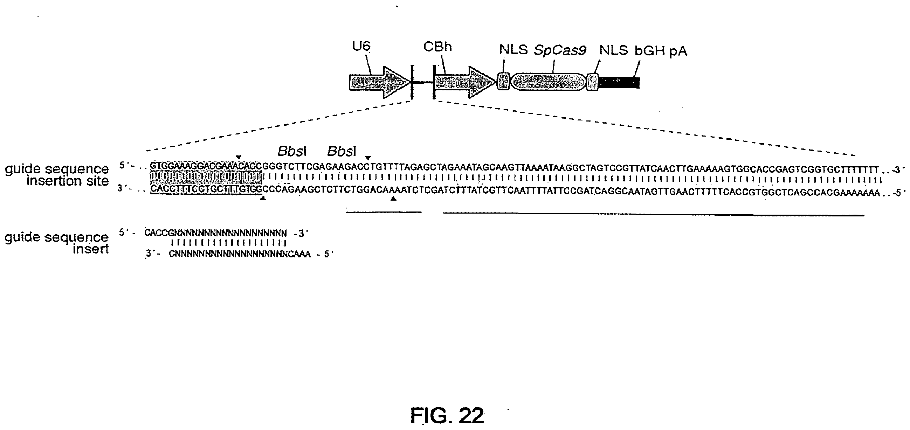

Figure 22 illustrates an exemplary bicistronic expression vector for expression of chimeric RNA including up to the +85 nucleotide of wild-type tracr RNA sequence, and SpCas9 with nuclear localization sequences. SpCas9 is expressed from a CBh promoter and terminated with the bGH polyA signal (bGH pA). The expanded sequence illustrated immediately below the schematic corresponds to the region surrounding the guide sequence insertion site, and includes, from 5' to 3', 3'-portion of the U6 promoter (first shaded region), BbsI cleavage sites (arrows), partial direct repeat (tracr mate sequence GTTTTAGAGCTA, underlined), loop sequence GAAA, and +85 tracr sequence (underlined sequence following loop sequence). An exemplary guide sequence insert is illustrated below the guide sequence insertion site, with nucleotides of the guide sequence for a selected target represented by an "N".

Sequences described in the above examples are as follows (polynucleotide sequences are 5' to 3'):

U6-short tracrRNA (Streptococcus pyogenes SF370):

GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACC**GGAACCATTCAAAACAGCATAGCAAGTTA**
**AAATAAGGCTAGTCCGTTATCAACTTGAAAAAGTGGCACCGAGTCGGTGC**<u>TTTT</u>

<u>TTT</u> (bold = tracrRNA sequence; underline = terminator sequence)
U6-long tracrRNA (Streptococcus pyogenes SF370):

GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGTAGTATTAAGTATTGTTTTATGGCTGATA
AATTTCTTTGAATTTCTCCTTGATTATTTGTTATAAAAGTTATAAAATAATCTTGTTG
GAACCATTCAAAACAGCATAGCAAGTTAAAATAAGGCTAGTCCGTTATCAACTTGA
AAAAGTGGCACCGAGTCGGTGCTTTTTTT

U6-DR-BbsI backbone-DR (Streptococcus pyogenes SF370):

GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGGGTTTTAGAGCTATGCTGTTTTGAATGGTC
CCAAAACGGGTCTTCGAGAAGACGTTTTAGAGCTATGCTGTTTTGAATGGTCCCAAA
AC

U6-chimeric RNA-BbsI backbone (Streptococcus pyogenes SF370)

GAGGGCCTATTTCCCATGATTCCTTCATATTTGCATATACGATACAAGGCT GTTAGAGAGATAATTGGAATTAATTTGACTGTAAACACAAGATATTAGTACAAAA TACGTGACGTAGAAGTAATAATTTCTTGGGTAGTTTGCAGTTTTAAAATTATGTTTT AAAATGGACTATCATATGCTTACCGTAACTTGAAAGTATTTCGATTTCTTGGCTTTAT ATATCTTGTGGAAAGGACGAAACACCGGGTCTTCGAGAAGACCTGTTTTAGAGCTA GAAATAGCAAGTTAAAATAAGGCTAGTCCG

NLS-SpCas9-EGFP:

MDYKDHDGDYKDHDIDYKDDDDKMAPKKKRKVGIHGVPAADKKYSIGLDI GTNSVGWAVITDEYKVPSKKFKVLGNTDRHSIKKNLIGALLFDSGETAEATRLKRTARR RYTRRKNRICYLQEIFSNEMAKVDDSFFHRLEESFLVEEDKKHERHPIFGNIVDEVAYHE KYPTIYHLRKKLVDSTDKADLRLIYLALAHMIKFRGHFLIEGDLNPDNSDVDKLFIQLVQ TYNQLFEENPINASGVDAKAILSARLSKSRRLENLIAQLPGEKKNGLFGNLIALSLGLTPN FKSNFDLAEDAKLQLSKDTYDDDLDNLLAQIGDQYADLFLAAKNLSDAILLSDILRVNT EITKAPLSASMIKRYDEHHQDLTLLKALVRQQLPEKYKEIFFDQSKNGYAGYIDGGASQ EEFYKFIKPILEKMDGTEELLVKLNREDLLRKQRTFDNGSIPHQIHLGELHAILRRQEDFY PFLKDNREKIEKILTFRIPYYVGPLARGNSRFAWMTRKSEETITPWNFEEVVDKGASAQS FIERMTNFDKNLPNEKVLPKHSLLYEYFTVYNELTKVKYVTEGMRKPAFLSGEQKKAIV DLLFKTNRKVTVKQLKEDYFKKIECFDSVEISGVEDRFNASLGTYHDLLKIIKDKDFLDN EENEDILEDIVLTLTLFEDREMIEERLKTYAHLFDDKVMKQLKRRRYTGWGRLSRKLIN GIRDKQSGKTILDFLKSDGFANRNFMQLIHDDSLTFKEDIQKAQVSGQGDSLHEHIANLA GSPAIKKGILQTVKVVDELVKVMGRHKPENIVIEMARENQTTQKGQKNSRERMKRIEEG IKELGSQILKEHPVENTQLQNEKLYLYYLQNGRDMYVDQELDINRLSDYDVDHIVPQSF

LKDDSIDNKVLTRSDKNRGKSDNVPSEEVVKKMKNYWRQLLNAKLITQRKFDNLTKAE
RGGLSELDKAGFIKRQLVETRQITKHVAQILDSRMNTKYDENDKLIREVKVITLKSKLVS
DFRKDFQFYKVREINNYHHAHDAYLNAVVGTALIKKYPKLESEFVYGDYKVYDVRKMI
AKSEQEIGKATAKYFFYSNIMNFFKTEITLANGEIRKRPLIETNGETGEIVWDKGRDFATV
RKVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPKYS
LFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDAAAVSKGEELFTG
VVPILVELDGDVNGHKFSVSGEGEGDATYGKLTLKFICTTGKLPVPWPTLVTTLTYGVQ
CFSRYPDHMKQHDFFKSAMPEGYVQERTIFFKDDGNYKTRAEVKFEGDTLVNRIELKGI
DFKEDGNILGHKLEYNYNSHNVYIMADKQKNGIKVNFKIRHNIEDGSVQLADHYQQNT
PIGDGPVLLPDNHYLSTQSALSKDPNEKRDHMVLLEFVTAAGITLGMDELYK

SpCas9-EGFP-NLS:

MDKKYSIGLDIGTNSVGWAVITDEYKVPSKKFKVLGNTDRHSIKKNLIGALLF
DSGETAEATRLKRTARRRYTRRKNRICYLQEIFSNEMAKVDDSFFHRLEESFLVEEDKK
HERHPIFGNIVDEVAYHEKYPTIYHLRKKLVDSTDKADLRLIYLALAHMIKFRGHFLIEG
DLNPDNSDVDKLFIQLVQTYNQLFEENPINASGVDAKAILSARLSKSRRLENLIAQLPGE
KKNGLFGNLIALSLGLTPNFKSNFDLAEDAKLQLSKDTYDDDLDNLLAQIGDQYADLFL
AAKNLSDAILLSDILRVNTEITKAPLSASMIKRYDEHHQDLTLLKALVRQQLPEKYKEIFF
DQSKNGYAGYIDGGASQEEFYKFIKPILEKMDGTEELLVKLNREDLLRKQRTFDNGSIPH
QIHLGELHAILRRQEDFYPFLKDNREKIEKILTFRIPYYVGPLARGNSRFAWMTRKSEETI
TPWNFEEVVDKGASAQSFIERMTNFDKNLPNEKVLPKHSLLYEYFTVYNELTKVKYVTE
GMRKPAFLSGEQKKAIVDLLFKTNRKVTVKQLKEDYFKKIECFDSVEISGVEDRFNASL
GTYHDLLKIIKDKDFLDNEENEDILEDIVLTLTLFEDREMIEERLKTYAHLFDDKVMKQL
KRRRYTGWGRLSRKLINGIRDKQSGKTILDFLKSDGFANRNFMQLIHDDSLTFKEDIQKA
QVSGQGDSLHEHIANLAGSPAIKKGILQTVKVVDELVKVMGRHKPENIVIEMARENQTT
QKGQKNSRERMKRIEEGIKELGSQILKEHPVENTQLQNEKLYLYYLQNGRDMYVDQEL
DINRLSDYDVDHIVPQSFLKDDSIDNKVLTRSDKNRGKSDNVPSEEVVKKMKNYWRQL
LNAKLITQRKFDNLTKAERGGLSELDKAGFIKRQLVETRQITKHVAQILDSRMNTKYDE
NDKLIREVKVITLKSKLVSDFRKDFQFYKVREINNYHHAHDAYLNAVVGTALIKKYPKL

ESEFVYGDYKVYDVRKMIAKSEQEIGKATAKYFFYSNIMNFFKTEITLANGEIRKRPLIET

NGETGEIVWDKGRDFATVRKVLSMPQVNIVKKTEVQTGGFSKESILPKRNSDKLIARKK

DWDPKKYGGFDSPTVAYSVLVVAKVEKGKSKKLKSVKELLGITIMERSSFEKNPIDFLE

AKGYKEVKKDLIIKLPKYSLFELENGRKRMLASAGELQKGNELALPSKYVNFLYLASHY

EKLKGSPEDNEQKQLFVEQHKHYLDEIIEQISEFSKRVILADANLDKVLSAYNKHRDKPI

REQAENIIHLFTLTNLGAPAAFKYFDTTIDRKRYTSTKEVLDATLIHQSITGLYETRIDLSQ

LGGDAAAVSKGEELFTGVVPILVELDGDVNGHKFSVSGEGEGDATYGKLTLKFICTTGK

LPVPWPTLVTTLTYGVQCFSRYPDHMKQHDFFKSAMPEGYVQERTIFFKDDGNYKTRA

EVKFEGDTLVNRIELKGIDFKEDGNILGHKLEYNYNSHNVYIMADKQKNGIKVNFKIRH

NIEDGSVQLADHYQQNTPIGDGPVLLPDNHYLSTQSALSKDPNEKRDHMVLLEFVTAAG

ITLGMDELYKKRPAATKKAGQAKKKK

NLS-SpCas9-EGFP-NLS:

[00249] MDYKDHDGDYKDHDIDYKDDDDKMAPKKKRKVGIHGVPAADKKYSIGLDI

GTNSVGWAVITDEYKVPSKKFKVLGNTDRHSIKKNLIGALLFDSGETAEATRLKRTARR

RYTRRKNRICYLQEIFSNEMAKVDDSFFHRLEESFLVEEDKKHERHPIFGNIVDEVAYHE

KYPTIYHLRKKLVDSTDKADLRLIYLALAHMIKFRGHFLIEGDLNPDNSDVDKLFIQLVQ

TYNQLFEENPINASGVDAKAILSARLSKSRRLENLIAQLPGEKKNGLFGNLIALSLGLTPN

FKSNFDLAEDAKLQLSKDTYDDDLDNLLAQIGDQYADLFLAAKNLSDAILLSDILRVNT

EITKAPLSASMIKRYDEHHQDLTLLKALVRQQLPEKYKEIFFDQSKNGYAGYIDGGASQ

EEFYKFIKPILEKMDGTEELLVKLNREDLLRKQRTFDNGSIPHQIHLGELHAILRRQEDFY

PFLKDNREKIEKILTFRIPYYVGPLARGNSRFAWMTRKSEETITPWNFEEVVDKGASAQS

FIERMTNFDKNLPNEKVLPKHSLLYEYFTVYNELTKVKYVTEGMRKPAFLSGEQKKAIV

DLLFKTNRKVTVKQLKEDYFKKIECFDSVEISGVEDRFNASLGTYHDLLKIIKDKDFLDN

EENEDILEDIVLTLTLFEDREMIEERLKTYAHLFDDKVMKQLKRRRYTGWGRLSRKLIN

GIRDKQSGKTILDFLKSDGFANRNFMQLIHDDSLTFKEDIQKAQVSGQGDSLHEHIANLA

GSPAIKKGILQTVKVVDELVKVMGRHKPENIVIEMARENQTTQKGQKNSRERMKRIEEG

IKELGSQILKEHPVENTQLQNEKLYLYYLQNGRDMYVDQELDINRLSDYDVDHIVPQSF

LKDDSIDNKVLTRSDKNRGKSDNVPSEEVVKKMKNYWRQLLNAKLITQRKFDNLTKAE

RGGLSELDKAGFIKRQLVETRQITKHVAQILDSRMNTKYDENDKLIREVKVITLKSKLVS

DFRKDFQFYKVREINNYHHAHDAYLNAVVGTALIKKYPKLESEFVYGDYKVYDVRKMI

AKSEQEIGKATAKYFFYSNIMNFFKTEITLANGEIRKRPLIETNGETGEIVWDKGRDFATV

RKVLSMPQVNIVKKTEVQTGGFSKESILPKRNSDKLIARKKDWDPKKYGGFDSPTVAYS VLVVAKVEKGKSKKLKSVKELLGITIMERSSFEKNPIDFLEAKGYKEVKKDLIIKLPKYS LFELENGRKRMLASAGELQKGNELALPSKYVNFLYLASHYEKLKGSPEDNEQKQLFVE QHKHYLDEIIEQISEFSKRVILADANLDKVLSAYNKHRDKPIREQAENIIHLFTLTNLGAP AAFKYFDTTIDRKRYTSTKEVLDATLIHQSITGLYETRIDLSQLGGDAAAVSKGEELFTG VVPILVELDGDVNGHKFSVSGEGEGDATYGKLTLKFICTTGKLPVPWPTLVTTLTYGVQ CFSRYPDHMKQHDFFKSAMPEGYVQERTIFFKDDGNYKTRAEVKFEGDTLVNRIELKGI DFKEDGNILGHKLEYNYNSHNVYIMADKQKNGIKVNFKIRHNIEDGSVQLADHYQQNT PIGDGPVLLPDNHYLSTQSALSKDPNEKRDHMVLLEFVTAAGITLGMDELYKKRPAATK KAGQAKKKK

NLS-SpCas9-NLS:

**[00251]** MDYKDHDGDYKDHDIDYKDDDDKMAPKKKRKVGIHGVPAADKKYSIGLDI GTNSVGWAVITDEYKVPSKKFKVLGNTDRHSIKKNLIGALLFDSGETAEATRLKRTARR RYTRRKNRICYLQEIFSNEMAKVDDSFFHRLEESFLVEEDKKHERHPIFGNIVDEVAYHE KYPTIYHLRKKLVDSTDKADLRLIYLALAHMIKFRGHFLIEGDLNPDNSDVDKLFIQLVQ TYNQLFEENPINASGVDAKAILSARLSKSRRLENLIAQLPGEKKNGLFGNLIALSLGLTPN FKSNFDLAEDAKLQLSKDTYDDDLDNLLAQIGDQYADLFLAAKNLSDAILLSDILRVNT EITKAPLSASMIKRYDEHHQDLTLLKALVRQQLPEKYKEIFFDQSKNGYAGYIDGGASQ EEFYKFIKPILEKMDGTEELLVKLNREDLLRKQRTFDNGSIPHQIHLGELHAILRRQEDFY PFLKDNREKIEKILTFRIPYYVGPLARGNSRFAWMTRKSEETITPWNFEEVVDKGASAQS FIERMTNFDKNLPNEKVLPKHSLLYEYFTVYNELTKVKYVTEGMRKPAFLSGEQKKAIV DLLFKTNRKVTVKQLKEDYFKKIECFDSVEISGVEDRFNASLGTYHDLLKIIKDKDFLDN EENEDILEDIVLTLTLFEDREMIEERLKTYAHLFDDKVMKQLKRRRYTGWGRLSRKLIN GIRDKQSGKTILDFLKSDGFANRNFMQLIHDDSLTFKEDIQKAQVSGQGDSLHEHIANLA GSPAIKKGILQTVKVVDELVKVMGRHKPENIVIEMARENQTTQKGQKNSRERMKRIEEG IKELGSQILKEHPVENTQLQNEKLYLYYLQNGRDMYVDQELDINRLSDYDVDHIVPQSF LKDDSIDNKVLTRSDKNRGKSDNVPSEEVVKKMKNYWRQLLNAKLITQRKFDNLTKAE RGGLSELDKAGFIKRQLVETRQITKHVAQILDSRMNTKYDENDKLIREVKVITLKSKLVS DFRKDFQFYKVREINNYHHAHDAYLNAVVGTALIKKYPKLESEFVYGDYKVYDVRKMI AKSEQEIGKATAKYFFYSNIMNFFKTEITLANGEIRKRPLIETNGETGEIVWDKGRDFATV RKVLSMPQVNIVKKTEVQTGGFSKESILPKRNSDKLIARKKDWDPKKYGGFDSPTVAYS

VLVVAKVEKGKSKKLKSVKELLGITIMERSSFEKNPIDFLEAKGYKEVKKDLIIKLPKYS
LFELENGRKRMLASAGELQKGNELALPSKYVNFLYLASHYEKLKGSPEDNEQKQLFVE
QHKHYLDEIIEQISEFSKRVILADANLDKVLSAYNKHRDKPIREQAENIIHLFTLTNLGAP
AAFKYFDTTIDRKRYTSTKEVLDATLIHQSITGLYETRIDLSQLGGDKRPAATKKAGQAK
KKK

NLS-mCherry-SpRNase3:

MFLFLSLTSFLSSSRTLVSKGEEDNMAIIKEFMRFKVHMEGSVNGHEFEIEGE
GEGRPYEGTQTAKLKVTKGGPLPFAWDILSPQFMYGSKAYVKHPADIPDYLKLSFPEGF
KWERVMNFEDGGVVTVTQDSSLQDGEFIYKVKLRGTNFPSDGPVMQKKTMGWEASSE
RMYPEDGALKGEIKQRLKLKDGGHYDAEVKTTYKAKKPVQLPGAYNVNIKLDITSHNE
DYTIVEQYERAEGRHSTGGMDELYKGSKQLEELLSTSFDIQFNDLTLLETAFTHTSYANE
HRLLNVSHNERLEFLGDAVLQLIISEYLFAKYPKKTEGDMSKLRSMIVREESLAGFSRFC
SFDAYIKLGKGEEKSGGRRRDTILGDLFEAFLGALLLDKGIDAVRRFLKQVMIPQVEKG
NFERVKDYKTCLQEFLQTKGDVAIDYQVISEKGPAHAKQFEVSIVVNGAVLSKGLGKSK
KLAEQDAAKNALAQLSEV

SpRNase3-mCherry-NLS:

MKQLEELLSTSFDIQFNDLTLLETAFTHTSYANEHRLLNVSHNERLEFLGDAV
LQLIISEYLFAKYPKKTEGDMSKLRSMIVREESLAGFSRFCSFDAYIKLGKGEEKSGGRR
RDTILGDLFEAFLGALLLDKGIDAVRRFLKQVMIPQVEKGNFERVKDYKTCLQEFLQTK
GDVAIDYQVISEKGPAHAKQFEVSIVVNGAVLSKGLGKSKKLAEQDAAKNALAQLSEV
GSVSKGEEDNMAIIKEFMRFKVHMEGSVNGHEFEIEGEGEGRPYEGTQTAKLKVTKGGP
LPFAWDILSPQFMYGSKAYVKHPADIPDYLKLSFPEGFKWERVMNFEDGGVVTVTQDS
SLQDGEFIYKVKLRGTNFPSDGPVMQKKTMGWEASSERMYPEDGALKGEIKQRLKLKD
GGHYDAEVKTTYKAKKPVQLPGAYNVNIKLDITSHNEDYTIVEQYERAEGRHSTGGMD
ELYKKRPAATKKAGQAKKKK

NLS-SpCas9n-NLS (the D10A nickase mutation is lowercase):

MDYKDHDGDYKDHDIDYKDDDDKMAPKKKRKVGIHGVPAADKKYSIGLaI
GTNSVGWAVITDEYKVPSKKFKVLGNTDRHSIKKNLIGALLFDSGETAEATRLKRTARR
RYTRRKNRICYLQEIFSNEMAKVDDSFFHRLEESFLVEEDKKHERHPIFGNIVDEVAYHE
KYPTIYHLRKKLVDSTDKADLRLIYLALAHMIKFRGHFLIEGDLNPDNSDVDKLFIQLVQ
TYNQLFEENPINASGVDAKAILSARLSKSRRLENLIAQLPGEKKNGLFGNLIALSLGLTPN

FKSNFDLAEDAKLQLSKDTYDDDLDNLLAQIGDQYADLFLAAKNLSDAILLSDILRVNT

EITKAPLSASMIKRYDEHHQDLTLLKALVRQQLPEKYKEIFFDQSKNGYAGYIDGGASQ

EEFYKFIKPILEKMDGTEELLVKLNREDLLRKQRTFDNGSIPHQIHLGELHAILRRQEDFY

PFLKDNREKIEKILTFRIPYYVGPLARGNSRFAWMTRKSEETITPWNFEEVVDKGASAQS

FIERMTNFDKNLPNEKVLPKHSLLYEYFTVYNELTKVKYVTEGMRKPAFLSGEQKKAIV

DLLFKTNRKVTVKQLKEDYFKKIECFDSVEISGVEDRFNASLGTYHDLLKIIKDKDFLDN

EENEDILEDIVLTLTLFEDREMIEERLKTYAHLFDDKVMKQLKRRRYTGWGRLSRKLIN

GIRDKQSGKTILDFLKSDGFANRNFMQLIHDDSLTFKEDIQKAQVSGQGDSLHEHIANLA

GSPAIKKGILQTVKVVDELVKVMGRHKPENIVIEMARENQTTQKGQKNSRERMKRIEEG

IKELGSQILKEHPVENTQLQNEKLYLYYLQNGRDMYVDQELDINRLSDYDVDHIVPQSF

LKDDSIDNKVLTRSDKNRGKSDNVPSEEVVKKMKNYWRQLLNAKLITQRKFDNLTKAE

RGGLSELDKAGFIKRQLVETRQITKHVAQILDSRMNTKYDENDKLIREVKVITLKSKLVS

DFRKDFQFYKVREINNYHHAHDAYLNAVVGTALIKKYPKLESEFVYGDYKVYDVRKMI

AKSEQEIGKATAKYFFYSNIMNFFKTEITLANGEIRKRPLIETNGETGEIVWDKGRDFATV

RKVLSMPQVNIVKKTEVQTGGFSKESILPKRNSDKLIARKKDWDPKKYGGFDSPTVAYS

VLVVAKVEKGKSKKLKSVKELLGITIMERSSFEKNPIDFLEAKGYKEVKKDLIIKLPKYS

LFELENGRKRMLASAGELQKGNELALPSKYVNFLYLASHYEKLKGSPEDNEQKQLFVE

QHKHYLDEIIEQISEFSKRVILADANLDKVLSAYNKHRDKPIREQAENIIHLFTLTNLGAP

AAFKYFDTTIDRKRYTSTKEVLDATLIHQSITGLYETRIDLSQLGGDKRPAATKKAGQAK

KKK

hEMX1 -HR Template-HindII-NheI:

GAATGCTGCCCTCAGACCCGCTTCCTCCCTGTCCTTGTCTGTCCAAGGAGA

ATGAGGTCTCACTGGTGGATTTCGGACTACCCTGAGGAGCTGGCACCTGAGGGACA

AGGCCCCCCACCTGCCCAGCTCCAGCCTCTGATGAGGGGTGGGAGAGAGCTACATG

AGGTTGCTAAGAAAGCCTCCCCTGAAGGAGACCACACAGTGTGTGAGGTTGGAGTC

TCTAGCAGCGGGTTCTGTGCCCCCAGGGATAGTCTGGCTGTCCAGGCACTGCTCTTG

ATATAAACACCACCTCCTAGTTATGAAACCATGCCCATTCTGCCTCTCTGTATGGAA

AAGAGCATGGGGCTGGCCCGTGGGGTGGTGTCCACTTTAGGCCCTGTGGGAGATCA

TGGGAACCCACGCAGTGGGTCATAGGCTCTCTCATTTACTACTCACATCCACTCTGT

GAAGAAGCGATTATGATCTCTCCTCTAGAAACTCGTAGAGTCCCATGTCTGCCGGCT

TCCAGAGCCTGCACTCCTCCACCTTGGCTTGGCTTTGCTGGGGCTAGAGGAGCTAGG

ATGCACAGCAGCTCTGTGACCCTTTGTTTGAGAGGAACAGGAAAACCACCCTTCTCT
CTGGCCCACTGTGTCCTCTTCCTGCCCTGCCATCCCCTTCTGTGAATGTTAGACCCAT
GGGAGCAGCTGGTCAGAGGGGACCCCGGCCTGGGGCCCCTAACCCTATGTAGCCTC
AGTCTTCCCATCAGGCTCTCAGCTCAGCCTGAGTGTTGAGGCCCCAGTGGCTGCTCT
GGGGGCCTCCTGAGTTTCTATCTGTGCCCCTCCCTCCCTGGCCCAGGTGAAGGTGT
GGTTCCAGAACCGGAGGACAAAGTACAAACGGCAGAAGCTGGAGGAGGAAGGGCC
TGAGTCCGAGCAGAAGAAGAAGGGCTCCCATCACATCAACCGGTGGCGCATTGCCA
CGAAGCAGGCCAATGGGGAGGACATCGATGTCACCTCCAATGACaagcttgctagcGGTGG
GCAACCACAAACCCACGAGGGCAGAGTGCTGCTTGCTGCTGGCCAGGCCCCTGCGT
GGGCCCAAGCTGGACTCTGGCCACTCCCTGGCCAGGCTTTGGGGAGGCCTGGAGTC
ATGGCCCCACAGGGCTTGAAGCCCGGGGCCGCCATTGACAGAGGGACAAGCAATGG
GCTGGCTGAGGCCTGGGACCACTTGGCCTTCTCCTCGGAGAGCCTGCCTGCCTGGGC
GGGCCCGCCCGCCACCGCAGCCTCCCAGCTGCTCTCCGTGTCTCCAATCTCCCTTTTG
TTTTGATGCATTTCTGTTTTAATTTATTTTCCAGGCACCACTGTAGTTTAGTGATCCCC
AGTGTCCCCTTCCCTATGGGAATAATAAAGTCTCTCTCTTAATGACACGGGCATC
CAGCTCCAGCCCCAGAGCCTGGGGTGGTAGATTCCGGCTCTGAGGGCCAGTGGGGG
CTGGTAGAGCAAACGCGTTCAGGGCCTGGGAGCCTGGGGTGGGGTACTGGTGGAGG
GGGTCAAGGGTAATTCATTAACTCCTCTCTTTTGTTGGGGGACCCTGGTCTCTACCTC
CAGCTCCACAGCAGGAGAAACAGGCTAGACATAGGGAAGGGCCATCCTGTATCTTG
AGGGAGGACAGGCCCAGGTCTTTCTTAACGTATTGAGAGGTGGGAATCAGGCCCAG
GTAGTTCAATGGGAGAGGGAGAGTGCTTCCCTCTGCCTAGAGACTCTGGTGGCTTCT
CCAGTTGAGGAGAAACCAGAGGAAAGGGGAGGATTGGGGTCTGGGGGAGGGAACA
CCATTCACAAAGGCTGACGGTTCCAGTCCGAAGTCGTGGGCCCACCAGGATGCTCA
CCTGTCCTTGGAGAACCGCTGGGCAGGTTGAGACTGCAGAGACAGGGCTTAAGGCT
GAGCCTGCAACCAGTCCCCAGTGACTCAGGGCCTCCTCAGCCCAAGAAAGAGCAAC
GTGCCAGGGCCCGCTGAGCTCTTGTGTTCACCTG

NLS-StCsn1-NLS:

MKRPAATKKAGQAKKKKSDLVLGLDIGIGSVGVGILNKVTGEIIHKNSRIFPA
AQAENNLVRRTNRQGRRLARRKKHRRVRLNRLFEESGLITDFTKISINLNPYQLRVKGL
TDELSNEELFIALKNMVKHRGISYLDDASDDGNSSVGDYAQIVKENSKQLETKTPGQIQL
ERYQTYGQLRGDFTVEKDGKKHRLINVFPTSAYRSEALRILQTQQEFNPQITDEFINRYL

EILTGKRKYYHGPGNEKSRTDYGRYRTSGETLDNIFGILIGKCTFYPDEFRAAKASYTAQ

EFNLLNDLNNLTVPTETKKLSKEQKNQIINYVKNEKAMGPAKLFKYIAKLLSCDVADIK

GYRIDKSGKAEIHTFEAYRKMKTLETLDIEQMDRETLDKLAYVLTLNTEREGIQEALEHE

FADGSFSQKQVDELVQFRKANSSIFGKGWHNFSVKLMMELIPELYETSEEQMTILTRLG

KQKTTSSSNKTKYIDEKLLTEEIYNPVVAKSVRQAIKIVNAAIKEYGDFDNIVIEMARETN

EDDEKKAIQKIQKANKDEKDAAMLKAANQYNGKAELPHSVFHGHKQLATKIRLWHQQ

GERCLYTGKTISIHDLINNSNQFEVDHILPLSITFDDSLANKVLVYATANQEKGQRTPYQ

ALDSMDDAWSFRELKAFVRESKTLSNKKKEYLLTEEDISKFDVRKKFIERNLVDTRYAS

RVVLNALQEHFRAHKIDTKVSVVRGQFTSQLRRHWGIEKTRDTYHHHAVDALIIAASSQ

LNLWKKQKNTLVSYSEDQLLDIETGELISDDEYKESVFKAPYQHFVDTLKSKEFEDSILF

SYQVDSKFNRKISDATIYATRQAKVGKDKADETYVLGKIKDIYTQDGYDAFMKIYKKD

KSKFLMYRHDPQTFEKVIEPILENYPNKQINEKGKEVPCNPFLKYKEEHGYIRKYSKKGN

GPEIKSLKYYDSKLGNHIDITPKDSNNKVVLQSVSPWRADVYFNKTTGKYEILGLKYAD

LQFEKGTGTYKISQEKYNDIKKKEGVDSDSEFKFTLYKNDLLLVKDTETKEQQLFRFLSR

TMPKQKHYVELKPYDKQKFEGGEALIKVLGNVANSGQCKKGLGKSNISIYKVRTDVLG

NQHIIKNEGDKPKLDFKRPAATKKAGQAKKKK

U6-St_tracrRNA(7-97):

GAGGGCCTATTTCCCATGATTCCTTCATATTTGCATATACGATACAAGGCT
GTTAGAGAGATAATTGGAATTAATTTGACTGTAAACACAAAGATATTAGTACAAAA
TACGTGACGTAGAAAGTAATAATTTCTTGGGTAGTTTGCAGTTTTAAAATTATGTTTT
AAAATGGACTATCATATGCTTACCGTAACTTGAAAGTATTTCGATTTCTTGGCTTTAT
ATATCTTGTGGAAAGGACGAAACACCGTTACTTAAATCTTGCAGAAGCTACAAAGA
TAAGGCTTCATGCCGAAATCAACACCCTGTCATTTTATGGCAGGGTGTTTTCGTTATT
TAA

U6-DR-spacer-DR (S. pyogenes SF370)

gagggcctatttcccatgattccttcatatttgcatatacgatacaaggctgttagagagataattggaattaatttgactgtaaa
cacaaagatattagtacaaaatacgtgacgtagaaagtaataatttcttgggtagtttgcagttttaaaattatgttttaaaatggactatcatatgc
ttaccgtaacttgaaagtatttcgatttcttggctttatatatcttgtggaaaggacgaaacacc<u>gggttttagagctatgctgttttgaatggtccc
aaaac</u>NNNNNNNNNNNNNNNNNNNNNNNNNNNNNNNN<u>gttttagagctatgctgttttgaatggtcccaaaac</u>**T
TTTTT** (lowercase underline = direct repeat; N = guide sequence; bold = terminator)

Chimeric RNA containing +48 tracr RNA (S. pyogenes SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacaccNNNNNNNNNNNNNNNNNNN NNgttttagagctagaaatagcaagttaaaataaggctagtccgTTTTTTT (N = guide sequence; first underline = tracr mate sequence; second underline = tracr sequence; bold = terminator)

Chimeric RNA containing +54 tracr RNA (S. pyogenes SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacaccNNNNNNNNNNNNNNNNNNN NNgttttagagctagaaatagcaagttaaaataaggctagtccgttatcaTTTTTTTT (N = guide sequence; first underline = tracr mate sequence; second underline = tracr sequence; bold = terminator)

Chimeric RNA containing +67 tracr RNA (S. pyogenes SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacaccNNNNNNNNNNNNNNNNNNN NNgttttagagctagaaatagcaagttaaaataaggctagtccgttatcaacttgaaaaagtgTTTTTTT (N = guide sequence; first underline = tracr mate sequence; second underline = tracr sequence; bold = terminator)

Chimeric RNA containing +85 tracr RNA (S. pyogenes SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacaccNNNNNNNNNNNNNNNNNNN NNgttttagagctagaaatagcaagttaaaataaggctagtccgttatcaacttgaaaaagtggcaccgagtcggtgcTTTTTTT (N = guide sequence; first underline = tracr mate sequence; second underline = tracr sequence; bold = terminator)

CBh-NLS-SpCas9-NLS

CGTTACATAACTTACGGTAAATGGCCCGCCTGGCTGACCGCCCAACGACC
CCCGCCCATTGACGTCAATAATGACGTATGTTCCCATAGTAACGCCAATAGGGACTT
TCCATTGACGTCAATGGGTGGAGTATTTACGGTAAACTGCCCACTTGGCAGTACATC
AAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTA

CGTATTAGTCATCGCTATTACCATGGTCGAGGTGAGCCCCACGTTCTGCTTCACTCTC

CCCATCTCCCCCCCCTCCCCACCCCCAATTTTGTATTTATTTATTTTTAATTATTTTG

TGCAGCGATGGGGGCGGGGGGGGGGGGGGGGCGCGCGCCAGGCGGGGCGGGGCGG

GGCGAGGGGCGGGGCGGGGCGAGGCGGAGAGGTGCGGCGGCAGCCAATCAGAGCG

GCGCGCTCCGAAAGTTTCCTTTTATGGCGAGGCGGCGGCGGCGGCGGCCCTATAAA

AAGCGAAGCGCGCGGCGGGCGGGAGTCGCTGCGACGCTGCCTTCGCCCCGTGCCCC

GCTCCGCCGCCGCCTCGCGCCGCCCGCCCCGGCTCTGACTGACCGCGTTACTCCCAC

AGGTGAGCGGGCGGGACGGCCCTTCTCCTCCGGGCTGTAATTAGCTGAGCAAGAGG

TAAGGGTTTAAGGGATGGTTGGTTGGTGGGGTATTAATGTTTAATTACCTGGAGCAC

CTGCCTGAAATCACTTTTTTTCAGGTTGGaccggtgccacc<u>ATGGACTATAAGGACCACGA</u>

<u>CGGAGACTACAAGGATCATGATATTGATTACAAAGACGATGACGATAAGATGGCCC</u>

<u>CAAAGAAGAAGCGGAAGGTCGGTATCCACGGAGTCCCAGCAGCCGACAAGAAGTA</u>

<u>CAGCATCGGCCTGGACATCGGCACCAACTCTGTGGGCTGGGCCGTGATCACCGACG</u>

<u>AGTACAAGGTGCCCAGCAAGAAATTCAAGGTGCTGGGCAACACCGACCGGCACAGC</u>

<u>ATCAAGAAGAACCTGATCGGAGCCCTGCTGTTCGACAGCGGCGAAACAGCCGAGGC</u>

<u>CACCCGGCTGAAGAGAACCGCCAGAAGAAGATACACCAGACGGAAGAACCGGATC</u>

<u>TGCTATCTGCAAGAGATCTTCAGCAACGAGATGGCCAAGGTGGACGACAGCTTCTTC</u>

<u>CACAGACTGGAAGAGTCCTTCCTGGTGGAAGAGGATAAGAAGCACGAGCGGCACCC</u>

<u>CATCTTCGGCAACATCGTGGACGAGGTGGCCTACCACGAGAAGTACCCCACCATCT</u>

<u>ACCACCTGAGAAAGAAACTGGTGGACAGCACCGACAAGGCCGACCTGCGGCTGATC</u>

<u>TATCTGGCCCTGGCCCACATGATCAAGTTCCGGGGCCACTTCCTGATCGAGGGCGAC</u>

<u>CTGAACCCCGACAACAGCGACGTGGACAAGCTGTTCATCCAGCTGGTGCAGACCTA</u>

<u>CAACCAGCTGTTCGAGGAAAACCCCATCAACGCCAGCGGCGTGGACGCCAAGGCCA</u>

<u>TCCTGTCTGCCAGACTGAGCAAGAGCAGACGGCTGGAAAATCTGATCGCCCAGCTG</u>

<u>CCCGGCGAGAAGAAGAATGGCCTGTTCGGCAACCTGATTGCCCTGAGCCTGGGCCT</u>

<u>GACCCCCAACTTCAAGAGCAACTTCGACCTGGCCGAGGATGCCAAACTGCAGCTGA</u>

<u>GCAAGGACACCTACGACGACGACCTGGACAACCTGCTGGCCCAGATCGGCGACCAG</u>

<u>TACGCCGACCTGTTTCTGGCCGCCAAGAACCTGTCCGACGCCATCCTGCTGAGCGAC</u>

<u>ATCCTGAGAGTGAACACCGAGATCACCAAGGCCCCCCTGAGCGCCTCTATGATCAA</u>

<u>GAGATACGACGAGCACCACCAGGACCTGACCCTGCTGAAAGCTCTCGTGCGGCAGC</u>

<u>AGCTGCCTGAGAAGTACAAAGAGATTTTCTTCGACCAGAGCAAGAACGGCTACGCC</u>

GGCTACATTGACGGCGGAGCCAGCCAGGAAGAGTTCTACAAGTTCATCAAGCCCAT
CCTGGAAAAGATGGACGGCACCGAGGAACTGCTCGTGAAGCTGAACAGAGAGGAC
CTGCTGCGGAAGCAGCGGACCTTCGACAACGGCAGCATCCCCCACCAGATCCACCT
GGGAGAGCTGCACGCCATTCTGCGGCGGCAGGAAGATTTTTACCCATTCCTGAAGG
ACAACCGGGAAAAGATCGAGAAGATCCTGACCTTCCGCATCCCCTACTACGTGGGC
CCTCTGGCCAGGGGAAACAGCAGATTCGCCTGGATGACCAGAAAGAGCGAGGAAA
CCATCACCCCCTGGAACTTCGAGGAAGTGGTGGACAAGGGCGCTTCCGCCCAGAGC
TTCATCGAGCGGATGACCAACTTCGATAAGAACCTGCCCAACGAGAAGGTGCTGCC
CAAGCACAGCCTGCTGTACGAGTACTTCACCGTGTATAACGAGCTGACCAAAGTGA
AATACGTGACCGAGGGAATGAGAAAGCCCGCCTTCCTGAGCGGCGAGCAGAAAAA
GGCCATCGTGGACCTGCTGTTCAAGACCAACCGGAAAGTGACCGTGAAGCAGCTGA
AAGAGGACTACTTCAAGAAAATCGAGTGCTTCGACTCCGTGGAAATCTCCGGCGTG
GAAGATCGGTTCAACGCCTCCCTGGGCACATACCACGATCTGCTGAAAATTATCAAG
GACAAGGACTTCCTGGACAATGAGGAAACGAGGACATTCTGGAAGATATCGTGCT
GACCCTGACACTGTTTGAGGACAGAGAGATGATCGAGGAACGGCTGAAAACCTATG
CCCACCTGTTCGACGACAAAGTGATGAAGCAGCTGAAGCGGCGGAGATACACCGGC
TGGGGCAGGCTGAGCCGGAAGCTGATCAACGGCATCCGGGACAAGCAGTCCGGCAA
GACAATCCTGGATTTCCTGAAGTCCGACGGCTTCGCCAACAGAAACTTCATGCAGCT
GATCCACGACGACAGCCTGACCTTTAAAGAGGACATCCAGAAAGCCCAGGTGTCCG
GCCAGGGCGATAGCCTGCACGAGCACATTGCCAATCTGGCCGGCAGCCCCGCCATT
AAGAAGGGCATCCTGCAGACAGTGAAGGTGGTGGACGAGCTCGTGAAAGTGATGGG
CCGGCACAAGCCCGAGAACATCGTGATCGAAATGGCCAGAGAGAACCAGACCACCC
AGAAGGGACAGAAGAACAGCCGCGAGAGAATGAAGCGGATCGAAGAGGGCATCAA
AGAGCTGGGCAGCCAGATCCTGAAAGAACACCCGTGGAAAACACCCAGCTGCAGA
ACGAGAAGCTGTACCTGTACTACCTGCAGAATGGGCGGGATATGTACGTGGACCAG
GAACTGGACATCAACCGGCTGTCCGACTACGATGTGGACCATATCGTGCCTCAGAG
CTTTCTGAAGGACGACTCCATCGACAACAAGGTGCTGACCAGAAGCGACAAGAACC
GGGGCAAGAGCGACAACGTGCCCTCCGAAGAGGTCGTGAAGAAGATGAAGAACTA
CTGGCGGCAGCTGCTGAACGCCAAGCTGATTACCCAGAGAAAGTTCGACAATCTGA
CCAAGGCCGAGAGAGGCGGCCTGAGCGAACTGGATAAGGCCGGCTTCATCAAGAG
ACAGCTGGTGGAAACCCGGCAGATCACAAAGCACGTGGCACAGATCCTGGACTCCC

GGATGAACACTAAGTACGACGAGAATGACAAGCTGATCCGGGAAGTGAAAGTGATC
ACCCTGAAGTCCAAGCTGGTGTCCGATTTCCGGAAGGATTTCCAGTTTTACAAAGTG
CGCGAGATCAACAACTACCACCACGCCCACGACGCCTACCTGAACGCCGTCGTGGG
AACCGCCCTGATCAAAAAGTACCCTAAGCTGGAAAGCGAGTTCGTGTACGGCGACT
ACAAGGTGTACGACGTGCGGAAGATGATCGCCAAGAGCGAGCAGGAAATCGGCAA
GGCTACCGCCAAGTACTTCTTCTACAGCAACATCATGAACTTTTTCAAGACCGAGAT
TACCCTGGCCAACGGCGAGATCCGGAAGCGGCCTCTGATCGAGACAAACGGCGAAA
CCGGGGAGATCGTGTGGGATAAGGGCCGGGATTTTGCCACCGTGCGGAAAGTGCTG
AGCATGCCCCAAGTGAATATCGTGAAAAAGACCGAGGTGCAGACAGGCGGCTTCAG
CAAAGAGTCTATCCTGCCCAAGAGGAACAGCGATAAGCTGATCGCCAGAAAGAAGG
ACTGGGACCCTAAGAAGTACGGCGGCTTCGACAGCCCCACCGTGGCCTATTCTGTGC
TGGTGGTGGCCAAAGTGGAAAAGGGCAAGTCCAAGAAACTGAAGAGTGTGAAAGA
GCTGCTGGGGATCACCATCATGGAAAGAAGCAGCTTCGAGAAGAATCCCATCGACT
TTCTGGAAGCCAAGGGCTACAAAGAAGTGAAAAAGGACCTGATCATCAAGCTGCCT
AAGTACTCCCTGTTCGAGCTGGAAAACGGCCGGAAGAGAATGCTGGCCTCTGCCGG
CGAACTGCAGAAGGGAAACGAACTGGCCCTGCCCTCCAAATATGTGAACTTCCTGT
ACCTGGCCAGCCACTATGAGAAGCTGAAGGGCTCCCCCGAGGATAATGAGCAGAAA
CAGCTGTTTGTGGAACAGCACAAGCACTACCTGGACGAGATCATCGAGCAGATCAG
CGAGTTCTCCAAGAGAGTGATCCTGGCCGACGCTAATCTGGACAAAGTGCTGTCCGC
CTACAACAAGCACCGGGATAAGCCCATCAGAGAGCAGGCCGAGAATATCATCCACC
TGTTTACCCTGACCAATCTGGGAGCCCCTGCCGCCTTCAAGTACTTTGACACCACCA
TCGACCGGAAGAGGTACACCAGCACCAAAGAGGTGCTGGACGCCACCCTGATCCAC
CAGAGCATCACCGGCCTGTACGAGACACGGATCGACCTGTCTCAGCTGGGAGGCGA
CTTTCTTTTTCTTAGCTTGACCAGCTTTCTTAGTAGCAGCAGGACGCTTAA (underline
= NLS-hSpCas9-NLS)

Example chimeric RNA for S. thermophilus LMD-9 CRISPR1 Cas9 (with PAM of NNAGAAW)

NNNNNNNNNNNNNNNNNNNNgtttttgtactctcaagatttaGAAAtaaatcttgcagaagctacaa
agataaggcttcatgccgaaatcaacaccctgtcattttatggcagggtgttttcgttatttaaTTTTTT (N = guide sequence;
first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNNNgttttgtactctcaGAAAtgcagaagctacaaagataaggcttca tgccgaaatcaacaccctgtcattttatggcagggtgttttcgttatttaa**TTTTTT** (N = guide sequence; first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNNNgttttgtactctcaGAAAtgcagaagctacaaagataaggcttca tgccgaaatcaacaccctgtcattttatggcagggtgt**TTTTTT** (N = guide sequence; first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNNNgttattgtactctcaagatttaGAAAtaaatcttgcagaagctacaa agataaggcttcatgccgaaatcaacaccctgtcattttatggcagggtgttttcgttatttaa**TTTTTT** (N = guide sequence; first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNNNgttattgtactctcaGAAAtgcagaagctacaaagataaggcttca tgccgaaatcaacaccctgtcattttatggcagggtgttttcgttatttaa**TTTTTT** (N = guide sequence; first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNNNgttattgtactctcaGAAAtgcagaagctacaaagataaggcttca tgccgaaatcaacaccctgtcattttatggcagggtgt**TTTTTT** (N = guide sequence; first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNNNgttattgtactctcaagatttaGAAAtaaatcttgcagaagctacaa tgataaggcttcatgccgaaatcaacaccctgtcattttatggcagggtgttttcgttatttaa**TTTTTT** (N = guide sequence; first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNgttattgtactctcaGAAAtgcagaagctacaatgataaggcttca tgccgaaatcaacaccctgtcattttatggcagggtgttttcgttatttaa**TTTTTT** (N = guide sequence; first underline = tracr mate sequence; second underline = tracr sequence; bold = terminator)

Example chimeric RNA for S. thermophilus LMD-9 CRISPR1 Cas9 (with PAM of NNAGAAW)

NNNNNNNNNNNNNNNNNNNNgttattgtactctcaGAAAtgcagaagctacaatgataaggcttca tgccgaaatcaacaccctgtcattttatggcagggtgt**TTTTTT** (N = guide sequence; first underline = tracr mate sequence; second underline = tracr sequence; bold = terminator)

Example chimeric RNA for S. thermophilus LMD-9 CRISPR3 Cas9 (with PAM of NGGNG)

NNNNNNNNNNNNNNNNNNNNgttttagagctgtgGAAAcacagcgagttaaaataaggcttagtc cgtactcaacttgaaaaggtggcaccgattcggtgt**TTTTTT** (N = guide sequence; first underline = tracr mate sequence; second underline = tracr sequence; bold = terminator)

Codon-optimized version of Cas9 from S. thermophilus LMD-9 CRISPR3 locus (with an NLS at both 5' and 3' ends)

ATGAAAAGGCCGGCGGCCACGAAAAAGGCCGGCCAGGCAAAAAAGAAA AAGACCAAGCCCTACAGCATCGGCCTGGACATCGGCACCAATAGCGTGGGCTGGGC CGTGACCACCGACAACTACAAGGTGCCCAGCAAGAAAATGAAGGTGCTGGGCAACA CCTCCAAGAAGTACATCAAGAAAAACCTGCTGGGCGTGCTGCTGTTCGACAGCGGC ATTACAGCCGAGGGCAGACGGCTGAAGAGAACCGCCAGACGGCGGTACACCCGGC GGAGAAACAGAATCCTGTATCTGCAAGAGATCTTCAGCACCGAGATGGCTACCCTG GACGACGCCTTCTTCCAGCGGCTGGACGACAGCTTCCTGGTGCCCGACGACAAGCG GGACAGCAAGTACCCCATCTTCGGCAACCTGGTGGAAGAGAAGGCCTACCACGACG AGTTCCCCACCATCTACCACCTGAGAAAGTACCTGGCCGACAGCACCAAGAAGGCC GACCTGAGACTGGTGTATCTGGCCCTGGCCCACATGATCAAGTACCGGGGCCACTTC CTGATCGAGGGCGAGTTCAACAGCAAGAACAACGACATCCAGAAGAACTTCCAGGA CTTCCTGGACACCTACAACGCCATCTTCGAGAGCGACCTGTCCCTGGAAAACAGCAA GCAGCTGGAAGAGATCGTGAAGGACAAGATCAGCAAGCTGGAAAAGAAGGACCGC ATCCTGAAGCTGTTCCCCGGCGAGAAGAACAGCGGAATCTTCAGCGAGTTTCTGAA

GCTGATCGTGGGCAACCAGGCCGACTTCAGAAAGTGCTTCAACCTGGACGAGAAAG
CCAGCCTGCACTTCAGCAAAGAGAGCTACGACGAGGACCTGGAAACCCTGCTGGGA
TATATCGGCGACGACTACAGCGACGTGTTCCTGAAGGCCAAGAAGCTGTACGACGC
TATCCTGCTGAGCGGCTTCCTGACCGTGACCGACAACGAGACAGAGGCCCCACTGA
GCAGCGCCATGATTAAGCGGTACAACGAGCACAAAGAGGATCTGGCTCTGCTGAAA
GAGTACATCCGGAACATCAGCCTGAAAACCTACAATGAGGTGTTCAAGGACGACAC
CAAGAACGGCTACGCCGGCTACATCGACGGCAAGACCAACCAGGAAGATTTCTATG
TGTACCTGAAGAAGCTGCTGGCCGAGTTCGAGGGGGCCGACTACTTTCTGGAAAAA
ATCGACCGCGAGGATTTCCTGCGGAAGCAGCGGACCTTCGACAACGGCAGCATCCC
CTACCAGATCCATCTGCAGGAAATGCGGGCCATCCTGGACAAGCAGGCCAAGTTCT
ACCCATTCCTGGCCAAGAACAAAGAGCGGATCGAGAAGATCCTGACCTTCCGCATC
CCTTACTACGTGGGCCCCCTGGCCAGAGGCAACAGCGATTTTGCCTGGTCCATCCGG
AAGCGCAATGAGAAGATCACCCCCTGGAACTTCGAGGACGTGATCGACAAAGAGTC
CAGCGCCGAGGCCTTCATCAACCGGATGACCAGCTTCGACCTGTACCTGCCCGAGG
AAAAGGTGCTGCCCAAGCACAGCCTGCTGTACGAGACATTCAATGTGTATAACGAG
CTGACCAAAGTGCGGTTTATCGCCGAGTCTATGCGGGACTACCAGTTCCTGGACTCC
AAGCAGAAAAAGGACATCGTGCGGCTGTACTTCAAGGACAAGCGGAAAGTGACCG
ATAAGGACATCATCGAGTACCTGCACGCCATCTACGGCTACGATGGCATCGAGCTG
AAGGGCATCGAGAAGCAGTTCAACTCCAGCCTGAGCACATACCACGACCTGCTGAA
CATTATCAACGACAAAGAATTTCTGGACGACTCCAGCAACGAGGCCATCATCGAAG
AGATCATCCACACCCTGACCATCTTTGAGGACCGCGAGATGATCAAGCAGCGGCTG
AGCAAGTTCGAGAACATCTTCGACAAGAGCGTGCTGAAAAAGCTGAGCAGACGGCA
CTACACCGGCTGGGGCAAGCTGAGCGCCAAGCTGATCAACGGCATCCGGGACGAGA
AGTCCGGCAACACAATCCTGGACTACCTGATCGACGACGGCATCAGCAACCGGAAC
TTCATGCAGCTGATCCACGACGACGCCCTGAGCTTCAAGAAGAAGATCCAGAAGGC
CCAGATCATCGGGGACGAGGACAAGGGCAACATCAAAGAAGTCGTGAAGTCCCTGC
CCGGCAGCCCCGCCATCAAGAAGGGAATCCTGCAGAGCATCAAGATCGTGGACGAG
CTCGTGAAAGTGATGGGCGGCAGAAAGCCCGAGAGCATCGTGGTGGAAATGGCTAG
AGAGAACCAGTACACCAATCAGGGCAAGAGCAACAGCCAGCAGAGACTGAAGAGA
CTGGAAAAGTCCCTGAAAGAGCTGGGCAGCAAGATTCTGAAAGAGAATATCCCTGC
CAAGCTGTCCAAGATCGACAACAACGCCCTGCAGAACGACCGGCTGTACCTGTACT

ACCTGCAGAATGGCAAGGACATGTATACAGGCGACGACCTGGATATCGACCGCCTG
AGCAACTACGACATCGACCATATTATCCCCCAGGCCTTCCTGAAAGACAACAGCATT
GACAACAAAGTGCTGGTGTCCTCCGCCAGCAACCGCGGCAAGTCCGATGATGTGCC
CAGCCTGGAAGTCGTGAAAAAGAGAAAGACCTTCTGGTATCAGCTGCTGAAAAGCA
AGCTGATTAGCCAGAGGAAGTTCGACAACCTGACCAAGGCCGAGAGAGGCGGCCTG
AGCCCTGAAGATAAGGCCGGCTTCATCCAGAGACAGCTGGTGGAAACCCGGCAGAT
CACCAAGCACGTGGCCAGACTGCTGGATGAGAAGTTTAACAACAAGAAGGACGAGA
ACAACCGGGCCGTGCGGACCGTGAAGATCATCACCCTGAAGTCCACCCTGGTGTCC
CAGTTCCGGAAGGACTTCGAGCTGTATAAAGTGCGCGAGATCAATGACTTTCACCAC
GCCCACGACGCCTACCTGAATGCCGTGGTGGCTTCCGCCCTGCTGAAGAAGTACCCT
AAGCTGGAACCCGAGTTCGTGTACGGCGACTACCCCAAGTACAACTCCTTCAGAGA
GCGGAAGTCCGCCACCGAGAAGGTGTACTTCTACTCCAACATCATGAATATCTTTAA
GAAGTCCATCTCCCTGGCCGATGGCAGAGTGATCGAGCGGCCCCTGATCGAAGTGA
ACGAAGAGACAGGCGAGAGCGTGTGGAACAAAGAAAGCGACCTGGCCACCGTGCG
GCGGGTGCTGAGTTATCCTCAAGTGAATGTCGTGAAGAAGGTGGAAGAACAGAACC
ACGGCCTGGATCGGGGCAAGCCCAAGGGCCTGTTCAACGCCAACCTGTCCAGCAAG
CCTAAGCCCAACTCCAACGAGAATCTCGTGGGGGCCAAAGAGTACCTGGACCCTAA
GAAGTACGGCGGATACGCCGGCATCTCCAATAGCTTCACCGTGCTCGTGAAGGGCA
CAATCGAGAAGGGCGCTAAGAAAAGATCACAAACGTGCTGGAATTTCAGGGGATC
TCTATCCTGGACCGGATCAACTACCGGAAGGATAAGCTGAACTTTCTGCTGGAAAAA
GGCTACAAGGACATTGAGCTGATTATCGAGCTGCCTAAGTACTCCCTGTTCGAACTG
AGCGACGGCTCCAGACGGATGCTGGCCTCCATCCTGTCCACCAACAACAAGCGGGG
CGAGATCCACAAGGGAAACCAGATCTTCCTGAGCCAGAAATTTGTGAAACTGCTGT
ACCACGCCAAGCGGATCTCCAACACCATCAATGAGAACCACCGGAAATACGTGGAA
AACCACAAGAAAGAGTTTGAGGAACTGTTCTACTACATCCTGGAGTTCAACGAGAA
CTATGTGGGAGCCAAGAAGAACGGCAAACTGCTGAACTCCGCCTTCCAGAGCTGGC
AGAACCACAGCATCGACGAGCTGTGCAGCTCCTTCATCGGCCCTACCGGCAGCGAG
CGGAAGGGACTGTTTGAGCTGACCTCCAGAGGCTCTGCCGCCGACTTTGAGTTCCTG
GGAGTGAAGATCCCCCGGTACAGAGACTACACCCCCTCTAGTCTGCTGAAGGACGC
CACCCTGATCCACCAGAGCGTGACCGGCCTGTACGAAACCCGGATCGACCTGGCTA

AGCTGGGCGAGGGAAAGCGTCCTGCTGCTACTAAGAAAGCTGGTCAAGCTAAGAAA
AAGAAATAA

*Example 5: RNA-guided editing of bacterial genomes using CRISPR-Cas systems*

Applicants used the CRISPR-associated endonuclease Cas9 to introduce precise mutations in the genomes of *Streptococcus pneumoniae* and *Escherichia coli.* The approach relied on Cas9-directed cleavage at the targeted site to kill unmutated cells and circumvented the need for selectable markers or counter-selection systems. Cas9 specificity was reprogrammed by changing the sequence of short CRISPR RNA (crRNA) to make single- and multi-nucleotide changes carried on editing templates. Simultaneous use of two crRNAs enabled multiplex mutagenesis. In *S. pneumoniae,* nearly 100% of cells that survived Cas9 cleavage contained the desired mutation, and 65% when used in combination with recombineering in *E. coli.* Applicants exhaustively analyzed Cas9 target requirements to define the range of targetable sequences and showed strategies for editing sites that do not meet these requirements, suggesting the versatility of this technique for bacterial genome engineering.

The understanding of gene function depends on the possibility of altering DNA sequences within the cell in a controlled fashion. Site-specific mutagenesis in eukaryotes is achieved by the use of sequence-specific nucleases that promote homologous recombination of a template DNA containing the mutation of interest. Zinc finger nucleases (ZFNs), transcription activator-like effector nucleases (TALENs) and homing meganucleases can be programmed to cleave genomes in specific locations, but these approaches require engineering of new enzymes for each target sequence. In prokaryotic organisms, mutagenesis methods either introduce a selection marker in the edited locus or require a two-step process that includes a counter-selection system. More recently, phage recombination proteins have been used for recombineering, a technique that promotes homologuous recombination of linear DNA or oligonucleotides. However, because there is no selection of mutations, recombineering efficiency can be relatively low (0.1-10% for point mutations down to $10^{-5}$-$10^{-6}$ for larger modifications), in many cases requiring the screening of a large number of colonies. Therefore new technologies that are affordable, easy to use and efficient are still in need for the genetic engineering of both eukaryotic and prokaryotic organisms.

Recent work on the CRISPR (clustered, regularly interspaced, short palindromic repeats) adaptive immune system of prokaryotes has led to the identification of nucleases whose sequence specificity is programmed by small RNAs. CRISPR loci are composed of a series of repeats separated by 'spacer' sequences that match the genomes of bacteriophages and other mobile genetic elements. The repeat-spacer array is transcribed as a long precursor and processed within repeat sequences to generate small crRNA that specify the target sequences (also known as protospacers) cleaved by CRISPR systems. Essential for cleavage is the presence of a sequence motif immediately downstream of the target region, known as the protospacer-adjacent motif (PAM). CRISPR-associated (*cas*) genes usually flank the repeat-spacer array and encode the enzymatic machinery responsible for crRNA biogenesis and targeting. Cas9 is a dsDNA endonuclease that uses a crRNA guide to specify the site of cleavage. Loading of the crRNA guide onto Cas9 occurs during the processing of the crRNA precursor and requires a small RNA antisense to the precursor, the tracrRNA, and RNAse III. In contrast to genome editing with ZFNs or TALENs, changing Cas9 target specificity does not require protein engineering but only the design of the short crRNA guide.

Applicants recently showed in *S. pneumoniae* that the introduction of a CRISPR system targeting a chromosomal locus leads to the killing of the transformed cells. It was observed that occasional survivors contained mutations in the target region, suggesting that Cas9 dsDNA endonuclease activity against endogenous targets could be used for genome editing. Applicants showed that marker-less mutations can be introduced through the transformation of a template DNA fragment that will recombine in the genome and eliminate Cas9 target recognition. Directing the specificity of Cas9 with several different crRNAs allows for the introduction of multiple mutations at the same time. Applicants also characterized in detail the sequence requirements for Cas9 targeting and show that the approach can be combined with recombineering for genome editing in *E. coli.*

RESULTS: Genome editing by Cas9 cleavage of a chromosomal target

*S. pneumoniae* strain crR6 contains a Cas9-based CRISPR system that cleaves a target sequence present in the bacteriophage φ8232.5. This target was integrated into the *srtA* chromosomal locus of a second strain R6$^{8232.5}$. An altered target sequence containing a mutation in the PAM region was integrated into the *srtA* locus of a third strain R6$^{370.1}$, rendering this strain 'immune' to CRISPR cleavage (Figure 28a). Applicants transformed R6$^{8232.5}$ and R6$^{370.1}$ cells with genomic DNA from crR6 cells, expecting that successful transformation of R6$^{8232.5}$ cells should lead to cleavage of the target locus and cell death. Contrary to this expectation, Applicants isolated R6$^{8232.5}$ transformants, albeit with approximately 10-fold less efficiency than R6$^{370.1}$ transformants (Figure 28b). Genetic analysis of eight R6$^{8232.5}$ transformants (Figure 28) revealed that the great majority are the product of a double recombination event that eliminates the toxicity of Cas9 targeting by replacing the φ8232.5 target with the crR6 genome's wild-type *srtA* locus, which does not contain the protospacer required for Cas9 recognition. These results were proof that the concurrent introduction of a CRISPR system targeting a genomic locus (the targeting construct) together with a template for recombination into the targeted locus (the editing template) led to targeted genome editing (Figure 23a).

To create a simplified system for genome editing, Applicants modified the CRISPR locus in strain crR6 by deleting *cas1*, *cas2* and *csn2*, genes which have been shown to be dispensable for CRISPR targeting, yielding strain crR6M (Figure 28a). This strain retained the same properties of crR6 (Figure 28b). To increase the efficiency of Cas9-based editing and demonstrate that a template DNA of choice can be used to control the mutation introduced, Applicants co-transformed R6$^{8232.5}$ cells with PCR products of the wild-type *srtA* gene or the mutant R6$^{370.1}$ target, either of which should be resistant to cleavage by Cas9. This resulted in a 5-to 10-fold increase of the frequency of transformation compared with genomic crR6 DNA alone (Figure 23b). The efficiency of editing was also substantially increased, with 8/8 transformants tested containing a wild-type *srtA* copy and 7/8 containing the PAM mutation present in the R6$^{370.1}$ target (Figure 23b and Figure 29a). Taken together, these results showed the potential of genome editing assisted by Cas9.

**Analysis of Cas9 target requirements:** To introduce specific changes in the genome, one must use an editing template carrying mutations that abolish Cas9-mediated cleavage, thereby preventing cell death. This is easy to achieve when the deletion of the target or its replacement by another sequence (gene insertion) is sought. When the goal is to produce gene fusions or to generate single-nucleotide mutations, the abolishment of Cas9 nuclease activity will only be possible by introducing mutations in the editing template that alter either the PAM or the protospacer sequences. To determine the constraints of CRISPR-mediated editing, Applicants performed an exhaustive analysis of PAM and protospacer mutations that abrogate CRISPR targeting.

Previous studies proposed that *S. pyogenes* Cas9 requires an NGG PAM immediately downstream of the protospacer. However, because only a very limited number of PAM-inactivating mutations have been described so far, Applicants conducted a systematic analysis to find all 5-nucleotide sequences following the protospacer that eliminate CRISPR cleavage. Applicants used randomized oligonucleotides to generate all possible 1,024 PAM sequences in a heterogeneous PCR product that was transformed into crR6 or R6 cells. Constructs carrying functional PAMs were expected to be recognized and destroyed by Cas9 in crR6 but not R6 cells (Figure 24a). More than $2\times10^5$ colonies were pooled together to extract DNA for use as template for the co-amplification of all targets. PCR products were deep sequenced and found to contain all 1,024 sequences, with coverage ranging from 5 to 42,472 reads (See section "Analysis of deep sequencing data"). The functionality of each PAM was estimated by the relative proportion of its reads in the crR6 sample over the R6 sample. Analysis of the first three bases of the PAM, averaging over the two last bases, clearly showed that the NGG pattern was under-represented in crR6 transformants (Figure 24b). Furthermore, the next two bases had no detectable effect on the NGG PAM (See section "Analysis of deep sequencing data"), demonstrating that the NGGNN sequence was sufficient to license Cas9 activity. Partial targeting was observed for NAG PAM sequences (Figure 24b). Also the NNGGN pattern partially inactivated CRISPR targeting (Table G), indicating that the NGG motif can still be recognized by Cas9 with reduced efficiency when shifted by 1 bp. These data shed light onto the molecular mechanism of Cas9 target recognition, and they revealed that NGG (or CCN on the complementary strand) sequences are sufficient for Cas9 targeting and that NGG to NAG or NNGGN mutations in the editing template should be avoided. Owing to the high frequency of these tri-nucleotide sequences (once every 8 bp), this means that almost any position of the genome can be edited. Indeed, Applicants tested ten randomly chosen targets carrying various PAMs and all were found to be functional (Figure 30).

Another way to disrupt Cas9-mediated cleavage is to introduce mutations in the protospacer region of the editing template. It is known that point mutations within the 'seed sequence' (the 8 to 10 protospacer nucleotides immediately adjacent to the PAM) can abolish cleavage by CRISPR nucleases. However, the exact length of this region is not known, and it is unclear whether mutations to any nucleotide in the seed can disrupt Cas9 target recognition. Applicants followed the same deep sequencing approach described above to randomize the entire protospacer sequence involved in base pair contacts with the crRNA and to determine all sequences that disrupt targeting. Each position of the 20 matching nucleotides (14) in the *spcl* target present in R6$^{8232.5}$ cells (Figure 23a) was randomized and transformed into crR6 and R6 cells (Figure 24a). Consistent with the presence of a seed sequence, only mutations in the 12 nucleotides immediately upstream of the PAM abrogated cleavage by Cas9 (Figure 24c). However, different mutations displayed markedly different effects. The distal (from the PAM) positions of the seed (12 to 7) tolerated most mutations and only one particular base substitution abrogated targeting. In contrast, mutations to any nucleotide in the proximal positions (6 to 1, except 3) eliminated Cas9 activity, although at different levels for each particular substitution. At position 3, only two substitutions affected CRISPR activity and with different strength. Applicants concluded that, although seed sequence mutations can prevent CRISPR targeting, there are restrictions regarding the nucleotide changes that can be made in each position of the seed. Moreover, these restrictions can most likely vary for different spacer sequences. Therefore Applicants believe that mutations in the PAM sequence, if possible, should be the preferred editing strategy. Alternatively, multiple mutations in the seed sequence may be introduced to prevent Cas9 nuclease activity.

**Cas9-mediated genome editing in *S. pneumonia*:** To develop a rapid and efficient method for targeted genome editing, Applicants engineered strain crR6Rk, a strain in which spacers can be easily introduced by PCR (Figure 33). Applicants decided to edit the β-galactosidase (*bgaA*) gene of *S. pneumoniae,* whose activity can be easily measured. Applicants introduced alanine substitutions of amino acids in the active site of this enzyme: R481A (R→A) and N563A,E564A (NE→AA) mutations. To illustrate different editing strategies, Applicants designed mutations of both the PAM sequence and the protospacer seed. In both cases the same targeting construct with a crRNA complementary to a region of the β-galactosidase gene that is adjacent to a TGG PAM sequence (CCA in the complementary strand, Figure 26) was used. The R→A editing template created a three-nucleotide mismatch on the protospacer seed sequence (CGT to GCA, also introducing a BtgZI restriction site). In the NE→AA editing template Applicants simultaneously introduced a synonymous mutation that created an inactive PAM (TGG to TTG) along with mutations that are 218 nt downstream of the protospacer region (AAT GAA to GCT GCA, also generating a TseI restriction site). This last editing strategy demonstrated the possibility of using a remote PAM to make mutations in places where a proper target may be hard to choose. For example, although the *S. pneumoniae* R6 genome, which has a 39.7% GC content, contains on average one PAM motif every 12 bp, some PAM motifs are separated by up to 194 bp (Figure 33). In addition Applicants designed a Δ*bgaA* in-frame deletion of 6,664 bp. In all three cases, co-transformation of the targeting and editing templates produced 10-times more kanamycin-resistant cells than co-transformation with a control editing template containing wild-type *bgaA* sequences (Figure 25b). Applicants genotyped 24 transformants (8 for each editing experiment) and found that all but one incorporated the desired change (Figure 25c). DNA sequencing also confirmed not only the presence of the introduced mutations but also the absence of secondary mutations in the target region (Figure 29b,c). Finally, Applicants measured β-galactosidase activity to confirm that all edited cells displayed the expected phenotype (Figure 25d).

Cas9-mediated editing can also be used to generate multiple mutations for the study of biological pathways. Applicants decided to illustrate this for the sortase-dependent pathway that anchors surface proteins to the envelope of Gram-positive bacteria. Applicants introduced a sortase deletion by co-transformation of a chloramphenicol-resistant targeting construct and a Δ*srtA* editing template (Figure 33a,b), followed by a Δ*bgaA* deletion using a kanamycin-resistant targeting construct that replaced the previous one. In *S. pneumoniae,* β-galactosidase is covalently linked to the cell wall by sortase. Therefore, deletion of *srtA* results in the release of the surface protein into the supernatant, whereas the double deletion has no detectable β-galactosidase activity (Figure 34c). Such a sequential selection can be iterated as many times as required to generate multiple mutations.

These two mutations may also be introduced at the same time. Applicants designed a targeting construct containing two spacers, one matching *srtA* and the other matching *bgaA*, and co-transformed it with both editing templates at the same time (Figure 25e). Genetic analysis of transformants showed that editing occurred in 6/8 cases (Figure 25f). Notably, the remaining two clones each contained either a Δ*srtA* or a Δ*bgaA* deletion, suggesting the possibility of performing combinatorial mutagenesis using Cas9. Finally, to eliminate the CRISPR sequences, Applicants introduced a plasmid containing the *bgaA* target and a spectinomycin resistance gene along with genomic DNA from the wild-type strain R6. Spectinomycin-resistant transformants that retain the plasmid eliminated the CRISPR sequences (Figure 34a,d).

**Mechanism and efficiency of editing:** To understand the mechanisms underlying genome editing with Cas9, Applicants designed an experiment in which the editing efficiency was measured independently of Cas9 cleavage. Applicants integrated the *ermAM* erythromycin resistance gene in the *srtA* locus, and introduced a premature stop codon using Cas9-mediated editing (Figure 33). The resulting strain (JEN53) contains an *ermAM(stop)* allele and is sensitive to erythromycin. This strain may be used to assess the efficiency at which the *ermAM* gene is repaired by measuring the fraction of cells that restore antibiotic resistance with or without the use of Cas9 cleavage. JEN53 was transformed with an editing template that restores the wild-type allele, together with either a kanamycin-resistant CRISPR construct targeting the *ermAM(stop)* allele (CRISPR: :ermAM(stop)) or a control construct without a spacer (CRISPR::Ø) (Figure 26a,b). In the absence of kanamycin selection, the fraction of edited colonies was on the order of $10^{-2}$ (erythromycin-resistant cfu/total cfu) (Figure 26c), representing the baseline frequency of recombination without Cas9-mediated selection against unedited cells. However, if kanamycin selection was applied and the control CRISPR construct was co-transformed, the fraction of edited colonies increased to about $10^{-1}$ (kanamycin- and erythromycin-resistant cfu/kanamycin-resistant cfu) (Figure 26c). This result shows that selection for the recombination of the CRISPR locus co-selected for recombination in the *ermAM* locus independently of Cas9 cleavage of the genome, suggesting that a subpopulation of cells is more prone to transformation and/or recombination. Transformation of the CRISPR:: ermAM(stop) construct followed by kanamycin selection resulted in an increase of the fraction of erythromycin-resistant, edited cells to 99 % (Figure 26c). To determine if this increase is caused by the killing of non-edited cells, Applicants compared the kanamycin-resistant colony forming units (cfu) obtained after co-transformation of JEN53 cells with the CRISPR::ermAM(stop) or CRISPR::Ø constructs.

Applicants counted 5.3 times less kanamycin-resistant colonies after transformation of the ermAM(stop) construct ($2.5 \times 10^4 / 4.7 \times 10^3$, Figure 35a), a result that suggests that indeed targeting of a chromosomal locus by Cas9 leads to the killing of non-edited cells. Finally, because the introduction of dsDNA breaks in the bacterial chromosome is known to trigger repair mechanisms that increase the rate of recombination of the damaged DNA, Applicants investigated whether cleavage by Cas9 induces recombination of the editing template. Applicants counted 2.2 times more colonies after co-transformation with the CRISPR:: erm(stop) construct than with the CRISPR::Ø construct (Figure 26d), indicating that there was a modest induction of recombination. Taken together, these results showed that co-selection of transformable cells, induction of recombination by Cas9-mediated cleavage and selection against non-edited cells, each contributed to the high efficiency of genome editing in *S. pneumoniae.*

As cleavage of the genome by Cas9 should kill non-edited cells, one would not expect to recover any cells that received the kanamycin resistance-containing Cas9 cassette but not the editing template. However, in the absence of the editing template Applicants recovered many kanamycin-resistant colonies after transformation of the CRISPR:: ermAM(stop) construct (Figure 35a). These cells that 'escape' CRISPR-induced death produced a background that determined a limit of the method. This background frequency may be calculated as the ratio of CRISPR::ermAM(stop)/CRISPR::Ø cfu, $2.6 \times 10^{-3}$ ($7.1 \times 10^1/2.7 \times 10^4$) in this experiment, meaning that if the recombination frequency of the editing template is less than this value, CRISPR selection may not efficiently recover the desired mutants above the background. To understand the origin of these cells, Applicants genotyped 8 background colonies and found that 7 contained deletions of the targeting spacer (Figure 35b) and one harbored a presumably inactivating mutation in Cas9 (Figure 35c).

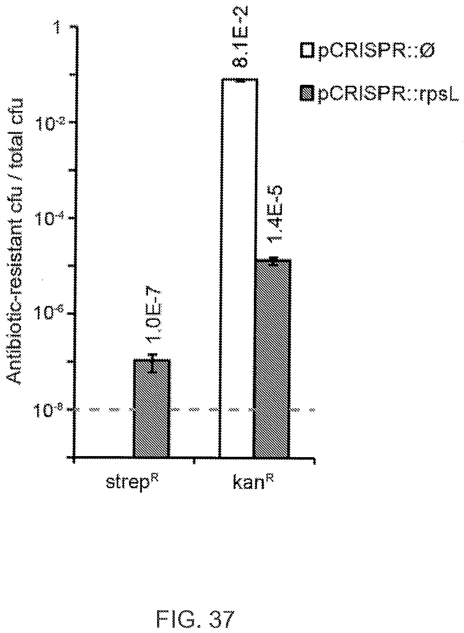

**Genome editing with Cas9 in *E. coli:*** The activation of Cas9 targeting through the chromosomal integration of a CRISPR-Cas system is only possible in organisms that are highly recombinogenic. To develop a more general method that is applicable to other microbes, Applicants decided to perform genome editing in *E. coli* using a plasmid-based CRISPR-Cas system. Two plasmids were constructed: a pCas9 plasmid carrying the tracrRNA, Cas9 and a chloramphenicol resistance cassette (Figure 36), and a pCRISPR kanamycin-resistant plasmid carrying the array of CRISPR spacers. To measure the efficiency of editing independently of CRISPR selection, Applicants sought to introduce an A to C transversion in the *rpsL* gene that confers streptomycin resistance. Applicants constructed a pCRISPR::rpsL plasmid harboring a spacer that would guide Cas9 cleavage of the wild-type, but not the mutant *rpsL* allele (Figure 27b). The pCas9 plasmid was first introduced into *E. coli* MG1655 and the resulting strain was co-transformed with the pCRISPR::rpsL plasmid and W542, an editing oligonucleotide containing the A to C mutation. streptomycin-resistant colonies after transformation of the pCRISPR::rpsL plasmid were only recovered, suggesting that Cas9 cleavage induces recombination of the oligonucleotide (Figure 37). However, the number of streptomycin-resistant colonies was two orders of magnitude lower than the number of kanamycin-resistant colonies, which are presumably cells that escape cleavage by Cas9. Therefore, in these conditions, cleavage by Cas9 facilitated the introduction of the mutation, but with an efficiency that was not enough to select the mutant cells above the background of 'escapers'.

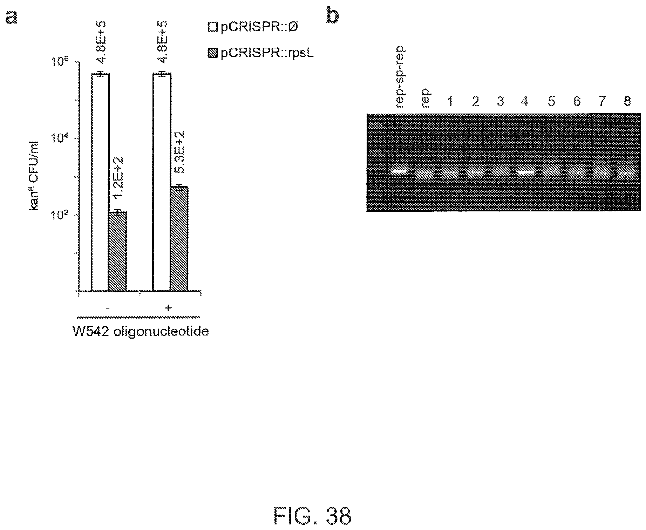

To improve the efficiency of genome editing in *E. coli,* Applicants applied their CRISPR system with recombineering, using Cas9-induced cell death to select for the desired mutations. The pCas9 plasmid was introduced into the recombineering strain HME63 (31), which contains the Gam, Exo and Beta functions of the □-red phage. The resulting strain was co-transformed with the pCRISPR::rpsL plasmid (or a pCRISPR::Ø control) and the W542 oligonucleotide (Figure 27a). The recombineering efficiency was $5.3 \times 10^{-5}$, calculated as the fraction of total cells that become streptomycin-resistant when the control plasmid was used (Figure 27c). In contrast, transformation with the pCRISPR::rpsL plasmid increased the percentage of mutant cells to $65 \pm 14$ % (Figures 27c and 29f). Applicants observed that the number of cfu was reduced by about three orders of magnitude after transformation of the pCRISPR::rpsL plasmid than the control plasmid ($4.8 \times 10^5/5.3 \times 10^2$, Figure 38a), suggesting that selection results from CRISPR-induced death of non-edited cells. To measure the rate at which Cas9 cleavage was inactivated, an important parameter of Applicants' method, Applicants transformed cells with either pCRISPR::rpsL or the control plasmid without the W542 editing oligonucleotide (Figure 38a). This background of CRISPR 'escapers', measured as the ratio of pCRISPR::rpsL/pCRISPR::Ø cfu, was $2.5 \times 10^{-4}$ ($1.2 \times 10^2/4.8 \times 10^5$). Genotyping eight of these escapers revealed that in all cases there was a deletion of the targeting spacer (Figure 38b). This background was higher than the recombineering efficiency of the *rpsL* mutation, $5.3 \times 10^{-5}$, which suggested that to obtain 65% of edited cells, Cas9 cleavage must induce oligonucleotide recombination. To confirm this, Applicants compared the number of kanamycin- and streptomycin-resistant cfu after transformation of pCRISPR::rpsL or pCRISPR::Ø (Figure 27d). As in the case for *S. pneumoniae,* Applicants observed a modest induction of recombination, about 6.7 fold ($2.0 \times 10^{-4}/3.0 \times 10^{-5}$). Taken together, these results indicated that the CRISPR system provided a method for selecting mutations introduced by recombineering.

Applicants showed that CRISPR-Cas systems may be used for targeted genome editing in bacteria by the co-introduction of a targeting construct that killed wild-type cells and an editing template that both eliminated CRISPR cleavage and introduced the desired mutations. Different types of mutations (insertions, deletions or scar-less single-nucleotide substitutions) may be generated. Multiple mutations may be introduced at the same time. The specificity and versatility of editing using the CRISPR system relied on several unique properties of the Cas9 endonuclease: (i) its target specificity may be programmed with a small RNA, without the need for enzyme engineering, (ii) target specificity was very high, determined by a 20 bp RNA-DNA interaction with low probability of non-target recognition, (iii) almost any sequence may be targeted, the only requirement being the presence of an adjacent NGG sequence, (iv) almost any mutation in the NGG sequence, as well as mutations in the seed sequence of the protospacer, eliminates targeting.

Applicants showed that genome engineering using the CRISPR system worked not only in highly recombinogenic bacteria such as *S. pneumoniae,* but also in *E. coli.* Results in *E. coli* suggested that the method may be applicable to other microorganisms for which plasmids may be introduced. In *E. coli*, the approach complements recombineering of mutagenic oligonucleotides. To use this methodology in microbes where recombineering is not a possible, the host homologous recombination machinery may be used by providing the editing template on a plasmid. In addition, because accumulated evidence indicates that CRISPR-mediated cleavage of the chromosome leads to cell death in many bacteria and archaea, it is possible to envision the use of endogenous CRISPR-Cas systems for editing purposes.

In both *S. pneumoniae* and *E. coli*, Applicants observed that although editing was facilitated by a co-selection of transformable cells and a small induction of recombination at the target site by Cas9 cleavage, the mechanism that contributed the most to editing was the selection against non-edited cells. Therefore the major limitation of the method was the presence of a background of cells that escape CRISPR-induced cell death and lack the desired mutation. Applicants showed that these 'escapers' arose primarily through the deletion of the targeting spacer, presumably after the recombination of the repeat sequences that flank the targeting spacer. Future improvements may focus on the engineering of flanking sequences that can still support the biogenesis of functional crRNAs but that are sufficiently different from one another to eliminate recombination. Alternatively, the direct transformation of chimeric crRNAs may be explored. In the particular case of *E. coli*, the construction of the CRISPR-Cas system was not possible if this organism was also used as a cloning host. Applicants solved this issue by placing Cas9 and the tracrRNA on a different plasmid than the CRISPR array. The engineering of an inducible system may also circumvent this limitation.

Although new DNA synthesis technologies provide the ability to cost-effectively create any sequence with a high throughput, it remains a challenge to integrate synthetic DNA in living cells to create functional genomes. Recently, the co-selection MAGE strategy was shown to improve the mutation efficiency of recombineering by selecting a subpopulation of cells that has an increased probability to achieve recombination at or around a given locus. In this method, the introduction of selectable mutations is used to increase the chances of generating nearby non-selectable mutations. As opposed to the indirect selection provided by this strategy, the use of the CRISPR system makes it possible to directly select for the desired mutation and to recover it with a high efficiency. These technologies add to the toolbox of genetic engineers, and together with DNA synthesis, they may substantially advance both the ability to decipher gene function and to manipulate organisms for biotechnological purposes. Two other studies also relate to CRISPR-assisted engineering of mammalian genomes. It is expected that these crRNA-directed genome editing technologies may be broadly useful in the basic and medical sciences.

Strains and culture conditions. *S. pneumoniae* strain R6 was provided by Dr. Alexander Tomasz. Strain crR6 was generated in a previous study. Liquid cultures of S. *pneumoniae* were grown in THYE medium (30g/l Todd-Hewitt agar, 5 g/l yeast extract). Cells were plated on tryptic soy agar (TSA) supplemented with 5 % defibrinated sheep blood. When appropriate, antibiotics were added as followings: kanamycin (400 $\mu$g/ml), chloramphenicol (5 $\mu$g/ml), erythromycin (1 $\mu$g/ml) streptomycin (100 $\mu$g/ml) or spectinomycin (100 $\mu$g/ml). Measurements of $\beta$-galactosidase activity were made using the Miller assay as previously described.

*E. coli* strains MG1655 and HME63 (derived from MG1655, $\Delta$(argF-lac) U169 $\lambda$ cl857 $\Delta$cro-bioA galK tyr 145 UAG mutS<>amp) (31) were provided by Jeff Roberts and Donald Court, respectively. Liquid cultures of *E. coli* were grown in LB medium (Difco). When appropriate, antibiotics were added as followings: chloramphenicol (25 $\mu$g/ml), kanamycin (25 $\mu$g/ml) and streptomycin (50 $\mu$g/ml).

*S. pneumoniae* **transformation.** Competent cells were prepared as described previously (23). For all genome editing transformations, cells were gently thawed on ice and resuspended in 10 volumes of M2 medium supplemented with 100 ng/ml of competence-stimulating peptide CSP1(40), and followed by addition of editing constructs (editing constructs were added to cells at a final concentration between 0.7 ng/$\mu$l to 2.5 $\mu$g/ul). Cells were incubated 20 min at 37 °C before the addition of 2 $\mu$l of targeting constructs and then incubated 40 min at 37 °C. Serial dilutions of cells were plated on the appropriate medium to determine the colony forming units (cfu) count.

*E. coli* **Lambda-red recombineering**. Strain HME63 was used for all recombineering experiments. Recombineering cells were prepared and handled according to a previously published protocol (6). Briefly, a 2 ml overnight culture (LB medium) inoculated from a single colony obtained from a plate was grown at 30 °C. The overnight culture was diluted 100-fold and grown at 30 °C with shaking (200rpm) until the OD$_{600}$ is from 0.4-0.5 (approximately 3 hrs). For Lambda-red induction, the culture was transferred to a 42 °C water bath to shake at 200rpm for 15 min. Immediately after induction, the culture was swirled in an ice-water slurry and chilled on ice for 5-10 min. Cells were then washed and aliquoted according to the protocol. For electro-transformation, 50 $\mu$l of cells were mixed with 1mM of salt-free oligos (IDT) or 100-150 ng of plasmid DNA (prepared by QIAprep Spin Miniprep Kit, Qiagen). Cells were electroporated using 1mm Gene Pulser cuvette (Bio-rad) at 1.8kV and were immediately resuspended in 1 ml of room temperature LB medium. Cells were recovered at 30 °C for 1-2 hrs before being plated on LB agar with appropriate antibiotic resistance and incubated at 32 °C overnight.

**Preparation of *S. pneumoniae* genomic DNA.** For transformation purposes, S. *pneumoniae* genomic DNA was extracted using the Wizard Genomic DNA Purification Kit, following instructions provided by the manufacturer (Promega). For genotyping purposes, 700ul of overnight *S. pneumoniae* cultures were pelleted, resuspended in 60ul of lysozyme solution (2mg/ml) and incubated 30min at 37°C. The genomic DNA was extracted using QIAprep Spin Miniprep Kit (Qiagen).

**Strain construction.** All primers used in this study are provided in Table G. To generate *S. pneumoniae* crR6M, an intermediate strain, LAM226, was made. In this strain the *aphA-3* gene (providing kanamycin resistance) adjacent to the CRISPR array of *S. pneumoniae* crR6 strain was replaced by a *cat* gene (providing chloramphenicol resistance). Briefly, crR6 genomic DNA was amplified using primers L448/L444 and L447/L481, respectively. The *cat* gene was amplified from plasmid pC194 using primers L445/L446. Each PCR product was gel-purified and all three were fused by SOEing PCR with primers L448/L481. The resulting PCR product was transformed into competent *S. pneumoniae* crR6 cells and chloramphenicol-resistant transformants were selected. To generate *S. pneumoniae* crR6M, *S. pneumoniae* crR6 genomic DNA was amplified by PCR using primers L409/L488 and L448/L481, respectively. Each PCR product was gel-purified and they were fused by SOEing PCR with primers L409/L481. The resulting PCR product was transformed into competent *S. pneumoniae* LAM226 cells and kanamycin-resistant transformants were selected.

To generate *S. pneumoniae* crR6Rc, *S. pneumoniae* crR6M genomic DNA was amplified by PCR using primers L430/W286, and *S. pneumoniae* LAM226 genomic DNA was amplified by PCR using primers W288/L481. Each PCR product was gel-purified and they were fused by SOEing PCR with primers L430/L481. The resulting PCR product was transformed into competent *S. pneumoniae* crR6M cells and chloramphenicol-resistant transformants were selected.

To generate *S. pneumoniae* crR6Rk, *S. pneumoniae* crR6M genomic DNA was amplified by PCR using primers L430/W286 and W287/L481, respectively. Each PCR product was gel-purified and they were fused by SOEing PCR with primers L430/L481. The resulting PCR product was transformed into competent *S. pneumoniae* crR6Rc cells and kanamycin-resistant transformants were selected.

To generate JEN37, *S. pneumoniae* crR6Rk genomic DNA was amplified by PCR using primers L430/W356 and W357/L481, respectively. Each PCR product was gel-purified and they were fused by SOEing PCR with primers L430/L481. The resulting PCR product was transformed into competent *S. pneumoniae* crR6Rc cells and kanamycin-resistant transformants were selected.

To generate JEN38, R6 genomic DNA was amplified using primers L422/L461 and L459/L426, respectively. The *ermAM* gene (specifying erythromycin resistance) was amplified from plasmid pFW15 [43] using primers L457/L458. Each PCR product was gel-purified and all three were fused by SOEing PCR with primers L422/L426. The resulting PCR product was transformed into competent *S. pneumoniae* crR6Rc cells and erythromycin-resistant transformants were selected.

*S. pneumoniae* JEN53 was generated in two steps. First JEN43 was constructed as illustrated in Figure 33. JEN53 was generated by transforming genomic DNA of JEN25 into competent JEN43 cells and selecting on both chloramphenicol and erythromycin.

To generate *S. pneumoniae* JEN62, *S. pneumoniae* crR6Rk genomic DNA was amplified by PCR using primers W256/W365 and W366/L403, respectively. Each PCR product was purified and ligated by Gibson assembly. The assembly product was transformed into competent *S. pneumoniae* crR6Rc cells and kanamycin-resistant transformants were selected.

**Plasmid construction.** pDB97 was constructed through phosphorylation and annealing of oligonucleotides B296/B297, followed by ligation in pLZ12spec digested by EcoRI/BamHI. Applicants fully sequenced pLZ12spec and deposited its sequence in genebank (accession: KC112384).

pDB98 was obtained after cloning the CRISPR leader sequence was cloned together with a repeat-spacer-repeat unit into pLZ12spec. This was achieved through amplification of crR6Rc DNA with primers B298/B320 and B299/B321, followed by SOEing PCR of both products and cloning in pLZ12spec with restriction sites BamHI/EcoRI. In this way the spacer sequence in pDB98 was engineered to contain two BsaI restriction sites in opposite directions that allow for the scar-less cloning of new spacers.

pDB99 to pDB108 were constructed by annealing of oligonucleotides B300/B301 (pDB99), B302/B303 (pDB100), B304/B305 (pDB101), B306/B307 (pDB102), B308/B309 (pDB103), B310/B311 (pDB104), B312/B313 (pDB105), B314/B315 (pDB106), B315/B317 (pDB107), B318/B319 (pDB108), followed by ligation in pDB98 cut by BsaI.

The pCas9 plasmid was constructed as follow. Essential CRISPR elements were amplified from *Streptococcos pyogenes* SF370 genomic DNA with flanking homology arms for Gibson Assembly. The tracrRNA and Cas9 were amplified with oligos HC008 and HC010. The leader and CRISPR sequences were amplified HC011/HC014 and HC015/HC009, so that two *Bsa*I type IIS sites were introduced in between two direct repeats to facilitate easy insertion of spacers.

pCRISPR was constructed by subcloning the pCas9 CRISPR array in pZE21-MCS1 through amplification with oligos B298+B299 and restriction with EcoRI and BamHI. The rpsL targeting spacer was cloned by annealing of oligos B352+B353 and cloning in the BsaI cut pCRISPR giving pCRISPR::rpsL.

**Generation of targeting and editing constructs.** Targeting constructs used for genome editing were made by Gibson assembly of Left PCRs and Right PCRs (Table G). Editing constructs were made by SOEing PCR fusing PCR products A (PCR A), PCR products B (PCR B) and PCR products C (PCR C) when applicable (Table G). The CRISPR::Ø and CRISPR: :ermAM(stop) targeting constructs were generated by PCR amplification of JEN62 and crR6 genomic DNA respectively, with oligos L409 and L481.

**Generation of targets with randomized PAM or protospacer sequences.** The 5 nucleotides following the spacer 1 target were randomized through amplification of R6[8232.5] genomic DNA with primers W377/L426. This PCR product was then assembled with the *cat* gene and the *srtA* upstream region that were amplified from the same template with primers L422/W376. 80 ng of the assembled DNA was used to transform strains R6 and crR6. Samples for the randomized targets were prepared using the following primers: B280-B290/L426 to randomize bases 1-10 of the target and B269-B278/L426 to randomize bases 10-20. Primers L422/B268 and L422/B279 were used to amplify the *cat* gene and *srtA* upstream region to be assembled with the first and last 10 PCR products respectively. The assembled constructs were pooled together and 30 ng was transformed in R6 and crR6. After transformation, cells were plated on chloramphenicol selection. For each sample more than $2\times10^5$ cells were pooled together in 1 ml of THYE and genomic DNA was extracted with the Promega Wizard kit. Primers B250/B251 were used to amplify the target region. PCR products were tagged and run on one Illumina MiSeq paired-end lane using 300 cycles.

**Analysis of deep sequencing data.**

Randomized PAM: For the randomized PAM experiment 3,429,406 reads were obtained for crR6 and 3,253,998 for R6. It is expected that only half of them will correspond to the PAM-target while the other half will sequence the other end of the PCR product. 1,623,008 of the crR6 reads and 1,537,131 of the R6 reads carried an error-free target sequence. The occurrence of each possible PAM among these reads is shown in supplementary file. To estimate the functionality of a PAM, its relative proportion in the crR6 sample over the R6 sample was computed and is denoted $r_{ijklm}$ where l,j,k,l,m are one of the 4 possible bases. The following statistical model was constructed:

$$\log(r_{ijklm}) = \mu + b2_i + b3_j + b4_k + b2b3_{i,j} + b3b4_{j,k} + \varepsilon_{ijklm},$$

where $\varepsilon$ is the residual error, b2 is the effect of the 2nd base of the PAM, b3 of the third, b4 of the fourth, b2b3 is the interaction between the second and third bases, b3b4 between the third and fourth bases. An analysis of variance was performed:

Anova table

|  | Df | Sum Sq | Mean Sq | F value | Pr (>F) | |
|---|---|---|---|---|---|---|
| b3 | 3 | 151.693 | 50.564 | 601.8450 | < 2.2e-16 | *** |
| b2 | 3 | 90.521 | 30.174 | 359.1454 | < 2.2e-16 | *** |
| b4 | 3 | 1.881 | 0.627 | 7.4623 | 6.070e-05 | *** |
| b3:b2 | 9 | 228.940 | 25.438 | 302.7738 | <2.2e-16 | *** |
| b3:b4 | 9 | 3.010 | 0.334 | 3.9809 | 5.227e-05 | *** |
| Residuals | 996 | 83.680 | 0.084 | | | |

When added to this model, b1 or b5 do not appear to be significant and other interactions than the ones included can also be discarded. The model choice was made through successive comparisons of more or less complete models using the anova method in R. Tukey's honest significance test was used to determine if pairwise differences between effects are significant.

NGGNN patterns are significantly different from all other patterns and carry the strongest effect (see table below).

In order to show that positions 1, 4 or 5 do not affect the NGGNN pattern Applicants looked at theses sequences only. Their effect appears to be normally distributed (see QQ plot in Figure 71), and model comparisons using the anova method in R shows that the null model is the best one, i.e. there is no significant role of b1, b4 and b5.

Model comparison using the anova method in R for the NGGNN sequences

Model 1:    ratio.log ~ 1
Model 2:    ratio. log ~ b1 + b4 + b5

(continued)

| | Res.Df | RSS | Df | Sum of Sq | F | Pr(>F) |
|---|---|---|---|---|---|---|
| 1 | 63 | 14.579 | | | | |
| 2 | 54 | 11.295 | 9 | 3.2836 | 1.7443 | 0.1013 |

Partial interference of NAGNN and NNGGN patterns

NAGNN patterns are significantly different from all other patterns but carry a much smaller effect than NGGNN (see Tukey's honest significance test below).

Finally, NTGGN and NCGGN patterns are similar and show significantly more CRISPR interference than NTGHN and NCGHN patterns (where H is A,T or C), as shown by a bonferroni adjusted pairwise student-test.

Pairwise comparisons of the effect of b4 on NYGNN sequences using t tests with pooled SD

Data: b4

| | A | C | G |
|---|---|---|---|
| C | 1.00 | | - |
| G | 9.2e-05 | 2.4e-06 | - |
| T | 0.31 | 1.00 | 1.2e-08 |

Taken together, these results allow concluding that NNGGN patterns in general produce either a complete interference in the case of NGGGN, or a partial interference in the case of NAGGN, NTGGN or NCGGN.

Tukey multiple comparisons of means: 95% family-wise confidence level

sb2:b3

| | diff | 1wr | upr | p adj |
|---|---|---|---|---|
| G:G-A:A | -2.76475 | -2.94075 | -2.58875 | <1E -07 |
| G:G-C:A | -2.76911 | -2.97511 | -2.62311 | <1E -07 |
| G:G-T:A | -2.7809 | -2.9569 | -2.6049 | <1E -07 |
| G:G-A:C | -2.81643 | -2.99244 | -2.64043 | <1E -07 |
| G:G-C:C | -2.77903 | -2.95504 | -2.60303 | <1E -07 |
| G:G-G:C | -2.64867 | -2.82468 | -2.47267 | <1E -07 |
| G:G-T:C | -2.79718 | -2.97319 | -2.62118 | <1E -07 |
| G:G-A:G | -2.67068 | -2.84668 | -2.49468 | <1E -07 |
| G:G-C:G | -2.73525 | -2.91125 | -2.5,5925 | <1E -07 |
| G:G-T:G | -2.7976 | -2.62159 | -2.9736 | <1E -07 |
| G:G-A:T | -2.76727 | -2.59127 | -2.94328 | <1E -07 |
| G:G-C:T | -2.84114 | -2.66513 | -3.01714 | <1E -0 7 |
| G:G-G:T | -2.76409 | -2.58809 | -2.94009 | <1E -07 |
| G:G-T:T | -2.76781 | -2.59181 | -2.94381 | <1E -07 |
| G:G-G:A | -2.13964 | -2.31565 | -1.96364 | <1E -07 |
| G:A-A:A | -0.62511 | -0.80111 | -0.4491 | <1E -07 |
| G:A-C:A | -0.65947 | -0.83547 | -0.43346 | <1E -07 |
| G:A-T:A | -0.64126 | -0.46525 | -0.81726 | <1E -07 |
| G:A-A:C | -0.67679 | -0.50078 | -0.85279 | <1E -07 |
| G:A-C:C | -0.63939 | -0.46339 | -0.81539 | <1E -07 |
| G:A-G:C | -0.50903 | -0.33303 | -0.68503 | <1E -07 |
| G:A-T:C | -0.65754 | -0.48154 | -0.83354 | <1E -07 |
| G:A-A:G | -0.53104 | -0.35503 | -0.70704 | <1E -07 |
| G:A-C:G | -0.59561 | -0.4196 | -0.77161 | <1E -07 |
| G:A-T:G | -0.65795 | -0.48195 | -0.83396 | <1E -07 |
| G:A-A:T | -0.62763 | -0.45163 | -0.80363 | <1E -07 |
| G:A-C:T | -0.70149 | -0.52549 | -0.3775 | <1E -07 |

(continued)

sb2:b3

| | diff | 1wr | upr | p adj |
|---|---|---|---|---|
| G:A-G:T | -0.62445 | -0.44844 | -0.80045 | <1E -07 |
| G:A-T:T | -0.62817 | -0.45216 | -0.80417 | <1 E -07 |

sb3:b4

| | diff | lwr | upr | P adj |
|---|---|---|---|---|
| G:G-G:A | -0.33532 | -0.51133 | -0.15932 | <1E-07 |
| G:G-G:C | -0.18118 | -0.35719 | -0.00518 | 0.036087 |
| G:G-G:T | -0.31626 | -0.14026 | -0.49226 | <1E-07 |

**Randomized target**

For the randomized target experiment 540,726 reads were obtained for crR6 and 753,570 for R6. As before, only half of the reads are expected to sequence the interesting end of the PCR product. After filtering for reads that carry a target that is error-free or with a single point mutation, 217,656 and 353,141 reads remained for crR6 and R6 respectively. The relative proportion of each mutant in the crR6 sample over the R6 sample was computed (Figure 24c). All mutations outside of the seed sequence (13-20 bases away from the PAM) show full interference. Those sequences were used as a reference to determine if other mutations inside the seed sequence can be said to significantly disrupt interference. A normal distribution was fitted to theses sequences using the fitdistr function of the MASS R package. The 0.99 quantile of the fitted distribution is shown as a dotted line in Figure 24c. Figure 72 shows a histogram of the data density with fitted normal distribution (black line) and .99 quantile (dotted line).

**Table F**. Relative abundance of PAM sequences in the crR6/R6 samples averaged over bases 1 and 5.

3rd position

| | | A | | C | | G | | T | | |
|---|---|---|---|---|---|---|---|---|---|---|
| A | AAA | 1.04 | ACA | 1.12 | AGA | 0.73 | ATA | 1.10 | A |
| | AAC | 1.07 | ACC | 1.04 | AGC | 0.64 | ATC | 0.97 | C |
| | AAG | 1.00 | ACG | 1.09 | AGG | 0.61 | ATG | 1.07 | G |
| | AAT | 0.98 | ACT | 1.02 | AGT | 0.65 | ATT | 1.01 | T |
| C | CAA | 1.05 | CCA | 1.05 | CGA | 0.99 | CTA | 1.07 | A |
| | CAC | 1.04 | CCC | 1.02 | CGC | 1.08 | CTC | 1.04 | C |
| | CAG | 1.08 | CCG | 1.08 | CGG | 0.81 | CTG | 1.05 | G |
| | CAT | 1.13 | CCT | 1.05 | CGT | 1.07 | CTT | 1.08 | T |
| G | GAA | 0.97 | GCA | 1.05 | GGA | 0.08 | GTA | 0.99 | A |
| | GAC | 0.92 | GCC | 1.00 | GGC | 0.05 | GTC | 1.15 | C |
| | GAG | 0.96 | GCG | 0.98 | GGG | 0.07 | GTG | 0.98 | G |
| | GAT | 0.98 | GCT | 0.99 | GGT | 0.06 | GTT | 1.05 | T |
| T | TAA | 1.08 | TCA | 1.16 | TGA | 1.05 | TTA | 1.14 | A |
| | TAC | 1.00 | TCC | 1.08 | TGC | 1.08 | TTC | 1.05 | C |
| | TAG | 1.02 | TCG | 1.11 | TGG | 0.77 | TTG | 1.01 | G |
| | TAT | 1.01 | TCT | 1.12 | TGT | 1.21 | TTT | 1.02 | T |

2nd position (left axis) — 4th position (right axis)

**Table G.** Primers used in this study.

| Primer | Sequence 5'-3' |
|--------|----------------|
| B217 | TCCTAGCAGGATTTCTGATATTACTGTCACGTTTTAGAGCTATGCTTTTGA |
| B218 | GTGACAGTAATATCAGAAATCCTGCTAGGAGTTTTGGGACCATTCAAAACAGC |
| B229 | GGGTTTCAAGTCTTTGTAGCAAGAG |
| B230 | GCCAATGAACGGGAACCCTTGGTC |
| B250 | NNNNGACGAGGCAATGGCTGAAATC |
| B251 | NNNNTTATTTGGCTCATATTTGCTG |
| B255 | CTTTACACCAATCGCTGCAACAGAC |
| B256 | CAAAATTTCTAGTCTTCTTTGCCTTTCCCCATAAAACCCTCCTTA |
| B257 | AGGGTTTTATGGGGAAAGGCAAAGAAGACTAGAAATTTTGATACC |
| B258 | CTTACGGTGCATAAAGTCAATTTCC |
| B269 | TGGCTCGATTTCAGCCATTGC |
| B270 | CTTTGACGAGGCAATGGCTGAAATCGAGCCAANAAAGCGCAAG |
| B271 | CTTTGACGAGGCAATGGCTGAAATCGAGCCAAANAAGCGCAAG |
| B272 | CTTTGACGAGGCAATGGCTGAAATCGAGCCAAAANAGCGCAAG |
| B273 | CTTTGACGAGGCAATGGCTGAAATCGAGCCAAAAANGCGCAAG |
| B274 | CTTTGACGAGGCAATGGCTGAAATCGAGCCAAAAAANCGCAAG |
| B275 | CTTTGACGAGGCAATGGCTGAAATCGAGCCAAAAAAGNGCAAG |
| B276 | CTTTGACGAGGCAATGGCTGAAATCGAGCCAAAAAAGCNCAAGAAG |
| B277 | CTTTGACGAGGCAATGGCTGAAATCGAGCCAAAAAAGCGNAAGAAG |
| B278 | CTTTGACGAGGCAATGGCTGAAATCGAGCCAAAAAAGCGCNAGAAG |
| B279 | GCGCTTTTTTGGCTCGATTTCAG |
| B280 | CAATGGCTGAAATCGAGCCAAAAAAGCGCANGAAGAAATC |
| B281 | CAATGGCTGAAATCGAGCCAAAAAAGCGCAANAAGAAATC |
| B282 | CAATGGCTGAAATCGAGCCAAAAAAGCGCAAGNAGAAATC |
| B283 | CAATGGCTGAAATCGAGCCAAAAAAGCGCAAGANGAAATC |
| B284 | CAATGGCTGAAATCGAGCCAAAAAAGCGCAAGAANAAATC |
| B285 | CAATGGCTGAAATCGAGCCAAAAAAGCGCAAGAAGNAATCAACC |
| B286 | CAATGGCTGAAATCGAGCCAAAAAAGCGCAAGAAGANATCAACC |
| B287 | CAATGGCTGAAATCGAGCCAAAAAAGCGCAAGAAGAANTCAACC |
| B288 | CAATGGCTGAAATCGAGCCAAAAAAGCGCAAGAAGAAANCAACC |
| B289 | CAATGGCTGAAATCGAGCCAAAAAAGCGCAAGAAGAAATNAACCAGC |
| B290 | CAATGGCTGAAATCGAGCCAAAAAAGCGCAAGAAGAAATCNACCAGC |
| B296 | gatccTCCATCCGTACAACCCACAACCCTGg |
| B297 | aattcCAGGGTTGTGGGTTGTACGGATGGAg |
| B298 | CATGGATCCTATTTCTTAATAACTAAAAATATGG |
| B299 | CATGAATTCAACTCAACAAGTCTCAGTGTGCTG |
| B300 | AAACATTTTTTCTCCATTTAGGAAAAAGGATGCTG |
| B301 | AAAACAGCATCCTTTTTCCTAAATGGAGAAAAAAT |
| B302 | AAACCTTAAATCAGTCACAAATAGCAGCAAAATTG |

(continued)

| Primer | Sequence 5'-3' |
|---|---|
| B303 | AAAACAATTTTGCTGCTATTTGTGACTGATTTAAG |
| B304 | AAACTTTTCATCATACGACCAATCTGCTTTATTTG |
| B305 | AAAACAAATAAAGCAGATTGGTCGTATGATGAAAA |
| B306 | AAACTCGTCCAGAAGTTATCGTAAAAGAAATCGAG |
| B307 | AAAAACTCGATTTCTTTTACGATAACTTCTGGACGA |
| B308 | AAACAATCTCTCCAAGGTTTCCTTAAAAATCTCTG |
| B309 | AAAACAGAGATTTTTAAGGAAACCTTGGAGAGATT |
| B310 | AAACGCCATCGTCAGGAAGAAGCTATGCTTGAGTG |
| B311 | AAAACACTCAAGCATAGCTTCTTCCTGACGATGGC |
| B312 | AAACATCTCTATACTTATTGAAATTTCTTTGTATG |
| B313 | AAAACATACAAAGAAATTTCAATAAGTATAGAGAT |
| B314 | AAACTAGCTGTGATAGTCCGCAAAACCAGCCTTCG |
| B315 | AAAACGAAGGCTGGTTTTGCGGACTATCACAGCTA |
| B316 | AAACATCGGAAGGTCGAGCAAGTAATTATCTTTTG |
| B317 | AAAACAAAAGATAATTACTTGCTCGACCTTCCGAT |
| B318 | AAACAAGATGGTATCGCAAAGTAAGTGACAATAAG |
| B319 | AAAACTTATTGTCACTTACTTTGCGATACCATCTT |
| B320 | GAGACCTTTGAGCTTCCGAGACTGGTCTCAGTTTTGGGACCATTCAAAACAG |
| B321 | TGAGACCAGTCTCGGAAGCTCAAAGGTCTCGTTTTAGAGCTATGCTGTTTTG |
| B352 | aaacTACTTTACGCAGCGCGGAGTTCGGTTTTTTg |
| B353 | aaaacAAAAAACCGAACTCCGCGCTGCGTAAAGTA |
| HC008_SP | ATGCCGGTACTGCCGGGCCTCTTGCGGGATTACGAAATCATCCTG |
| HC009_SP | GTGACTGGCGATGCTGTCGGAATGGACGATCACACTACTCTTCTT |
| HC010_SP | TTAAGAAATAATCTTCATCTAAAATATACTTCAGTCACCTCCTAGCTGAC |
| HC011_SP | ATTGATTTGAGTCAGCTAGGAGGTGACTGAAGTATATTTTAGATGAAG |
| HC014_SP | GAGACCTTTGAGCTTCCGAGACTGGTCTAGTTTTGGGACCATTCAAAACAG CATAGCTCTAAAACCTCGTAGACTATTTTTGTC |
| HC015_SP | GAGACCAGTCTCGGAAGCTCAAAGGTCTCGTTTTAGAGCTATGCTGTTTTG AATGGTCCCAAAACTTCAGCACACTGAGACTTG |
| L403 | AGTCATCCCAGCAACAAATGG |
| L409 | CGTGGTAAATCGGATAACGTTCCAAGTGAAG |
| L422 | Tgctcttcttcacaaacaaggg |
| L426 | AAGCCAAAGTTTGGCACCACC |
| L430 | GTAGCTTATTCAGTCCTAGTGG |
| L444 | CGTTTGTTGAACTAATGGGTGCAAATTACGAATCTTCTCCTGACG |
| L445 | CGTCAGGAGAAGATTCGTAATTTGCACCCATTAGTTCAACAAACG |
| L446 | GATATTATGGAGCCTATTTTTTGTGGGTTTTTAGGCATAAAACTATATG |
| L447 | CATATAGTTTTATGCCTAAAAACCcACAAAAATAGGCTCCATAATATC |

(continued)

| Primer | Sequence 5'-3' |
|--------|----------------|
| L448 | ATTATTTCTTAATAACTAAAAATATGG |
| L457 | CGTgtacaattgctagcgtacggc |
| L458 | GCACCGGTGATCACTAGTCCTAGG |
| L459 | cctaggactagtgatcaccggtGCAAATATGAGCCAAATAAATATAT |
| L461 | GCCGTACGCTAGCAATTGTACACGTTTGTTGAACTAATGGGTGC |
| L481 | TTCAAATTTTCCCATTTGATTCTCC |
| L488 | CCATATTTTTAGTTATTAAGAAATAATACCAGCCATCAGTCACCTCC |
| W256 | AGACGATTCAATAGACAATAAGG |
| W286 | GTTTTGGGACCATTCAAAACAGCATAGCTCTAAAACCTCGTAGAC |
| W287 | GCTATGCTGTTTTGAATGGTCCCAAAACcattattttaacacacgaggtg |
| W288 | GCTATGCTGTTTTGAATGGTCCCAAAACGCACCCATTAGTTCAACAAACG |
| W326 | AATTCTTTTCTTCATCATCGGTC |
| W327 | AAGAAAGAATGAAGATTGTTCATG |
| W341 | GGTACTAATCAAAATAGTGAGGAGG |
| W354 | GTTTTTCAAAATCTGCGGTTGCG |
| W355 | AAAAATTGAAAAAATGGTGGAAACAC |
| W356 | ATTTCGTAAACGGTATCGGTTTCTTTTAAAGTTTTGGGACCATTCAAAACAGC |
| W357 | TTTAAAAGAAACCGATACCGTTTACGAAATGTTTTAGAGCTATGCTGTTTTGA |
| W365 | AAACGGTATCGGTTTCTTTTAAATTCAATTGTTTTGGGACCATTCAAAACAGC |
| W366 | AATTGAATTTAAAAGAAACCGATACCGTTTGTTTTAGAGCTATGCTGTTTTGA |
| W370 | GTTCCTTAAACCAAAACGGTATCGGTTTCTTTTAAATTC |
| W371 | GAAACCGATACCGTTTTGGTTTAAGGAACAGGTAAAGGGCATTTAAC |
| W376 | CGATTTCAGCCATTGCCTCGTC |
| W377 | GCCTTTGACGAGGCAATGGCTGAAATCGNNNNNAAAAAGCGCAAGAAGAAATCAAC |
| W391 | TCCGTACAACCCACAACCCTGCTAGTGAGCGTTTTGGGACCATTCAAAACAGC |
| W392 | GCTCACTAGCAGGGTTGTGGGTTGTACGGAGTTTTAGAGCTATGCTGTTTTGA |
| W393 | TTGTTGCCACTCTTCCTTCTTTC |
| W397 | CAGGGTTGTGGGTTGTTGCGATGGAGTTAACTCCCATCTCC |
| W398 | GGGAGTTAACTCCATCGCAACAACCCACAACCCTGCTAGTG |
| W403 | GTGGTATCTATCGTGATGTGAC |
| W404 | TTACCGAAACGGAATTTATCTGC |
| W405 | AAAGCTAGAGTTCCGCAATTGG |
| W431 | GTGGGTTGTACGGATTGAGTTAACTCCCATCTCCTTC |
| W432 | GATGGGAGTTAACTCAATCCGTACAACCCACAACCCTG |
| W433 | GCTTCACCTATTGCAGCACCAATTGACCACATGAAGATAG |
| W434 | GTGGTCAATTGGTGCTGCAATAGGTGAAGCTAATGGTGATG |
| W463 | CTGATTTGTATTAATTTTGAGACATTATGCTTCACCTTC |
| W464 | GCATAATGTCTCAAAATTAATACAAATCAGTGAAATCATG |
| W465 | GTTTTGGGACCATTCAAAACAGCATAGCTCTAAAACGTGACAGTAATATCAG |

(continued)

| Primer | Sequence 5'-3' |
| --- | --- |
| W466 | GTTTTAGAGCTATGCTGTTTTGAATGGTCCCAAAACGCTCACTAGCAGGGTTG |
| W542 | ATACTTTTACGCAGCGGAGTTCGGTTTTgTAGGAGTGGTAGTATATACACGAGTACAT |

Table H. Design of targeting and editing constructs used in this study.

| Edition | Targeting Constructs<br><br>Template DNA | Left PCR | Right PCR | Spacer Sequence | PAM |
|---|---|---|---|---|---|
| bgaA R>A | crR6Rk | W256/W391 | W392/L403 | GCTCACTAGCAGGGTTGTGGGTTGTACGGA | TGG |
| bgaA NE>AA | crR6Rk | W256/W391 | W392/L403 | GCTCACTAGCAGGGTTGTGGGTTGTACGGA | TGG |
| ΔbgaA | crR6Rk | W256/W391 | W392/L403 | GCTCACTAGCAGGGTTGTGGGTTGTACGGA | TGG |
| ΔsrtA | crR6Rc | W256/B218 | B217/L403 | TCCTAGCAGGATTTCTGATATTACTGTCAC | TGG |
| ermB Stop | crR6Rk | W256/W356 | W357/L403 | TTTAAAAGAAACCGATACCGTTACGAAAT | TGG |
| ΔsrtA ΔbgaA | JEN51 (for Left PCR) and JEN52 (for Right PCR) | W256/W465 | W466/W403 | same as the ones used for ΔsrtA and ΔbgaA | TGG |

| Edition | Editing Constructs<br><br>Template DNA | PCR A | PCR B | PCR C | SOEing PCR | Name of resulting strains | Primers used to verify edited genotype |
|---|---|---|---|---|---|---|---|
| bgaA R>A | R6 | W403/W397 | W398/W404 | N/A | W403/W404 | JEN56 | W403/W404 |
| bgaA NE>AA | R6 | W403/W431 | W432/W433 | W434/W404 | W403/W404 | JEN60 | W403/W404 |
| ΔbgaA | R6 | B255/B256 | B257/B258 | N/A | B255/B258 | JEN52 | W393/W405 |
| ΔsrtA | R6 | B230/W463 | W464/B229 | N/A | B230/B229 | JEN51 | W422/W426 |
| ermB Stop | JEN38 | L422/W370 | W371/L426 | N/A | L422/L426 | JEN43 | L457/L458 |
| ΔsrtA ΔbgaA | Same as the ones used for ΔsrtA and ΔbgaA | | | | | JEN64 | Same as the ones used for ΔsrtA and ΔbgaA |

*Example 6: Optimization of the guide RNA for Streptococcus pyogenes Cas9 (referred to as SpCas9).*

Applicants mutated the tracrRNA and direct repeat sequences, or mutated the chimeric guide RNA to enhance the RNAs in cells.

The optimization is based on the observation that there were stretches of thymines (Ts) in the tracrRNA and guide RNA, which might lead to early transcription termination by the pol 3 promoter. Therefore Applicants generated the following optimized sequences. Optimized tracrRNA and corresponding optimized direct repeat are presented in pairs.

Optimized tracrRNA 1 (mutation underlined):

GGAACCATTCAtAACAGCATAGCAAGTTAtAATAAGGCTAGTCCGTTATCA

ACTTGAAAAAGTGGCACCGAGTCGGTGCTTTTT

Optimized direct repeat 1 (mutation underlined):
GTTaTAGAGCTATGCTGTTaTGAATGGTCCCAAAAC

Optimized tracrRNA 2 (mutation underlined):

GGAACCATTCAAtACAGCATAGCAAGTTAAtATAAGGCTAGTCCGTTATCA

ACTTGAAAAAGTGGCACCGAGTCGGTGCTTTTT

Optimized direct repeat 2 (mutation underlined):
GTaTTAGAGCTATGCTGTaTTGAATGGTCCCAAAAC

Applicants also optimized the chimeric guideRNA for optimal activity in eukaryotic cells.

Original guide RNA:

NNNNNNNNNNNNNNNNNNNNGTTTTAGAGCTAGAAATAGCAAGTTAAAA

TAAGGCTAGTCCGTTATCAACTTGAAAAAGTGGCACCGAGTCGGTGCTTTTTTT

Optimized chimeric guide RNA sequence 1:

NNNNNNNNNNNNNNNNNNNNGTATTAGAGCTAGAAATAGCAAGTTAATA

TAAGGCTAGTCCGTTATCAACTTGAAAAAGTGGCACCGAGTCGGTGCTTTTTTT

Optimized chimeric guide RNA sequence 2:

NNNNNNNNNNNNNNNNNNNNGTTTTAGAGCTATGCTGTTTTGGAAACAA

AACAGCATAGCAAGTTAAAATAAGGCTAGTCCGTTATCAACTTGAAAAAGTGGCAC

CGAGTCGGTGCTTTTTTT

Optimized chimeric guide RNA sequence 3:

NNNNNNNNNNNNNNNNNNNNGTATTAGAGCTATGCTGTATTGGAAACAA

TACAGCATAGCAAGTTAATATAAGGCTAGTCCGTTATCAACTTGAAAAAGTGGCACC

GAGTCGGTGCTTTTTTT

Applicants showed that optimized chimeric guide RNA works better as indicated in Figure 3. The experiment was conducted by co-transfecting 293FT cells with Cas9 and a U6-guide RNA DNA cassette to express one of the four RNA forms shown above. The target of the guide RNA is the same target site in the human Emx1 locus: "GTCACCTCCAATGACTAGGG"

*Example 7: Optimization of Streptococcus thermophiles LMD-9 CRISP1 Cas9 (referred to as St1Cas9).*

Applicants designed guide chimeric RNAs as shown in Figure 4.
The St1Cas9 guide RNAs can undergo the same type of optimization as for SpCas9 guide RNAs, by breaking the stretches of poly thymines (Ts)

*Example 8: Cas9 diversity and mutations*

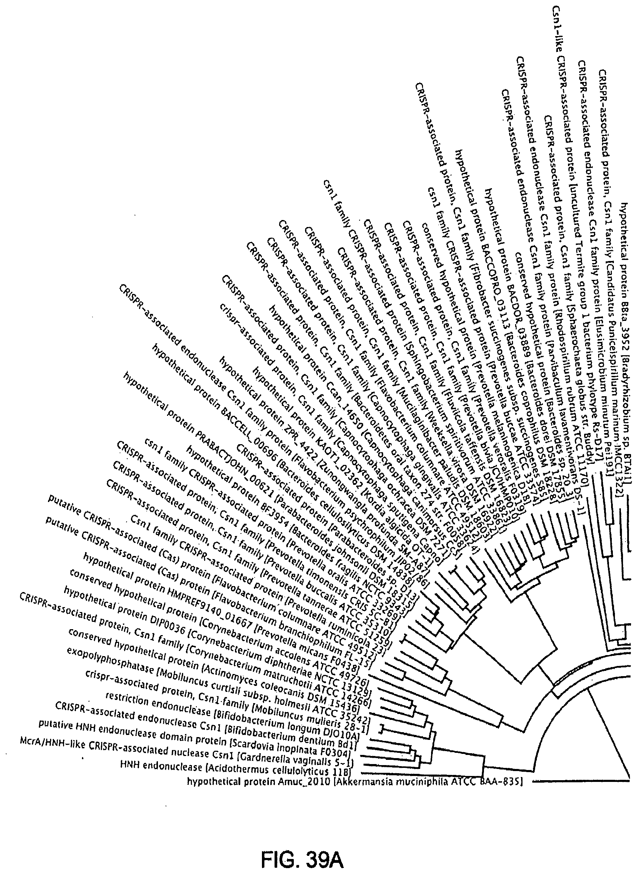

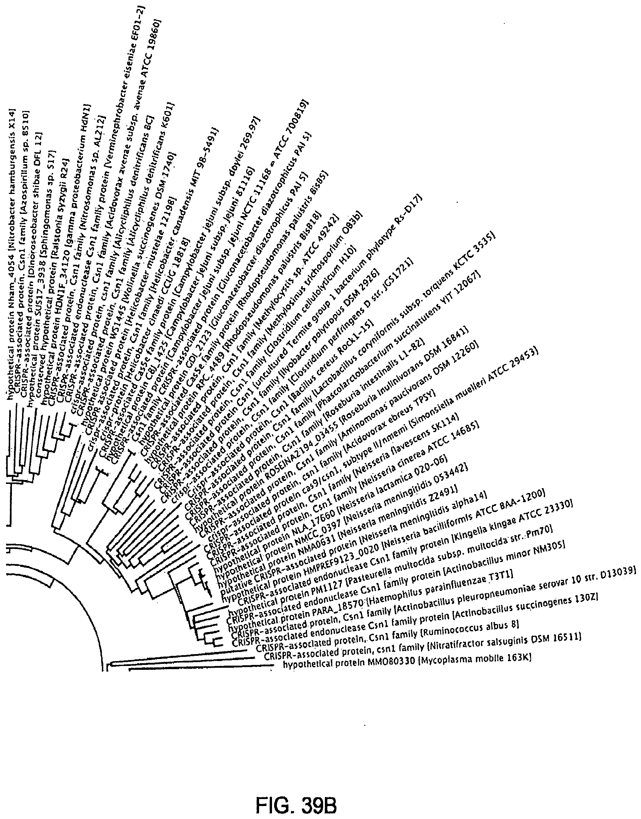

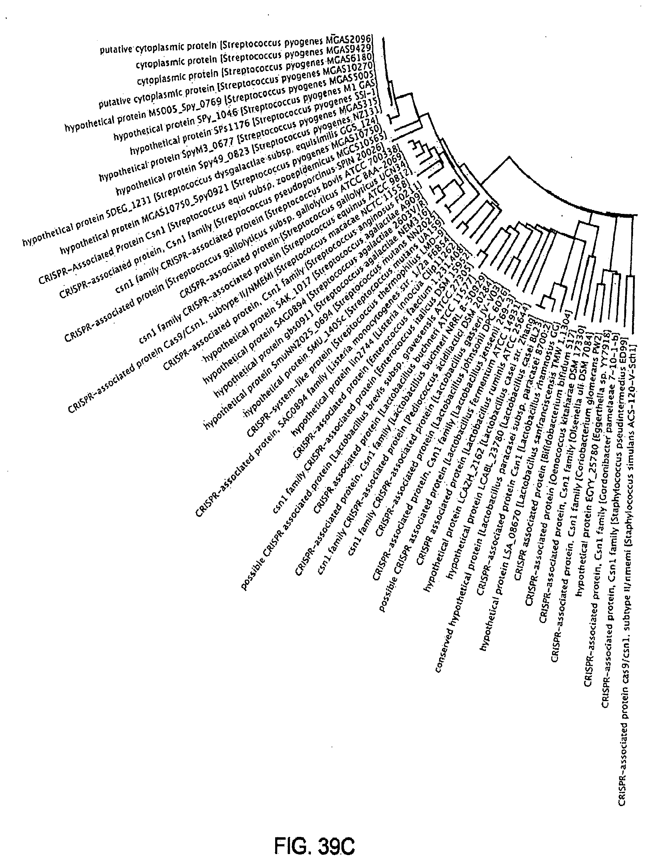

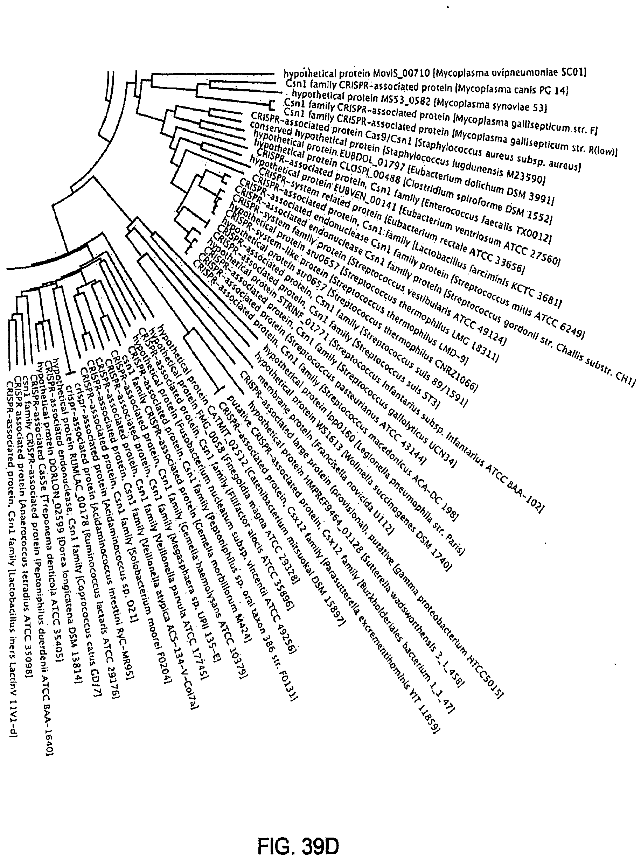

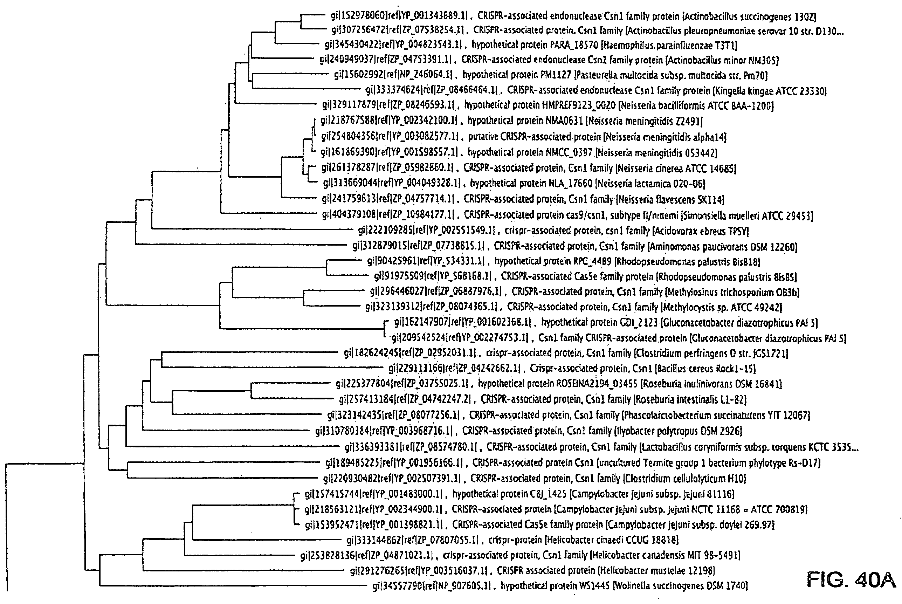

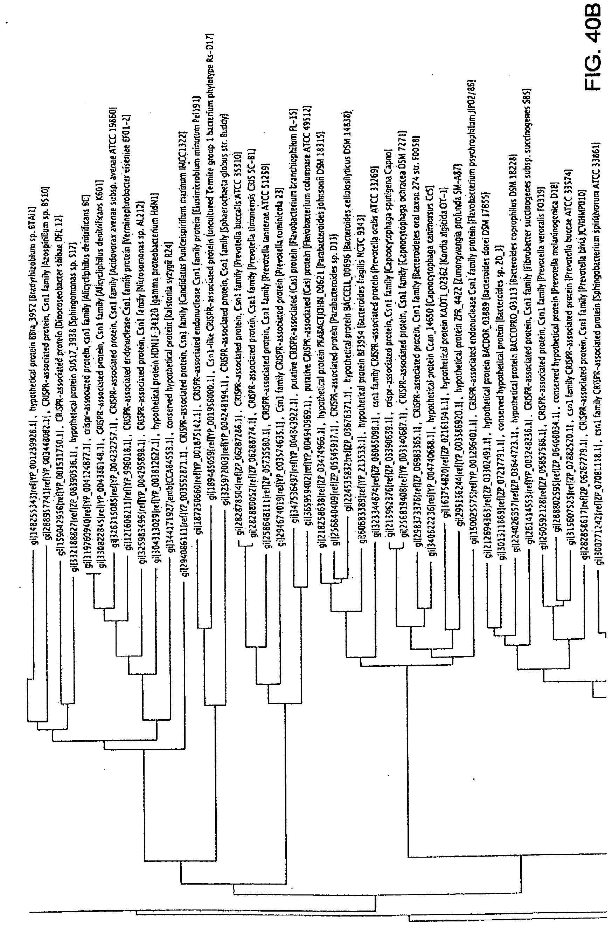

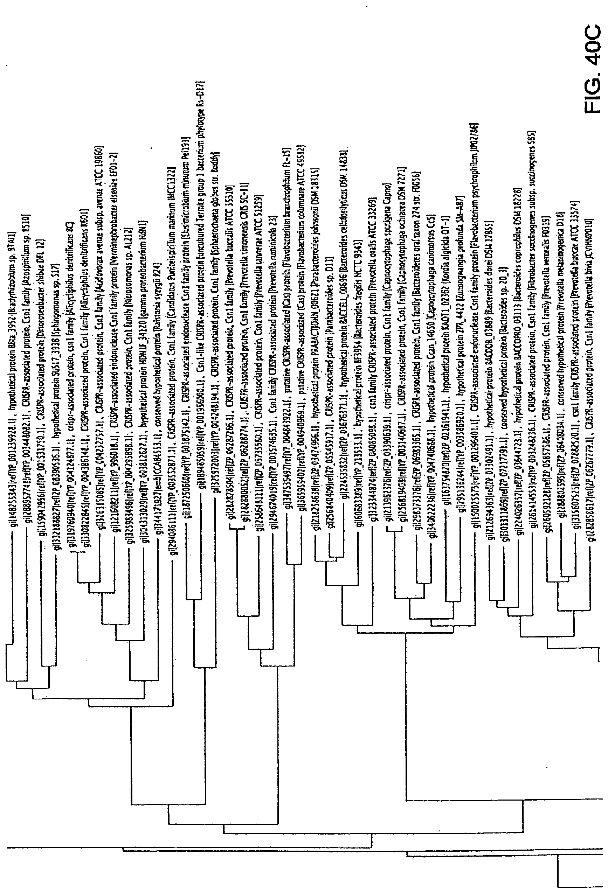

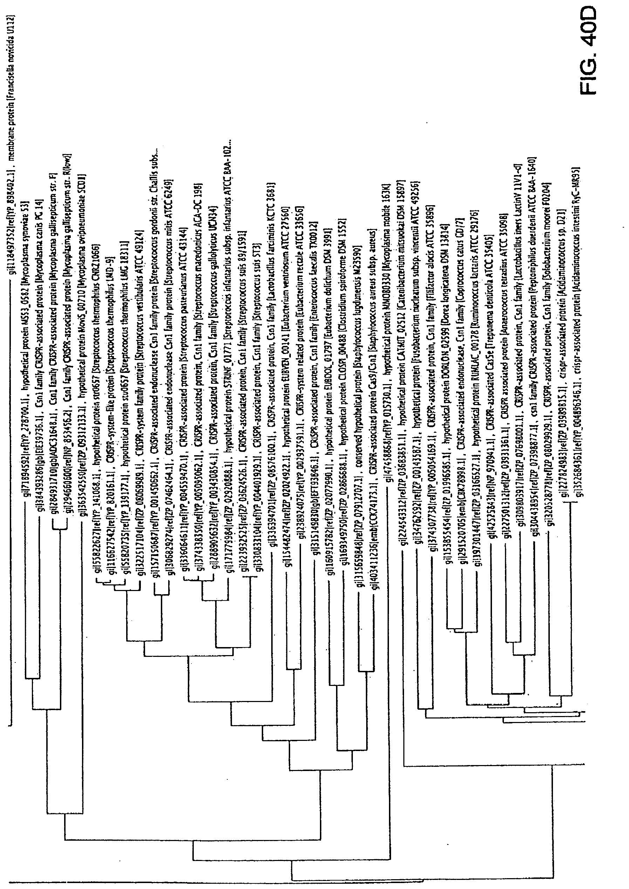

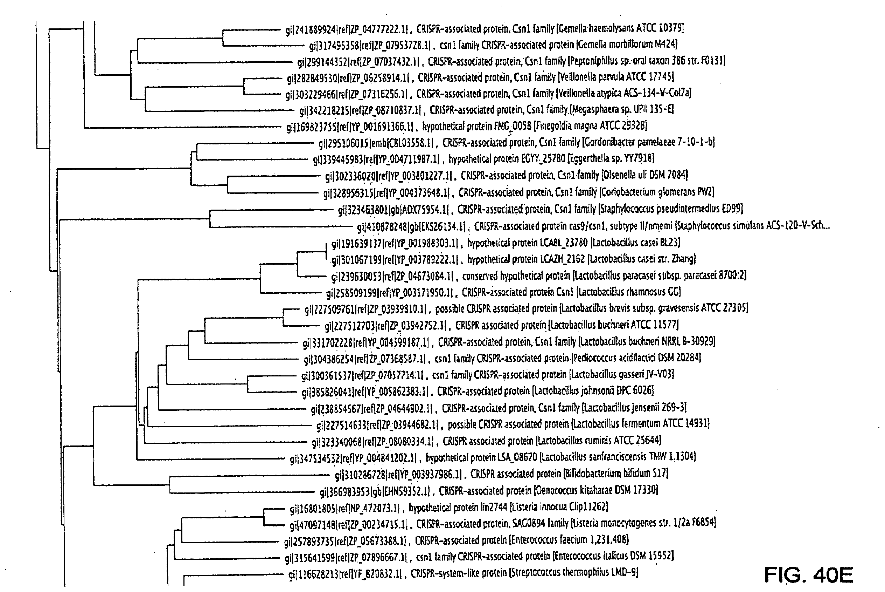

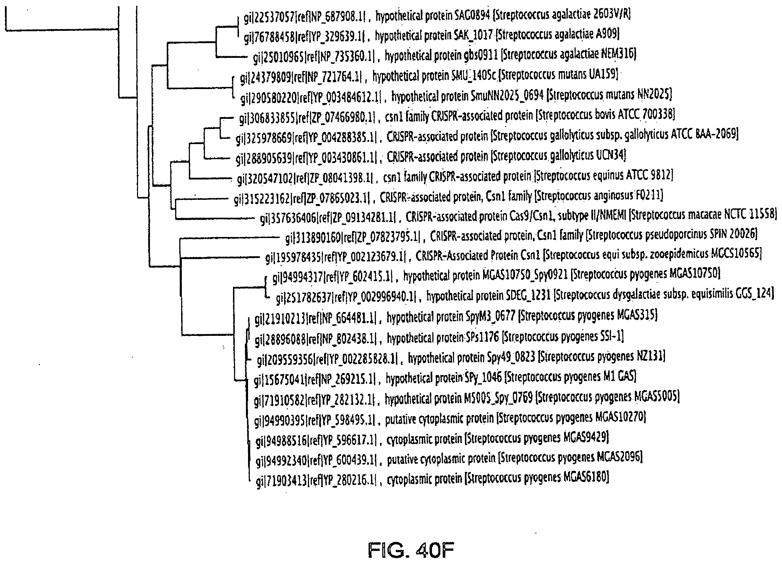

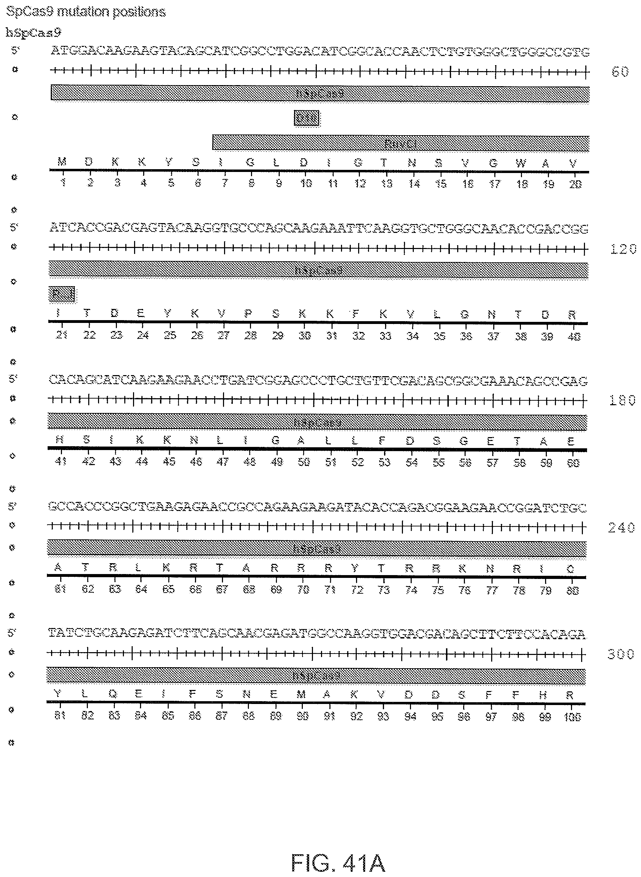

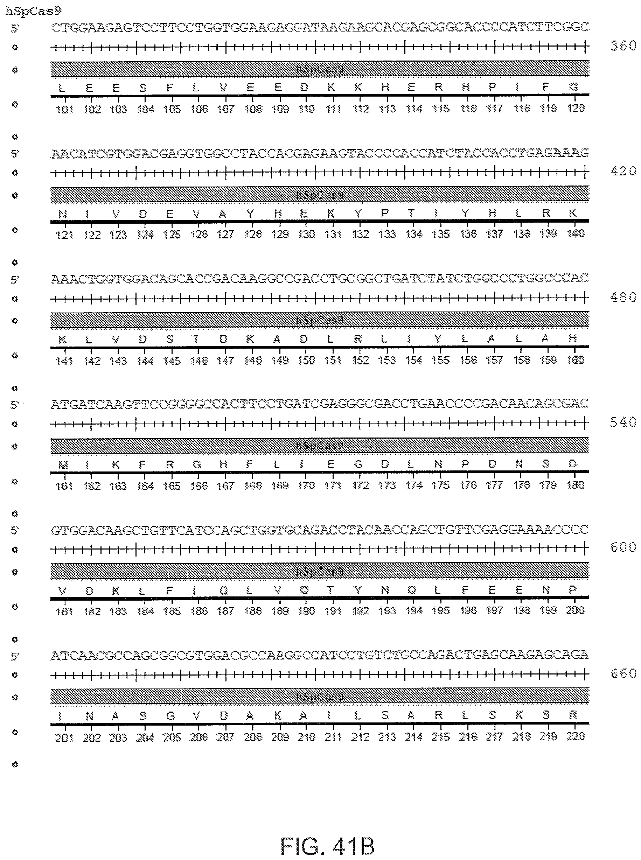

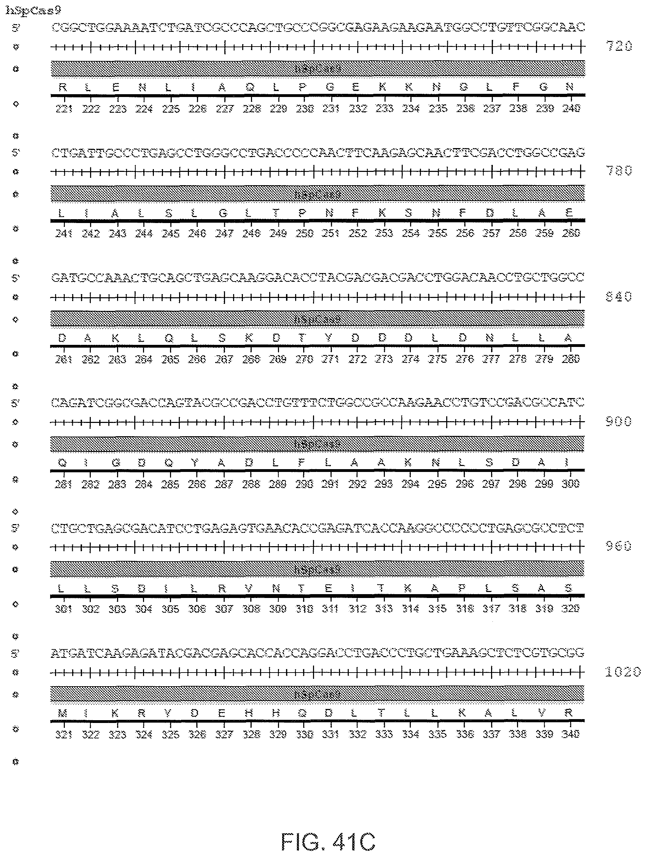

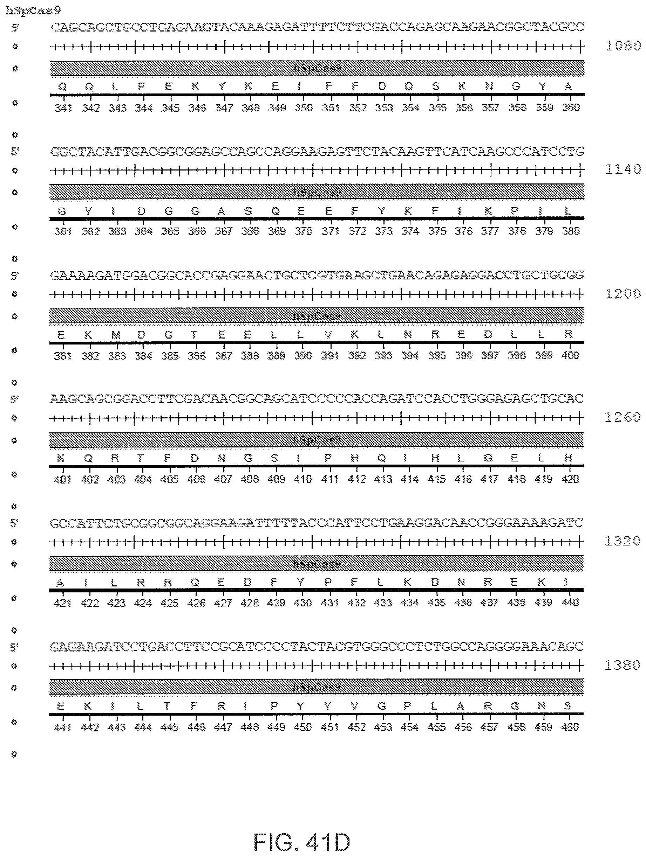

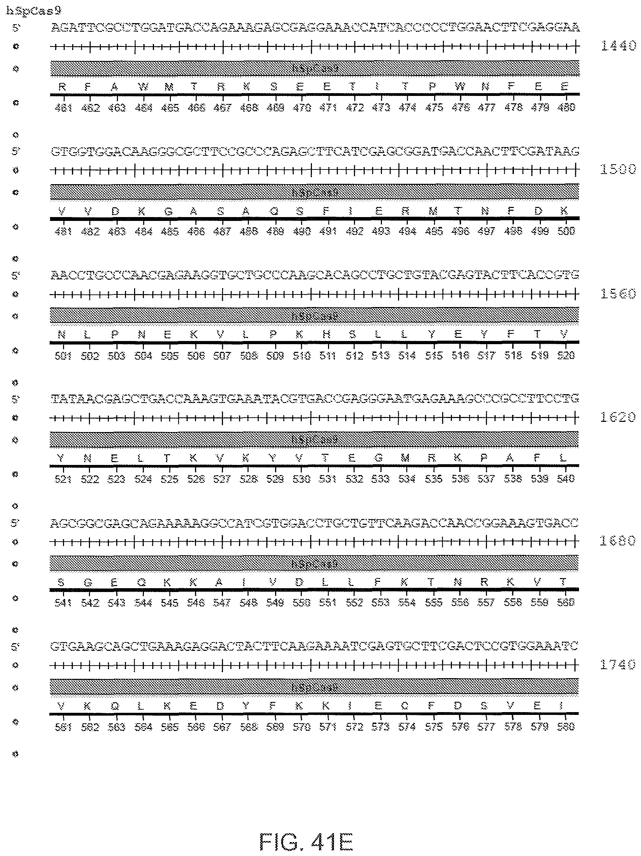

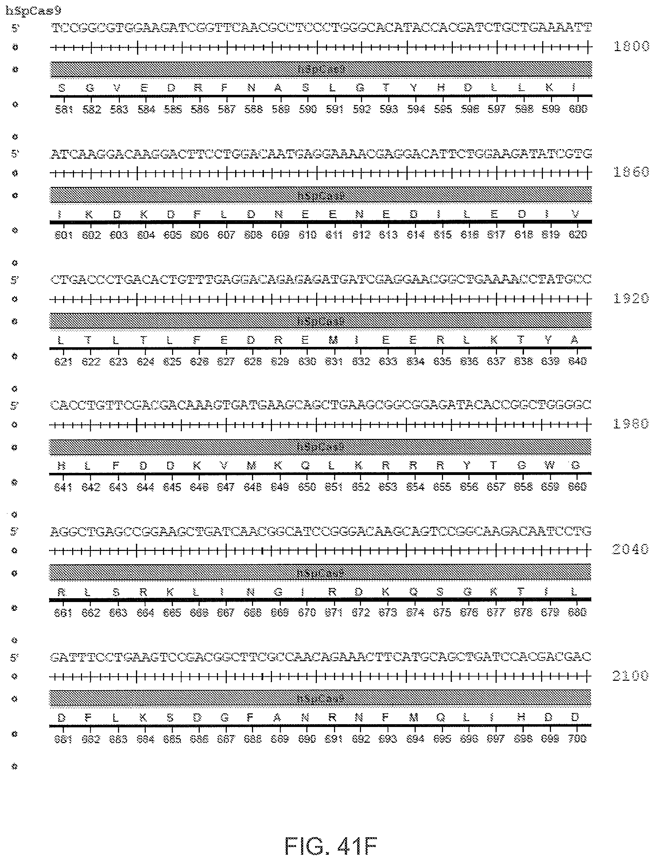

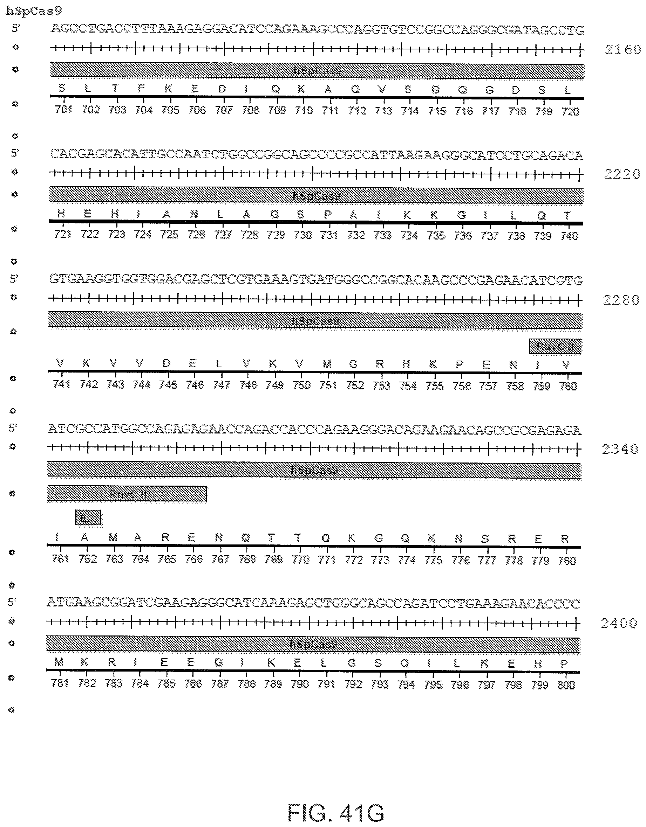

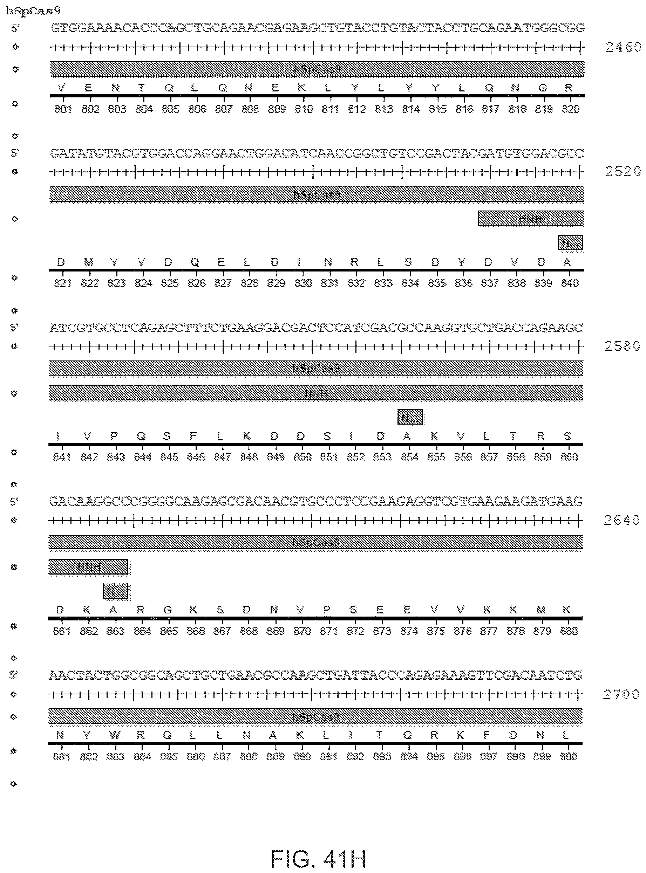

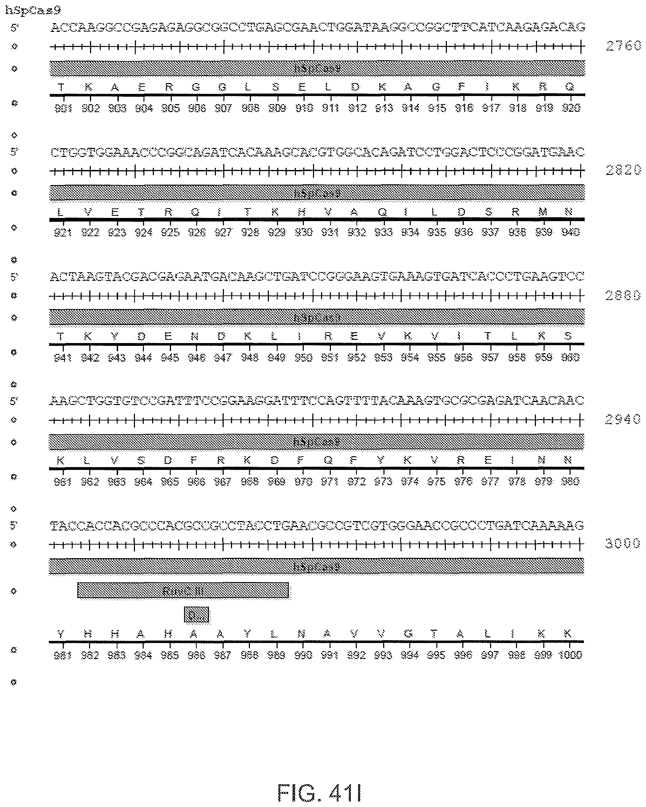

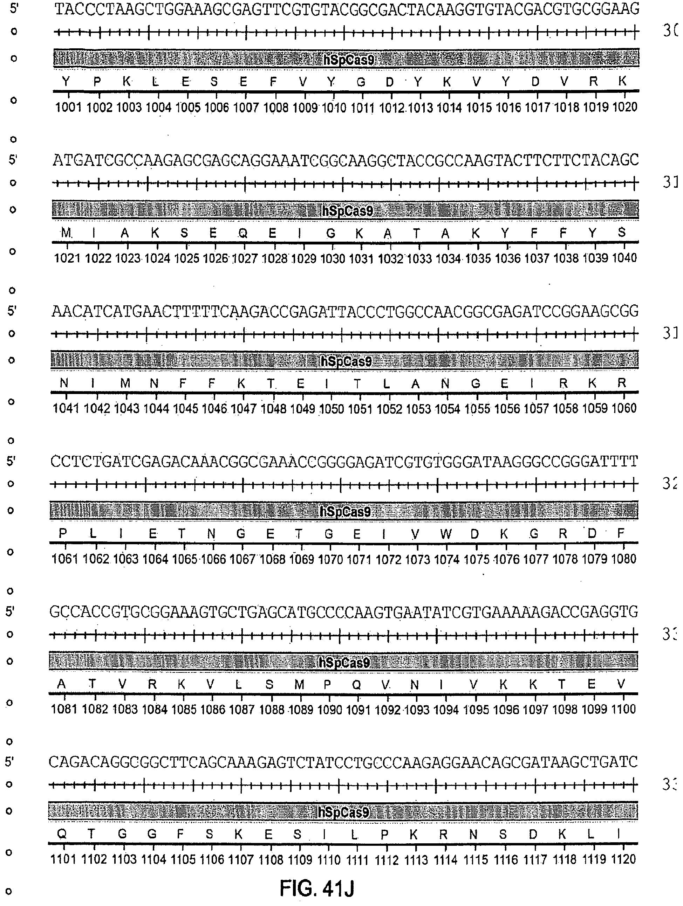

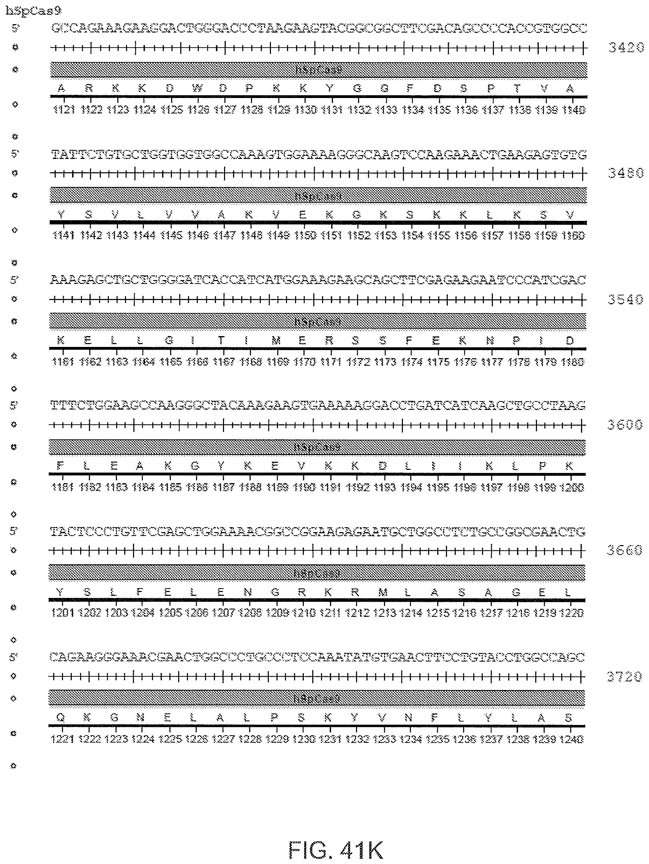

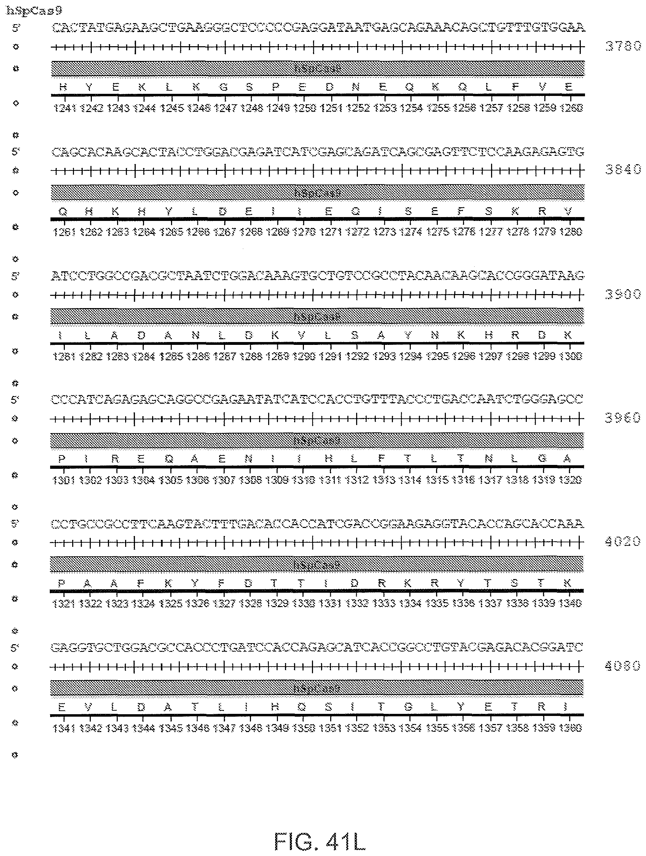

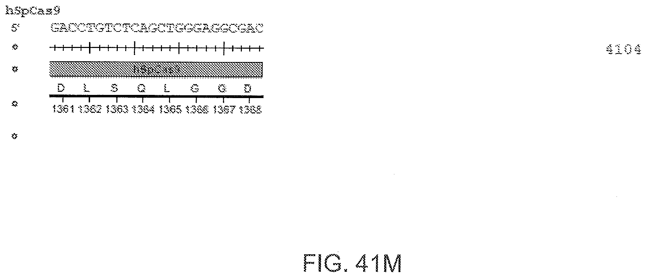

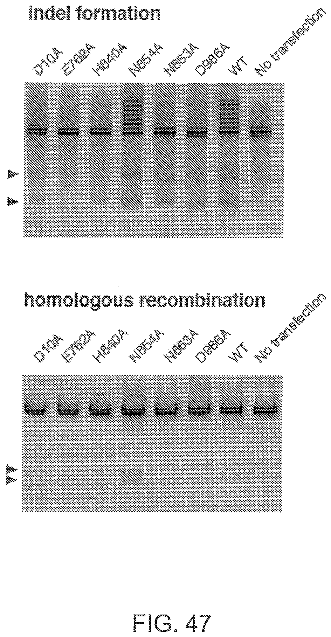

The CRISPR-Cas system is an adaptive immune mechanism against invading exogenous DNA employed by diverse species across bacteria and archaea. The type II CRISPR-Cas9 system consists of a set of genes encoding proteins responsible for the "acquisition" of foreign DNA into the CRISPR locus, as well as a set of genes encoding the "execution" of the DNA cleavage mechanism; these include the DNA nuclease (Cas9), a non-coding transactivating cr-RNA (tracrRNA), and an array of foreign DNA-derived spacers flanked by direct repeats (crRNAs). Upon maturation by Cas9, the tracRNA and crRNA duplex guide the Cas9 nuclease to a target DNA sequence specified by the spacer guide sequences, and mediates double-stranded breaks in the DNA near a short sequence motif in the target DNA that is required for cleavage and specific to each CRISPR-Cas system. The type II CRISPR-Cas systems are found throughout the bacterial kingdom and highly diverse in in Cas9 protein sequence and size, tracrRNA and crRNA direct repeat sequence, genome organization of these elements, and the motif requirement for target cleavage. One species may have multiple distinct CRISPR-Cas systems.
Applicants evaluated 207 putative Cas9s from bacterial species identified based on sequence homology to known Cas9s and structures orthologous to known subdomains, including the HNH endonuclease domain and the RuvC endonuclease domains [information from the Eugene Koonin and Kira Makarova]. Phylogenetic analysis based on the protein sequence conservation of this set revealed five families of Cas9s, including three groups of large Cas9s (~1400 amino acids) and two of small Cas9s (~1100 amino acids) (Figures 39 and 40A-F).
In this example, Applicants show that the following mutations can convert SpCas9 into a nicking enzyme: D10A, E762A, H840A, N854A, N863A, D986A.
Applicants provide sequences showing where the mutation points are located within the SpCas9 gene (Figure 41). Applicants also show that the nickases are still able to mediate homologous recombination (Assay indicated in Figure 2). Furthermore, Applicants show that SpCas9 with these mutations (individually) do not induce double strand break (Figure 47).

*Example 9: Supplement to DNA targeting specificity of the RNA-guided Cas9 nuclease*

Cell culture and transfection

Human embryonic kidney (HEK) cell line 293FT (Life Technologies) was maintained in Dulbecco's modified Eagle's Medium (DMEM) supplemented with 10% fetal bovine serum (HyClone), 2mM GlutaMAX (Life Technologies), 100U/mL penicillin, and 100$\mu$g/mL streptomycin at 37°C with 5% CO2 incubation.
293FT cells were seeded either onto 6-well plates, 24-well plates, or 96-well plates (Corning) 24 hours prior to transfection. Cells were transfected using Lipofectamine 2000 (Life Technologies) at 80-90% confluence following the manufacturer's recommended protocol. For each well of a 6-well plate, a total of 1 ug of Cas9+sgRNA plasmid was used. For each well of a 24-well plate, a total of 500ng Cas9+sgRNA plasmid was used unless otherwise indicated. For each well of a 96-well plate, 65 ng of Cas9 plasmid was used at a 1:1 molar ratio to the U6-sgRNA PCR product.
Human embryonic stem cell line HUES9 (Harvard Stem Cell Institute core) was maintained in feeder-free conditions on GelTrex (Life Technologies) in mTesR medium (Stemcell Technologies) supplemented with 100ug/ml Normocin (InvivoGen). HUES9 cells were transfected with Amaxa P3 Primary Cell 4-D Nucleofector Kit (Lonza) following the manufacturer's protocol.

SURVEYOR nuclease assay for genome modification

293FT cells were transfected with plasmid DNA as described above. Cells were incubated at 37°C for 72 hours post-transfection prior to genomic DNA extraction. Genomic DNA was extracted using the QuickExtract DNA Extraction Solution (Epicentre) following the manufacturer's protocol. Briefly, pelleted cells were resuspended in QuickExtract solution and incubated at 65°C for 15 minutes and 98°C for 10 minutes.
The genomic region flanking the CRISPR target site for each gene was PCR amplified (primers listed in Tables J and K), and products were purified using QiaQuick Spin Column (Qiagen) following the manufacturer's protocol. 400ng total of the purified PCR products were mixed with 2$\mu$l 10X Taq DNA Polymerase PCR buffer (Enzymatics) and ultrapure water to a final volume of 20$\mu$l, and subjected to a re-annealing process to enable heteroduplex formation: 95°C for 10min, 95°C to 85°C ramping at - 2°C/s, 85°C to 25°C at - 0.25°C/s, and 25°C hold for 1 minute. After re-annealing, products were treated with SURVEYOR nuclease and SURVEYOR enhancer S (Transgenomics) following the manufacturer's recommended protocol, and analyzed on 4-20% Novex TBE poly-acrylamide gels (Life Technologies). Gels were stained with SYBR Gold DNA stain (Life Technologies) for 30 minutes and imaged with a Gel Doc gel imaging system (Bio-rad). Quantification was based on relative band intensities.

Northern blot analysis of tracrRNA expression in human cells

Northern blots were performed as previously described1. Briefly, RNAs were heated to 95°C for 5 min before loading on 8% denaturing polyacrylamide gels (SequaGel, National Diagnostics). Afterwards, RNA was transferred to a pre-hybridized Hybond N+ membrane (GE Healthcare) and crosslinked with Stratagene UV Crosslinker (Stratagene). Probes were labeled with [gamma-32P] ATP (Perkin Elmer) with T4 polynucleotide kinase (New England Biolabs). After washing, membrane was exposed to phosphor screen for one hour and scanned with phosphorimager (Typhoon).

Bisulfite sequencing to assess DNA methylation status

HEK 293FT cells were transfected with Cas9 as described above. Genomic DNA was isolated with the DNeasy Blood & Tissue Kit (Qiagen) and bisulfite converted with EZ DNA Methylation-Lightning Kit (Zymo Research). Bisulfite PCR was conducted using KAPA2G Robust HotStart DNA Polymerase (KAPA Biosystems) with primers designed using the Bisulfite Primer Seeker (Zymo Research, Tables J and K). Resulting PCR amplicons were gel-purified, digested with EcoRI and HindIII, and ligated into a pUC19 backbone prior to transformation. Individual clones were then Sanger sequenced to assess DNA methylation status.

*In vitro* transcription and cleavage assay

HEK 293FT cells were transfected with Cas9 as described above. Whole cell lysates were then prepared with a lysis buffer (20 mM HEPES, 100 mM KC1, 5 mM MgCl2, 1 mM DTT, 5% glycerol, 0.1% Triton X-100) supplemented with Protease Inhibitor Cocktail (Roche). T7-driven sgRNA was in vitro transcribed using custom oligos (Example 10) and HiScribe T7 In Vitro Transcription Kit (NEB), following the manufacturer's recommended protocol. To prepare methylated target sites, pUC19 plasmid was methylated by M.SssI and then linearized by NheI. The in vitro cleavage assay was performed as follows: for a 20 uL cleavage reaction, 10 uL of cell lysate with incubated with 2 uL cleavage buffer (100 mM HEPES, 500 mM KCl, 25 mM MgCl2, 5 mM DTT, 25% glycerol), the in vitro transcribed RNA, and 300 ng pUC19 plasmid DNA.

Deep sequencing to assess targeting specificity

HEK 293FT cells plated in 96-well plates were transfected with Cas9 plasmid DNA and single guide RNA (sgRNA) PCR cassette 72 hours prior to genomic DNA extraction (Fig. 72). The genomic region flanking the CRISPR target site for each gene was amplified (Fig. 74, Fig. 80, (Example 10) by a fusion PCR method to attach the Illumina P5 adapters as well as unique sample-specific barcodes to the target amplicons (schematic described in Fig. 73). PCR products were purified using EconoSpin 96-well Filter Plates (Epoch Life Sciences) following the manufacturer's recommended protocol.

Barcoded and purified DNA samples were quantified by Quant-iT PicoGreen dsDNA Assay Kit or Qubit 2.0 Fluorometer (Life Technologies) and pooled in an equimolar ratio. Sequencing libraries were then deep sequenced with the Illumina MiSeq Personal Sequencer (Life Technologies).

Sequencing data analysis and indel detection

MiSeq reads were filtered by requiring an average Phred quality (Q score) of at least 23, as well as perfect sequence matches to barcodes and amplicon forward primers. Reads from on- and off-target loci were analyzed by first performing Smith-Waterman alignments against amplicon sequences that included 50 nucleotides upstream and downstream of the target site (a total of 120 bp). Alignments, meanwhile, were analyzed for indels from 5 nucleotides upstream to 5 nucleotides downstream of the target site (a total of 30 bp). Analyzed target regions were discarded if part of their alignment fell outside the MiSeq read itself, or if matched base-pairs comprised less than 85% of their total length.

Negative controls for each sample provided a gauge for the inclusion or exclusion of indels as putative cutting events. For each sample, an indel was counted only if its quality score exceeded $\mu - \sigma$, where $\mu$ was the mean quality-score of the negative control corresponding to that sample and $\sigma$ was the standard deviation of same. This yielded whole target-region indel rates for both negative controls and their corresponding samples. Using the negative control's per-target-region-per-read error rate, $q$, the sample's observed indel count $n$, and its read-count $R$, a maximum-likelihood estimate for the fraction of reads having target-regions with true-indels, $p$, was derived by applying a binomial error model, as follows.

Letting the (unknown) number of reads in a sample having target regions incorrectly counted as having at least 1 indel be $E$, we can write (without making any assumptions about the number of true indels)

$$\mathrm{Prob}(E|p) = \binom{R(1-p)}{E} q^E (1-q)^{R(1-p)-E}$$

since $R(1\text{-}p)$ is the number of reads having target-regions with no true indels. Meanwhile, because the number of reads observed to have indels is $n$, $n = E + Rp$, in other words the number of reads having target-regions with errors but no true indels plus the number of reads whose target-regions correctly have indels. We can then re-write the above

$$\mathrm{Prob}(E|p) = \mathrm{Prob}(n = E + Rp|p) = \binom{R(1-p)}{n-Rp} q^{n-Rp}(1-q)^{R-n}$$

Taking all values of the frequency of target-regions with true-indels $p$ to be equally probable a priori, $\mathrm{Prob}(n|p) \propto \mathrm{Prob}(p|n)$. The maximum-likelihood estimate (MLE) for the frequency of target regions with true-indels was therefore set as the value of $p$ that maximized $\mathrm{Prob}(n|p)$. This was evaluated numerically.

In order to place error bounds on the true-indel read frequencies in the sequencing libraries themselves, Wilson score intervals (2) were calculated for each sample, given the MLE-estimate for true-indel target-regions, $Rp$, and the number of reads $R$. Explicitly, the lower bound $l$ and upper bound $u$ were calculated as

$$l = \left(Rp + \frac{z^2}{2} - z\sqrt{Rp(1-p) + z^2/4}\right)/(R + z^2)$$

$$u = \left(Rp + \frac{z^2}{2} + z\sqrt{Rp(1-p) + z^2/4}\right)/(R + z^2)$$

where $z$, the standard score for the confidence required in normal distribution of variance 1, was set to 1.96, meaning a confidence of 95%. The maximum upper bounds and minimum lower bounds for each biological replicate are listed in Figs. 80-83.

qRT-PCR analysis of relative Cas9 and sgRNA expression

293FT cells plated in 24-well plates were transfected as described above. 72 hours post-transfection, total RNA was harvested with miRNeasy Micro Kit (Qiagen). Reverse-strand synthesis for sgRNAs was performed with qScript Flex cDNA kit (VWR) and custom first-strand synthesis primers (Tables J and K). qPCR analysis was performed with Fast SYBR Green Master Mix (Life Technologies) and custom primers (Tables J and K), using GAPDH as an endogenous control. Relative quantification was calculated by the $\Delta\Delta$CT method.

Table I | Target site sequences. Tested target sites for *S. pyogenes* type II CRISPR system with the requisite PAM. Cells were transfected with Cas9 and either crRNA-tracrRNA or chimeric sgRNA for each target.

| Target site ID | genomic target | Target site sequence (5' to 3') | PAM | strand |
|---|---|---|---|---|
| 1 | EMX1 | GTCACCTCCAATGACTAGGG | TGG | + |
| 2 | EMX1 | GACATCGATGTCCTCCCCAT | TGG | - |
| 3 | EMX1 | GAGTCCGAGCAGAAGAAGAA | GGG | + |
| 6 | EMX1 | GCGCCACCGGTTGATGTGAT | GGG | - |
| 10 | EMX1 | GGGGCACAGATGAGAAACTC | AGG | - |
| 11 | EMX1 | GTACAAACGGCAGAAGCTGG | AGG | + |

(continued)

| Target site ID | genomic target | Target site sequence (5' to 3') | PAM | strand |
|---|---|---|---|---|
| 12 | EMX1 | GGCAGAAGCTGGAGGAGGAA | GGG | + |
| 13 | EMX1 | GGAGCCCTTCTTCTTCTGCT | CGG | - |
| 14 | EMX1 | GGGCAACCACAAACCCACGA | GGG | + |
| 15 | EMX1 | GCTCCCATCACATCAACCGG | TGG | + |
| 16 | EMX1 | GTGGCGCATTGCCACGAAGC | AGG | + |
| 17 | EMX1 | GGCAGAGTGCTGCTTGCTGC | TGG | + |
| 18 | EMX1 | GCCCCTGCGTGGGCCCAAGC | TGG | + |
| 19 | EMX1 | GAGTGGCCAGAGTCCAGCTT | GGG | - |
| 20 | EMX1 | GGCCTCCCCAAAGCCTGGCC | AGG | - |
| 4 | PVALB | GGGGCCGAGATTGGGTGTTC | AGG | + |
| 5 | PVALB | GTGGCGAGAGGGGCCGAGAT | TGG | + |
| 1 | SERPINB5 | GAGTGCCGCCGAGGCGGGGC | GGG | + |
| 2 | SERPINB5 | GGAGTGCCGCCGAGGCGGGG | CGG | + |
| 3 | SERPINB5 | GGAGAGGAGTGCCGCCGAGG | CGG | + |

Table J | Primer sequences

| SURVEYOR assay | | |
|---|---|---|
| primer name | genomic target | primer sequence (5' to 3') |
| Sp-EMX1-F1 | EMX1 | AAAACCACCCTTCTCTCTGGC |
| Sp-EMX1-R1 | EMX1 | GGAGATTGGAGACACGGAGAG |
| Sp-EMX1-F2 | EMX1 | CCATCCCCTTCTGTGAATGT |
| Sp-EMX1-R2 | EMX1 | GGAGATTGGAGACACGGAGA |
| Sp-PVALB-F | PVALB | CTGGAAAGCCAATGCCTGAC |
| Sp-PVALB-R | PVALB | GGCAGCAAACTCCTTGTCCT |
| qRT-PCR for Cas9 and sgRNA expression | | |
| primer name | | primer sequence (5' to 3') |
| sgRNA reverse-strand synthesis | | AAGCACCGACTCGGTGCCAC |
| EMX1.1 sgRNA qPCR F | | TCACCTCCAATGACTAGGGG |
| EMX1.1 sgRNA qPCR R | | CAAGTTGATAACGGACTAGCCT |
| EMX1.3 sgRNA qPCR F | | AGTCCGAGCAGAAGAAGAAGTTT |
| EMX1.3 sgRNA qPCR R | | TTTCAAGTTGATAACGGACTAGCCT |
| Cas9 qPCR F | | AAACAGCAGATTCGCCTGGA |
| Cas9 qPCR R | | TCATCCGCTCGATGAAGCTC |
| GAPDH qPCR F | | TCCAAAATCAAGTGGGGCGA |
| GAPDH qPCR R | | TGATGACCCTTTTGGCTCCC |

(continued)

| Bisulfite PCR and sequencing | |
|---|---|
| **primer name** | **primer sequence (5' to 3')** |
| Bisulfite PCR F (SERPINB5 locus) | GAGGAATTCTTTTTTTGTTYGAATATGTTGGAGG TTTTTTGGAAG |
| Bisulfite PCR R (SERPINB5 locus) | GAGAAGCTTAAATAAAAAACRACAATACTCAAC CCAACAACC |
| pUC19 sequencing | CAGGAAACAGCTATGAC |

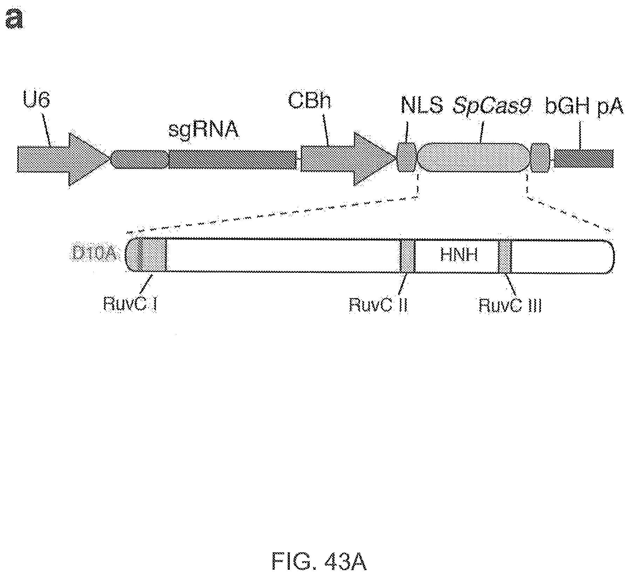

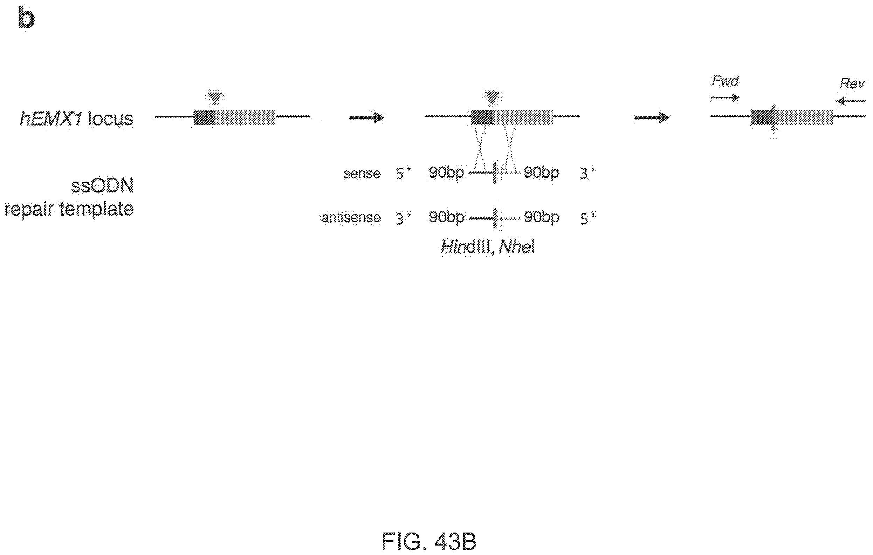

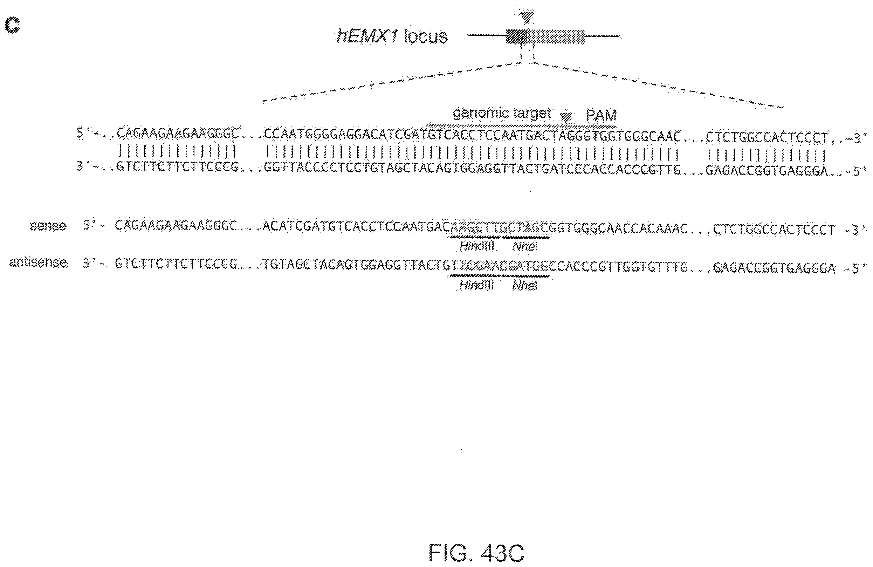

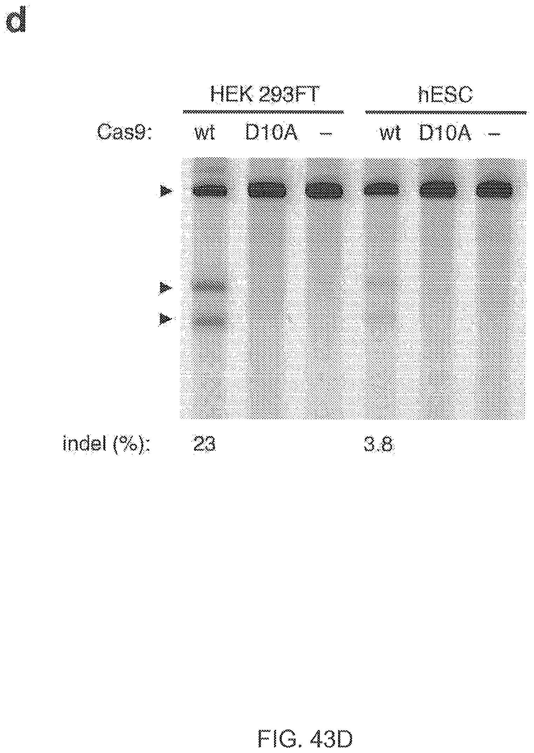

Table K | Sequences for primers to test sgRNA architecture. Primers hybridize to the reverse strand of the U6 promoter unless otherwise indicated. The U6 priming site is in *italics*, the guide sequence is indicated as a stretch of Ns, the direct repeat sequence is highlighted in bold, and the tracrRNA sequence underlined. The secondary structure of each sgRNA architecture is shown in Fig. 43.

| primer name | primer sequence (5' to 3') |
|---|---|
| **U6-Forward** | GCCTCTA*GAGGTACCTGAGGGCCTATTTCCCATGATTCC* |
| I: sgRNA(DR +12, tracrRNA +85) | ACCTCTAG<u>AAAAAAAGCACCGACTCGGTGCCACTTTTTCAA GTTGATAACGGACTAGCCTTATTTTAACTTGCTATTTC</u>**TAGC TCTAAAAC**NNNNNNNNNNNNNNNNNNNN*GGTGTTTCGTCCT TTCCACAAG* |
| II: sgRNA(DR +12, tracrRNA +85) mut2 | ACCTCTAG<u>AAAAAAAGCACCGACTCGGTGCCACTTTTTCAA GTTGATAACGGACTAGCCTTATATTAACTTGCTATTTC</u>**TAGC TCTAATAC**NNNNNNNNNNNNNNNNNNNN*GGTGTTTCGTCCT TTCCACAAG* |
| III: sgRNA(DR +22, tracrRNA +85) | ACCTCTAG<u>AAAAAAAGCACCGACTCGGTGCCACTTTTTCAA GTTGATAACGGACTAGCCTTATTTTAACTTGCTATGCTGTTTT GTTTC</u>**CAAAACAGCATAGCTCTAAAC**NNNNNNNNNNNNNNN NNNNNNNN*GGTGTTTCGTCCTTTCCACAAG* |
| IV: sgRNA(DR +22, tracrRNA +85) mut4 | ACCTCTAG<u>AAAAAAAGCACCGACTCGGTGCCACTTTTTCAA GTTGATAACGGACTAGCCTTATATTAACTTGCTATGCTGTAT TGTTTC</u>**CAATACAGCATAGCTCTAATAC**NNNNNNNNNNNNN NNNNNNNNN*GGTGTTTCGTCCTTTCCACAAG* |

Table L | Target sites with alternate PAMs for testing PAM specificity of Cas9. All target sites for PAM specificity testing are found within the human EMX1 locus.

| Target site sequence (5' to 3') | PAM |
|---|---|
| AGGCCCCAGTGGCTGCTCT | NAA |
| ACATCAACCGGTGGCGCAT | NAT |
| AAGGTGTGGTTCCAGAACC | NAC |
| CCATCACATCAACCGGTGG | NAG |
| AAACGGCAGAAGCTGGAGG | NTA |
| GGCAGAAGCTGGAGGAGGA | NTT |
| GGTGTGGTTCCAGAACCGG | NTC |

(continued)

| Target site sequence (5' to 3') | PAM |
|---|---|
| AACCGGAGGACAAAGTACA | NTG |
| TTCCAGAACCGGAGGACAA | NCA |
| GTGTGGTTCCAGAACCGGA | NCT |
| TCCAGAACCGGAGGACAAA | NCC |
| CAGAAGCTGGAGGAGGAAG | NCG |
| CATCAACCGGTGGCGCATT | NGA |
| GCAGAAGCTGGAGGAGGAA | NGT |
| CCTCCCTCCCTGGCCCAGG | NGC |
| TCATCTGTGCCCCTCCCTC | NAA |
| GGGAGGACATCGATGTCAC | NAT |
| CAAACGGCAGAAGCTGGAG | NAC |
| GGGTGGGCAACCACAAACC | NAG |
| GGTGGGCAACCACAAACCC | NTA |
| GGCTCCCATCACATCAACC | NTT |
| GAAGGGCCTGAGTCCGAGC | NTC |
| CAACCGGTGGCGCATTGCC | NTG |
| AGGAGGAAGGGCCTGAGTC | NCA |
| AGCTGGAGGAGGAAGGGCC | NCT |
| GCATTGCCACGAAGCAGGC | NCC |
| ATTGCCACGAAGCAGGCCA | NCG |
| AGAACCGGAGGACAAAGTA | NGA |
| TCAACCGGTGGCGCATTGC | NGT |
| GAAGCTGGAGGAGGAAGGG | NGC |

*Example 10: Supplementary Sequences*

All sequences are in the 5' to 3' direction. For U6 transcription, the string of underlined Ts serve as the transcriptional terminator.

> U6-short tracrRNA (*Streptococcus pyogenes* SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgttttaaaatggactatcatatgc ttaccgtaacttgaaagtatttcgatttcttggctttatatatcttgtggaaaggacgaaacacc**GGAACCATTCAAAACAGC ATAGCAAGTTAAAATAAGGCTAGTCCGTTATCAACTTGAAAAAGTGGCACCGA GTCGGTGC**<u>TTTTTTT</u>

(tracrRNA sequence in **bold**)
>U6-DR-guide sequence-DR (*Streptococcus pyogenes* SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacaccgggttttagagctatgctgttttgaatggtccc aaaac**NNNNNNNNNNNNNNNNNNNNNNNNNNNNNN**gttttagagctatgctgttttgaatggtcccaaaacT TTTTTT

(direct repeat sequence is highlighted in gray and the guide sequence is in bold Ns)
> sgRNA containing +48 tracrRNA (*Streptococcus pyogenes* SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacacc**NNNNNNNNNNNNNNNNNN NN**gttttagagctagaaa**tagcaagttaaaataaggctagtccg**TTTTTTT

(guide sequence is in bold Ns and the tracrRNA fragment is in bold)
> sgRNA containing +54 tracrRNA (*Streptococcus pyogenes* SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacacc**NNNNNNNNNNNNNNNNNN NN**gttttagagctagaaa**tagcaagttaaaataaggctagtccgttatca**TTTTTTT

(guide sequence is in bold Ns and the tracrRNA fragment is in **bold**)
> sgRNA containing +67 tracrRNA (*Streptococcus pyogenes* SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacacc**NNNNNNNNNNNNNNNNNN NN**gttttagagctagaaa**tagcaagttaaaataaggctagtccgttatcaacttgaaaaagtg**TTTTTTT

(guide sequence is in **bold Ns** and the tracrRNA fragment is in **bold**)
> sgRNA containing +85 tracrRNA (*Streptococcus pyogenes* SF370)

gagggcctatttcccatgattccttcatatttgcatatacgatacaaggctgttagagagataattggaattaatttgactgtaaa cacaaagatattagtacaaaatacgtgacgtagaaagtaataatttcttgggtagtttgcagttttaaaattatgtttaaaatggactatcatatgc ttaccgtaacttgaaagtatttcgatttcttggctttatatatcttgtggaaaggacgaaacacc**NNNNNNNNNNNNNNNNNN NN**gttttagagctagaaa**tagcaagttaaaataaggctagtccgttatcaacttgaaaaagtggcaccgagtcggtgc**TTTTTT T

(guide sequence is in **bold Ns** and the tracrRNA fragment is in **bold**)
> CBh-NLS-SpCas9-NLS

CGTTACATAACTTACGGTAAATGGCCCGCCTGGCTGACCGCCCAACGACC
CCCGCCCATTGACGTCAATAATGACGTATGTTCCCATAGTAACGCCAATAGGGACTT
TCCATTGACGTCAATGGGTGGAGTATTTACGGTAAACTGCCCACTTGGCAGTACATC
AAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTA
CGTATTAGTCATCGCTATTACCATGGTCGAGGTGAGCCCCACGTTCTGCTTCACTCTC
CCCATCTCCCCCCCCTCCCCACCCCCAATTTTGTATTTATTTATTTTTTAATTATTTTG
TGCAGCGATGGGGGCGGGGGGGGGGGGGGGGGCGCGCGCCAGGCGGGGCGGGGCGG
GGCGAGGGGCGGGGCGGGGCGAGGCGGAGAGGTGCGGCGGCAGCCAATCAGAGCG
GCGCGCTCCGAAAGTTTCCTTTTATGGCGAGGCGGCGGCGGCGGCGGCCCTATAAA
AAGCGAAGCGCGCGGCGGGCGGGAGTCGCTGCGACGCTGCCTTCGCCCCGTGCCCC
GCTCCGCCGCCGCCTCGCGCCGCCCGCCCCGGCTCTGACTGACCGCGTTACTCCCAC
AGGTGAGCGGGCGGGACGGCCCTTCTCCTCCGGGCTGTAATTAGCTGAGCAAGAGG
TAAGGGTTTAAGGGATGGTTGGTTGGTGGGGTATTAATGTTTAATTACCTGGAGCAC
CTGCCTGAAATCACTTTTTTTCAGGTTGGaccggtgccacc**ATGGACTATAAGGACCACG**
**ACGGAGACTACAAGGATCATGATATTGATTACAAAGACGATGACGATAAGATG**
**GCCCCAAAGAAGAAGCGGAAGGTCGGTATCCACGGAGTCCCAGCAGCCGACAA**
**GAAGTACAGCATCGGCCTGGACATCGGCACCAACTCTGTGGGCTGGGCCGTGA**
**TCACCGACGAGTACAAGGTGCCCAGCAAGAAATTCAAGGTGCTGGGCAACACC**
**GACCGGCACAGCATCAAGAAGAACCTGATCGGAGCCCTGCTGTTCGACAGCGG**
**CGAAACAGCCGAGGCCACCCGGCTGAAGAGAACCGCCAGAAGAAGATACACCA**
**GACGGAAGAACCGGATCTGCTATCTGCAAGAGATCTTCAGCAACGAGATGGCC**
**AAGGTGGACGACAGCTTCTTCCACAGACTGGAAGAGTCCTTCCTGGTGGAAGA**
**GGATAAGAAGCACGAGCGGCACCCCATCTTCGGCAACATCGTGGACGAGGTGG**
**CCTACCACGAGAAGTACCCCACCATCTACCACCTGAGAAAGAAACTGGTGGAC**
**AGCACCGACAAGGCCGACCTGCGGCTGATCTATCTGGCCCTGGCCCACATGAT**
**CAAGTTCCGGGGCCACTTCCTGATCGAGGGCGACCTGAACCCCGACAACAGCG**
**ACGTGGACAAGCTGTTCATCCAGCTGGTGCAGACCTACAACCAGCTGTTCGAG**
**GAAAACCCCATCAACGCCAGCGGCGTGGACGCCAAGGCCATCCTGTCTGCCAG**
**ACTGAGCAAGAGCAGACGGCTGGAAAATCTGATCGCCCAGCTGCCCGGCGAGA**

AGAAGAATGGCCTGTTCGGCAACCTGATTGCCCTGAGCCTGGGCCTGACCCCC

AACTTCAAGAGCAACTTCGACCTGGCCGAGGATGCCAAACTGCAGCTGAGCAA

GGACACCTACGACGACGACCTGGACAACCTGCTGGCCCAGATCGGCGACCAGT

ACGCCGACCTGTTTCTGGCCGCCAAGAACCTGTCCGACGCCATCCTGCTGAGC

GACATCCTGAGAGTGAACACCGAGATCACCAAGGCCCCCCTGAGCGCCTCTAT

GATCAAGAGATACGACGAGCACCACCAGGACCTGACCCTGCTGAAAGCTCTCG

TGCGGCAGCAGCTGCCTGAGAAGTACAAAGAGATTTTCTTCGACCAGAGCAAG

AACGGCTACGCCGGCTACATTGACGGCGGAGCCAGCCAGGAAGAGTTCTACAA

GTTCATCAAGCCCATCCTGGAAAAGATGGACGGCACCGAGGAACTGCTCGTGA

AGCTGAACAGAGAGGACCTGCTGCGGAAGCAGCGGACCTTCGACAACGGCAG

CATCCCCCACCAGATCCACCTGGGAGAGCTGCACGCCATTCTGCGGCGGCAGG

AAGATTTTTACCCATTCCTGAAGGACAACCGGGAAAAGATCGAGAAGATCCTG

ACCTTCCGCATCCCCTACTACGTGGGCCCTCTGGCCAGGGGAAACAGCAGATT

CGCCTGGATGACCAGAAAGAGCGAGGAAACCATCACCCCCTGGAACTTCGAGG

AAGTGGTGGACAAGGGCGCTTCCGCCCAGAGCTTCATCGAGCGGATGACCAAC

TTCGATAAGAACCTGCCCAACGAGAAGGTGCTGCCCAAGCACAGCCTGCTGTA

CGAGTACTTCACCGTGTATAACGAGCTGACCAAAGTGAAATACGTGACCGAGG

GAATGAGAAAGCCCGCCTTCCTGAGCGGCGAGCAGAAAAAGGCCATCGTGGAC

CTGCTGTTCAAGACCAACCGGAAAGTGACCGTGAAGCAGCTGAAAGAGGACTA

CTTCAAGAAAATCGAGTGCTTCGACTCCGTGGAAATCTCCGGCGTGGAAGATC

GGTTCAACGCCTCCCTGGGCACATACCACGATCTGCTGAAAATTATCAAGGAC

AAGGACTTCCTGGACAATGAGGAAACGAGGACATTCTGGAAGATATCGTGCT

GACCCTGACACTGTTTGAGGACAGAGAGATGATCGAGGAACGGCTGAAAACCT

ATGCCCACCTGTTCGACGACAAAGTGATGAAGCAGCTGAAGCGGCGGAGATAC

ACCGGCTGGGGCAGGCTGAGCCGGAAGCTGATCAACGGCATCCGGGACAAGC

AGTCCGGCAAGACAATCCTGGATTTCCTGAAGTCCGACGGCTTCGCCAACAGA

AACTTCATGCAGCTGATCCACGACGACAGCCTGACCTTTAAAGAGGACATCCA

GAAAGCCCAGGTGTCCGGCCAGGGCGATAGCCTGCACGAGCACATTGCCAATC

TGGCCGGCAGCCCCGCCATTAAGAAGGGCATCCTGCAGACAGTGAAGGTGGTG

GACGAGCTCGTGAAAGTGATGGGCCGGCACAAGCCCGAGAACATCGTGATCGA

AATGGCCAGAGAGAACCAGACCACCCAGAAGGGACAGAAGAACAGCCGCGAG

AGAATGAAGCGGATCGAAGAGGGCATCAAAGAGCTGGGCAGCCAGATCCTGAA
AGAACACCCCGTGGAAAACACCCAGCTGCAGAACGAGAAGCTGTACCTGTACT
ACCTGCAGAATGGGCGGGATATGTACGTGGACCAGGAACTGGACATCAACCGG
CTGTCCGACTACGATGTGGACCATATCGTGCCTCAGAGCTTTCTGAAGGACGA
CTCCATCGACAACAAGGTGCTGACCAGAAGCGACAAGAACCGGGGCAAGAGCG
ACAACGTGCCCTCCGAAGAGGTCGTGAAGAAGATGAAGAACTACTGGCGGCAG
CTGCTGAACGCCAAGCTGATTACCCAGAGAAAGTTCGACAATCTGACCAAGGC
CGAGAGAGGCGGCCTGAGCGAACTGGATAAGGCCGGCTTCATCAAGAGACAG
CTGGTGGAAACCCGGCAGATCACAAAGCACGTGGCACAGATCCTGGACTCCCG
GATGAACACTAAGTACGACGAGAATGACAAGCTGATCCGGGAAGTGAAAGTGA
TCACCCTGAAGTCCAAGCTGGTGTCCGATTTCCGGAAGGATTTCCAGTTTTACA
AAGTGCGCGAGATCAACAACTACCACCACGCCCACGACGCCTACCTGAACGCC
GTCGTGGGAACCGCCCTGATCAAAAAGTACCCTAAGCTGGAAAGCGAGTTCGT
GTACGGCGACTACAAGGTGTACGACGTGCGGAAGATGATCGCCAAGAGCGAGC
AGGAAATCGGCAAGGCTACCGCCAAGTACTTCTTCTACAGCAACATCATGAACT
TTTTCAAGACCGAGATTACCCTGGCCAACGGCGAGATCCGGAAGCGGCCTCTG
ATCGAGACAAACGGCGAAACCGGGGAGATCGTGTGGGATAAGGGCCGGGATT
TTGCCACCGTGCGGAAAGTGCTGAGCATGCCCCAAGTGAATATCGTGAAAAAG
ACCGAGGTGCAGACAGGCGGCTTCAGCAAAGAGTCTATCCTGCCCAAGAGGAA
CAGCGATAAGCTGATCGCCAGAAAGAAGGACTGGGACCCTAAGAAGTACGGCG
GCTTCGACAGCCCCACCGTGGCCTATTCTGTGCTGGTGGTGGCCAAAGTGGAA
AAGGGCAAGTCCAAGAAACTGAAGAGTGTGAAAGAGCTGCTGGGGATCACCAT
CATGGAAAGAAGCAGCTTCGAGAAGAATCCCATCGACTTTCTGGAAGCCAAGG
GCTACAAAGAAGTGAAAAAGGACCTGATCATCAAGCTGCCTAAGTACTCCCTG
TTCGAGCTGGAAAACGGCCGGAAGAGAATGCTGGCCTCTGCCGGCGAACTGCA
GAAGGGAAACGAACTGGCCCTGCCCTCCAAATATGTGAACTTCCTGTACCTGG
CCAGCCACTATGAGAAGCTGAAGGGCTCCCCGAGGATAATGAGCAGAAACAG
CTGTTTGTGGAACAGCACAAGCACTACCTGGACGAGATCATCGAGCAGATCAG
CGAGTTCTCCAAGAGAGTGATCCTGGCCGACGCTAATCTGGACAAAGTGCTGT
CCGCCTACAACAAGCACCGGGATAAGCCCATCAGAGAGCAGGCCGAGAATATC
ATCCACCTGTTTACCCTGACCAATCTGGGAGCCCCTGCCGCCTTCAAGTACTTT

**GACACCACCATCGACCGGAAGAGGTACACCAGCACCAAAGAGGTGCTGGACGC CACCCTGATCCACCAGAGCATCACCGGCCTGTACGAGACACGGATCGACCTGT CTCAGCTGGGAGGCGACTTTCTTTTTCTTAGCTTGACCAGCTTTCTTAGTAGCA GCAGGACGCTTTAA**

(NLS-hSpCas9-NLS is highlighted in **bold**)
> Sequencing amplicon for EMX1 guides 1.1, 1.14, 1.17

CCAATGGGGAGGACATCGATGTCACCTCCAATGACTAGGGTGGGCAACC ACAAACCCACGAGGGCAGAGTGCTGCTTGCTGCTGGCCAGGCCCCTGCGTGGGCCC AAGCTGGACTCTGGCCAC

> Sequencing amplicon for EMX1 guides 1.2, 1.16

CGAGCAGAAGAAGAAGGGCTCCCATCACATCAACCGGTGGCGCATTGCC ACGAAGCAGGCCAATGGGGAGGACATCGATGTCACCTCCAATGACTAGGGTGGGCA ACCACAAACCCACGAG

> Sequencing amplicon for EMX1 guides 1.3, 1.13, 1.15

GGAGGACAAAGTACAAACGGCAGAAGCTGGAGGAGGAAGGGCCTGAGTC CGAGCAGAAGAAGAAGGGCTCCCATCACATCAACCGGTGGCGCATTGCCACGAAGC AGGCCAATGGGGAGGACATCGAT

> Sequencing amplicon for EMX1 guides 1.6

AGAAGCTGGAGGAGGAAGGGCCTGAGTCCGAGCAGAAGAAGAAGGGCTC CCATCACATCAACCGGTGGCGCATTGCCACGAAGCAGGCCAATGGGGAGGACATCG ATGTCACCTCCAATGACTAGGGTGG

> Sequencing amplicon for EMX1 guides 1.10

CCTCAGTCTTCCCATCAGGCTCTCAGCTCAGCCTGAGTGTTGAGGCCCCAG TGGCTGCTCTGGGGGCCTCCTGAGTTTCTCATCTGTGCCCCTCCCTCCCTGGCCCAGG TGAAGGTGTGGTTCCA

> Sequencing amplicon for EMX1 guides 1.11, 1.12

TCATCTGTGCCCCTCCCTCCCTGGCCCAGGTGAAGGTGTGGTTCCAGAACC GGAGGACAAAGTACAAACGGCAGAAGCTGGAGGAGGAAGGGCCTGAGTCCGAGCA GAAGAAGAAGGGCTCCCATCACA

> Sequencing amplicon for EMX1 guides 1.18, 1.19

CTCCAATGACTAGGGTGGGCAACCACAAACCCACGAGGGCAGAGTGCTG
CTTGCTGCTGGCCAGGCCCCTGCGTGGGCCCAAGCTGGACTCTGGCCACTCCCTGGC
CAGGCTTTGGGGAGGCCTGGAGT

> Sequencing amplicon for EMX1 guides 1.20

CTGCTTGCTGCTGGCCAGGCCCCTGCGTGGGCCCAAGCTGGACTCTGGCC
ACTCCCTGGCCAGGCTTTGGGGAGGCCTGGAGTCATGGCCCCACAGGGCTTGAAGC
CCGGGGCCGCCATTGACAGAG

>T7 promoter F primer for annealing with target strand
GAAATTAATACGACTCACTATAGGG
>oligo containing pUC19 target site 1 for methylation (T7 reverse)

AAAAAAGCACCGACTCGGTGCCACTTTTTCAAGTTGATAACGGACTAGCC
TTATTTTAACTTGCTATTTCTAGCTCTAAAACAACGACGAGCGTGACACCACCCTAT
AGTGAGTCGTATTAATTTC

>oligo containing pUC19 target site 2 for methylation (T7 reverse)

AAAAAAGCACCGACTCGGTGCCACTTTTTCAAGTTGATAACGGACTAGCC
TTATTTTAACTTGCTATTTCTAGCTCTAAAACGCAACAATTAATAGACTGGACCTATA
GTGAGTCGTATTAATTTC

*Example 11: Oligo-mediated Cas9-induced Homologous Recombination*

The oligo homologous recombination test is a comparison of efficiency across different Cas9 variants and different HR template (oligo vs. plasmid).

293FT cells were used. SpCas9 = Wildtype Cas9 and SpCas9n = nickase Cas9 (D10A). The chimeric RNA target is the same *EMX1* Protospacer Target 1 as in Examples 5, 9 and 10 and oligos synthesized by IDT using PAGE purification.

Figure 44 depicts a design of the oligo DNA used as Homologous Recombination (HR) template in this experiment. Long oligos contain 100bp homology to the *EMX1* locus and a HindIII restriction site. 293FT cells were co-transfected with: first, a plasmid containing a chimeric RNA targeting human *EMX1* locus and wild-type cas9 protein, and second, the oligo DNA as HR template. Samples are from 293FT cells collected 96 hours post transfection with Lipofectamine 2000. All products were amplified with an *EMX1* HR Primer, gel purified, followed by digestion with HindIII to detect the efficiency of integration of HR template into the human genome.

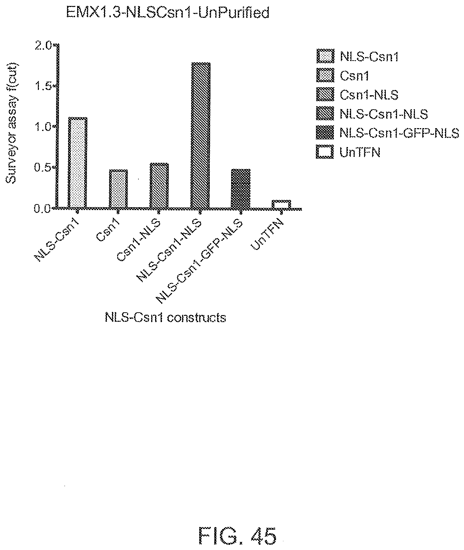

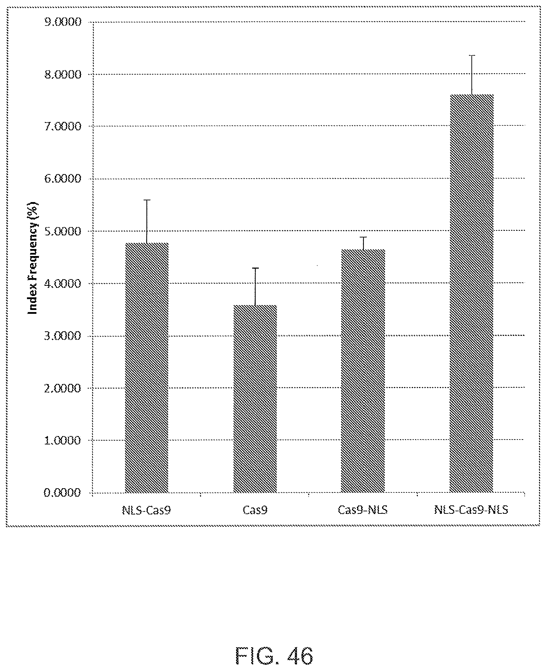

Figures 45 and 46 depict a comparison of HR efficiency induced by different combination of Cas9 protein and HR template. The Cas9 construct used were either wild-type Cas9 or the nickase version of Cas9 (Cas9n). The HR template used were: antisense oligo DNA (Antisense-Oligo in above figure), or sense oligo DNA (Sense-Oligo in above figure), or plasmid HR template (HR template in above figure). The sense/anti-sense definition is that the actively-transcribed strand with sequence corresponding to the transcribed mRNA is defined as the sense strand of genome. HR Efficiency is shown as percentage of HindIII digestion band as against all genomic PCR amplified product (bottom numbers).

*Example 12: Autistic Mouse*

Recent large-scale sequencing initiatives have produced a large number of genes associated with disease. Discovering the genes is only the beginning in understanding what the gene does and how it leads to a diseased phenotype. Current technologies and approaches to study candidate genes are slow and laborious. The gold standards, gene targeting and genetic knockouts, require a significant investment in time and resources, both monetary and in terms of research personnel. Applicants set out to utilize the hSpCas9 nuclease to target many genes and do so with higher efficiency and lower turnaround compared to any other technology. Because of the high efficiency of hSpCas9 Applicants can do RNA injection into mouse zygotes and immediately get genome-modified animals without the need to do any preliminary gene targeting in mESCs.

Chromodomain helicase DNA binding protein 8 (CHD8) is a pivotal gene in involved in early vertebrate development and morphogenesis. Mice lacking CHD8 die during embryonic development. Mutations in the CHD8 gene have been associated with autism spectrum disorder in humans. This association was made in three different papers published simultaneously in Nature. The same three studies identified a plethora of genes associated with autism spectrum disorder. Applicants' aim was to create knockout mice for the four genes that were found in all papers, Chd8, Katnal2, Kctd13, and Scn2a. In addition, Applicants chose two other genes associated with autism spectrum disorder, schizophrenia, and ADHD, GIT1, CACNA1C, and CACNB2. And finally, as a positive control Applicants decide to target MeCP2.

For each gene Applicants designed three gRNAs that would likely knockout the gene. A knockout would occur after the hSpCas9 nuclease makes a double strand break and the error prone DNA repair pathway, non-homologous end joining, corrects the break, creating a mutation. The most likely result is a frameshift mutation that would knockout the gene. The targeting strategy involved finding proto-spacers in the exons of the gene that had a PAM sequence, NGG, and was unique in the genome. Preference was given to proto-spacers in the first exon, which would be most deleterious to the gene.

Each gRNA was validated in the mouse cell line, Neuro-N2a, by liposomal transient co-transfection with hSpCas9. 72 hours post-transfection genomic DNA was purified using QuickExtract DNA from Epicentre. PCR was performed to amplify the locus of interest. Subsequently the SURVEYOR Mutation Detection Kit from Transgenomics was followed. The SURVEYOR results for each gRNA and respective controls are shown in Figure A1. A positive SURVEYOR result is one large band corresponding to the genomic PCR and two smaller bands that are the product of the SURVEYOR nuclease making a double-strand break at the site of a mutation. The average cutting efficiency of each gRNA was also determined for each gRNA. The gRNA that was chosen for injection was the highest efficiency gRNA that was the most unique within the genome.

RNA (hSpCas9+gRNA RNA) was injected into the pronucleus of a zygote and later transplanted into a foster mother. Mothers were allowed to go full term and pups were sampled by tail snip 10 days postnatal. DNA was extracted and used as a template for PCR, which was then processed by SURVEYOR. Additionally, PCR products were sent for sequencing. Animals that were detected as being positive in either the SURVEYOR assay or PCR sequencing would have their genomic PCR products cloned into a pUC19 vector and sequenced to determine putative mutations from each allele.

So far, mice pups from the Chd8 targeting experiment have been fully processed up to the point of allele sequencing. The Surveyor results for 38 live pups (lanes 1-38) 1 dead pup (lane 39) and 1 wild-type pup for comparison (lane 40) are shown in Figure A2. Pups 1-19 were injected with gRNA Chd8.2 and pups 20-38 were injected with gRNA Chd8.3. Of the 38 live pups, 13 were positive for a mutation. The one dead pup also had a mutation. There was no mutation detected in the wild-type sample. Genomic PCR sequencing was consistent with the SURVEYOR assay findings.

*Example 13: CRISPR/Cas-Mediated Transcriptional Modulation*

Figure 67 depicts a design of the CRISPR-TF (Transcription Factor) with transcriptional activation activity. The chimeric RNA is expressed by U6 promoter, while a human-codon-optimized, double-mutant version of the Cas9 protein (hSpCas9m), operably linked to triple NLS and a VP64 functional domain is expressed by a EF1a promoter. The double mutations, D10A and H840A, renders the cas9 protein unable to introduce any cleavage but maintained its capacity to bind to target DNA when guided by the chimeric RNA.

Figure 68 depicts transcriptional activation of the human SOX2 gene with CRISPR-TF system (Chimeric RNA and the Cas9-NLS-VP64 fusion protein). 293FT cells were transfected with plasmids bearing two components: (1) U6-driven different chimeric RNAs targeting 20-bp sequences within or around the human SOX2 genomic locus, and (2) EF1a-driven hSpCas9m (double mutant)-NLS-VP64 fusion protein. 96 hours post transfection, 293FT cells were harvested and the level of activation is measured by the induction of mRNA expression using a qRT-PCR assay. All expression levels are normalized against the control group (grey bar), which represents results from cells transfected with the CRISPR-TF backbone plasmid without chimeric RNA. The qRT-PCR probes used for detecting the SOX2 mRNA is Taqman Human Gene Expression Assay (Life Technologies). All experiments represents data from 3 biological replicates, n=3, error bars show s.e.m.

*Example 14: NLS: Cas9 NLS*

293FT cells were transfected with plasmid containing two components: (1) EF1a promoter driving the expression of Cas9 (wild-type human-codon-optimized Sp Cas9) with different NLS designs (2) U6 promoter driving the same chimeric RNA targeting human EMX1 locus.

Cells were collect at 72h time point post transfection, and then extracted with 50 $\mu$l of the QuickExtract genomic DNA extraction solution following manufacturer's protocol. Target EMX1 genomic DNA were PCR amplified and then Gel-purify with 1% agarose gel. Genomic PCR product were re-anneal and subjected to the Surveyor assay following manufacturer's protocol. The genomic cleavage efficiency of different constructs were measured using SDS-PAGE on a 4-12% TBE-PAGE gel (Life Technologies), analyzed and quantified with ImageLab (Bio-rad) software, all following manufacturer's protocol.

Figure 69 depicts a design of different Cas9 NLS constructs. All Cas9 were the human-codon-optimized version of the Sp Cas9. NLS sequences are linked to the cas9 gene at either N-terminus or C-terminus. All Cas9 variants with different NLS designs were cloned into a backbone vector containing so it is driven by EF1a promoter. On the same vector there is a chimeric RNA targeting human EMX1 locus driven by U6 promoter, together forming a two-component system.

Table **M.** Cas9 NLS Design Test Results. Quantification of genomic cleavage of different cas9-nls constructs by surveyor assay.

| Percentage Genome Cleavage as measured by Surveyor assay | Biological Replicate 1 (%) | Biological Replicate 2 (%) | Biological Replicate 3 (%) | Average (%) | Error (S.E.M., standard error of the mean) |
|---|---|---|---|---|---|
| Cas9 (No NLS) | 2.50 | 3.30 | 2.73 | 2.84 | 0.24 |
| Cas9 with N-term NLS | 7.61 | 6.29 | 5.46 | 6.45 | 0.63 |
| Cas9 with C-term NLS | 5.75 | 4.86 | 4.70 | 5.10 | 0.33 |
| Cas9 with Double (N-term and C-term) NLS | 9.08 | 9.85 | 7.78 | 8.90 | 0.60 |

Figure 70 depicts the efficiency of genomic cleavage induced by Cas9 variants bearing different NLS designs. The percentage indicate the portion of human EMX1 genomic DNA that were cleaved by each construct. All experiments are from 3 biological replicates. n = 3, error indicates S.E.M.

*Example 15: Engineering of Microalgae using Cas9*

Methods of delivering Cas9

Method 1: Applicants deliver Cas9 and guide RNA using a vector that expresses Cas9 under the control of a constitutive promoter such as Hsp70A-Rbc S2 or Beta2-tubulin.

Method 2: Applicants deliver Cas9 and T7 polymerase using vectors that expresses Cas9 and T7 polymerase under the control of a constitutive promoter such as Hsp70A-Rbc S2 or Beta2-tubulin. Guide RNA will be delivered using a vector containing T7 promoter driving the guide RNA.

Method 3: Applicants deliver Cas9 mRNA and *in vitro* transcribed guide RNA to algae cells. RNA can be *in vitro* transcribed. Cas9 mRNA will consist of the coding region for Cas9 as well as 3'UTR from Cop1 to ensure stabilization of the Cas9 mRNA.

For Homologous recombination, Applicants provide an additional homology directed repair template.

Sequence for a cassette driving the expression of Cas9 under the control of beta-2 tubulin promoter, followed by the 3' UTR of Cop1.

TCTTTCTTGCGCTATGACACTTCCAGCAAAAGGTAGGGCGGGCTGCGAGA
CGGCTTCCCGGCGCTGCATGCAACACCGATGATGCTTCGACCCCCCGAAGCTCCTTC
GGGGCTGCATGGGCGCTCCGATGCCGCTCCAGGGCGAGCGCTGTTTAAATAGCCAG
GCCCCCGATTGCAAAGACATTATAGCGAGCTACCAAAGCCATATTCAAACACCTAG
ATCACTACCACTTCTACACAGGCCACTCGAGCTTGTGATCGCACTCCGCTAAGGGGG
CGCCTCTTCCTCTTCGTTTCAGTCACAACCCGCAAACATGTACCCATACGATGTTCCA
GATTACGCTTCGCCGAAGAAAAGCGCAAGGTCGAAGCGTCCGACAAGAAGTACAG
CATCGGCCTGGACATCGGCACCAACTCTGTGGGCTGGGCCGTGATCACCGACGAGT
ACAAGGTGCCCAGCAAGAAATTCAAGGTGCTGGGCAACACCGACCGGCACAGCATC
AAGAAGAACCTGATCGGAGCCCTGCTGTTCGACAGCGGCGAAACAGCCGAGGCCAC
CCGGCTGAAGAGAACCGCCAGAAGAAGATACACCAGACGGAAGAACCGGATCTGC
TATCTGCAAGAGATCTTCAGCAACGAGATGGCCAAGGTGGACGACAGCTTCTTCCAC
AGACTGGAAGAGTCCTTCCTGGTGGAAGAGGATAAGAAGCACGAGCGGCACCCCAT
CTTCGGCAACATCGTGGACGAGGTGGCCTACCACGAGAAGTACCCCACCATCTACC
ACCTGAGAAAGAAACTGGTGGACAGCACCGACAAGGCCGACCTGCGGCTGATCTAT
CTGGCCCTGGCCCACATGATCAAGTTCCGGGGCCACTTCCTGATCGAGGGCGACCTG
AACCCCGACAACAGCGACGTGGACAAGCTGTTCATCCAGCTGGTGCAGACCTACAA
CCAGCTGTTCGAGGAAAACCCCATCAACGCCAGCGGCGTGGACGCCAAGGCCATCC
TGTCTGCCAGACTGAGCAAGAGCAGACGGCTGGAAAATCTGATCGCCCAGCTGCCC
GGCGAGAAGAAGAATGGCCTGTTCGGCAACCTGATTGCCCTGAGCCTGGGCCTGAC
CCCCAACTTCAAGAGCAACTTCGACCTGGCCGAGGATGCCAAACTGCAGCTGAGCA
AGGACACCTACGACGACGACCTGGACAACCTGCTGGCCCAGATCGGCGACCAGTAC

GCCGACCTGTTTCTGGCCGCCAAGAACCTGTCCGACGCCATCCTGCTGAGCGACATC
CTGAGAGTGAACACCGAGATCACCAAGGCCCCCCTGAGCGCCTCTATGATCAAGAG
ATACGACGAGCACCACCAGGACCTGACCCTGCTGAAAGCTCTCGTGCGGCAGCAGC
TGCCTGAGAAGTACAAAGAGATTTTCTTCGACCAGAGCAAGAACGGCTACGCCGGC
TACATTGACGGCGGAGCCAGCCAGGAAGAGTTCTACAAGTTCATCAAGCCCATCCT
GGAAAAGATGGACGGCACCGAGGAACTGCTCGTGAAGCTGAACAGAGAGGACCTG
CTGCGGAAGCAGCGGACCTTCGACAACGGCAGCATCCCCCACCAGATCCACCTGGG
AGAGCTGCACGCCATTCTGCGGCGGCAGGAAGATTTTTACCCATTCCTGAAGGACAA
CCGGGAAAAGATCGAGAAGATCCTGACCTTCCGCATCCCCTACTACGTGGGCCCTCT
GGCCAGGGGAAACAGCAGATTCGCCTGGATGACCAGAAAGAGCGAGGAAACCATC
ACCCCCTGGAACTTCGAGGAAGTGGTGGACAAGGGCGCTTCCGCCCAGAGCTTCAT
CGAGCGGATGACCAACTTCGATAAGAACCTGCCCAACGAGAAGGTGCTGCCCAAGC
ACAGCCTGCTGTACGAGTACTTCACCGTGTATAACGAGCTGACCAAAGTGAAATAC
GTGACCGAGGGAATGAGAAAGCCCGCCTTCCTGAGCGGCGAGCAGAAAAAGGCCA
TCGTGGACCTGCTGTTCAAGACCAACCGGAAAGTGACCGTGAAGCAGCTGAAAGAG
GACTACTTCAAGAAAATCGAGTGCTTCGACTCCGTGGAAATCTCCGGCGTGGAAGAT
CGGTTCAACGCCTCCCTGGGCACATACCACGATCTGCTGAAAATTATCAAGGACAA
GGACTTCCTGGACAATGAGGAAAACGAGGACATTCTGGAAGATATCGTGCTGACCC
TGACACTGTTTGAGGACAGAGAGATGATCGAGGAACGGCTGAAAACCTATGCCCAC
CTGTTCGACGACAAAGTGATGAAGCAGCTGAAGCGGCGGAGATACACCGGCTGGGG
CAGGCTGAGCCGGAAGCTGATCAACGGCATCCGGGACAAGCAGTCCGGCAAGACA
ATCCTGGATTTCCTGAAGTCCGACGGCTTCGCCAACAGAAACTTCATGCAGCTGATC
CACGACGACAGCCTGACCTTTAAAGAGGACATCCAGAAAGCCCAGGTGTCCGGCCA
GGGCGATAGCCTGCACGAGCACATTGCCAATCTGGCCGGCAGCCCCGCCATTAAGA
AGGGCATCCTGCAGACAGTGAAGGTGGTGGACGAGCTCGTGAAAGTGATGGGCCGG
CACAAGCCCGAGAACATCGTGATCGAAATGGCCAGAGAGAACCAGACCACCCAGA
AGGGACAGAAGAACAGCCGCGAGAGAATGAAGCGGATCGAAGAGGGCATCAAAGA
GCTGGGCAGCCAGATCCTGAAGAACACCCCGTGGAAAACACCCAGCTGCAGAACG
AGAAGCTGTACCTGTACTACCTGCAGAATGGGCGGGATATGTACGTGGACCAGGAA
CTGGACATCAACCGGCTGTCCGACTACGATGTGGACCATATCGTGCCTCAGAGCTTT
CTGAAGGACGACTCCATCGACAACAAGGTGCTGACCAGAAGCGACAAGAACCGGG

GCAAGAGCGACAACGTGCCCTCCGAAGAGGTCGTGAAGAAGATGAAGAACTACTGG
CGGCAGCTGCTGAACGCCAAGCTGATTACCCAGAGAAAGTTCGACAATCTGACCAA
GGCCGAGAGAGGCGGCCTGAGCGAACTGGATAAGGCCGGCTTCATCAAGAGACAG
CTGGTGGAAACCCGGCAGATCACAAAGCACGTGGCACAGATCCTGGACTCCCGGAT
GAACACTAAGTACGACGAGAATGACAAGCTGATCCGGGAAGTGAAAGTGATCACCC
TGAAGTCCAAGCTGGTGTCCGATTTCCGGAAGGATTTCCAGTTTTACAAAGTGCGCG
AGATCAACAACTACCACCACGCCCACGACGCCTACCTGAACGCCGTCGTGGGAACC
GCCCTGATCAAAAAGTACCCTAAGCTGGAAAGCGAGTTCGTGTACGGCGACTACAA
GGTGTACGACGTGCGGAAGATGATCGCCAAGAGCGAGCAGGAAATCGGCAAGGCT
ACCGCCAAGTACTTCTTCTACAGCAACATCATGAACTTTTTCAAGACCGAGATTACC
CTGGCCAACGGCGAGATCCGGAAGCGGCCTCTGATCGAGACAAACGGCGAAACCGG
GGAGATCGTGTGGGATAAGGGCCGGGATTTTGCCACCGTGCGGAAAGTGCTGAGCA
TGCCCCAAGTGAATATCGTGAAAAAGACCGAGGTGCAGACAGGCGGCTTCAGCAAA
GAGTCTATCCTGCCCAAGAGGAACAGCGATAAGCTGATCGCCAGAAAGAAGGACTG
GGACCCTAAGAAGTACGGCGGCTTCGACAGCCCCACCGTGGCCTATTCTGTGCTGGT
GGTGGCCAAAGTGGAAAAGGGCAAGTCCAAGAAACTGAAGAGTGTGAAAGAGCTG
CTGGGGATCACCATCATGGAAAGAAGCAGCTTCGAGAAGAATCCCATCGACTTTCT
GGAAGCCAAGGGCTACAAAGAAGTGAAAAAGGACCTGATCATCAAGCTGCCTAAGT
ACTCCCTGTTCGAGCTGGAAAACGGCCGGAAGAGAATGCTGGCCTCTGCCGGCGAA
CTGCAGAAGGGAAACGAACTGGCCCTGCCCTCCAAATATGTGAACTTCCTGTACCTG
GCCAGCCACTATGAGAAGCTGAAGGGCTCCCCCGAGGATAATGAGCAGAAACAGCT
GTTTGTGGAACAGCACAAGCACTACCTGGACGAGATCATCGAGCAGATCAGCGAGT
TCTCCAAGAGAGTGATCCTGGCCGACGCTAATCTGGACAAAGTGCTGTCCGCCTACA
ACAAGCACCGGGATAAGCCCATCAGAGAGCAGGCCGAGAATATCATCCACCTGTTT
ACCCTGACCAATCTGGGAGCCCCTGCCGCCTTCAAGTACTTTGACACCACCATCGAC
CGGAAGAGGTACACCAGCACCAAAGAGGTGCTGGACGCCACCCTGATCCACCAGAG
CATCACCGGCCTGTACGAGACACGGATCGACCTGTCTCAGCTGGGAGGCGACAGCC
CCAAGAAGAAGAGAAAGGTGGAGGCCAGCTAAGGATCCGGCAAGACTGGCCCCGC
TTGGCAACGCAACAGTGAGCCCCTCCCTAGTGTGTTTGGGGATGTGACTATGTATTC
GTGTGTTGGCCAACGGGTCAACCCGAACAGATTGATACCCGCCTTGGCATTTCCTGT
CAGAATGTAACGTCAGTTGATGGTACT

Sequence for a cassette driving the expression of T7 polymerase under the control of beta-2 tubulin promoter, followed by the 3' UTR of Cop1:

TCTTTCTTGCGCTATGACACTTCCAGCAAAAGGTAGGGCGGGCTGCGAGA
CGGCTTCCCGGCGCTGCATGCAACACCGATGATGCTTCGACCCCCCGAAGCTCCTTC
GGGGCTGCATGGGCGCTCCGATGCCGCTCCAGGGCGAGCGCTGTTTAAATAGCCAG
GCCCCCGATTGCAAAGACATTATAGCGAGCTACCAAAGCCATATTCAAACACCTAG
ATCACTACCACTTCTACACAGGCCACTCGAGCTTGTGATCGCACTCCGCTAAGGGGG
CGCCTCTTCCTCTTCGTTTCAGTCACAACCCGCAAACatgcctaagaagaagaggaaggttaacacgatt
aacatcgctaagaacgacttctctgacatcgaactggctgctatcccgttcaacactctggctgaccattacggtgagcgtttagctcgcgaa
cagttggcccttgagcatgagtcttacgagatgggtgaagcacgcttccgcaagatgtttgagcgtcaacttaaagctggtgaggttgcggat
aacgctgccgccaagcctctcatcactaccctactccctaagatgattgcacgcatcaacgactggtttgaggaagtgaaagctaagcgcg
gcaagcgcccgacagccttccagttcctgcaagaaatcaagccggaagccgtagcgtacatcaccattaagaccactctggcttgcctaac
cagtgctgacaatacaaccgttcaggctgtagcaagcgcaatcggtcgggccattgaggacgaggctcgcttcggtcgtatccgtgacctt
gaagctaagcacttcaagaaaaacgttgaggaacaactcaacaagcgcgtagggcacgtctacaagaaagcatttatgcaagttgtcgag
gctgacatgctctctaagggtctactcggtggcgaggcgtggtcttcgtggcataaggaagactctattcatgtaggagtacgctgcatcgag
atgctcattgagtcaaccggaatggttagcttacaccgccaaaatgctggcgtagtaggtcaagactctgagactatcgaactcgcacctga
atacgctgaggctatcgcaacccgtgcaggtgcgctggctggcatctctccgatgttccaaccttgcgtagttcctcctaagccgtggactgg
cattactggtggtggctattgggctaacggtcgtcgtcctctggcgctggtgcgtactcacagtaagaaagcactgatgcgctacgaagacg
tttacatgcctgaggtgtacaaagcgattaacattgcgcaaaacaccgcatggaaaatcaacaagaaagtcctagcggtcgccaacgtaatc
accaagtggaagcattgtccggtcgaggacatccctgcgattgagcgtgaagaactcccgatgaaaccggaagacatcgacatgaatcct
gaggctctcaccgcgtggaaacgtgctgccgctgctgtgtaccgcaaggacaaggctcgcaagtctcgccgtatcagccttgagttcatgc
ttgagcaagccaataagtttgctaaccataaggccatctggttcccttacaacatggactggcgcggtcgtgtttacgctgtgtcaatgttcaac
ccgcaaggtaacgatatgaccaaaggactgcttacgctggcgaaaggtaaaccaatcggtaaggaaggttactactggctgaaaatccac
ggtgcaaactgtgcgggtgtcgacaaggttccgttccctgagcgcatcaagttcattgaggaaaaccacgagaacatcatggcttgcgctaa
gtctccactggagaacacttggtgggctgagcaagattctccgttctgcttccttgcgttctgctttgagtacgctgggggtacagcaccacggc
ctgagctataactgctcccttccgctggcgtttgacgggtcttgctctggcatccagcacttctccgcgatgctccgagatgaggtaggtggtc
gcgcggttaacttgcttcctagtgaaaccgttcaggacatctacgggattgttgctaagaaagtcaacgagattctacaagcagacgcaatca
atgggaccgataacgaagtagttaccgtgaccgatgagaacactggtgaaatctctgagaaagtcaagctgggcactaaggcactggctg
gtcaatggctggcttacggtgttactcgcagtgtgactaagcgttcagtcatgacgctggcttacgggtccaaagagttcggcttccgtcaac
aagtgctggaagataccattcagccagctattgattccggcaagggtctgatgttcactcagccgaatcaggctgctggatacatggctaag
ctgatttgggaatctgtgagcgtgacggtggtagctgcggttgaagcaatgaactggcttaagtctgctgctaagctgctggctgctgaggtc

aaagataagaagactggagagattcttcgcaagcgttgcgctgtgcattgggtaactcctgatggtttccctgtgtggcaggaatacaagaa

gcctattcagacgcgcttgaacctgatgttcctcggtcagttccgcttacagcctaccattaacaccaacaaagatagcgagattgatgcaca

caaacaggagtctggtatcgctcctaactttgtacacagccaagacggtagccaccttcgtaagactgtagtgtgggcacacgagaagtac

ggaatcgaatcttttgcactgattcacgactccttcggtacgattccggctgacgctgcgaacctgttcaaagcagtgcgcgaaactatggttg

acacatatgagtcttgtgatgtactggctgatttctacgaccagttcgctgaccagttgcacgagtctcaattggacaaaatgccagcacttcc

ggctaaaggtaacttgaacctccgtgacatcttagagtcggacttcgcgttcgcgtaa**GGATCCGGCAAGACTGGCCCC**
**GCTTGGCAACGCAACAGTGAGCCCCTCCCTAGTGTGTTTGGGGATGTGACTATGTAT**
**TCGTGTGTTGGCCAACGGGTCAACCCGAACAGATTGATACCCGCCTTGGCATTTCCT**
**GTCAGAATGTAACGTCAGTTGATGGTACT**

Sequence of guide RNA driven by the T7 promoter (T7 promoter, Ns represent targeting sequence):

gaaat**TAATACGACTCACTATA**<u>NNNNNNNNNNNNNNNNNNNNN</u>gttttagagctaG

AAAtagcaagttaaaataaggctagtccgttatcaacttgaaaaagtggcaccgagtcggtgctttttt

Gene delivery:

Chlamydomonas reinhardtii strain CC-124 and CC-125 from the Chlamydomonas Resource Center will be used for electroporation. Electroporation protocol follows standard recommended protocol from the GeneArt Chlamydomonas Engineering kit.

Also, Applicants generate a line of Chlamydomonas reinhardtii that expresses Cas9 constitutively. This can be done by using pChlamy1 (linearized using PvuI) and selecting for hygromycin resistant colonies. Sequence for pChlamy1 containing Cas9 is below. In this way to achieve gene knockout one simply needs to deliver RNA for the guideRNA. For homologous recombination Applicants deliver guideRNA as well as a linearized homologous recombination template.

pChlamy1-Cas9:

TGCGGTATTTCACACCGCATCAGGTGGCACTTTTCGGGGAAATGTGCGCG
GAACCCCTATTTGTTTATTTTTCTAAATACATTCAAATATGTATCCGCTCATGAGATT
ATCAAAAAGGATCTTCACCTAGATCCTTTTAAATTAAAAATGAAGTTTTAAATCAAT
CTAAAGTATATATGAGTAAACTTGGTCTGACAGTTACCAATGCTTAATCAGTGAGGC
ACCTATCTCAGCGATCTGTCTATTTCGTTCATCCATAGTTGCCTGACTCCCCGTCGTG
TAGATAACTACGATACGGGAGGGCTTACCATCTGGCCCCAGTGCTGCAATGATACCG
CGAGACCCACGCTCACCGGCTCCAGATTTATCAGCAATAAACCAGCCAGCCGGAAG

GGCCGAGCGCAGAAGTGGTCCTGCAACTTTATCCGCCTCCATCCAGTCTATTAATTG
TTGCCGGGAAGCTAGAGTAAGTAGTTCGCCAGTTAATAGTTTGCGCAACGTTGTTGC
CATTGCTACAGGCATCGTGGTGTCACGCTCGTCGTTTGGTATGGCTTCATTCAGCTCC
GGTTCCCAACGATCAAGGCGAGTTACATGATCCCCCATGTTGTGCAAAAAAGCGGTT
AGCTCCTTCGGTCCTCCGATCGTTGTCAGAAGTAAGTTGGCCGCAGTGTTATCACTC
ATGGTTATGGCAGCACTGCATAATTCTCTTACTGTCATGCCATCCGTAAGATGCTTTT
CTGTGACTGGTGAGTACTCAACCAAGTCATTCTGAGAATAGTGTATGCGGCGACCGA
GTTGCTCTTGCCCGGCGTCAATACGGGATAATACCGCGCCACATAGCAGAACTTTAA
AAGTGCTCATCATTGGAAAACGTTCTTCGGGGCGAAAACTCTCAAGGATCTTACCGC
TGTTGAGATCCAGTTCGATGTAACCCACTCGTGCACCCAACTGATCTTCAGCATCTTT
TACTTTCACCAGCGTTTCTGGGTGAGCAAAAACAGGAAGGCAAAATGCCGCAAAAA
AGGGAATAAGGGCGACACGGAAATGTTGAATACTCATACTCTTCCTTTTTCAATATT
ATTGAAGCATTTATCAGGGTTATTGTCTCATGACCAAAATCCCTTAACGTGAGTTTTC
GTTCCACTGAGCGTCAGACCCCGTAGAAAAGATCAAAGGATCTTCTTGAGATCCTTT
TTTTCTGCGCGTAATCTGCTGCTTGCAAACAAAAAAACCACCGCTACCAGCGGTGGT
TTGTTTGCCGGATCAAGAGCTACCAACTCTTTTTCCGAAGGTAACTGGCTTCAGCAG
AGCGCAGATACCAAATACTGTTCTTCTAGTGTAGCCGTAGTTAGGCCACCACTTCAA
GAACTCTGTAGCACCGCCTACATACCTCGCTCTGCTAATCCTGTTACCAGTGGCTGTT
GCCAGTGGCGATAAGTCGTGTCTTACCGGGTTGGACTCAAGACGATAGTTACCGGAT
AAGGCGCAGCGGTCGGGCTGAACGGGGGGTTCGTGCACACAGCCCAGCTTGGAGCG
AACGACCTACACCGAACTGAGATACCTACAGCGTGAGCTATGAGAAAGCGCCACGC
TTCCCGAAGGGAGAAAGGCGGACAGGTATCCGGTAAGCGGCAGGGTCGGAACAGG
AGAGCGCACGAGGGAGCTTCCAGGGGGAAACGCCTGGTATCTTTATAGTCCTGTCG
GGTTTCGCCACCTCTGACTTGAGCGTCGATTTTTGTGATGCTCGTCAGGGGGGCGGA
GCCTATGGAAAAACGCCAGCAACGCGGCCTTTTTACGGTTCCTGGCCTTTTGCTGGC
CTTTTGCTCACATGTTCTTTCCTGCGTTATCCCCTGATTCTGTGGATAACCGTATTACC
GCCTTTGAGTGAGCTGATACCGCTCGCCGCAGCCGAACGACCGAGCGCAGCGAGTC
AGTGAGCGAGGAAGCGGTCGCTGAGGCTTGACATGATTGGTGCGTATGTTTGTATGA
AGCTACAGGACTGATTTGGCGGGCTATGAGGGCGGGGGAAGCTCTGGAAGGGCCGC
GATGGGGCGCGCGGCGTCCAGAAGGCGCCATACGGCCCGCTGGCGGCACCCATCCG
GTATAAAAGCCCGCGACCCCGAACGGTGACCTCCACTTTCAGCGACAAACGAGCAC

TTATACATACGCGACTATTCTGCCGCTATACATAACCACTCAGCTAGCTTAAGATCC

CATCAAGCTTGCATGCCGGGCGCGCCAGAAGGAGCGCAGCCAAACCAGGATGATGT

TTGATGGGGTATTTGAGCACTTGCAACCCTTATCCGGAAGCCCCCTGGCCCACAAAG

GCTAGGCGCCAATGCAAGCAGTTCGCATGCAGCCCCTGGAGCGGTGCCCTCCTGAT

AAACCGGCCAGGGGGCCTATGTTCTTTACTTTTTTACAAGAGAAGTCACTCAACATC

TTAAAATGGCCAGGTGAGTCGACGAGCAAGCCCGGCGGATCAGGCAGCGTGCTTGC

AGATTTGACTTGCAACGCCCGCATTGTGTCGACGAAGGCTTTTGGCTCCTCTGTCGCT

GTCTCAAGCAGCATCTAACCCTGCGTCGCCGTTTCCATTTGCAGGAGATTCGAGGTA

CCATGTACCCATACGATGTTCCAGATTACGCTTCGCCGAAGAAAAAGCGCAAGGTC

GAAGCGTCCGACAAGAAGTACAGCATCGGCCTGGACATCGGCACCAACTCTGTGGG

CTGGGCCGTGATCACCGACGAGTACAAGGTGCCCAGCAAGAAATTCAAGGTGCTGG

GCAACACCGACCGGCACAGCATCAAGAAGAACCTGATCGGAGCCCTGCTGTTCGAC

AGCGGCGAAACAGCCGAGGCCACCCGGCTGAAGAGAACCGCCAGAAGAAGATACA

CCAGACGGAAGAACCGGATCTGCTATCTGCAAGAGATCTTCAGCAACGAGATGGCC

AAGGTGGACGACAGCTTCTTCCACAGACTGGAAGAGTCCTTCCTGGTGGAAGAGGA

TAAGAAGCACGAGCGGCACCCCATCTTCGGCAACATCGTGGACGAGGTGGCCTACC

ACGAGAAGTACCCCACCATCTACCACCTGAGAAAGAAACTGGTGGACAGCACCGAC

AAGGCCGACCTGCGGCTGATCTATCTGGCCCTGGCCCACATGATCAAGTTCCGGGGC

CACTTCCTGATCGAGGGCGACCTGAACCCCGACAACAGCGACGTGGACAAGCTGTT

CATCCAGCTGGTGCAGACCTACAACCAGCTGTTCGAGGAAAACCCCATCAACGCCA

GCGGCGTGGACGCCAAGGCCATCCTGTCTGCCAGACTGAGCAAGAGCAGACGGCTG

GAAAATCTGATCGCCCAGCTGCCCGGCGAGAAGAAGAATGGCCTGTTCGGCAACCT

GATTGCCCTGAGCCTGGGCCTGACCCCCAACTTCAAGAGCAACTTCGACCTGGCCGA

GGATGCCAAACTGCAGCTGAGCAAGGACACCTACGACGACGACCTGGACAACCTGC

TGGCCCAGATCGGCGACCAGTACGCCGACCTGTTTCTGGCCGCCAAGAACCTGTCCG

ACGCCATCCTGCTGAGCGACATCCTGAGAGTGAACACCGAGATCACCAAGGCCCCC

CTGAGCGCCTCTATGATCAAGAGATACGACGAGCACCACCAGGACCTGACCCTGCT

GAAAGCTCTCGTGCGGCAGCAGCTGCCTGAGAAGTACAAAGAGATTTTCTTCGACC

AGAGCAAGAACGGCTACGCCGGCTACATTGACGGCGGAGCCAGCCAGGAAGAGTTC

TACAAGTTCATCAAGCCCATCCTGGAAAAGATGGACGGCACCGAGGAACTGCTCGT

GAAGCTGAACAGAGAGGACCTGCTGCGGAAGCAGCGGACCTTCGACAACGGCAGC

ATCCCCCACCAGATCCACCTGGGAGAGCTGCACGCCATTCTGCGGCGGCAGGAAGA

TTTTTACCCATTCCTGAAGGACAACCGGGAAAAGATCGAGAAGATCCTGACCTTCCG

CATCCCCTACTACGTGGGCCCTCTGGCCAGGGGAAACAGCAGATTCGCCTGGATGA

CCAGAAAGAGCGAGGAAACCATCACCCCCTGGAACTTCGAGGAAGTGGTGGACAA

GGGCGCTTCCGCCCAGAGCTTCATCGAGCGGATGACCAACTTCGATAAGAACCTGC

CCAACGAGAAGGTGCTGCCCAAGCACAGCCTGCTGTACGAGTACTTCACCGTGTAT

AACGAGCTGACCAAAGTGAAATACGTGACCGAGGGAATGAGAAAGCCCGCCTTCCT

GAGCGGCGAGCAGAAAAAGGCCATCGTGGACCTGCTGTTCAAGACCAACCGGAAA

GTGACCGTGAAGCAGCTGAAAGAGGACTACTTCAAGAAAATCGAGTGCTTCGACTC

CGTGGAAATCTCCGGCGTGGAAGATCGGTTCAACGCCTCCCTGGGCACATACCACG

ATCTGCTGAAAATTATCAAGGACAAGGACTTCCTGGACAATGAGGAAAACGAGGAC

ATTCTGGAAGATATCGTGCTGACCCTGACACTGTTTGAGGACAGAGAGATGATCGA

GGAACGGCTGAAAACCTATGCCCACCTGTTCGACGACAAAGTGATGAAGCAGCTGA

AGCGGCGGAGATACACCGGCTGGGGCAGGCTGAGCCGGAAGCTGATCAACGGCATC

CGGGACAAGCAGTCCGGCAAGACAATCCTGGATTTCCTGAAGTCCGACGGCTTCGC

CAACAGAAACTTCATGCAGCTGATCCACGACGACAGCCTGACCTTTAAAGAGGACA

TCCAGAAAGCCCAGGTGTCCGGCCAGGGCGATAGCCTGCACGAGCACATTGCCAAT

CTGGCCGGCAGCCCCGCCATTAAGAAGGGCATCCTGCAGACAGTGAAGGTGGTGGA

CGAGCTCGTGAAAGTGATGGGCCGGCACAAGCCCGAGAACATCGTGATCGAAATGG

CCAGAGAGAACCAGACCACCCAGAAGGGACAGAAGAACAGCCGCGAGAGAATGAA

GCGGATCGAAGAGGGCATCAAAGAGCTGGGCAGCCAGATCCTGAAAGAACACCCC

GTGGAAAACACCCAGCTGCAGAACGAGAAGCTGTACCTGTACTACCTGCAGAATGG

GCGGGATATGTACGTGGACCAGGAACTGGACATCAACCGGCTGTCCGACTACGATG

TGGACCATATCGTGCCTCAGAGCTTTCTGAAGGACGACTCCATCGACAACAAGGTGC

TGACCAGAAGCGACAAGAACCGGGGCAAGAGCGACAACGTGCCCTCCAAGAGGT

CGTGAAGAAGATGAAGAACTACTGGCGGCAGCTGCTGAACGCCAAGCTGATTACCC

AGAGAAAGTTCGACAATCTGACCAAGGCCGAGAGAGGCGGCCTGAGCGAACTGGAT

AAGGCCGGCTTCATCAAGAGACAGCTGGTGGAAACCCGGCAGATCACAAAGCACGT

GGCACAGATCCTGGACTCCCGGATGAACACTAAGTACGACGAGAATGACAAGCTGA

TCCGGGAAGTGAAAGTGATCACCCTGAAGTCCAAGCTGGTGTCCGATTTCCGGAAG

GATTTCCAGTTTTACAAAGTGCGCGAGATCAACAACTACCACCACGCCCACGACGCC

TACCTGAACGCCGTCGTGGGAACCGCCCTGATCAAAAAGTACCCTAAGCTGGAAAG
CGAGTTCGTGTACGGCGACTACAAGGTGTACGACGTGCGGAAGATGATCGCCAAGA
GCGAGCAGGAAATCGGCAAGGCTACCGCCAAGTACTTCTTCTACAGCAACATCATG
AACTTTTTCAAGACCGAGATTACCCTGGCCAACGGCGAGATCCGGAAGCGGCCTCT
GATCGAGACAAACGGCGAAACCGGGGAGATCGTGTGGGATAAGGGCCGGGATTTTG
CCACCGTGCGGAAAGTGCTGAGCATGCCCCAAGTGAATATCGTGAAAAAGACCGAG
GTGCAGACAGGCGGCTTCAGCAAAGAGTCTATCCTGCCCAAGAGGAACAGCGATAA
GCTGATCGCCAGAAAGAAGGACTGGGACCCTAAGAAGTACGGCGGCTTCGACAGCC
CCACCGTGGCCTATTCTGTGCTGGTGGTGGCCAAAGTGGAAAAGGGCAAGTCCAAG
AAACTGAAGAGTGTGAAAGAGCTGCTGGGGATCACCATCATGGAAAGAAGCAGCTT
CGAGAAGAATCCCATCGACTTTCTGGAAGCCAAGGGCTACAAAGAAGTGAAAAAGG
ACCTGATCATCAAGCTGCCTAAGTACTCCCTGTTCGAGCTGGAAAACGGCCGGAAG
AGAATGCTGGCCTCTGCCGGCGAACTGCAGAAGGGAAACGAACTGGCCCTGCCCTC
CAAATATGTGAACTTCCTGTACCTGGCCAGCCACTATGAGAAGCTGAAGGGCTCCCC
CGAGGATAATGAGCAGAAACAGCTGTTTGTGGAACAGCACAAGCACTACCTGGACG
AGATCATCGAGCAGATCAGCGAGTTCTCCAAGAGAGTGATCCTGGCCGACGCTAAT
CTGGACAAAGTGCTGTCCGCCTACAACAAGCACCGGGATAAGCCCATCAGAGAGCA
GGCCGAGAATATCATCCACCTGTTTACCCTGACCAATCTGGGAGCCCCTGCCGCCTT
CAAGTACTTTGACACCACCATCGACCGGAAGAGGTACACCAGCACCAAAGAGGTGC
TGGACGCCACCCTGATCCACCAGAGCATCACCGGCCTGTACGAGACACGGATCGAC
CTGTCTCAGCTGGGAGGCGACAGCCCCAAGAAGAAGAGAAAGGTGGAGGCCAGCT
AACATATGATTCGAATGTCTTTCTTGCGCTATGACACTTCCAGCAAAAGGTAGGGCG
GGCTGCGAGACGGCTTCCCGGCGCTGCATGCAACACCGATGATGCTTCGACCCCCCG
AAGCTCCTTCGGGGCTGCATGGGCGCTCCGATGCCGCTCCAGGGCGAGCGCTGTTTA
AATAGCCAGGCCCCCGATTGCAAAGACATTATAGCGAGCTACCAAAGCCATATTCA
AACACCTAGATCACTACCACTTCTACACAGGCCACTCGAGCTTGTGATCGCACTCCG
CTAAGGGGGCGCCTCTTCCTCTTCGTTTCAGTCACAACCCGCAAACATGACACAAGA
ATCCCTGTTACTTCTCGACCGTATTGATTCGGATGATTCCTACGCGAGCCTGCGGAA
CGACCAGGAATTCTGGGAGGTGAGTCGACGAGCAAGCCCGGCGGATCAGGCAGCGT
GCTTGCAGATTTGACTTGCAACGCCCGCATTGTGTCGACGAAGGCTTTTGGCTCCTCT
GTCGCTGTCTCAAGCAGCATCTAACCCTGCGTCGCCGTTTCCATTTGCAGCCGCTGG

CCCGCCGAGCCCTGGAGGAGCTCGGGCTGCCGGTGCCGCCGGTGCTGCGGGTGCCC

GGCGAGAGCACCAACCCCGTACTGGTCGGCGAGCCCGGCCCGGTGATCAAGCTGTT

CGGCGAGCACTGGTGCGGTCCGGAGAGCCTCGCGTCGGAGTCGGAGGCGTACGCGG

TCCTGGCGGACGCCCCGGTGCCGGTGCCCCGCCTCCTCGGCCGCGGCGAGCTGCGGC

CCGGCACCGGAGCCTGGCCGTGGCCCTACCTGGTGATGAGCCGGATGACCGGCACC

ACCTGGCGGTCCGCGATGGACGGCACGACCGACCGGAACGCGCTGCTCGCCCTGGC

CCGCGAACTCGGCCGGGTGCTCGGCCGGCTGCACAGGGTGCCGCTGACCGGGAACA

CCGTGCTCACCCCCCATTCCGAGGTCTTCCCGGAACTGCTGCGGGAACGCCGCGCGG

CGACCGTCGAGGACCACCGCGGGTGGGGCTACCTCTCGCCCCGGCTGCTGGACCGC

CTGGAGGACTGGCTGCCGGACGTGGACACGCTGCTGGCCGGCCGCGAACCCCGGTT

CGTCCACGGCGACCTGCACGGGACCAACATCTTCGTGGACCTGGCCGCGACCGAGG

TCACCGGGATCGTCGACTTCACCGACGTCTATGCGGGAGACTCCCGCTACAGCCTGG

TGCAACTGCATCTCAACGCCTTCCGGGGCGACCGCGAGATCCTGGCCGCGCTGCTCG

ACGGGGCGCAGTGGAAGCGGACCGAGGACTTCGCCCGCGAACTGCTCGCCTTCACC

TTCCTGCACGACTTCGAGGTGTTCGAGGAGACCCCGCTGGATCTCTCCGGCTTCACC

GATCCGGAGGAACTGGCGCAGTTCCTCTGGGGGCCGCCGGACACCGCCCCCGGCGC

CTGATAAGGATCCGGCAAGACTGGCCCCGCTTGGCAACGCAACAGTGAGCCCCTCC

CTAGTGTGTTTGGGGATGTGACTATGTATTCGTGTGTTGGCCAACGGGTCAACCCGA

ACAGATTGATACCCGCCTTGGCATTTCCTGTCAGAATGTAACGTCAGTTGATGGTAC

T

For all modified Chlamydomonas reinhardtii cells, Applicants used PCR, SURVEYOR nuclease assay, and DNA sequencing to verify successful modification.

*Example 16: Use of Cas9 as a transcriptional repressor in bacteria*

The ability to artificially control transcription is essential both to the study of gene function and to the construction of synthetic gene networks with desired properties. Applicants describe here the use of the RNA-guided Cas9 protein as a programmable transcriptional repressor.

Applicants have previously demonstrated how the Cas9 protein of *Streptococcus pyogenes* SF370 can be used to direct genome editing in *Streptococcus pneumoniae.* In this study Applicants engineered the crR6Rk strain containing a minimal CRISPR system, consisting of *cas9*, the tracrRNA and a repeat. The D10A-H840 mutations were introduced into *cas9* in this strain, giving strain crR6Rk**. Four spacers targeting different positions of the *bgaA* β-galactosidase gene promoter were cloned in the CRISPR array carried by the previously described pDB98 plasmid. Applicants observed a X to Y fold reduction in β-galactosidase activity depending on the targeted position, demonstrating the potential of Cas9 as a programmable repressor (Figure 73).

To achieve Cas9** repression in *Escherichia coli* a green fluorescence protein (GFP) reporter plasimd (pDB127) was constructred to express the *gfpmut2* gene from a constituitive promoter. The promoter was designed to carry several NPP PAMs on both strands, to measure the effect of Cas9** binding at various positions. Applicants introduced the D10A-H840 mutations into pCas9, a plasmid described carrying the tracrRNA, *cas9* and a minimal CRISPR array designed for the easy cloning of new spacers. Twenty-two different spacers were designed to target different regions of the *gfpmut2* promoter and open reading frame. An approximately 20-fold reduction of fluorescence of was observed upon targeting regions overlapping or adjacent to the - 35 and -10 promoter elements and to the Shine-Dalgarno sequence. Targets on both strands showed similar repression levels. These results suggest that the binding of Cas9** to any position of the promoter region prevents transcription initiation, presumably through steric inhibition of RNAP binding.

To determine whether Cas9** could prevent transcription elongation, Applicants directed it to the reading frame of *gpfmut2.* A reduction in fluorescence was observed both when the coding and non-coding strands where targeted, suggesting that Cas9 binding is actually strong enough to represent an obstacle to the running RNAP. However, while a 40% reduction in expression was observed when the coding strand was the target, a 20-fold reduction was observed for the non-coding strand (Fig 21b, compare T9, T10 and T11 to B9, B10 and B11). To directly determine the effects of Cas9** binding on transcription, Applicants extracted RNA from strains carrying either the T5, T10, B10 or a control construct that does not target pDB127 and subjected it to Northern blot analysis using either a probe binding before (B477) or after (B510) the B10 and T10 target sites. Consistent with Applicants' fluorescence methods, no *gfpmut2* transcription was detected when Cas9** was directed to the promoter region (T5 target) and a transcription was observed after the targeting of the T10 region. Interestingly, a smaller transcript was observed with the B477 probe. This band corresponds to the expected size of a transcript that would be interrupted by Cas9**, and is a direct indication of a transcriptional termination caused by dgRNA::Cas9** binding to the coding strand. Surprisingly, Applicants detected no transcript when the non-coding strand was targeted (B10). Since Cas9** binding to the B10 region is unlikely to interfere with transcription initiation, this result suggests that the mRNA was degraded. DgRNA::Cas9 was shown to bind ssRNA *in vitro.* Applicants speculate that binding may trigger degradation of the mRNA by host nucleases. Indeed, ribosome stalling can induce cleavage on the translated mRNA in *E. coli.*

Some applications require a precise tuning gene expression rather than its complete repression. Applicants sought to achieve intermediate repression levels through the introduction of mismatches that will weaken the crRNA/target interactions. Applicants created a series of spacers based on the B1, T5 and B10 constructs with increasing numbers of mutations in the 5' end of the crRNA. Up to 8 mutations in B1 and T5 did not affect the repression level, and a progressive increased in fluorescence was observed for additional mutations.

The observed repression with only an 8nt match between the crRNA and its target raises the question of off-targeting effects of the use of Cas9** as a transcriptional regulator. Since a good PAM (NGG) is also required for Cas9 binding, the number of nucleotides to match to obtain some level of respiration is 10. A 10nt match occurs randomly once every ~1 Mbp, and such sites are thus likely to be found even in small bacterial genomes. However, to effectively repress transcription, such site needs to be in the promoter region of gene, which makes off-targeting much less likely. Applicants also showed that gene expression can be affected if the non-coding strand of a gene is targeted. For this to happen, a random target would have to be in the right orientation, but such events relatively more likely to happen. As a matter of fact, during the course of this study Applicants were unable to construct one of the designed spacer on pCas9**. Applicants later found this spacer showed a 12bp match next to a good PAM in the essential *murC* gene. Such off-targeting could easily be avoided by a systematic blast of the designed spacers.

References:

1. Urnov, F.D., Rebar, E.J., Holmes, M.C., Zhang, H.S. & Gregory, P.D. Genome editing with engineered zinc finger nucleases. Nat. Rev. Genet. 11, 636-646 (2010).
2. Bogdanove, A.J. & Voytas, D.F. TAL effectors: customizable proteins for DNA targeting. Science 333, 1843-1846 (2011).
3. Stoddard, B.L. Homing endonuclease structure and function. Q. Rev. Biophys. 38, 49-95 (2005).
4. Bae, T. & Schneewind, O. Allelic replacement in Staphylococcus aureus with inducible counter-selection. Plasmid 55, 58-63 (2006).
5. Sung, C.K., Li, H., Claverys, J.P. & Morrison, D.A. An rpsL cassette, janus, for gene replacement through negative selection in Streptococcus pneumoniae. Appl. Environ. Microbiol. 67, 5190-5196 (2001).
6. Sharan, S.K., Thomason, L.C., Kuznetsov, S.G. & Court, D.L. Recombineering: a homologous recombination-based method of genetic engineering. Nat. Protoc. 4, 206-223 (2009).
7. Jinek, M. et al. A programmable dual-RNA-guided DNA endonuclease in adaptive bacterial immunity. Science 337, 816-821 (2012).
8. Deveau, H., Garneau, J.E. & Moineau, S. CRISPR/Cas system and its role in phage-bacteria interactions. Annu. Rev. Microbiol. 64, 475-493 (2010).
9. Horvath, P. & Barrangou, R. CRISPR/Cas, the immune system of bacteria and archaea. Science 327, 167-170 (2010).
10. Terns, M.P. & Terns, R.M. CRISPR-based adaptive immune systems. Curr. Opin. Microbiol. 14, 321-327 (2011).
11. van der Oost, J., Jore, M.M., Westra, E.R., Lundgren, M. & Brouns, S.J. CRISPR-based adaptive and heritable immunity in prokaryotes. Trends. Biochem. Sci. 34, 401-407 (2009).
12. Brouns, S.J. et al. Small CRISPRRNAs guide antiviral defense in prokaryotes. Science 321, 960-964 (2008).

13. Carte, J., Wang, R., Li, H., Terns, R.M. & Terns, M.P. Cas6 is an endoribonuclease that generates guide RNAs for invader defense in prokaryotes. Genes Dev. 22, 3489-3496 (2008).

14. Deltcheva, E. et al. CRISPR RNA maturation by trans-encoded small RNA and host factor RNase III. Nature 471, 602-607 (2011).

15. Hatoum-Aslan, A., Maniv, I. & Marraffini, L.A. Mature clustered, regularly interspaced, short palindromic repeats RNA (crRNA) length is measured by a ruler mechanism anchored at the precursor processing site. Proc. Natl. Acad. Sci. U.S.A. 108, 21218-21222 (2011).

16. Haurwitz, R.E., Jinek, M., Wiedenheft, B., Zhou, K. & Doudna, J.A. Sequence- and structure-specific RNA processing by a CRISPR endonuclease. Science 329, 1355-1358 (2010).

17. Deveau, H. et al. Phage response to CRISPR-encoded resistance in Streptococcus thermophilus. J. Bacteriol. 190, 1390-1400 (2008).

18. Gasiunas, G., Barrangou, R., Horvath, P. & Siksnys, V. Cas9-crRNA ribonucleoprotein complex mediates specific DNA cleavage for adaptive immunity in bacteria. Proc. Natl. Acad. Sci. U.S.A. (2012).

19. Makarova, K.S., Aravind, L., Wolf, Y.I. & Koonin, E.V. Unification of Cas protein families and a simple scenario for the origin and evolution of CRISPR-Cas systems. Biol. Direct. 6, 38 (2011).

20. Barrangou, R. RNA-mediated programmable DNA cleavage. Nat. Biotechnol. 30, 836-838 (2012).

21. Brouns, S.J. Molecular biology. A Swiss army knife of immunity. Science 337, 808-809 (2012).

22. Carroll, D. A CRISPR Approach to Gene Targeting. Mol. Ther. 20, 1658-1660 (2012).

23. Bikard, D., Hatoum-Aslan, A., Mucida, D. & Marraffini, L.A. CRISPR interference can prevent natural transformation and virulence acquisition during in vivo bacterial infection. Cell Host Microbe 12, 177-186 (2012).

24. Sapranauskas, R. et al. The Streptococcus thermophilus CRISPR/Cas system provides immunity in Escherichia coli. Nucleic Acids Res. (2011).

25. Semenova, E. et al. Interference by clustered regularly interspaced short palindromic repeat (CRISPR) RNA is governed by a seed sequence. Proc. Natl. Acad. Sci. U.S.A. (2011).

26. Wiedenheft, B. et al. RNA-guided complex from a bacterial immune system enhances target recognition through seed sequence interactions. Proc. Natl. Acad. Sci. U.S.A. (2011).

27. Zahner, D. & Hakenbeck, R. The Streptococcus pneumoniae beta-galactosidase is a surface protein. J. Bacteriol. 182, 5919-5921 (2000).

28. Marraffini, L.A., Dedent, A.C. & Schneewind, O. Sortases and the art of anchoring proteins to the envelopes of gram-positive bacteria. Microbiol. Mol. Biol. Rev. 70, 192-221 (2006).

29. Motamedi, M.R., Szigety, S.K. & Rosenberg, S.M. Double-strand-break repair recombination in Escherichia coli: physical evidence for a DNA replication mechanism in vivo. Genes Dev. 13, 2889-2903 (1999).

30. Hosaka, T. et al. The novel mutation K87E in ribosomal protein S12 enhances protein synthesis activity during the late growth phase in Escherichia coli. Mol. Genet. Genomics 271, 317-324 (2004).

31. Costantino, N. & Court, D.L. Enhanced levels of lambda Red-mediated recombinants in mismatch repair mutants. Proc. Natl. Acad. Sci. U.S.A. 100, 15748-15753 (2003).

32. Edgar, R. & Qimron, U. The Escherichia coli CRISPR system protects from lambda lysogenization, lysogens, and prophage induction. J. Bacteriol. 192, 6291-6294 (2010).

33. Marraffini, L.A. & Sontheimer, E.J. Self versus non-self discrimination during CRISPR RNA-directed immunity. Nature 463, 568-571 (2010).

34. Fischer, S. et al. An archaeal immune system can detect multiple Protospacer Adjacent Motifs (PAMs) to target invader DNA. J. Biol. Chem. 287, 33351-33363 (2012).

35. Gudbergsdottir, S. et al. Dynamic properties of the Sulfolobus CRISPR/Cas and CRISPR/Cmr systems when challenged with vector-borne viral and plasmid genes and protospacers. Mol. Microbiol. 79, 35-49 (2011).

36. Wang, H.H. et al. Genome-scale promoter engineering by coselection MAGE. Nat Methods 9, 591-593 (2012).

37. Cong, L. et al. Multiplex Genome Engineering Using CRISPR/Cas Systems. Science In press (2013).

38. Mali, P. et al. RNA-Guided Human Genome Engineering via Cas9. Science In press (2013).

39. Hoskins, J. et al. Genome of the bacterium Streptococcus pneumoniae strain R6. J. Bacteriol. 183, 5709-5717 (2001).

40. Havarstein, L.S., Coomaraswamy, G. & Morrison, D.A. An unmodified heptadecapeptide pheromone induces competence for genetic transformation in Streptococcus pneumoniae. Proc. Natl. Acad. Sci. U.S.A. 92, 11140-11144 (1995).

41. Horinouchi, S. & Weisblum, B. Nucleotide sequence and functional map of pC194, a plasmid that specifies inducible chloramphenicol resistance. J. Bacteriol. 150, 815-825 (1982).

42. Horton, R.M. In Vitro Recombination and Mutagenesis of DNA : SOEing Together Tailor-Made Genes. Methods Mol. Biol. 15, 251-261 (1993).

43. Podbielski, A., Spellerberg, B., Woischnik, M., Pohl, B. & Lutticken, R. Novel series of plasmid vectors for gene inactivation and expression analysis in group A streptococci (GAS). Gene 177, 137-147 (1996).

44. Husmann, L.K., Scott, J.R., Lindahl, G. & Stenberg, L. Expression of the Arp protein, a member of the M protein family, is not sufficient to inhibit phagocytosis of Streptococcus pyogenes. Infection and immunity 63, 345-348 (1995).
45. Gibson, D.G. et al. Enzymatic assembly of DNA molecules up to several hundred kilobases. Nat Methods 6, 343-345 (2009).

**Claims**

1. A method of selecting one or more prokaryotic cell(s), comprising:

   (a) introducing to a population of prokaryotic cells an engineered, programmable, non-naturally occurring CRISPR-Cas system comprising a Cas protein and one or more guide RNAs that target genomic loci of DNA molecules encoding one or more gene products in prokaryotic cell(s) of the population, wherein the one or more guide RNAs comprise a guide sequence, a tracr sequence, and a tracr mate sequence, and wherein the Cas protein and the one or more guide RNAs do not naturally occur together; and an editing template comprising one or more mutations compared to the genomic loci which abolish Cas protein cleavage;
   (b) allowing homologous recombination of the editing template with the genomic loci to obtain one or more prokaryotic cell(s) having one or more mutations in the genomic loci; and
   (c) allowing the Cas protein to cleave the genomic loci of the DNA molecules encoding the one or more gene products,

   wherein cleavage of the genomic loci induces cell death, thereby allowing one or more prokaryotic cell(s) having one or more mutations in the genomic loci to be selected.

2. The method of claim 1, wherein the Cas protein is a type II CRISPR system Cas protein.

3. The method of claim 2, wherein the Cas protein is Cas9 enzyme.

4. The method of claim 3, wherein the Cas9 enzyme is a Streptococcus pyogenes Cas9 enzyme.

5. The method of claim 1, wherein the prokaryotic cells are S. pneumoniae.

6. The method of claim 1, wherein the one or more guide RNAs target an antibiotic resistance gene.

7. The method of any preceding claim wherein one or more mutations are introduced into the genomic locus in one or more prokaryotic cell(s) of the population, in which the mutated genomic locus is not to be cleaved by the Cas9 protein.

**Patentansprüche**

1. Verfahren zum Auswählen von einer oder mehreren prokaryotischen Zelle(n), umfassend:

   (a) Einbringen, in eine Population prokaryotischer Zellen, eines hergestellten, programmierbaren, nicht natürlich vorkommenden CRISPR-Cas-Systems, umfassend ein Cas-Protein und eine oder mehrere Führungs-RNAs, welche genomische Loki von DNA-Molekülen, die ein oder mehrere Genprodukte codieren, in einer bzw. in prokaryotischen Zelle(n) der Population anzielen, wobei die eine oder mehreren Führungs-RNAs eine Führungssequenz, eine tracr-Sequenz und eine tracr-Mate-Sequenz umfassen, und wobei das Cas-Protein und die eine oder mehreren Führungs-RNAs in der Natur nicht gemeinsam vorkommen; und ein Editier-Template, umfassend eine oder mehrere Mutationen im Vergleich zu den genomischen Loki, welche Cas-Protein-Spaltung unterbinden;
   (b) Ermöglichen homologer Rekombination des Editier-Templates mit den genomischen Loki, um eine oder mehrere prokaryotische Zelle(n) mit einer oder mehreren Mutationen in den genomischen Loki zu erhalten; und
   (c) Ermöglichen, dass das Cas-Protein die genomischen Loki der DNA-Moleküle, die das eine oder die mehreren Genprodukte codieren, spaltet,

   wobei Spaltung der genomischen Loki Zelltod herbeiführt, wodurch ermöglicht wird, dass eine oder mehrere prokaryotische Zelle(n) mit einer oder mehreren Mutationen in den genomischen Loki ausgewählt wird bzw. werden.

**2.** Verfahren nach Anspruch 1, wobei das Cas-Protein ein Typ-II-CRISPR-System-Cas-Protein ist.

**3.** Verfahren nach Anspruch 2, wobei das Cas-Protein Cas9-Enzym ist.

**4.** Verfahren nach Anspruch 3, wobei das Cas9-Enzym ein *Streptococcus pyogenes* Cas9-Enzym ist.

**5.** Verfahren nach Anspruch 1, wobei die prokaryotischen Zellen *S. pneumoniae* sind.

**6.** Verfahren nach Anspruch 1, wobei die eine oder mehreren Führungs-RNAs ein Antibiotikaresistenzgen anzielen.

**7.** Verfahren nach einem vorstehenden Anspruch, wobei eine oder mehrere Mutationen in den genomischen Lokus in einer oder mehreren prokaryotischen Zelle(n) der Population eingebracht werden, wobei der mutierte genomische Lokus nicht vom Cas9-Protein gespalten werden soll.

**Revendications**

**1.** Procédé pour la sélection d'une ou de plusieurs cellule(s) procaryote(s), consistant à:

(a) introduire dans une population de cellules procaryotes un système CRISPR-Cas fabriqué, programmable, d'origine non naturelle comprenant une protéine Cas et un ou plusieurs ARNs de guidage qui ciblent des loci génomiques de molécules d'ADN codant pour un ou plusieurs produits de gène dans une ou des cellule(s) procaryote(s) de la population, dans laquelle les un ou plusieurs ARNs de guidage comprennent une séquence de guidage, une séquence tracr et une séquence d'appariement de tracr et dans laquelle la protéine Cas et les un ou plusieurs ARNs de guidage ne se présentent pas ensemble dans la nature; et une matrice d'édition comprenant une ou plusieurs mutations en comparaison avec les loci génomiques qui abolissent le clivage de la protéine Cas;
(b) permettre une recombinaison homologue de la matrice d'édition avec les loci génomiques pour obtenir une ou plusieurs cellule(s) procaryote(s) ayant une ou plusieurs mutations dans les loci génomiques; et
(c) permettre à la protéine Cas de cliver les loci génomiques des molécules d'ADN codant pour les un ou plusieurs produits de gène,

dans laquelle le clivage des loci génomiques induit une mort cellulaire, permettant ainsi à une ou plusieurs cellule(s) procaryote(s) qui ont une ou plusieurs mutations dans les loci génomiques d'être sélectionnée(s).

**2.** Procédé selon la revendication 1, dans laquelle la protéine Cas est une protéine Cas d'un système CRISPR de type II.

**3.** Procédé selon la revendication 2, dans laquelle la protéine Cas est une enzyme Cas9.

**4.** Procédé selon la revendication 3, dans laquelle l'enzyme Cas9 est une enzyme Cas9 de *Streptococcuspyogenes*.

**5.** Procédé selon la revendication 1, dans laquelle les cellules procaryotes sont *S. pneumoniae*.

**6.** Procédé selon la revendication 1, dans laquelle les un ou plusieurs ARNs de guidage ciblent un gène de résistance aux antibiotiques.

**7.** Procédé selon l'une quelconque revendication précédente, dans laquelle une ou plusieurs mutations sont introduites dans le locus génomique dans une ou plusieurs cellule(s) procaryote(s) de la population, dans laquelle le locus génomique muté ne doit pas être clivé par la protéine Cas9.

**FIG. 1**

FIG. 2A

FIG. 2B

**FIG. 2C**

D

| 2xNLS-SpCas9 | + | + | + | + | + |
| SpRNAse III | − | + | + | − | + |
| short tracrRNA | − | + | − | + | + |
| DR-EMX1-DR | + | − | + | + | + |

684bp ►

367bp ►
317bp ►

indel (%):                  4.7   5.0

FIG. 2D

E

Target locus 5'-..AGCTGGAGGAGGAAGGGCCTGAGTCCGAGCAGAAGAAGAAGGGCTCCCAC..-3'
                   |||||||||||||||||||||                    |||||||||||
              3'-..TCGACCTCCTCCTTCCCGGACTCAGGCTCGTCTTCTTCTTCCCGAGGGTG..-5'
                                    ||||||||||||||||||||||||
crRNA                            5'- GAGUCCGAGCAGAAGAAGAAGUUUUAGAGC..-3'

indel      AGCTGGAGGAGGAAGGGCCTGAGTCCGAGCAGAAGA-GAAGGGCTCCCAT

F

human *EMX1* protospacer target (mutation in 5 of 43 sequenced clones = 11.6%)

WT      5'-..CTGGAGGAGGAAGGGCCTGAGTCCGAGCAGAAG AAGAAGGGCTCCCATCACAT..-3'
Δ1         CTGGAGGAGGAAGGGCCTGAGTCCGAGCAGAAG--AGAAGGGCTCCCATCACAT
+1         CTGGAGGAGGAAGGGCCTGAGTCCGAGCAGAAGAAAGAAGGGCTCCCATCACAT
Δ3         CTGGAGGAGGAAGGGCCTGAGTCCGAGCAGAAG----AAGGGCTCCCATCACAT
m1,Δ6      CTGGAGGAGGAAGGGCCTGAGCCCGAGCAGAAG------GGCTCCCATCACAT

FIG. 2E-F

FIG. 3

FIG. 4A-C

D

FIG. 4D

FIG. 5A-B

**C**

human *EMX1* locus

200bp

HR Template

*Hind*III, *Nhe*I

**D**

| hSpCas9 | + | − | − |
| hSpCas9n | − | + | − |
| HR template | + | + | + |

2281bp ►

1189bp ►
1092bp ►

HR (%)  0.70  0.46

**E**

A A T G A C A A G C T T G C T A G C G G T G G G

*Hind*III   *Nhe*I

FIG. 5C-E

FIG. 5F

FIG. 5G

<table>
<tr><th>Cas9</th><th>target species</th><th>gene</th><th>protospacer ID</th><th>protospacer sequence (5' to 3')</th><th>PAM</th><th>strand</th><th>cell line tested</th><th>% indel (pre-crRNA + tracrRNA)</th><th>% indel (chimeric RNA)</th></tr>
<tr><td rowspan="14">S. pyogenes SF370 type II CRISPR</td><td rowspan="8">Homo sapiens</td><td>EMX1</td><td>1</td><td>GGAAGGGCCTGAGTCCGAGCAGAAGAAGAA</td><td>GGG</td><td>+</td><td>293FT</td><td>20 ± 1.8</td><td>6.7 ± 0.62</td></tr>
<tr><td>EMX1</td><td>2</td><td>CATTGGAGGTGACATCGATGTCCTCCCCAT</td><td>TGG</td><td>−</td><td>293FT</td><td>2.1 ± 0.31</td><td>N.D.</td></tr>
<tr><td>EMX1</td><td>3</td><td>GGACATCGATGTCACCTCCAATGACTAGGG</td><td>TGG</td><td>+</td><td>293FT</td><td>14 ± 1.1</td><td>N.D.</td></tr>
<tr><td>EMX1</td><td>4</td><td>CATCGATGTCCTCCCCATTGGCCTGCTTCG</td><td>TGG</td><td>−</td><td>293FT</td><td>11 ± 1.7</td><td>N.D.</td></tr>
<tr><td>EMX1</td><td>5</td><td>TTCGTGGCAATGCGCCACCGGTTGATGTGA</td><td>TGG</td><td>−</td><td>293FT</td><td>4.3 ± 0.48</td><td>2.1 ± 0.51</td></tr>
<tr><td>EMX1</td><td>6</td><td>TCGTGGCAATGCGCCACCGGTTGATGTGAT</td><td>GGG</td><td>−</td><td>293FT</td><td>4.0 ± 0.88</td><td>0.41 ± 0.25</td></tr>
<tr><td>EMX1</td><td>7</td><td>TCCAGCTTCTGCCGTTTGTACTTTGTCCTC</td><td>CGG</td><td>−</td><td>293FT</td><td>1.5 ± 0.12</td><td>N.D.</td></tr>
<tr><td>EMX1</td><td>8</td><td>GGAGGGAGGGGCACAGATGAGAAACTCAGG</td><td>AGG</td><td>−</td><td>293FT</td><td>7.8 ± 3.83</td><td>2.3 ± 1.2</td></tr>
<tr><td rowspan="3">Homo sapiens</td><td>PVALB</td><td>9</td><td>AGGGGCCCAGATTGGGTGTTCAGGGCAGAG</td><td>AGG</td><td>+</td><td>293FT</td><td>21 ± 2.6</td><td>6.5 ± 0.32</td></tr>
<tr><td>PVALB</td><td>10</td><td>ATGCAGGAGGGTGGCCGAGAGGGGCCGAGAT</td><td>TGG</td><td>+</td><td>293FT</td><td>N.D.</td><td>N.D.</td></tr>
<tr><td>PVALB</td><td>11</td><td>GGTGGCGAGAGGGGCCGAGATTGGGTGTTC</td><td>AGG</td><td>+</td><td>293FT</td><td>N.D.</td><td>N.D.</td></tr>
<tr><td rowspan="3">Mus musculus</td><td>Th</td><td>12</td><td>CAAGCACTGAGTGCCATTAGCTAAATGCAT</td><td>AGG</td><td>−</td><td>Neuro2A</td><td>27 ± 4.3</td><td>4.1 ± 2.2</td></tr>
<tr><td>Th</td><td>13</td><td>AATGCATAGGGTACCACCCACAGGTGCCAG</td><td>GGG</td><td>−</td><td>Neuro2A</td><td>4.8 ± 1.2</td><td>N.D.</td></tr>
<tr><td>Th</td><td>14</td><td>ACACACATGGGAAAGCCTCTGGGCCAGGAA</td><td>AGG</td><td>+</td><td>Neuro2A</td><td>11.3 ± 1.3</td><td>N.D.</td></tr>
<tr><td></td><td></td><td></td><td></td><td></td><td></td><td></td><td></td><td></td></tr>
<tr><td rowspan="2">S. thermophilus LMD-9 CRISPR1</td><td rowspan="2">Homo sapiens</td><td>EMX1</td><td>15</td><td>GGAGGAGGTAGTATACAGAAACACAGAAA</td><td>GTAGAAT</td><td>−</td><td>293FT</td><td>14 ± 0.88</td><td>N.T.</td></tr>
<tr><td>EMX1</td><td>16</td><td>AGAATGTAGAGGAGTCACAGAAACTCAGCA</td><td>CTAGAAA</td><td>−</td><td>293FT</td><td>7.8 ± 0.77</td><td>N.T.</td></tr>
</table>

<div style="text-align:center">FIG. 6</div>

**A**

U6 → short tracrRNA (89bp)

northern blot probe target

+1 →

..GGACGAAACACCGGAACCATTCAAAACAGCATAGCAAGTTAAAATAAGG
CTAGTCCGTTATCAACTTGAAAAAGTGGCACCGAGTCGGTGCTTTTTTT..

U6 terminator

U6 → long tracrRNA (171bp)

+1 →

..GGACGAAACACCGGTAGTATTAAGTATTGTTTTATGGCTGATAAATTTC
TTTGAATTTCTCCTTGATTATTTGTTATAAAAGTTATAAAATAATCTTGTT
GGAACCATTCAAAACAGCATAGCAAGTTAAAATAAGGCTAGTCCGTTATCA
ACTTGAAAAAGTGGCACCGAGTCGGTGCTTTTTTT..

U6 terminator

**B**

| | | | | |
|---|---|---|---|---|
| SpCas9 | + | + | + | + |
| long tracrRNA | + | + | – | – |
| short tracrRNA | – | – | + | + |
| SpRNase III | + | + | + | + |
| DR-*EMX1*(1)-DR | + | + | + | + |

684bp ►

367bp ►
317bp ►

indel (%)          0.60    14    11

FIG. 7A-B

C

FIG. 7C

FIG. 8

FIG. 9A-B

EP 2 848 690 B1

FIG. 10

A

*Streptococcus pyogenes* SF370 type II CRISPR
PAM occurence in human genome (NGG)

Median: 8bp
Mean: 12.7bp

B

*Streptococcus thermophilus* LMD-9 CRISPR1
PAM occurence in human genome (NNAGAAW)

Median: 65bp
Mean: 106.6bp

C

| Chr | NGG median | NGG mean | NNAGAAW median | NNAGAAW mean |
|-----|--------|------|--------|------|
| 1 | 7 | 12.8 | 67 | 115.8 |
| 2 | 8 | 12.7 | 64 | 100.8 |
| 3 | 8 | 13.0 | 63 | 98.5 |
| 4 | 9 | 14.0 | 61 | 94.5 |
| 5 | 8 | 13.1 | 63 | 97.9 |
| 6 | 8 | 13.1 | 63 | 98.5 |
| 7 | 8 | 12.4 | 64 | 102.9 |
| 8 | 8 | 12.8 | 64 | 100.9 |
| 9 | 7 | 13.9 | 65 | 120.5 |
| 10 | 7 | 12.1 | 66 | 107.0 |
| 11 | 7 | 12.0 | 65 | 105.8 |
| 12 | 8 | 12.4 | 65 | 103.5 |
| 13 | 8 | 13.6 | 62 | 94.6 |
| 14 | 8 | 12.0 | 65 | 101.5 |
| 15 | 7 | 11.5 | 68 | 107.7 |
| 16 | 7 | 11.7 | 74 | 136.8 |
| 17 | 6 | 10.3 | 76 | 127.9 |
| 18 | 8 | 13.4 | 63 | 101.8 |
| 19 | 6 | 9.4 | 82 | 145.4 |
| 20 | 7 | 11.1 | 72 | 121.8 |
| 21 | 7 | 13.4 | 64 | 111.4 |
| 22 | 6 | 9.2 | 85 | 140.3 |
| X | 8 | 13.2 | 63 | 99.0 |
| Y | 8 | 29.2 | 62 | 223.7 |

FIG. 11A-C

FIG. 12A-C

FIG. 13A-C

EP 2 848 690 B1

FIG. 14A-B

FIG. 15

FIG. 16

| Primer name | Assay | Genomic Target | Primer sequence |
|---|---|---|---|
| Sp-EMX1-F | SURVEYOR assay, sequencing | *EMX1* | AAAACCACCCTTCTCTCTGGC |
| Sp-EMX1-R | SURVEYOR assay, sequencing | *EMX1* | GGAGATTGGAGACACGGAGAG |
| Sp-PVALB-F | SURVEYOR assay, sequencing | *PVALB* | CTGGAAAGCCAATGCCTGAC |
| Sp-PVALB-R | SURVEYOR assay, sequencing | *PVALB* | GGCAGCAAACTCCTTGTCCT |
| Sp-Th-F | SURVEYOR assay, sequencing | *Th* | GTGCTTTGCAGAGGCCTACC |
| Sp-Th-R | SURVEYOR assay, sequencing | *Th* | CCTGGAGCGCATGCAGTAGT |
| St-EMX1-F | SURVEYOR assay, sequencing | *EMX1* | ACCTTCTGTGTTTCCACCATTC |
| St-EMX1-R | SURVEYOR assay, sequencing | *EMX1* | TTGGGGAGTGCACAGACTTC |
| Sp-EMX1-RFLP-F | RFLP, sequencing | *EMX1* | GGCTCCCTGGGTTCAAAGTA |
| Sp-EMX1-RFLP-R | RFLP, sequencing | *EMX1* | AGAGGGGTCTGGATGTCGTAA |
| Pb_EMX1_sp1 | Northern Blot Probe | Not applicable | TAGCTCTAAAACTTCTTCTTCTGCTCGGAC |
| Pb_tracrRNA | Northern Blot Probe | Not applicable | CTAGCCTTATTTTAACTTGCTATGCTGTTT |

FIG. 17

FIG. 18A

**FIG. 18B**

FIG. 18C

FIG. 19A-B

FIG. 20

FIG. 21

FIG. 22

FIG. 23

## a

crR6

R6$^{8232.5}$

5' TCGGTGCGGTGGTTGATTTCTTCTTGCCTTTTTGGCTT 3'

3' AGCCACGCCACCAACTAAAGAAGAACGGCAAAAACCGAA 5'

PAM

crRNA 5' GAUUUCUUCUUGCGCUUUUUGUUUUA 3'

Randomized PAM

Randomized protospacer

Transformation and chloramphenicol selection

Isolation of cells with inactive PAMs (2x10³ colonies)

Deep sequencing

## b

2nd PAM position

| | | A | | C | | G | | T | | |
|---|---|---|---|---|---|---|---|---|---|---|
| A | AAA | 1.02 | ACA | 1.06 | AGA | 0.95 | ATA | 1.02 | | A |
| C | CAA | 1.03 | CCA | 1.03 | CGA | 0.97 | CTA | 1.00 | | |
| G | GAA | 1.10 | GCA | 1.09 | GGA | 0.93 | GTA | 1.01 | | |
| T | TAA | 1.02 | TCA | 1.11 | TGA | 0.91 | TTA | 1.11 | | |
| A | AAC | 1.07 | ACC | 1.02 | AGC | 1.00 | ATC | 1.09 | | C |
| C | CAC | 1.06 | CCC | 1.04 | CGC | 1.00 | CTC | 1.09 | | |
| G | GAC | 1.05 | GCC | 1.02 | GGC | 1.02 | GTC | 1.22 | | |
| T | TAC | 1.06 | TCC | 1.05 | TGC | 1.03 | TTC | 1.06 | | |
| A | AAG | 0.49 | ACG | 0.97 | AGG | 0.06 | ATG | 0.96 | | G |
| C | CAG | 0.78 | CCG | 1.08 | CGG | 0.06 | CTG | 1.03 | | |
| G | GAG | 0.50 | GCG | 1.00 | GGG | 0.06 | GTG | 1.20 | | |
| T | TAG | 0.86 | TCG | 0.91 | TGG | 0.07 | TTG | 1.06 | | |
| A | AAT | 1.01 | ACT | 1.09 | AGT | 1.04 | ATT | 1.03 | | T |
| C | CAT | 1.03 | CCT | 1.03 | CGT | 1.07 | CTT | 1.08 | | |
| G | GAT | 1.04 | GCT | 1.08 | GGT | 1.08 | GTT | 1.12 | | |
| T | TAT | 1.06 | TCT | 1.03 | TGT | 1.06 | TTT | 1.01 | | |

1st PAM position

3rd PAM position

## c

No CRISPR targeting

CRISPR targeting

Position | 20 | 19 | 18 | 17 | 16 | 15 | 14 | 13 | 12 | 11 | 10 | 9 | 8 | 7 | 6 | 5 | 4 | 3 | 2 | 1 | TGG

Protospacer

PAM

A C G T

FIG. 24

FIG. 25

FIG. 26

FIG. 27

FIG. 28

FIG. 29

FIG. 30

FIG. 31

FIG. 32

FIG. 33

FIG. 34

FIG. 35

FIG. 36

FIG. 37

FIG. 38

FIG. 39A

FIG. 39B

FIG. 39C

FIG. 39D

FIG. 40A

**FIG. 40B**

gi|148255343|ref|YP_001239928.1|, hypothetical protein BBta_3952 [Bradyrhizobium sp. BTAi1]
gi|288957741|ref|YP_003448082.1|, CRISPR-associated protein, Csn1 family [Azospirillum sp. B510]
gi|159042956|ref|YP_001531750.1|, CRISPR-associated protein [Dinoroseobacter shibae DFL 12]
gi|332188827|ref|ZP_08390536.1|, hypothetical protein SUS17_3938 [Sphingomonas sp. S17]
gi|319760940|ref|YP_004124877.1|, crispr-associated protein, csn1 family [Alicycliphilus denitrificans K601]
gi|330822845|ref|YP_004386148.1|, CRISPR-associated protein, Csn1 family [Alicycliphilus denitrificans K601]
gi|326315085|ref|YP_004232757.1|, CSPR-associated protein, Csn1 family [Acidovorax avenae subsp. avenae ATCC 19860]
gi|121608211|ref|YP_996018.1|, CRISPR-associated endonuclease Csn1 family protein [Verminephrobacter eiseniae EF01-2]
gi|325983496|ref|YP_004295898.1|, CRISPR-associated protein, Csn1 family [Nitrosomonas sp. AL212]
gi|304313029|ref|YP_003812627.1|, hypothetical protein HDN1F_34120 [gamma proteobacterium HdN1]
gi|34171927|emb|CCA84553.1|, conserved hypothetical protein [Ralstonia syzygii R24]
gi|294008611|ref|YP_003552871.1|, CRISPR-associated protein, Csn1 family [Candidatus Puniceispirillum marinum IMCC1322]
gi|187259660|ref|YP_001875142.1|, CRISPR-associated endonuclease Csn1 family protein [Elusimicrobium minutum Pei191]
gi|118945059|ref|YP_001956000.1|, Csn1-like CRISPR-associated protein [uncultured Termite group 1 bacterium phylotype Rs-D17]
gi|325972003|ref|YP_004248194.1|, CRISPR-associated protein, Csn1 family [Sphaerochaeta globus str. Buddy]
gi|282878504|ref|ZP_06287286.1|, CRISPR-associated protein, Csn1 family [Prevotella buccalis ATCC 35310]
gi|282880052|ref|ZP_06288774.1|, CRISPR-associated protein, Csn1 family [Prevotella timonensis CRIS 5C-B1]
gi|258646111|ref|ZP_05735580.1|, CRISPR-associated protein, Csn1 family [Prevotella tannerae ATCC 51259]
gi|294674019|ref|YP_003574635.1|, Csn1 family CRISPR-associated protein [Prevotella ruminicola 23]
gi|347536497|ref|YP_004843922.1|, putative CRISPR-associated (Cas) protein [Flavobacterium branchiophilum FL-15]
gi|365959402|ref|YP_004940569.1|, putative CRISPR-associated (Cas) protein [Flavobacterium columnare ATCC 49512]
gi|218258638|ref|ZP_03474956.1|, hypothetical protein PRABACTJOHN_00621 [Parabacteroides johnsonii DSM 18315]
gi|256840409|ref|ZP_05545917.1|, CRISPR-associated protein [Parabacteroides sp. D13]
gi|224535832|ref|ZP_03676371.1|, hypothetical protein BACCELL_00696 [Bacteroides cellulosilyticus DSM 14838]
gi|60683389|ref|YP_211533.1|, hypothetical protein BF3954 [Bacteroides fragilis NCTC 9343]
gi|213962376|ref|ZP_03390639.1|, crispr-associated protein, Csn1 family [Prevotella oralis ATCC 33269]
gi|256819408|ref|YP_003140687.1|, CRISPR-associated protein, Csn1 family [Capnocytophaga spurigena Capno]
gi|298373376|ref|ZP_06983365.1|, CRISPR-associated protein, Csn1 family [Capnocytophaga ochracea DSM 7271]
gi|346622236|ref|YP_004760688.1|, hypothetical protein Ccan_14650 [Capnocytophaga canimorsus Cc5]
gi|163754820|ref|ZP_02161941.1|, hypothetical protein [Bacteroidetes oral taxon 274 str. F0058]
gi|295136244|ref|YP_003586920.1|, hypothetical protein ZPR_4422 [Zunongwangia profunda SM-A87]
gi|150025575|ref|YP_001296401.1|, CRISPR-associated endonuclease Csn1 family protein [Flavobacterium psychrophilum JIP02/86]
gi|212694363|ref|ZP_03302491.1|, hypothetical protein BACDOR_03889 [Bacteroides dorei DSM 17855]
gi|301311869|ref|ZP_07217791.1|, conserved hypothetical protein [Bacteroides sp. 20_3]
gi|224026357|ref|ZP_03644723.1|, hypothetical protein BACCOPRO_03113 [Bacteroides coprophilus DSM 18228]
gi|261414553|ref|YP_003248236.1|, CRISPR-associated protein, Csn1 family [Fibrobacter succinogenes subsp. succinogenes S85]
gi|260592128|ref|ZP_05857566.1|, conserved hypothetical protein [Prevotella melaninogenica D18]
gi|288802595|ref|ZP_06408034.1|, CRISPR-associated protein, Csn1 family [Prevotella veroralis F0319]
gi|315607525|ref|ZP_07882520.1|, csn1 family CRISPR-associated protein [Prevotella buccae ATCC 33574]
gi|282858617|ref|ZP_06267779.1|, CRISPR-associated protein, Csn1 family [Prevotella bivia JCVIHMP010]
gi|300771242|ref|ZP_07081118.1|, csn1 family CRISPR-associated protein [Sphingobacterium spiritivorum ATCC 33861]

FIG. 40C

FIG. 40D

EP 2 848 690 B1

gi|241889924|ref|ZP_04777222.1|, CRISPR-associated protein, Csn1 family [Gemella haemolysans ATCC 10379]

gi|317495358|ref|ZP_07953728.1|, csn1 family CRISPR-associated protein [Gemella morbillorum M424]

gi|299144352|ref|ZP_07037432.1|, CRISPR-associated protein, Csn1 family [Peptoniphilus sp. oral taxon 386 str. F0131]

gi|282849530|ref|ZP_06258914.1|, CRISPR-associated protein, Csn1 family [Veillonella parvula ATCC 17745]

gi|303229466|ref|ZP_07316256.1|, CRISPR-associated protein, Csn1 family [Veillonella atypica ACS-134-V-Col7a]

gi|342218215|ref|ZP_08710837.1|, CRISPR-associated protein, Csn1 family [Megasphaera sp. UPII 135-E]

gi|169823755|ref|YP_001691366.1|, hypothetical protein FMG_0058 [Finegoldia magna ATCC 29328]

gi|295106015|emb|CBL03558.1|, CRISPR-associated protein, Csn1 family [Gordonibacter pamelaeae 7-10-1-b]

gi|339445983|ref|YP_004711987.1|, hypothetical protein EGYY_25780 [Eggerthella sp. YY7918]

gi|302336020|ref|YP_003801227.1|, CRISPR-associated protein, Csn1 family [Olsenella uli DSM 7084]

gi|328956315|ref|YP_004373648.1|, CRISPR-associated protein, Csn1 family [Coriobacterium glomerans PW2]

gi|323463801|gb|ADX75954.1|, CRISPR-associated protein, Csn1 family [Staphylococcus pseudintermedius ED99]

gi|410878248|gb|EKS26134.1|, CRISPR-associated protein cas9/csn1, subtype II/nmemi [Staphylococcus simulans ACS-120-V-Sch...

gi|191639137|ref|YP_001988303.1|, hypothetical protein LCABL_23780 [Lactobacillus casei BL23]

gi|301067199|ref|YP_003789222.1|, hypothetical protein LCAZH_2162 [Lactobacillus casei str. Zhang]

gi|239630053|ref|ZP_04673084.1|, conserved hypothetical protein [Lactobacillus paracasei subsp. paracasei 8700:2]

gi|258509199|ref|YP_003171950.1|, CRISPR-associated protein Csn1 [Lactobacillus rhamnosus GG]

gi|227509761|ref|ZP_03939810.1|, possible CRISPR associated protein [Lactobacillus brevis subsp. gravesensis ATCC 27305]

gi|227512703|ref|ZP_03942752.1|, CRISPR associated protein [Lactobacillus buchneri ATCC 11577]

gi|331702228|ref|YP_004399187.1|, CRISPR-associated protein, Csn1 family [Lactobacillus buchneri NRRL B-30929]

gi|304386254|ref|ZP_07368587.1|, csn1 family CRISPR-associated protein [Pediococcus acidilactici DSM 20284]

gi|300361537|ref|ZP_07057714.1|, csn1 family CRISPR-associated protein [Lactobacillus gasseri JV-V03]

gi|385826041|ref|YP_005862383.1|, CRISPR-associated protein [Lactobacillus johnsonii DPC 6026]

gi|238854567|ref|ZP_04644902.1|, CRISPR-associated protein, Csn1 family [Lactobacillus jensenii 269-3]

gi|227514633|ref|ZP_03944682.1|, possible CRISPR associated protein [Lactobacillus fermentum ATCC 14931]

gi|323340068|ref|ZP_08080334.1|, CRISPR associated protein [Lactobacillus ruminis ATCC 25644]

gi|347534532|ref|YP_004841202.1|, hypothetical protein LSA_08670 [Lactobacillus sanfranciscensis TMW 1.1304]

gi|310286728|ref|YP_003937986.1|, CRISPR associated protein [Bifidobacterium bifidum S17]

gi|366983953|gb|EHN59352.1|, CRISPR-associated protein [Oenococcus kitaharae DSM 17330]

gi|16801805|ref|NP_472073.1|, hypothetical protein lin2744 [Listeria innocua Clip11262]

gi|47097148|ref|ZP_00234715.1|, CRISPR-associated protein, SAG0894 family [Listeria monocytogenes str. 1/2a F6854]

gi|257893735|ref|ZP_05673388.1|, CRISPR-associated protein [Enterococcus faecium 1,231,408]

gi|315641599|ref|ZP_07896667.1|, csn1 family CRISPR-associated protein [Enterococcus italicus DSM 15952]

gi|116628213|ref|YP_820832.1|, CRISPR-system-like protein [Streptococcus thermophilus LMD-9]

**FIG. 40E**

gi|22537057|ref|NP_687908.1| , hypothetical protein SAG0894 [Streptococcus agalactiae 2603V/R]

gi|76788458|ref|YP_329639.1| , hypothetical protein SAK_1017 [Streptococcus agalactiae A909]

gi|25010965|ref|NP_735360.1| , hypothetical protein gbs0911 [Streptococcus agalactiae NEM316]

gi|24379809|ref|NP_721764.1| , hypothetical protein SMU_1405c [Streptococcus mutans UA159]

gi|290580220|ref|YP_003484612.1| , hypothetical protein SmuNN2025_0694 [Streptococcus mutans NN2025]

gi|306833855|ref|ZP_07466980.1| , csn1 family CRISPR-associated protein [Streptococcus bovis ATCC 700338]

gi|325978669|ref|YP_004288385.1| , CRISPR-associated protein [Streptococcus gallolyticus subsp. gallolyticus ATCC BAA-2069]

gi|288905639|ref|YP_003430861.1| , CRISPR-associated protein [Streptococcus gallolyticus UCN34]

gi|320547102|ref|ZP_08041398.1| , csn1 family CRISPR-associated protein [Streptococcus equinus ATCC 9812]

gi|315223162|ref|ZP_07865023.1| , CRISPR-associated protein, Csn1 family [Streptococcus anginosus F0211]

gi|357636406|ref|ZP_09134281.1| , CRISPR-associated protein Cas9/Csn1, subtype II/NMEMI [Streptococcus macacae NCTC 11558]

gi|313890160|ref|ZP_07823795.1| , CRISPR-associated protein, Csn1 family [Streptococcus pseudoporcinus SPIN 20026]

gi|195978435|ref|YP_002123679.1| , CRISPR-Associated Protein Csn1 [Streptococcus equi subsp. zooepidemicus MGCS10565]

gi|94994317|ref|YP_602415.1| , hypothetical protein MGAS10750_Spy0921 [Streptococcus pyogenes MGAS10750]

gi|251782637|ref|YP_002996940.1| , hypothetical protein SDEG_1231 [Streptococcus dysgalactiae subsp. equisimilis GGS_124]

gi|21910213|ref|NP_664481.1| , hypothetical protein SpyM3_0677 [Streptococcus pyogenes MGAS315]

gi|28896088|ref|NP_802438.1| , hypothetical protein SPs1176 [Streptococcus pyogenes SSI-1]

gi|209559356|ref|YP_002285828.1| , hypothetical protein Spy49_0823 [Streptococcus pyogenes NZ131]

gi|15675041|ref|NP_269215.1| , hypothetical protein SPy_1046 [Streptococcus pyogenes M1 GAS]

gi|71910582|ref|YP_282132.1| , hypothetical protein M5005_Spy_0769 [Streptococcus pyogenes MGAS5005]

gi|94990395|ref|YP_598495.1| , putative cytoplasmic protein [Streptococcus pyogenes MGAS10270]

gi|94988516|ref|YP_596617.1| , cytoplasmic protein [Streptococcus pyogenes MGAS9429]

gi|94992340|ref|YP_600439.1| , putative cytoplasmic protein [Streptococcus pyogenes MGAS2096]

gi|71903413|ref|YP_280216.1| , cytoplasmic protein [Streptococcus pyogenes MGAS6180]

FIG. 40F

SpCas9 mutation positions

hSpCas9

5'  ATGGACAAGAAGTACAGCATCGGCCTGGACATCGGCACCAACTCTGTGGGCTGGGCCGTG
                                                                        60

| | hSpCas9 | | |
|---|---|---|---|

| | | HtH | |

| | | RuvC1 | |

| M | D | K | K | Y | S | I | G | L | D | I | G | T | N | S | V | G | W | A | V |
|---|---|---|---|---|---|---|---|---|---|---|---|---|---|---|---|---|---|---|---|
| 1 | 2 | 3 | 4 | 5 | 6 | 7 | 8 | 9 | 10 | 11 | 12 | 13 | 14 | 15 | 16 | 17 | 18 | 19 | 20 |

5'  ATCACCGACGAGTACAAGGTGCCCAGCAAGAAATTCAAGGTGCTGGGCAACACCGACCGG
                                                                        120

| | | hSpCas9 | |

| RuvC1 | |

| I | T | D | E | Y | K | V | P | S | K | K | F | K | V | L | G | N | T | D | R |
|---|---|---|---|---|---|---|---|---|---|---|---|---|---|---|---|---|---|---|---|
| 21 | 22 | 23 | 24 | 25 | 26 | 27 | 28 | 29 | 30 | 31 | 32 | 33 | 34 | 35 | 36 | 37 | 38 | 39 | 40 |

5'  CACAGCATCAAGAAGAACCTGATCGGAGCCCTGCTGTTCGACAGCGGCGAAACAGCCGAG
                                                                        180

| | | hSpCas9 | |

| H | S | I | K | K | N | L | I | G | A | L | L | F | D | S | G | E | T | A | E |
|---|---|---|---|---|---|---|---|---|---|---|---|---|---|---|---|---|---|---|---|
| 41 | 42 | 43 | 44 | 45 | 46 | 47 | 48 | 49 | 50 | 51 | 52 | 53 | 54 | 55 | 56 | 57 | 58 | 59 | 60 |

5'  GCCACCCGGCTGAAGAGAACCGCCAGAAGAAGATACACCAGACGGAAGAACCGGATCTGC
                                                                        240

| | | hSpCas9 | |

| A | T | R | L | K | R | T | A | R | R | R | Y | T | R | R | K | N | R | I | C |
|---|---|---|---|---|---|---|---|---|---|---|---|---|---|---|---|---|---|---|---|
| 61 | 62 | 63 | 64 | 65 | 66 | 67 | 68 | 69 | 70 | 71 | 72 | 73 | 74 | 75 | 76 | 77 | 78 | 79 | 80 |

5'  TATCTGCAAGAGATCTTCAGCAACGAGATGGCCAAGGTGGACGACAGCTTCTTCCACAGA
                                                                        300

| | | hSpCas9 | |

| Y | L | Q | E | I | F | S | N | E | M | A | K | V | D | D | S | F | F | H | R |
|---|---|---|---|---|---|---|---|---|---|---|---|---|---|---|---|---|---|---|---|
| 81 | 82 | 83 | 84 | 85 | 86 | 87 | 88 | 89 | 90 | 91 | 92 | 93 | 94 | 95 | 96 | 97 | 98 | 99 | 100 |

FIG. 41A

170

FIG. 41B

hSpCas9

5' CGGCTGGAAAATCTGATCGCCCAGCTGCCCGGCGAGAAGAAGAATGGCCTGTTCGGCAAC

720

hSpCas9

R L E N L I A Q L P G E K K N G L F G N
221 222 223 224 225 226 227 228 229 230 231 232 233 234 235 236 237 238 239 240

5' CTGATTGCCCTGAGCCTGGGCCTGACCCCCAACTTCAAGAGCAACTTCGACCTGGCCGAG

780

hSpCas9

L I A L S L G L T P N F K S N F D L A E
241 242 243 244 245 246 247 248 249 250 251 252 253 254 255 256 257 258 259 260

5' GATGCCAAACTGCAGCTGAGCAAGGACACCTACGACGACGACCTGGACAACCTGCTGGCC

840

hSpCas9

D A K L Q L S K D T Y D D D L D N L L A
261 262 263 264 265 266 267 268 269 270 271 272 273 274 275 276 277 278 279 280

5' CAGATCGGCGACCAGTACGCCGACCTGTTTCTGGCCGCCAAGAACCTGTCCGACGCCATC

900

hSpCas9

Q I G D Q Y A D L F L A A K N L S D A I
281 282 283 284 285 286 287 288 289 290 291 292 293 294 295 296 297 298 299 300

5' CTGCTGAGCGACATCCTGAGAGTGAACACCGAGATCACCAAGGCCCCCCTGAGCGCCTCT

960

hSpCas9

L L S D I L R V N T E I T K A P L S A S
301 302 303 304 305 306 307 308 309 310 311 312 313 314 315 316 317 318 319 320

5' ATGATCAAGAGATACGACGAGCACCACCAGGACCTGACCCTGCTGAAAGCTCTCGTGCGG

1020

hSpCas9

M I K R Y D E H H Q D L T L L K A L V R
321 322 323 324 325 326 327 328 329 330 331 332 333 334 335 336 337 338 339 340

FIG. 41C

hSpCas9

5' CAGCAGCTGCCTGAGAAGTACAAAGAGATTTTCTTCGACCAGAGCAAGAACGGCTACGCC 1080

hSpCas9

Q Q L P E K Y K E I F F D Q S K N G Y A
341 342 343 344 345 346 347 348 349 350 351 352 353 354 355 356 357 358 359 360

5' GGCTACATTGACGGCGGAGCCAGCCAGGAAGAGTTCTACAAGTTCATCAAGCCCATCCTG 1140

hSpCas9

G Y I D G G A S Q E E F Y K F I K P I L
361 362 363 364 365 366 367 368 369 370 371 372 373 374 375 376 377 378 379 380

5' GAAAAGATGGACGGCACCGAGGAACTGCTCGTGAAGCTGAACAGAGAGGACCTGCTGCGG 1200

hSpCas9

E K M D G T E E L L V K L N R E D L L R
381 382 383 384 385 386 387 388 389 390 391 392 393 394 395 396 397 398 399 400

5' AAGCAGCGGACCTTCGACAACGGCAGCATCCCCCACCAGATCCACCTGGGAGAGCTGCAC 1260

hSpCas9

K Q R T F D N G S I P H Q I H L G E L H
401 402 403 404 405 406 407 408 409 410 411 412 413 414 415 416 417 418 419 420

5' GCCATTCTGCGGCGGCAGGAAGATTTTTACCCATTCCTGAAGGACAACCGGGAAAAGATC 1320

hSpCas9

A I L R R Q E D F Y P F L K D N R E K I
421 422 423 424 425 426 427 428 429 430 431 432 433 434 435 436 437 438 439 440

5' GAGAAGATCCTGACCTTCCGCATCCCCTACTACGTGGGCCCTCTGGCCAGGGGAAACAGC 1380

hSpCas9

E K I L T F R I P Y Y V G P L A R G N S
441 442 443 444 445 446 447 448 449 450 451 452 453 454 455 456 457 458 459 460

FIG. 41D

hSpCas9

5' AGATTCGCCTGGATGACCAGAAAGAGCGAGGAAACCATCACCCCCTGGAACTTCGAGGAA
1440

hSpCas9

R F A W M T R K S E E T I T P W N F E E
461 462 463 464 465 466 467 468 469 470 471 472 473 474 475 476 477 478 479 480

5' GTGGTGGACAAGGGCGCTTCCGCCCAGAGCTTCATCGAGCGGATGACCAACTTCGATAAG
1500

hSpCas9

V V D K G A S A Q S F I E R M T N F D K
481 482 483 484 485 486 487 488 489 490 491 492 493 494 495 496 497 498 499 500

5' AACCTGCCCAACGAGAAGGTGCTGCCCAAGCACAGCCTGCTGTACGAGTACTTCACCGTG
1560

hSpCas9

N L P N E K V L P K H S L L Y E Y F T V
501 502 503 504 505 506 507 508 509 510 511 512 513 514 515 516 517 518 519 520

5' TATAACGAGCTGACCAAAGTGAAATACGTGACCGAGGGAATGAGAAAGCCCGCCTTCCTG
1620

hSpCas9

Y N E L T K V K Y V T E G M R K P A F L
521 522 523 524 525 526 527 528 529 530 531 532 533 534 535 536 537 538 539 540

5' AGCGGCGAGCAGAAAAAGGCCATCGTGGACCTGCTGTTCAAGACCAACCGGAAAGTGACC
1680

hSpCas9

S G E Q K K A I V D L L F K T N R K V T
541 542 543 544 545 546 547 548 549 550 551 552 553 554 555 556 557 558 559 560

5' GTGAAGCAGCTGAAAGAGGACTACTTCAAGAAAATCGAGTGCTTCGACTCCGTGGAAATC
1740

hSpCas9

V K Q L K E D Y F K K I E C F D S V E I
561 562 563 564 565 566 567 568 569 570 571 572 573 574 575 576 577 578 579 580

FIG. 41E

EP 2 848 690 B1

hSpCas9

5'  TCCGGCGTGGAAGATCGGTTCAACGCCTCCCTGGGCACATACCACGATCTGCTGAAAATT

hSpCas9
S    G    V    E    D    R    F    N    A    S    L    G    T    Y    H    D    L    L    K    I
581  582  583  584  585  586  587  588  589  590  591  592  593  594  595  596  597  598  599  600

5'  ATCAAGGACAAGGACTTCCTGGACAATGAGGAAAACGAGGACATTCTGGAAGATATCGTG

hSpCas9
I    K    D    K    D    F    L    D    N    E    E    N    E    D    I    L    E    D    I    V
601  602  603  604  605  606  607  608  609  610  611  612  613  614  615  616  617  618  619  620

5'  CTGACCCTGACACTGTTTGAGGACAGAGAGATGATCGAGGAACGGCTGAAAACCTATGCC

hSpCas9
L    T    L    T    L    F    E    D    R    E    M    I    E    E    R    L    K    T    Y    A
621  622  623  624  625  626  627  628  629  630  631  632  633  634  635  636  637  638  639  640

5'  CACCTGTTCGACGACAAAGTGATGAAGCAGCTGAAGCGGCGGAGATACACCGGCTGGGGC

hSpCas9
H    L    F    D    D    K    V    M    K    Q    L    K    R    R    R    Y    T    G    W    G
641  642  643  644  645  646  647  648  649  650  651  652  653  654  655  656  657  658  659  660

5'  AGGCTGAGCCGGAAGCTGATCAACGGCATCCGGGACAAGCAGTCCGGCAAGACAATCCTG

hSpCas9
R    L    S    R    K    L    I    N    G    I    R    D    K    Q    S    G    K    T    I    L
661  662  663  664  665  666  667  668  669  670  671  672  673  674  675  676  677  678  679  680

5'  GATTCCTGAAGTCCGACGGCTTCGCCAACAGAAACTTCATGCAGCTGATCCACGACGAC

hSpCas9
D    F    L    K    S    D    G    F    A    N    R    N    F    M    Q    L    I    H    D    D
681  682  683  684  685  686  687  688  689  690  691  692  693  694  695  696  697  698  699  700

FIG. 41F

175

hSpCas9

5' AGCCTGACCTTTAAAGAGGACATCCAGAAAGCCCAGGTGTCCGGCCAGGGCGATAGCCTG

2160

hSpCas9

S   L   T   F   K   E   D   I   Q   K   A   Q   V   S   G   Q   G   D   S   L
701 702 703 704 705 706 707 708 709 710 711 712 713 714 715 716 717 718 719 720

5' CACGAGCACATTGCCAATCTGGCCGGCAGCCCCGCCATTAAGAAGGGCATCCTGCAGACA

2220

hSpCas9

H   E   H   I   A   N   L   A   G   S   P   A   I   K   K   G   I   L   Q   T
721 722 723 724 725 726 727 728 729 730 731 732 733 734 735 736 737 738 739 740

5' GTGAAGGTGGTGGACGAGCTCGTGAAAGTGATGGGCCGGCACAAGCCCGAGAACATCGTG

2280

hSpCas9

RuvC II

V   K   V   V   D   E   L   V   K   V   M   G   R   H   K   P   E   N   I   V
741 742 743 744 745 746 747 748 749 750 751 752 753 754 755 756 757 758 759 760

5' ATCGCCATGGCCAGAGAGAACCAGACCACCCAGAAGGGACAGAAGAACAGCCGCGAGAGA

2340

hSpCas9

RuvC II

E

I   A   M   A   R   E   N   Q   T   T   Q   K   G   Q   K   N   S   R   E   R
761 762 763 764 765 766 767 768 769 770 771 772 773 774 775 776 777 778 779 780

5' ATGAAGCGGATCGAAGAGGGCATCAAAGAGCTGGGCAGCCAGATCCTGAAAGAACACCCC

2400

hSpCas9

M   K   R   I   E   E   G   I   K   E   L   G   S   Q   I   L   K   E   H   P
781 782 783 784 785 786 787 788 789 790 791 792 793 794 795 796 797 798 799 800

FIG. 41G

hSpCas9

5' GTGGAAAACACCCAGCTGCAGAACGAGAAGCTGTACCTGTACTACCTGCAGAATGGGCGG
    ++++|++++|++++|++++|++++|++++|++++|++++|++++|++++|++++|++++|    2460

hSpCas9

    V  E  N  T  Q  L  Q  N  E  K  L  Y  L  Y  Y  L  Q  N  G  R
   801 802 803 804 805 806 807 808 809 810 811 812 813 814 815 816 817 818 819 820

5' GATATGTACGTGGACCAGGAACTGGACATCAACCGGCTGTCCGACTACGATGTGGACGCC
    ++++|++++|++++|++++|++++|++++|++++|++++|++++|++++|++++|++++|    2520

hSpCas9

                                                        HNH

                                                              H

    D  M  Y  V  D  Q  E  L  D  I  N  R  L  S  D  Y  D  V  D  A
   821 822 823 824 825 826 827 828 829 830 831 832 833 834 835 836 837 838 839 840

5' ATCGTGCCTCAGAGCTTTCTGAAGGACGACTCCATCGACGCCAAGGTGCTGACCAGAAGC
    ++++|++++|++++|++++|++++|++++|++++|++++|++++|++++|++++|++++|    2580

hSpCas9

HNH

                                     N

    I  V  P  Q  S  F  L  K  D  D  S  I  D  A  K  V  L  T  R  S
   841 842 843 844 845 846 847 848 849 850 851 852 853 854 855 856 857 858 859 860

5' GACAAGGCCCGGGGCAAGAGCGACAACGTGCCCTCCGAAGAGGTCGTGAAGAAGATGAAG
    ++++|++++|++++|++++|++++|++++|++++|++++|++++|++++|++++|++++|    2640

hSpCas9

HNH

      N

    D  K  A  R  G  K  S  D  N  V  P  S  E  E  V  V  K  K  M  K
   861 862 863 864 865 866 867 868 869 870 871 872 873 874 875 876 877 878 879 880

5' AACTACTGGCGGCAGCTGCTGAACGCCAAGCTGATTACCCAGAGAAAGTTCGACAATCTG
    ++++|++++|++++|++++|++++|++++|++++|++++|++++|++++|++++|++++|    2700

hSpCas9

    N  Y  W  R  Q  L  L  N  A  K  L  I  T  Q  R  K  F  D  N  L
   881 882 883 884 885 886 887 888 889 890 891 892 893 894 895 896 897 898 899 900

FIG. 41H

hSpCas9

5' ACCAAGGCCGAGAGAGGCGGCCTGAGCGAACTGGATAAGGCCGGCTTCATCAAGAGACAG

2760

hSpCas9

T K A E R G G L S E L D K A G F I K R Q

901 902 903 904 905 906 907 908 909 910 911 912 913 914 915 916 917 918 919 920

5' CTGGTGGAAACCCGGCAGATCACAAAGCACGTGGCACAGATCCTGGACTCCCGGATGAAC

2820

hSpCas9

L V E T R Q I T K H V A Q I L D S R M N

921 922 923 924 925 926 927 928 929 930 931 932 933 934 935 936 937 938 939 940

5' ACTAAGTACGACGAGAATGACAAGCTGATCCGGGAAGTGAAAGTGATCACCCTGAAGTCC

2880

hSpCas9

T K Y D E N D K L I R E V K V I T L K S

941 942 943 944 945 946 947 948 949 950 951 952 953 954 955 956 957 958 959 960

5' AAGCTGGTGTCCGATTTCCGGAAGGATTTCCAGTTTTACAAAGTGCGCGAGATCAACAAC

2940

hSpCas9

K L V S D F R K D F Q F Y K V R E I N N

961 962 963 964 965 966 967 968 969 970 971 972 973 974 975 976 977 978 979 980

5' TACCACCACGCCCACGACGCCTACCTGAACGCCGTCGTGGGAACCGCCCTGATCAAAAAG

3000

hSpCas9

RuvC II

D...

Y H H A H D A Y L N A V V G T A L I K K

981 982 983 984 985 986 987 988 989 990 991 992 993 994 995 996 997 998 999 1000

FIG. 41I

178

5' TACCCTAAGCTGGAAAGCGAGTTCGTGTACGGCGACTACAAGGTGTACGACGTGCGGAAG
30

hSpCas9

Y P K L E S E F V Y G D Y K V Y D V R K

1001 1002 1003 1004 1005 1006 1007 1008 1009 1010 1011 1012 1013 1014 1015 1016 1017 1018 1019 1020

5' ATGATCGCCAAGAGCGAGCAGGAAATCGGCAAGGCTACCGCCAAGTACTTCTTCTACAGC
31

hSpCas9

M I A K S E Q E I G K A T A K Y F F Y S

1021 1022 1023 1024 1025 1026 1027 1028 1029 1030 1031 1032 1033 1034 1035 1036 1037 1038 1039 1040

5' AACATCATGAACTTTTTCAAGACCGAGATTACCCTGGCCAACGGCGAGATCCGGAAGCGG
31

hSpCas9

N I M N F F K T E I T L A N G E I R K R

1041 1042 1043 1044 1045 1046 1047 1048 1049 1050 1051 1052 1053 1054 1055 1056 1057 1058 1059 1060

5' CCTCTGATCGAGACAAACGGCGAAACCGGGGAGATCGTGTGGGATAAGGGCCGGGATTTT
32

hSpCas9

P L I E T N G E T G E I V W D K G R D F

1061 1062 1063 1064 1065 1066 1067 1068 1069 1070 1071 1072 1073 1074 1075 1076 1077 1078 1079 1080

5' GCCACCGTGCGGAAAGTGCTGAGCATGCCCCAAGTGAATATCGTGAAAAAGACCGAGGTG
33

hSpCas9

A T V R K V L S M P Q V N I V K K T E V

1081 1082 1083 1084 1085 1086 1087 1088 1089 1090 1091 1092 1093 1094 1095 1096 1097 1098 1099 1100

5' CAGACAGGCGGCTTCAGCAAAGAGTCTATCCTGCCCAAGAGGAACAGCGATAAGCTGATC
33

hSpCas9

Q T G G F S K E S I L P K R N S D K L I

1101 1102 1103 1104 1105 1106 1107 1108 1109 1110 1111 1112 1113 1114 1115 1116 1117 1118 1119 1120

# FIG. 41J

hSpCas9

```
5'   GCCAGAAAGAAGGACTGGGACCCTAAGAAGTACGGCGGCTTCGACAGCCCCACCGTGGCC
0    +++++++++++++++++++++++++++++++++++++++++++++++++++++++++++++   3420
0                                hSpCas9
     A   R   K   K   D   W   D   P   K   K   Y   G   G   F   D   S   P   T   V   A
0    1121 1122 1123 1124 1125 1126 1127 1128 1129 1130 1131 1132 1133 1134 1135 1136 1137 1138 1139 1140
```

```
5'   TATTCTGTGCTGGTGGTGGCCAAAGTGGAAAAGGGCAAGTCCAAGAAACTGAAGAGTGTG
0    +++++++++++++++++++++++++++++++++++++++++++++++++++++++++++++   3480
0                                hSpCas9
     Y   S   V   L   V   V   A   K   V   E   K   G   K   S   K   K   L   K   S   V
0    1141 1142 1143 1144 1145 1146 1147 1148 1149 1150 1151 1152 1153 1154 1155 1156 1157 1158 1159 1160
```

```
5'   AAAGAGCTGCTGGGGATCACCATCATGGAAAGAAGCAGCTTCGAGAAGAATCCCATCGAC
0    +++++++++++++++++++++++++++++++++++++++++++++++++++++++++++++   3540
0                                hSpCas9
     K   E   L   L   G   I   T   I   M   E   R   S   S   F   E   K   N   P   I   D
0    1161 1162 1163 1164 1165 1166 1167 1168 1169 1170 1171 1172 1173 1174 1175 1176 1177 1178 1179 1180
```

```
5'   TTTCTGGAAGCCAAGGGCTACAAAGAAGTGAAAAAGGACCTGATCATCAAGCTGCCTAAG
0    +++++++++++++++++++++++++++++++++++++++++++++++++++++++++++++   3600
0                                hSpCas9
     F   L   E   A   K   G   Y   K   E   V   K   K   D   L   I   I   K   L   P   K
0    1181 1182 1183 1184 1185 1186 1187 1188 1189 1190 1191 1192 1193 1194 1195 1196 1197 1198 1199 1200
```

```
5'   TACTCCCTGTTCGAGCTGGAAAACGGCCGGAAGAGAATGCTGGCCTCTGCCGGCGAACTG
0    +++++++++++++++++++++++++++++++++++++++++++++++++++++++++++++   3660
0                                hSpCas9
     Y   S   L   F   E   L   E   N   G   R   K   R   M   L   A   S   A   G   E   L
0    1201 1202 1203 1204 1205 1206 1207 1208 1209 1210 1211 1212 1213 1214 1215 1216 1217 1218 1219 1220
```

```
5'   CAGAAGGGAAACGAACTGGCCCTGCCCTCCAAATATGTGAACTTCCTGTACCTGGCCAGC
0    +++++++++++++++++++++++++++++++++++++++++++++++++++++++++++++   3720
0                                hSpCas9
     Q   K   G   N   E   L   A   L   P   S   K   Y   V   N   F   L   Y   L   A   S
0    1221 1222 1223 1224 1225 1226 1227 1228 1229 1230 1231 1232 1233 1234 1235 1236 1237 1238 1239 1240
```

FIG. 41K

hSpCas9

5' CACTATGAGAAGCTGAAGGGCTCCCCCGAGGATAATGAGCAGAAACAGCTGTTTGTGGAA
3780

hSpCas9

H  Y  E  K  L  K  G  S  P  E  D  N  E  Q  K  Q  L  F  V  E

1241 1242 1243 1244 1245 1246 1247 1248 1249 1250 1251 1252 1253 1254 1255 1256 1257 1258 1259 1260

5' CAGCACAAGCACTACCTGGACGAGATCATCGAGCAGATCAGCGAGTTCTCCAAGAGAGTG
3840

hSpCas9

Q  H  K  H  Y  L  D  E  I  I  E  Q  I  S  E  F  S  K  R  V

1261 1262 1263 1264 1265 1266 1267 1268 1269 1270 1271 1272 1273 1274 1275 1276 1277 1278 1279 1280

5' ATCCTGGCCGACGCTAATCTGGACAAAGTGCTGTCCGCCTACAACAAGCACCGGGATAAG
3900

hSpCas9

I  L  A  D  A  N  L  D  K  V  L  S  A  Y  N  K  H  R  D  K

1281 1282 1283 1284 1285 1286 1287 1288 1289 1290 1291 1292 1293 1294 1295 1296 1297 1298 1299 1300

5' CCCATCAGAGAGCAGGCCGAGAATATCATCCACCTGTTTACCCTGACCAATCTGGGAGCC
3960

hSpCas9

P  I  R  E  Q  A  E  N  I  I  H  L  F  T  L  T  N  L  G  A

1301 1302 1303 1304 1305 1306 1307 1308 1309 1310 1311 1312 1313 1314 1315 1316 1317 1318 1319 1320

5' CCTGCCGCCTTCAAGTACTTTGACACCACCATCGACCGGAAGAGGTACACCAGCACCAAA
4020

hSpCas9

P  A  A  F  K  Y  F  D  T  T  I  D  R  K  R  Y  T  S  T  K

1321 1322 1323 1324 1325 1326 1327 1328 1329 1330 1331 1332 1333 1334 1335 1336 1337 1338 1339 1340

5' GAGGTGCTGGACGCCACCCTGATCCACCAGAGCATCACCGGCCTGTACGAGACACGGATC
4080

hSpCas9

E  V  L  D  A  T  L  I  H  Q  S  I  T  G  L  Y  E  T  R  I

1341 1342 1343 1344 1345 1346 1347 1348 1349 1350 1351 1352 1353 1354 1355 1356 1357 1358 1359 1360

FIG. 41L

hSpCas9

5'    GACCTGTCTCAGCTGGGAGGCGAC

+++++++++++++++++++++++++    4104

```
[===================================]
          hSpCas9
```

D   L   S   Q   L   G   G   D

1361 1362 1363 1364 1365 1366 1367 1368

FIG. 41M

FIG. 42

FIG. 43A

FIG. 43B

FIG. 43C

FIG. 43D

FIG. 44A

b

dir.
guide 1   repeat guide 2

↓ insert

direct
repeat          *Bbs*I      *Bbs*I              direct repeat

guide oligo
insertion site

5'-...CAGAACGGGTCTTCGAGAAGACG|TTTAGAGCTATGCTGTTTTGAATGGTCCCA...-3'

3'-...GTTTTGCCCAGAAGCTCTTCTGCAAAAT|CTCGATACGACAAAACTTACCAGGGT...-5'

PX260

U6                    3x
                      Cbh FLAG *NLS* SpCas9 *NLS* bGH pA H1  tracrRNA

*Bbs*I*Bbs*I

*PciI,*        *XbaI,*    *AgeI*              *EcoRI*  *NotI*        *BsrGI*
*AflIII*       *KpnI*

FIG. 44B

EP 2 848 690 B1

EMX1.3-NLSCsn1-UnPurified

FIG. 45

FIG. 46

FIG. 47

A

B

| SpCas9 | + | – | + | + | – | – | – |
| SpCas9n | – | + | – | – | + | + | – |
| Antisense-Oligo | – | – | + | – | + | – | – |
| Sense-Oligo | – | – | – | + | – | + | – |
| HR template | + | + | – | – | – | – | + |

2281bp ►

1182bp ►
1099bp ►

HR (%): 0.41  0.27  1.1  0.43  0.073

FIG. 48

Conditional Cas9, Rosa26 targeting vector map

FIG. 49A

Constitutive Cas9, Rosa26 targeting vector map

FIG. 49B

pCAG
ccgtttaaacaattctgcaggaatctagttattaatagtaatcaattacggggtcattagttcatagcccatatatggagttccgcgttacataactt
acggtaaatggcccgcctggctgaccgcccaacgacccccgcccattgacgtcaataatgacgtatgttcccatagtaacgccaatagggga
ctttccattgacgtcaatgggtggagtatttacggtaaactgcccacttggcagtacatcaagtgtatcatatgccaagtacgccccctattgac
gtcaatgacggtaaatggcccgcctggcattatgcccagtacatgaccttatgggactttcctacttggcagtacatctacgtattagtcatcgc
tattaccatggtcgaggtgagccccacgttctgcttcactctccccatctcccccccctccccacccccaattttgtatttatttattttttaattatttt
gtgcagcgatgggggcggggggggggggggggcgcgcgccaggcggggcggggcggggcgaggggcggggcggggcgaggc
ggagaggtgcggcggcagccaatcagagcggcgcgctccgaaagtttccttttatggcgaggcggcggcggcggcggccctataaaaa
gcgaagcgcgcggcgggcggaAGTCGCTGCGcgctgccttcgccccgtgccccgctccgccgccgcctcgcgccgcccgcc
ccggctctgactgaccgcgttactcccacaggtgagcgggcgggacggcccttctcctccgggctgtaattagcgcttggtttaatgacggc
ttgtttctttctgtggctgcgtgaaagccttgaggggctccgggagggccctttgtgcggggggggagcggctcggggggtgcgtgcgtgtgt
gtgtgcgtggggagcgccgcgtgcggctccgcgctgcccggcggctgtgagcgctgcgggcgcggcgcggggctttgtgcgctccgc
agtgtgcgcgaggggagcgcggccgggggcggtgccccgcggtgcggggggggggctgcgaggggaacaaaggctgcgtgcggggt
gtgtgcgtgggggggtgagcagggggtgtgggcgcgtcggtcgggctgcaacccccctgcacccccctccccgagttgctgagcacg
gcccggcttcgggtgcggggctccgtacggggcgtggcgcggggctcgccgtgccgggcggggggtggcggcaggtggggggtgcc
gggcggggcggggccgcctcgggccggggagggctcgggggaggggcgcggcggcccccggagcgccggcggctgtcgaggcg
cggcgagccgcagccattgccttttatggtaatcgtgcgagagggcgcagggacttcctttgtcccaaatctgtgcggagccgaaatctgg
gaggcgccgccgcacccccctctagcgggcgcgggggcgaagcggtgcggcgccggcaggaaggaaatgggcggggagggccttcgt
gcgtcgccgcgccgccgtcccccttctccctctccagcctcggggctgtccgcggggggggacggctgccttcggggggggacggggcaggg
cggggttcggcttctggcgtgtgaccggcggctctagagcctctgctaaccatgttcatgccttcttcttttcctacagctcctgggcaacgtg
ctggttattgtgctgtctcatcattttggcaaa

NLS-Cas9-NLS
ATGGACTATAAGGACCACGACGGAGACTACAAGGATCATGATATTGATTACAAAGA
CGATGACGATAAGATGGCCCCAAAGAAGAAGCGGAAGGTCGGTATCCACGGAGTCC
CAGCAGCCGACAAGAAGTACAGCATCGGCCTGGACATCGGCACCAACTCTGTGGGC
TGGGCCGTGATCACCGACGAGTACAAGGTGCCCAGCAAGAAATTCAAGGTGCTGGG
CAACACCGACCGGCACAGCATCAAGAAGAACCTGATCGGAGCCCTGCTGTTCGACA
GCGGCGAAACAGCCGAGGCCACCCGGCTGAAGAGAACCGCCAGAAGAAGATACAC
CAGACGGAAGAACCGGATCTGCTATCTGCAAGAGATCTTCAGCAACGAGATGGCCA
AGGTGGACGACAGCTTCTTCCACAGACTGGAAGAGTCCTTCCTGGTGGAAGAGGAT
AAGAAGCACGAGCGGCACCCCATCTTCGGCAACATCGTGGACGAGGTGGCCTACCA
CGAGAAGTACCCCACCATCTACCACCTGAGAAAGAAACTGGTGGACAGCACCGACA
AGGCCGACCTGCGGCTGATCTATCTGGCCCTGGCCCACATGATCAAGTTCCGGGGCC
ACTTCCTGATCGAGGGCGACCTGAACCCCGACAACAGCGACGTGGACAAGCTGTTC

FIG. 50A

ATCCAGCTGGTGCAGACCTACAACCAGCTGTTCGAGGAAAACCCCATCAACGCCAG
CGGCGTGGACGCCAAGGCCATCCTGTCTGCCAGACTGAGCAAGAGCAGACGGCTGG
AAAATCTGATCGCCCAGCTGCCCGGCGAGAAGAAGAATGGCCTGTTCGGaAACCTG
ATTGCCCTGAGCCTGGGCCTGACCCCCAACTTCAAGAGCAACTTCGACCTGGCCGAG
GATGCCAAACTGCAGCTGAGCAAGGACACCTACGACGACGACCTGGACAACCTGCT
GGCCCAGATCGGCGACCAGTACGCCGACCTGTTTCTGGCCGCCAAGAACCTGTCCG
ACGCCATCCTGCTGAGCGACATCCTGAGAGTGAACACCGAGATCACCAAGGCCCCC
CTGAGCGCCTCTATGATCAAGAGATACGACGAGCACCACCAGGACCTGACCCTGCT
GAAAGCTCTCGTGCGGCAGCAGCTGCCTGAGAAGTACAAAGAGATTTTCTTCGACC
AGAGCAAGAACGGCTACGCCGGCTACATTGACGGCGGAGCCAGCCAGGAAGAGTTC
TACAAGTTCATCAAGCCCATCCTGGAAAAGATGGACGGCACCGAGGAACTGCTCGT
GAAGCTGAACAGAGAGGACCTGCTGCGGAAGCAGCGGACCTTCGACAACGGCAGC
ATCCCCCACCAGATCCACCTGGGAGAGCTGCACGCCATTCTGCGGCGGCAGGAAGA
TTTTTACCCATTCCTGAAGGACAACCGGGAAAAGATCGAGAAGATCCTGACCTTCCG
CATCCCCTACTACGTGGGCCCTCTGGCCAGGGGAAACAGCAGATTCGCCTGGATGA
CCAGAAAGAGCGAGGAAACCATCACCCCCTGGAACTTCGAGGAAGTGGTGGACAA
GGGCGCTTCCGCCCAGAGCTTCATCGAGCGGATGACCAACTTCGATAAGAACCTGC
CCAACGAGAAGGTGCTGCCCAAGCACAGCCTGCTGTACGAGTACTTCACCGTGTAT
AACGAGCTGACCAAAGTGAAATACGTGACCGAGGGAATGAGAAAGCCCGCCTTCCT
GAGCGGCGAGCAGAAAAAGGCCATCGTGGACCTGCTGTTCAAGACCAACCGGAAA
GTGACCGTGAAGCAGCTGAAAGAGGACTACTTCAAGAAAATCGAGTGCTTCGACTC
CGTGGAAATCTCCGGCGTGGAAGATCGGTTCAACGCCTCCCTGGGCACATACCACG
ATCTGCTGAAAATTATCAAGGACAAGGACTTCCTGGACAATGAGGAAAACGAGGAC
ATTCTGGAAGATATCGTGCTGACCCTGACACTGTTTGAGGACAGAGAGATGATCGA
GGAACGGCTGAAAACCTATGCCCACCTGTTCGACGACAAAGTGATGAAGCAGCTGA
AGCGGCGGAGATACACCGGCTGGGGCAGGCTGAGCCGGAAGCTGATCAACGGCATC
CGGGACAAGCAGTCCGGCAAGACAATCCTGGATTTCCTGAAGTCCGACGGCTTCGC
CAACAGAAACTTCATGCAGCTGATCCACGACGACAGCCTGACCTTTAAAGAGGACA
TCCAGAAAGCCCAGGTGTCCGGCCAGGGCGATAGCCTGCACGAGCACATTGCCAAT
CTGGCCGGCAGCCCCGCCATTAAGAAGGGCATCCTGCAGACAGTGAAGGTGGTGGA
CGAGCTCGTGAAAGTGATGGGCCGGCACAAGCCCGAGAACATCGTGATCGAAATGG
CCAGAGAGAACCAGACCACCCAGAAGGGACAGAAGAACAGCCGCGAGAGAATGAA
GCGGATCGAAGAGGGCATCAAAGAGCTGGGCAGCCAGATCCTGAAAGAACACCCC
GTGGAAAACACCCAGCTGCAGAACGAGAAGCTGTACCTGTACTACCTGCAGAATGG
GCGGGATATGTACGTGGACCAGGAACTGGACATCAACCGGCTGTCCGACTACGAT

FIG. 50B

GTGGACCATATCGTGCCTCAGAGCTTTCTGAAGGACGACTCCATCGACAACAAGGTG
CTGACCAGAAGCGACAAGAACCGGGGCAAGAGCGACAACGTGCCCTCCGAAGAGG
TCGTGAAGAAGATGAAGAACTACTGGCGGCAGCTGCTGAACGCCAAGCTGATTACC
CAGAGAAAGTTCGACAATCTGACCAAGGCCGAGAGAGGCGGCCTGAGCGAACTGG
ATAAGGCCGGCTTCATCAAGAGACAGCTGGTGGAAACCCGGCAGATCACAAAGCAC
GTGGCACAGATCCTGGACTCCCGGATGAACACTAAGTACGACGAGAATGACAAGCT
GATCCGGGAAGTGAAAGTGATCACCCTGAAGTCCAAGCTGGTGTCCGATTTCCGGA
AGGATTTCCAGTTTTACAAAGTGCGCGAGATCAACAACTACCACCACGCCCACGAC
GCCTACCTGAACGCCGTCGTGGGAACCGCCCTGATCAAAAAGTACCCTAAGCTGGA
AAGCGAGTTCGTGTACGGCGACTACAAGGTGTACGACGTGCGGAAGATGATCGCCA
AGAGCGAGCAGGAAATCGGCAAGGCTACCGCCAAGTACTTCTTCTACAGCAACATC
ATGAACTTTTTCAAGACCGAGATTACCCTGGCCAACGGCGAGATCCGGAAGCGGCC
TCTGATCGAGACAAACGGCGAAACCGGGGAGATCGTGTGGGATAAGGGCCGGGATT
TTGCCACCGTGCGGAAAGTGCTGAGCATGCCCCAAGTGAATATCGTGAAAAAGACC
GAGGTGCAGACAGGCGGCTTCAGCAAAGAGTCTATCCTGCCCAAGAGGAACAGCGA
TAAGCTGATCGCCAGAAAGAAGGACTGGGACCCTAAGAAGTACGGCGGCTTCGACA
GCCCCACCGTGGCCTATTCTGTGCTGGTGGTGGCCAAAGTGGAAAAGGGCAAGTCC
AAGAAACTGAAGAGTGTGAAAGAGCTGCTGGGGATCACCATCATGGAAAGAAGCA
GCTTCGAGAAGAATCCCATCGACTTTCTGGAAGCCAAGGGCTACAAAGAAGTGAAA
AAGGACCTGATCATCAAGCTGCCTAAGTACTCCCTGTTCGAGCTGGAAAACGGCCG
GAAGAGAATGCTGGCCTCTGCCGGCGAACTGCAGAAGGGAAACGAACTGGCCCTGC
CCTCCAAATATGTGAACTTCCTGTACCTGGCCAGCCACTATGAGAAGCTGAAGGGCT
CCCCCGAGGATAATGAGCAGAAACAGCTGTTTGTGGAACAGCACAAGCACTACCTG
GACGAGATCATCGAGCAGATCAGCGAGTTCTCCAAGAGAGTGATCCTGGCCGACGC
TAATCTGGACAAAGTGCTGTCCGCCTACAACAAGCACCGGGATAAGCCCATCAGAG
AGCAGGCCGAGAATATCATCCACCTGTTTACCCTGACCAATCTGGGAGCCCCTGCCG
CCTTCAAGTACTTTGACACCACCATCGACCGGAAGAGGTACACCAGCACCAAAGAG
GTGCTGGACGCCACCCTGATCCACCAGAGCATCACCGGCCTGTACGAGACACGGAT
CGACCTGTCTCAGCTGGGAGGCGACAAAAGGCCGGCGGCCACGAAAAAGGCCGGC
CAGGCAAAAAGAAAAAG

P2A-EGFP
ggaagcggagccactaacttctccctgttgaaacaagcaggggatgtcgaagagaatcccgggccaGTGAGCAAGGGCGA
GGAGCTGTTCACCGGGGTGGTGCCCATCCTGGTCGAGCTGGACGGCGACGTAAACG
GCCACAAGTTCAGCGTGTCCGGCGAGGGCGAGGGCGATGCCACCTACGGCAAGCTG
ACCCTGAAGTTCATCT

FIG. 50C

GCACCACCGGCAAGCTGCCCGTGCCCTGGCCCACCCTCGTGACCACCCTGACCTACG
GCGTGCAGTGCTTCAGCCGCTACCCCGACCACATGAAGCAGCACGACTTCTTCAAGT
CCGCCATGCCCGAAGGCTACGTCCAGGAGCGCACCATCTTCTTCAAGGACGACGGC
AACTACAAGACCCGCGCCGAGGTGAAGTTCGAGGGCGACACCCTGGTGAACCGCAT
CGAGCTGAAGGGCATCGACTTCAAGGAGGACGGCAACATCCTGGGGCACAAGCTGG
AGTACAACTACAACAGCCACAACGTCTATATCATGGCCGACAAGCAGAAGAACGGC
ATCAAGGTGAACTTCAAGATCCGCCACAACATCGAGGACGGCAGCGTGCAGCTCGC
CGACCACTACCAGCAGAACACCCCCATCGGCGACGGCCCCGTGCTGCTGCCCGACA
ACCACTACCTGAGCACCCAGTCCGCCCTGAGCAAAGACCCCAACGAGAAGCGCGAT
CACATGGTCCTGCTGGAGTTCGTGACCGCCGCCGGGATCACTCTCGGCATGGACGAG
CTGTACAAG

WPRE
Cgataatcaacctctggattacaaaatttgtgaaagattgactggtattcttaactatgttgctcctttacgctatgtggatacgctgctttaatgc
ctttgtatcatgctattgcttcccgtatggctttcattttctcctccttgtataaatcctggttgctgtctctttatgaggagttgtggcccgttgtcagg
caacgtggcgtggtgtgcactgtgtttgctgacgcaacccccactggttggggcattgccaccacctgtcagctcctttccgggactttcgctt
tccccctccctattgccacggcggaactcatcgccgcctgccttgcccgctgctggacaggggctcggctgttgggcactgacaattccgtg
gtgttgtcggggaaatcatcgtcctttccttggctgctcgcctgtgttgccacctggattctgcgcgggacgtccttctgctacgtcccttcggc
cctcaatccagcggaccttccttcccgcggcctgctgccggctctgcggcctcttccgcgtcttcgccttcgccctcagacgagtcggatctc
cctttgggccgcctccccgcatcg

bGHpolyA
cgacCTCGACtgtgccttctagttgccagccatctgttgtttgcccctcccccgtgccttccttgaccctggaaggtgccactcccactgt
cctttcctaataaaatgaggaaattgcatcgcattgtctgagtaggtgtcattctattctggggggtggggtggggcaggacagcaaggggg
aggattgggaagacaatGgcaggcatg

loxP-SV40polyAx3-loxP
ataacttcgtataatgtatgctatacgaagttattcgcgatgaataaatgaaagcttgcagatctgcgactctagaggatctgcgactctagagg
atcataatcagccntaccacattttgtagaggttttactngctttaaaaaaacctcccacacctcccctgaacctgaaacataaaatgaatgcaa
ttgttgttgttaacttgttgttattgcagcttataatggttacaaataaagcaatagcatcacaaatttcacaaataaagcattttttcactgcattctagt
tgtggtttgtccaaactcatcaatgtatcttatcatgtctggatctgcgactctagaggatcataatcagccataccacattgtagaggttttactt
gctttaaaaaacctcccacacctcccctgaacctgaaacataaaatgaatgcaattgttgttgttaacttgtttattgcagcttataatggttaca
aataaagcaatagcatcacaaatttcacaaataaagcattttttcactgcattctagttgtggtttgtccaaactcatcaatgtatcttatcatgtct
ggatctgcgactctagaggatcataatcagccataccacattgtagaggttttacttgctttaaaaaacctcccacacctcccctgaacctga
aacataaaatgaatgcaattgttgttgttaacttgtttattgcagcttataatggttacaaata

FIG. 50D

aagcaatagcatcacaaatttcacaaataaagcatttttttcactgcattctagttgtggtttgtccaaactcatcaatgtatcttatcatgtctggat
ccccatcaagctgatccggaacccttaatataacttcgtataatgtatgctatacgaagttat

Rosa26 short homology arm
caggccctccgagcgtggtggagccgttctgtgagacagccgggtacgagtcgtgacgctggaagggggcaagcgggtggtgggcagg
aatgcggtccgccctgcagcaaccggaggggggagggagaaggggagcggaaaagtctccaccggacgcggccatggctcggggggg
gggggcagcggaggaGcgcttccggccgacgtctcgtcgctgattggcttCttttcctccccgccgtgtgtgaaaacacaaatggcgtgtt
ttggttggcgtaaggcgcctgtcagttaacggcagccggagtgcgcagccgccggcagcctcgctctgcccactgggtggggcgggag
gtaggtggggtgaggcgagctgGacgtgcgggcgcggtcggcctctggcggggcgggggagggggagggagggtcagcgaaagta
gctcgcgcgcgagcggccgcccaccctcccccttcctctggggggagtcgttttacccgccgccggccgggcctcgtcgtctgattggctctc
gggggcccagaaaaactggcccttgccattggctcgtgttcgtgcaagttgagtccatccgccggccagcggggggcggcgaggaggcgctc
ccaggttccggccctcccctcggccccgcgccgcagagtctggccgcgcgccctgcgcaacgtggcaggaagcgcgcgctggggggc
ggggacgggcagtagggctgagcggctgcggggcgggtgcaagcacgtttccgacttgagttgcctcaagaggggcgtgctgagccag
acctccatcgcgcactccggggagtggagggaaggagcgagggctcagttgggctgttttggaggcaggaagcacttgctctcccaaagt
cgctctgagttgttatcagtaagggagctgcagtggagtaggcggggagaaggccgcaccttctccggagggggggagggggagtgttgc
aataccttctgggagttctctgctgcctcctggcttctgaggaccgccctgggcctgggagaatcccttcccctcttccctcgtgatctgcaa
ctccagtctttctag

Rosa26 long homology arm
agatgggcgggagtcttctgggcaggcttaaaggctaacctggtgtgtgggcgttgtcctgcaggggaattgaacaggtgtaaaattggag
ggacaagacttcccacagattttcggttttgtcgggaagttttttaatagggggcaaataaggaaaatgggaggataggtagtcatctgggggtttt
atgcagcaaaactacaggttattattgcttgtgatccgcctcggagtattttccatcgaggtagattaaagacatgctcacccgagttttatactct
cctgcttGAGATCCTTACTACAGTATGAAATTACAGTGTCGCGAGTTAGACTATGTAAGC
AGAATTTTAATCATttttaaagagcccagtacttcatatccatttctcccgctccttctgcagccttatcaaaaggtaTtttagaaca
ctcattttagccccattttcatttattatactggcttatccaacccctagacagagcattggcattttcccttcctgatcttagaagtctgatgactca
tgaaaccagacagattagttacatacaccacaaatcgaggctgtagctgggggcctcaacactgcagttctttataactccttagtacacttttttg
ttgatcctttgccttgatccttaattttcagtgtctatcacctctcccgtcaggtggtgttccacatttgggcctattctcagtccaggggagttttaca
acaatagatgtattgagaatccaacctaaagcttaactttccactcccatgaatgcctctctccttttttctccattTATAAACTGAGCT
ATTAACCATTAATGGTTTCCAGGTGGATGTCTCCTCCCCCAATATTACCTGATGTATC
TTACATATTGCCAGGCTGATATTTTAAGACATTAAAAGGTATATTTCATTATTGAGCC
ACATGGTATTGATTACTGCTtactaaaattttgtcattgtacacatctgtaaaaggtggttccttttggaatgcaaagttcaggt
gtttgttgtctttcctgacctaaggtcttgtgagcttgtattttttctatttaagcagtgctttctcttggactggcttgactcatggcattctacacgtta
ttgctggtctaaatgtgattttgccaagcttcttcaggacctataattttgcttgacttgtagccaaacacaagtaaaatgattaagcaacaaatgt
atttgtgaagcttggtttttaggttgttgtgttgtgtgtgcttgtgctctataataatactatccaggggctggagaggtggctcggagttcaagag
cacagactgctcttccagaagtc

## FIG. 50E

ctgagttcaattcccagcaaccacatggtggctcacaaccatctgtaatgggatctgatgccctcttctggtgtgtctgaagaccacaagtgta
ttcacattaaataaataaaTCCTCCTTCTTCTTCTTTTTTTTTTTTtAAAGAGAATACTGTCTCCAG
TAGAAtTTACTGAAGTAATGAAATACTTTGTGTTTGTTCCAATATGGTAGCCAATAAT
CAAATtACTCTTTaAGCACTGGAAATGTtACCAAGGAACTAaTTTTtATTTgAAGTGTaA
CTGTGGACAGAGGAGCCATAACTGCAGACTTGTGGGATACAGAAGACCAATGCAGA
CtTTAATGTCTTTTCTCTTACACTAAGCAATAAAGAAATAAAAATTGAACTTCTAGTA
TCCTATTTGTTtAAACTGCTAGCTTTACtTAACTTTTGTGCTTCATCTATACAAAGCTG
AAAGCTAAGTCTGCAGCCATTACTAAACATGAAAGCAAGTAATGATAATTTTGGATT
TCAAAAATGTAGGGCCAGAGTTTAGCCAGCCAGTGGTGGTGCTTGCCTTTATGCCtTT
AATCCCAGCACTCTGGAGGCAGAGACAGGCAGATCTCTGAGTTTGAGCCCAGCCTG
GtCTACACATCAAGTTCTATCTAGGATAGCCAGGAATACACACAGAAACCCTGTTGG
GGAGGGGGGCTCTGAGATTTCATAAAATTATAATTGAAGCATTCCCTAATGAGCCAC
TATGGATGTGGCTAAATCCGTCTACCTTTCTGATGAGATTTGGGTATTATTTTTTCTG
TCTCTGCTGTTGGTTGGGTCTTTTGACACTGTGGGCTTTCTTtAAAGCCTCCTTCCTGC
CATGTGGTCTCTTGTTTGCTACTAACTTCCCATGGCTTAAATGGCATGGCTTTTTGCC
TTCTAAGGGCAGCTGCTGAGATTTGCAGCCTGATTTCCAGGGTGGGGTTGGGAAATC
TTTCAAACACTAAAATTGTCCTTTAAtTTTTTTTTAAAAAATGGGTTATATAATAAA
CCTCATAAAATAGTTATGAGGAGTGAGGTGGACTAATATTAAaTGAGTCCCTCCCCT
ATAAAAGAGCTATTAAGGCTTTTTGTCTTATACTtAACTTTTTTTTAAATGTGGTATC
TTTAGAACCAAGGGTCTTAGAGTTTTAGTATACAGAAACTGTTGCATCGCTTAATCA
GATTTTCTAGTTTCAAATCCAGAGAATCCAAATTCTTCACAGCCAAAGTCAAATTAA
GAATTTCTGACTTTtAATGTTAaTTTGCtTACTGTGAATATaAAAATGATAGCTTTTCCT
GAGGCAGGGTCTCACTATGTATCTCTGCCTGATCTGCAACAAGATATGTAGACTAAA
GTTCTGCCTGCTTTTGTCTCCTGAATACTAAGGTTAAAATGTAGTAATACTTTTGGAA
CTTGCAGGTCAGATTCTTTTATAGGGGACACACTAAGGGAGCTTGGGTGATAGTTGG
TAAAtgtgtttaagtgatgaaaacttgaattattatcaccgcaacctacttttaaaaaaaaaaagccaggcctgttagagcatgctTaaggg
atccctaggacttgctgagcacacaAGAGTAGtTACTTGGCAGGCTCCTGGTGAGAGCATATTTCAA
AAAACAAGGCAGACAACCAAGAAACTACAGTtAAGGTTACCTGTCTTTaAACCATCT
GCATATACACAGGGATATTAAAATATTCCAAATAATATTTCATTCAAGTTTTCCCCC
ATCAAATTGGGACATGGATTTCTCCGGTGAATAGGCAGAGTTGGAAACTAAACAAA
TGTTGGTTTTGTGATTTGTGAAATTGTTTTCAAGTGATAGTTAAAGCCCATGAGATAC
AGAACAAAGCTGCTATTTCGAGGTCTCTTGGTtATACTCAGAAGCACTTCTTTGGGT
TTCCCTGCACTATCCTGATCATGTGCTAGGCCTACCTTAGGCTGATTGTTGTTCAAAT
aAACTTAAGTTTCCTGTCAGGTGATGTCATATGATTTCATATATCAAGGCAAAACATG
TTATATATGTTAAACATTTGTACTTAATGTGAAAGTTAGGTCTTTGTGGGTT

FIG. 50F

TGATTTTtAAtTTTCAAAACCTGAGCTAAATAAGTCATTTTtACATGTCTTACATTTGGT
GgAATTGTATaATTGTGGTTTGCAGGCAAGACTCTCTGACCTAGTAACCCTaCCTATA
GAGCACTTTGCTGGGTCACAAGTCTAGGAGTCAAGCATTTCACCTTGAAGTTGAGAC
GTTTTGTTAGTGTATACTAGTTtATATGTTGGAGGACATGTTTATCCAGAAGATATTC
AGGACTATTTTTGACTGGGCTAAGGAATTGATTCTGATTAGCACTGTTAGTGAGCAT
TGAGTGGCCTTTAGGCTTGAATTggagtcacttgtatatctcaaataatgctggcctttttaaaagcccttgttctttatca
ccctgttttctacataattttgttcaaagaaatacttgtttggaTCTCCTTTTGACAACAATAGCATGTTTTCAAG
CCATATTTTTTTTCCTTTTTTTTTTTTTTTTGGTTTTTCGAGACAGGGTTTCTCTGTAT
AGCCCTGGCTGTCCTGGAACTCACTTTGTAGACCAGGCTGGCCTCGAACTCAGAAAT
CCGCCTGCCTCTGCCTCCTGAGTGCCGGGATTAAAGGCGTGCACCACCACGCCTGGC
TAAGTTGGATATTTTGTtATATAACTATAACCAATACTAACTCCACTGGGTGGATTTT
TAATTCAGTCAGTAGTCTTAAGTGGTCTTTATTGGCCCTTcATTAAAATCTACTGTTC
ACTCTAACAGAGGCTGTTGGtACTAGTGGCACtAAGCAACTTCCTACGGATATACTA
GCAGAtTAAGGGTCAGGGATAGAAACTAGTCTAGCGTTTTGTATACCTACCAGCTTtA
TACTACCTTGTTCTGATAGAAATATTTcAGGACATCTAGCTT

pPGK-Neo-pPGK-polyA

aattctaccgggtagggggaggcgcttttcccaaggcagtctggagcatgcgctttagcagccccgctgggcacttggcgctacacaagtgg
cctctggcctcgcacacattccacatccaccggtaggcgccaaccggctccgttcttggtggccccttcgcgccaccttctactcctcccct
agtcaggaagttccccccccgccccgcagctcgcgtcgtgcaggacgtgacaaatggaagtagcacgtctcactagtctcgtgcagatgga
cagcaccgctgagcaatggaagcgggtaggcctttggggcagcggccaatagcagctttgctccttcgctttctgggctcagaggctggg
aaggggtggtccggggcgggctcaggggcgggctcaggggcggggcgggcgcccgaaggtcctccggaggcccggcattctgc
acgcttcaaaagcgcacgtctgccgcgctgttctcctcttcctcatctccgggcctttcgacctgcaatcgccgctagcgaagttcctattctct
agaaagtataggaacttcgccaccatgggatcggccattgaacaagatggattgcacgcaggttctccggccgcttgggtggagaggctat
tcggctatgactgggcacaacagacaatcggctgctctgatgccgccgtgttccggctgtcagcgcaggggcgcccggttcttttgtcaag
accgacctgtccggtgccctgaatgaactgcaggacgaggcagcgcggctatcgtggctggccacgacgggcgttccttgcgcagctgt
gctcgacgttgtcactgaagcgggaagggactggctgctattgggcgaagtgccgggggcaggatctcctgtcatctcaccttgctcctgcc
gagaaagtatccatcatggctgatgcaatgcggcggctgcatacgcttgatccggctacctgcccattcgaccaccaagcgaaacatcgca
tcgagcgagcacgtactcggatggaagccggtcttgtcgatcaggatgatctggacgaagagcatcagggggctcgcgccagccgaactg
ttcgccaggctcaaggcgcgcatgcccgacggcgatgatctcgtcgtgacccatggcgatgcctgcttgccgaatatcatggtggaaaatg
gccgcttttctggattcatcgactgtggccggctggtgtggcggaccgctatcaggacatagcgttggctacccgtgatattgctgaagag
cttggcggcgaatgggctgaccgcttcctcgtgctttacggtatcgccgctcccgattcgcagcgcatcgccttctatcgccttcttgacgagt
tcttctgaggggatccgctgtaagtctgcagaaattgatgatctattaaacaataaagatgtccactaaaatggaagttttcctgtcatactttgtt
aagaagggtgagaacagagtacctacattttgaatggaaggattggagctacggggggtgggggtggggtgggattagataaatgcctgct
ctttactgaaggctctttactattgctttatgataatgtttcatagttg

FIG. 50G

gatatcataatttaaacaagcaaaaccaaattaagggccagctcattcctcccactcatgatctatagatctatagatctctcgtgggatcattgt
ttttctcttgattcccactttgtggttctaagtactgtggtttccaaatgtgtcagtttcatagcctgaagaacgagatcagcagcctctgttccaca
tacacttcattctcagtattgttttgccaagttctaattccatcagaaagc

pPGK-DTA
TACCGGGTAGGGGAGGCGCTTTTCCcAAGGCAGTCTGgAGCATGCGCtTTAGCAGCCC
CGCTgGGCACTTGGCGCTACACAAGTGGCCTCTGGCCTCGCACACATTCCACATCCA
CCGGTAGGCGCCAACCGGCTCCGTTCTTTGGTGGCCCCTTCGCGCCACCTTCTACTCC
TCCCCTAGTCAGGAAGTTCCCCCCCGCCCCGCAGCTCGCGTCGTGcAGGACGTGACA
AATGGAAGTAGCACGTCTCACTAGTCTCGTgCAGATGGACAGCACCGCTGAGCAATG
GAAGCGGGTAGGCCTTTGGGGCAGCGGCCAATAGCAGCTTTGCTCCTTCGCTTTCTG
GGCTCAGAGGCTGGGAAGGGGTGGGTCCGGGGGCGGGCTCAGGGGCGGGCTCAGG
GGCGGGGCGGGCGCCCGAAGGTCCTCCGGAGGCCCGGCATTCTGCACGCTTCAAAA
GCGCACGTCTGCCGCGCTGTTCTCCTCTTCCTCATCTCCGGGCCTTTCGACCTGCAGG
TCCTCGCCATggatcctgatgatgttgttGattcttctaaAtcttttgtGatggaaaacttttcttcgtaccacgggactaaacctggtt
atgtagattccattcaaaaaggtatacaaaagccaaaatctggtacacaaggaaattatgacgatgattggaaagggttttatagtaccgacaa
taaatacgacgctgcgggatactctgtagataatgaaaacccgctctctggaaaagctggaggcgtggtcaaagtgacgtatccaggactg
acgaaggttctcgcactaaaagtggataatgccgaaactattaagaaagagttaggtttaagtctcactgaaccgttgatggagcaagtcgga
acggaagagtttatcaaaaggttcggtgatggtgcttcgcgtgtagtgctcagccttcccttcgctgaggggagttctagcgttgaatatattaa
taactgggaacaggcgaaagcgttaagcgtagaacttgagattaattttgaaacccgtggaaaacgtggccaagatgcgatgtatgagtata
tggctcaagcctgtgcaggaaatcgtgtcaggcgatctctttgtgaaggaaccttacttctgtggtgtgacataattggacaaactacctacag
agatttaaagctctaaggtaaatataaaaatttttaagtgtataatgtgttaaactactgattctaattgtttgtgtattttagattccaacctatggaact
gatgaatgggagcagtggtggaatgcagatcctagagctcgctgatcagcctcgactgtgccttctagttgccagccatctgttgtttgcccct
cccccgtgccttccttgaccctggaaggtgccactcccactgtcctttcctaataaaatgaggaaattgcatcgcattgtctgagtaggtgtcat
tctattctggggggtggggtggggcaggacagcaagggggaggattgggaagacaatagcaggcatg

FIG. 50H

FIG. 51

FIG. 52

Validation of Cas9 nuclease activity by Surveyor

FIG. 53

|  |  | Average | StDev |
|---|---|---|---|
| pCAG-loxp(pA)loxp-NLS-hSpCas9-NLS-2A-GFP | Clone 1 | 32.1 | 7.1 |
|  | Clone 2 | 27.3 | 3.5 |
|  | Clone 3 | 35.9 | 1.4 |
|  | Clone 4 | 39.0 | 4.7 |
| pCAG-NLS-hSpCas9-NLS-2A-GFP | Clone 1 | 26.9 | 1.3 |
|  | Clone 2 | 33.1 | 2.7 |

FIG. 54

| SA (1 kb) | Construct | pPGK-Neo | LA (4.3 kb) | pPGK-DTA |

Rosa26 Locus

FIG. 55

FIG. 56

FIG. 57

left guide RNA target (L)          spacer (0 - n BP)

5'-NNNNNNNNNNNNNNNNNNNNNNNNNGG....CCNNNNNNNNNNNNNNNNNNNNNNNNN-3'
  |||||||||||||||||||||||||||     |||||||||||||||||||||||||||
3'-NNNNNNNNNNNNNNNNNNNNNNNNNCC....GGNNNNNNNNNNNNNNNNNNNNNNNNN-5'
                                 right guide RNA target (R)

processing by nickase
(to improve NHEJ efficiency, can also co-express with TREX1)

5'-NNNNNNNNNNNNNNNNNNNN        NNNNGG....CCNNNNNNNNNNNNNNNNNNNNNNNNN-3'
  ||||||||||||||||||||                       ||||||||||||||||||||||||
3'-NNNNNNNNNNNNNNNNNNNNNNNNNCC....GGNNN        NNNNNNNNNNNNNNNNNNNNNNNN-5'

FIG. 58

FIG. 59

FIG. 60

FIG. 61

FIG. 62

FIG. 63A-C

**FIG. 64**

FIG. 65

gRNA sequences for Chd8 targeting:

Chd8.1 -- agctgttttactggtcggct
Chd8.2 -- aatggatacacctggtcgaa
Chd8.3 -- caatggatacacctggtcga

FIG. 66

FIG. NLS1

FIG. 67

FIG. 68

FIG. 69

NLS architecture optimization for SpCas9

indel (%)  4.7    3.6    4.6    7.6

FIG. 70

QQ plot for the NGGNN sequences

# FIG. 71

# FIG. 72

FIG. 73

FIG. 74

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4873316 A
- EP 264166 A
- US 20110059502 A
- US 5049386 A
- US 4946787 A
- US 4897355 A
- WO 9117424 A
- WO 9116024 A
- US 4186183 A
- US 4217344 A
- US 4235871 A
- US 4261975 A
- US 4485054 A
- US 4501728 A
- US 4774085 A
- US 4837028 A
- US 9405700 W
- US 4797368 A
- WO 9324641 A
- US 5173414 A
- US 20030087817 A
- US 6603061 B
- US 7868149 B
- US 20090100536 A
- US 61736527
- WO 61748427 A

### Non-patent literature cited in the description

- **IN GOEDDEL.** GENE EXPRESSION TECHNOLOGY: METHODS IN ENZYMOLOGY. Academic Press, 1990, vol. 185
- **BOSHART et al.** *Cell,* 1985, vol. 41, 521-530
- *Mol. Cell. Biol.,* 1988, vol. 8 (1), 466-472
- *Proc. Natl. Acad. Sci. USA.,* 1981, vol. 78 (3), 1527-31
- Overview of principles of hybridization and the strategy of nucleic acid probe assay. **TIJSSEN.** Laboratory Techniques In Biochemistry And Molecular Biology-Hybridization With Nucleic Acid Probes Part I. Elsevier, 1993
- **SAMBROOK ; FRITSCH ; MANIATIS.** MOLECULAR CLONING: A LABORATORY MANUAL. 1989
- CURRENT PROTOCOLS IN MOLECULAR BIOLOGY. 1987
- A PRACTICAL APPROACH. the series METHODS IN ENZYMOLOGY. Academic Press, Inc, 1995
- ANTIBODIES, A LABORATORY MANUAL, and ANIMAL CELL CULTURE. 1987
- **GOEDDEL.** GENE EXPRESSION TECHNOLOGY: METHODS IN ENZYMOLOGY. Academic Press, 1990, vol. 185
- **SMITH ; JOHNSON.** Gene. Pharmacia Biotech Inc, 1988, vol. 67, 31-40
- **AMRANN et al.** *Gene,* 1988, vol. 69, 301-315
- **STUDIER et al.** GENE EXPRESSION TECHNOLOGY: METHODS IN ENZYMOLOGY. Academic Press, 1990, vol. 185, 60-89
- **BALDARI et al.** *EMBO J.,* 1987, vol. 6, 229-234
- **KUIJAN ; HERSKOWITZ.** *Cell,* 1982, vol. 30, 933-943
- **SCHULTZ et al.** *Gene,* 1987, vol. 54, 113-123
- **SMITH et al.** *Mol. Cell. Biol.,* 1983, vol. 3, 2156-2165
- **LUCKLOW ; SUMMERS.** *Virology,* 1989, vol. 170, 31-39
- **SEED.** *Nature,* 1987, vol. 329, 840
- **KAUFMAN et al.** *EMBO J.,* 1987, vol. 6, 187-195
- **SAMBROOK et al.** MOLECULAR CLONING: A LABORATORY MANUAL. Cold Spring Harbor Laboratory Press, 1989
- **PINKERT et al.** *Genes Dev.,* 1987, vol. 1, 268-277
- **CALAME ; EATON.** *Adv. Immunol.,* 1988, vol. 43, 235-275
- **WINOTO ; BALTIMORE.** *EMBO J.,* 1989, vol. 8, 729-733
- **BANEIJI et al.** *Cell,* 1983, vol. 33, 29-740
- **QUEEN ; BALTIMORE.** *Cell,* 1983, vol. 33, 741-748
- **BYRNE ; RUDDLE.** *Proc. Natl. Acad. Sci. USA,* 1989, vol. 86, 5473-5477
- **EDLUND et al.** *Science,* 1985, vol. 230, 912-916
- **KESSEL ; GRUSS.** *Science,* 1990, vol. 249, 374-379
- **CAMPES ; TILGHMAN.** *Genes Dev.,* 1989, vol. 3, 537-546

- **ISHINO et al.** *J. Bacteriol.,* 1987, vol. 169, 5429-5433
- **NAKATA et al.** *J. Bacteriol.,* 1989, vol. 171, 3553-3556
- **GROENEN et al.** *Mol. Microbiol.,* 1993, vol. 10, 1057-1065
- **HOE et al.** *Emerg. Infect. Dis.,* 1999, vol. 5, 254-263
- **MASEPOHL et al.** *Biochim. Biophys. Acta,* 1996, vol. 1307, 26-30
- **MOJICA et al.** *Mol. Microbiol.,* 1995, vol. 17, 85-93
- **JANSSEN et al.** *OMICS J. Integ. Biol.,* 2002, vol. 6, 23-33
- **MOJICA et al.** *Microbiol.,* 2000, vol. 36, 244-246
- **EMBDEN et al.** *J. Bacteriol.,* 2000, vol. 182, 2393-2401
- **JANSEN et al.** *Mol. Microbiol.,* 2002, vol. 43, 1565-1575
- **NAKAMURA, Y. et al.** Codon usage tabulated from the international DNA sequence databases: status for the year 2000. *Nucl. Acids Res.,* 2000, vol. 28, 292
- **ZUKER ; STIEGLER.** *Nucleic Acids Res.,* 1981, vol. 9, 133-148
- **A.R. GRUBER et al.** *Cell,* 2008, vol. 106 (1), 23-24
- **PA CARR ; GM CHURCH.** *Nature Biotechnology,* 2009, vol. 27 (12), 1151-62
- **ANDERSON.** *Science,* 1992, vol. 256, 808-813
- **NABEL ; FELGNER.** *TIBTECH,* 1993, vol. 11, 211-217
- **MITANI ; CASKEY.** *TIBTECH,* 1993, vol. 11, 162-166
- **DILLON.** *TIBTECH,* 1993, vol. 11, 167-175
- **MILLER.** *Nature,* 1992, vol. 357, 455-460
- **VAN BRUNT.** *Biotechnology,* 1988, vol. 6 (10), 1149-1154
- **VIGNE.** *Restorative Neurology and Neuroscience,* 1995, vol. 8, 35-36
- **KREMER ; PERRICAUDET.** *British Medical Bulletin,* 1995, vol. 51 (1), 31-44
- **HADDADA et al.** in Current Topics in Microbiology and Immunology. 1995
- **YU et al.** *Gene Therapy,* 1994, vol. 1, 13-26
- **CRYSTAL.** *Science,* 1995, vol. 270, 404-410
- **BLAESE et al.** *Cancer Gene Ther.,* 1995, vol. 2, 291-297
- **BEHR et al.** *Bioconjugate Chem.,* 1994, vol. 5, 382-389
- **REMY et al.** *Bioconjugate Chem.,* 1994, vol. 5, 647-654
- **GAO et al.** *Gene Therapy,* 1995, vol. 2, 710-722
- **AHMAD et al.** *Cancer Res.,* 1992, vol. 52, 4817-4820
- **BUCHSCHER et al.** *J. Virol.,* 1992, vol. 66, 2731-2739
- **JOHANN et al.** *J. Virol.,* 1992, vol. 66, 1635-1640
- **SOMMNERFELT et al.** *Virol.,* 1990, vol. 176, 58-59
- **WILSON et al.** *J. Virol.,* 1989, vol. 63, 2374-2378
- **MILLER et al.** *J. Virol.,* 1991, vol. 65, 2220-2224
- **WEST et al.** *Virology,* 1987, vol. 160, 38-47
- **KOTIN.** *Human Gene Therapy,* 1994, vol. 5, 793-801
- **MUZYCZKA.** *J. Clin. Invest.,* 1994, vol. 94, 1351
- **TRATSCHIN et al.** *Mol. Cell. Biol.,* 1985, vol. 5, 3251-3260
- **TRATSCHIN et al.** *Mol. Cell. Biol.,* 1984, vol. 4, 2072-2081
- **HERMONAT ; MUZYCZKA.** *PNAS,* 1984, vol. 81, 6466-6470
- **SAMULSKI et al.** *J. Virol.,* 1989, vol. 63, 03822-3828
- **MORRELL et al.** Crop genomics:advances and applications. *Nat Rev Genet.,* 29 December 2011, vol. 13 (2), 85-96
- **ROBERT D. WELLS ; TETSUO ASHIZAWA.** Genetic Instabilities and Neurological Diseases. Academic Press, 13 October 2011
- **MCIVOR EI ; POLAK U.** New insights into repeat instability: role of RNA•DNA hybrids. *Napierala M. RNA Biol.,* September 2010, vol. 7 (5), 551-8
- Genetics of Epilepsy and Genetic Epilepsies. Mariani Foundation Paediatric Neurology, vol. 20, 2009
- Genetic Diseases of the Eye. Oxford University Press, 2012
- **GUSCHIN et al.** *Methods Mol Biol,* 2010, vol. 649, 247
- **GRUBER et al.** *Nucleic Acids Research,* 2008, vol. 36, W70
- **SAPRANAUSKAS et al.** *Nucleic Acids Research,* 2011, vol. 39, 9275
- **GASIUNAS et al.** *Proc. Natl. Acad. Sci. USA,* 2012, vol. 109, E2579
- **JINEK et al.** *Science,* 2012, vol. 337, 816
- **MAKAROVA et al.** *Nat Rev Microbiol,* 2011, vol. 9, 467
- **URNOV, F.D. ; REBAR, E.J. ; HOLMES, M.C. ; ZHANG, H.S. ; GREGORY, P.D.** Genome editing with engineered zinc finger nucleases. *Nat. Rev. Genet.,* 2010, vol. 11, 636-646
- **BOGDANOVE, A.J. ; VOYTAS, D.F.** TAL effectors: customizable proteins for DNA targeting. *Science,* 2011, vol. 333, 1843-1846
- **STODDARD, B.L.** Homing endonuclease structure and function. *Q. Rev. Biophys.,* 2005, vol. 38, 49-95

- **BAE, T. ; SCHNEEWIND, O.** Allelic replacement in Staphylococcus aureus with inducible counter-selection. *Plasmid,* 2006, vol. 55, 58-63
- **SUNG, C.K. ; LI, H. ; CLAVERYS, J.P. ; MORRISON, D.A.** An rpsL cassette, janus, for gene replacement through negative selection in Streptococcus pneumoniae. *Appl. Environ. Microbiol,* 2001, vol. 67, 5190-5196
- **SHARAN, S.K. ; THOMASON, L.C. ; KUZNETSOV, S.G. ; COURT, D.L.** Recombineering: a homologous recombination-based method of genetic engineering. *Nat. Protoc.,* 2009, vol. 4, 206-223
- **JINEK, M. et al.** A programmable dual-RNA-guided DNA endonuclease in adaptive bacterial immunity. *Science,* 2012, vol. 337, 816-821
- **DEVEAU, H. ; GARNEAU, J.E. ; MOINEAU, S.** CRISPR/Cas system and its role in phage-bacteria interactions. *Annu. Rev. Microbiol.,* 2010, vol. 64, 475-493
- **HORVATH, P. ; BARRANGOU, R.** CRISPR/Cas, the immune system of bacteria and archaea. *Science,* 2010, vol. 327, 167-170
- **TERNS, M.P. ; TERNS, R.M.** CRISPR-based adaptive immune systems. *Curr. Opin. Microbiol.,* 2011, vol. 14, 321-327
- **VAN DER OOST, J. ; JORE, M.M. ; WESTRA, E.R. ; LUNDGREN, M. ; BROUNS, S.J.** CRISPR-based adaptive and heritable immunity in prokaryotes. *Trends. Biochem. Sci.,* 2009, vol. 34, 401-407
- **BROUNS, S.J. et al.** Small CRISPRRNAs guide antiviral defense in prokaryotes. *Science,* 2008, vol. 321, 960-964
- **CARTE, J. ; WANG, R. ; LI, H. ; TERNS, R.M. ; TERNS, M.P.** Cas6 is an endoribonuclease that generates guide RNAs for invader defense in prokaryotes. *Genes Dev.,* 2008, vol. 22, 3489-3496
- **DELTCHEVA, E. et al.** CRISPR RNA maturation by trans-encoded small RNA and host factor RNase III. *Nature,* 2011, vol. 471, 602-607
- **HATOUM-ASLAN, A. ; MANIV, I. ; MARRAFFINI, L.A.** Mature clustered, regularly interspaced, short palindromic repeats RNA (crRNA) length is measured by a ruler mechanism anchored at the precursor processing site. *Proc. Natl. Acad. Sci. U.S.A.,* 2011, vol. 108, 21218-21222
- **HAURWITZ, R.E. ; JINEK, M. ; WIEDENHEFT, B. ; ZHOU, K. ; DOUDNA, J.A.** Sequence- and structure-specific RNA processing by a CRISPR endonuclease. *Science,* 2010, vol. 329, 1355-1358
- **DEVEAU, H. et al.** Phage response to CRISPR-encoded resistance in Streptococcus thermophilus. *J. Bacteriol.,* 2008, vol. 190, 1390-1400
- **GASIUNAS, G. ; BARRANGOU, R. ; HORVATH, P. ; SIKSNYS, V.** Cas9-crRNA ribonucleoprotein complex mediates specific DNA cleavage for adaptive immunity in bacteria. *Proc. Natl. Acad. Sci. U.S.A.,* 2012
- **MAKAROVA, K.S. ; ARAVIND, L. ; WOLF, Y.I. ; KOONIN, E.V.** Unification of Cas protein families and a simple scenario for the origin and evolution of CRISPR-Cas systems. *Biol. Direct.,* 2011, vol. 6, 38
- **BARRANGOU, R.** RNA-mediated programmable DNA cleavage. *Nat. Biotechnol.,* 2012, vol. 30, 836-838
- **BROUNS, S.J.** Molecular biology. A Swiss army knife of immunity. *Science,* 2012, vol. 337, 808-809
- **CARROLL, D.** A CRISPR Approach to Gene Targeting. *Mol. Ther.,* 2012, vol. 20, 1658-1660
- **BIKARD, D. ; HATOUM-ASLAN, A. ; MUCIDA, D. ; MARRAFFINI, L.A.** CRISPR interference can prevent natural transformation and virulence acquisition during in vivo bacterial infection. *Cell Host Microbe,* 2012, vol. 12, 177-186
- **SAPRANAUSKAS, R. et al.** The Streptococcus thermophilus CRISPR/Cas system provides immunity in Escherichia coli. *Nucleic Acids Res.,* 2011
- **SEMENOVA, E. et al.** Interference by clustered regularly interspaced short palindromic repeat (CRISPR) RNA is governed by a seed sequence. *Proc. Natl. Acad. Sci. U.S.A.,* 2011
- **WIEDENHEFT, B. et al.** RNA-guided complex from a bacterial immune system enhances target recognition through seed sequence interactions. *Proc. Natl. Acad. Sci. U.S.A.,* 2011
- **ZAHNER, D. ; HAKENBECK, R.** The Streptococcus pneumoniae beta-galactosidase is a surface protein. *J. Bacteriol.,* 2000, vol. 182, 5919-5921
- **MARRAFFINI, L.A. ; DEDENT, A.C. ; SCHNEEWIND, O.** Sortases and the art of anchoring proteins to the envelopes of gram-positive bacteria. *Microbiol. Mol. Biol. Rev.,* 2006, vol. 70, 192-221
- **MOTAMEDI, M.R. ; SZIGETY, S.K. ; ROSENBERG, S.M.** Double-strand-break repair recombination in Escherichia coli: physical evidence for a DNA replication mechanism in vivo. *Genes Dev.,* 1999, vol. 13, 2889-2903
- **HOSAKA, T. et al.** The novel mutation K87E in ribosomal protein S12 enhances protein synthesis activity during the late growth phase in Escherichia coli. *Mol. Genet. Genomics,* 2004, vol. 271, 317-324
- **COSTANTINO, N. ; COURT, D.L.** Enhanced levels of lambda Red-mediated recombinants in mismatch repair mutants. *Proc. Natl. Acad. Sci. U.S.A.,* 2003, vol. 100, 15748-15753
- **EDGAR, R. ; QIMRON, U.** The Escherichia coli CRISPR system protects from lambda lysogenization, lysogens, and prophage induction. *J. Bacteriol.,* 2010, vol. 192, 6291-6294
- **MARRAFFINI, L.A. ; SONTHEIMER, E.J.** Self versus non-self discrimination during CRISPR RNA-directed immunity. *Nature,* 2010, vol. 463, 568-571

- **FISCHER, S. et al.** An archaeal immune system can detect multiple Protospacer Adjacent Motifs (PAMs) to target invader DNA. *J. Biol. Chem,* 2012, vol. 287, 33351-33363
- **GUDBERGSDOTTIR, S. et al.** Dynamic properties of the Sulfolobus CRISPR/Cas and CRISPR/Cmr systems when challenged with vector-borne viral and plasmid genes and protospacers. *Mol. Microbiol,* 2011, vol. 79, 35-49
- **WANG, H.H. et al.** Genome-scale promoter engineering by coselection MAGE. *Nat Methods,* 2012, vol. 9, 591-593
- **CONG, L. et al.** Multiplex Genome Engineering Using CRISPR/Cas Systems. *Science,* 2013
- **MALI, P. et al.** RNA-Guided Human Genome Engineering via Cas9. *Science,* 2013
- **HOSKINS, J. et al.** Genome of the bacterium Streptococcus pneumoniae strain R6. *J. Bacteriol,* 2001, vol. 183, 5709-5717
- **HAVARSTEIN, L.S. ; COOMARASWAMY, G. ; MORRISON, D.A.** An unmodified heptadecapeptide pheromone induces competence for genetic transformation in Streptococcus pneumoniae. *Proc. Natl. Acad. Sci. U.S.A.,* 1995, vol. 92, 11140-11144
- **HORINOUCHI, S. ; WEISBLUM, B.** Nucleotide sequence and functional map of pC194, a plasmid that specifies inducible chloramphenicol resistance. *J. Bacteriol,* 1982, vol. 150, 815-825
- **HORTON, R.M.** In Vitro Recombination and Mutagenesis of DNA : SOEing Together Tailor-Made Genes. *Methods Mol. Biol,* 1993, vol. 15, 251-261
- **PODBIELSKI, A. ; SPELLERBERG, B. ; WOISCHNIK, M. ; POHL, B. ; LUTTICKEN, R.** Novel series of plasmid vectors for gene inactivation and expression analysis in group A streptococci (GAS). *Gene,* 1996, vol. 177, 137-147
- **HUSMANN, L.K. ; SCOTT, J.R. ; LINDAHL, G. ; STENBERG, L.** Expression of the Arp protein, a member of the M protein family, is not sufficient to inhibit phagocytosis of Streptococcus pyogenes. *Infection and immunity,* 1995, vol. 63, 345-348
- **GIBSON, D.G. et al.** Enzymatic assembly of DNA molecules up to several hundred kilobases. *Nat Methods,* 2009, vol. 6, 343-345